# EUROPEAN PATENT SPECIFICATION

(11) **EP 1 414 969 B1**
(45) Date of publication and mention of the grant of the patent: **26.05.2010**
(21) Application number: 02733919.1
(22) Date of filing: 28.03.2002
(51) Int. Cl.: C12N 15/52, C12N 1/21, C12P 19/62

(54) **BIOSYNTHETIC GENES FOR BUTENYL-SPINOSYN INSECTICIDE PRODUCTION**
BIOSYNTHETISCHE GENE ZUR HERSTELLUNG VON BUTENYL-SPINOSYN-HALTIGEM INSEKTIZID
GENES BIOSYNTHETIQUES DESTINES A LA PRODUCTION D'UN INSECTICIDE DE BUTENYLE-SPINOSYNE

(30) Priority: 30.03.2001 US 280175 P
(43) Date of publication of application: 06.05.2004
(73) Proprietor: Dow AgroSciences LLC, Indianapolis, Indiana 46268 (US)
(72) Inventor: HAHN, Donald, R., Zionsville, IN 46077 (US); JACKSON, James, D., Michigan 49002 (US); BULLARD, Brian, S., Kokomo, IN 46902 (US); GUSTAFSON, Gary, D., Zionville, IN 46077 (US); WALDRON, Clive, Indianapolis, IN 46220 (US); MITCHELL, Jon, C., West Lafayette, IN 47906 (US)
(74) Representative: Raynor, John
(86) International application number: PCT/US2002/009968
(87) International publication number: WO 2002/079477

(56) References cited:
- WO-A-01/16303
- WO-A-01/16303
- WO-A-01/19840
- WO-A-01/19840
- WO-A-99/46387
- WO-A-99/46387
- WALDRON, C. ET AL.: "A cluster of genes for the biosynthesis of spinosyns, novel macrolide insect control agents produced by Saccharopolyspora spinosa" ANTONIE VAN LEEUWENHOEK, vol. 78, no. 3-4, December 2000 (2000-12), pages 385-390, XP008015874
- DONALD R HAHN ET AL: "Butenyl-spinosyns, a natural example of genetic engineering of antibiotic biosynthetic genes" JOURNAL OF INDUSTRIAL MICROBIOLOGY & BIOTECHNOLOGY ; OFFICIAL JOURNAL OF THE SOCIETY FOR INDUSTRIAL MICROBIOLOGY, SPRINGER-VERLAG, BE, vol. 33, no. 2, 1 February 2006 (2006-02-01), pages 94-104, XP019357769 ISSN: 1476-5535
- WALDRON, C. ET AL.: "A cluster of genes for the biosynthesis of spinosyns, novel macrolide insect control agents produced by Saccharopolyspora spinosa" ANTONIE VAN LEEUWENHOEK, vol. 78, no. 3-4, December 2000 (2000-12), pages 385-390, XP008015874

### SEQUENCE LISTING

<110> Hahn, Donald
   Jackson, Jim
   Bullard, Brian
   Gustafson, Gary
   Waldron, Clive
   Mitchell, Jon
<120> Biosynthetic Genes for Butenyl-Spinosyn Insecticide Production
<130> 51609
<140>
   <141>
<150> US 60/280,175
   <151> 2001-03-01
<160> 40
<170> PatentIn Ver. 2.0
<210> 1
   <211> 75236
   <212> DNA
   <213> Saccharopolyspora sp. NRRL30141
<400> 1
   atgagcgaag ccgggaacct gatcgccgtc gtcggattct cctgccgcct accccaggca      60
   cctgacccgg cttctttctg gcggttgctg cgcaccggaa cggacgccat caccaccgtc     120
   ccggaagggc ggtggggcga cccgttgccc ggccgggatg cgcccaaggg cccggaatgg     180
   ggcggcttcc tggctgatgt cgactgcttc gatcccgagt tcttcgggat ctcgccgcga     240
   gaagcggccg ccatggaccc ccagcagagg ctggctctgg agctcgcctg ggaggctctc     300
   gaagacgccg gtatccccgc cggcgagctg cgcggcactg ccgcgggggt gttcatgggg     360
   gcgatctctg acgactacgc cgccctgctt cgcaagagcc cgccggaagt ggctgcgcag     420
   taccgtctca ccggcaccca tcgaagtctg atcgccaacc gcgtgtccta cgtgctcggc     480
   ctgcgcgggc caagcctgac ggtggattca ggtcagtcct cgtccctggt cggcgtgcat     540
   ctcgccagcg agagcctgcg acgtggcgag tgcgcgatcg ctctcgccgg cggcgtgaac     600
   ctcaacctgg ctgccgagag caacagagcc ctgatggact tcggcgcgct ctccccggac     660
   ggtcgctgct tcaccttcga tgcgcgggcg aacggttacg tccgcggcga aggcggcggc     720
   ctcgtcgtgc tgaagaaggc cgatcaggct cgcgccgatg gcgaccggat ctactgcctc     780
   atccgcggca gcgcggtcaa caacgacggg ggcggtgctg ggctcacggc tccggcggca     840
   gacgcccagg cggagttgct gcgacaggca taccggaacg cgggtgtcga cccggccgcc     900
   gtgcagtacg tcgagctcca cggcagcgcg accagagtcg gggaccccgt cgaagcagca      960
   gccctcggat ctgtcctggg tgtggcaaga cggcccggcg acaagctgcg tgtggggtcg 1020
   gcgaagacca acgtcggcca tctggaagca gcggcgggcg tcaccgggtt gctgaagacc 1080
   gcactcagca tctggcaccg cgaactgccg ccgagtcttc acttcaccgc ccccaacccg 1140
   gaaatcccgc tggacgaact gaatctacgc gtccagcgtg atctgcggcc gtggccggag 1200
   agcgagggcc cgctgctggc cggcgtcagc gccttcggaa tgggaggcac gaactgccac 1260
   ctggtgctct ccgattcgtc ccaggtggag cgaaggcgta gtggacccgc tgaggcgacc 1320
   atgccttggg tcttgtcggc cagaacaccg gtcgcattgc gtgcgcaggc ggcgcgcttg 1380
   cacacgcacc tcaatactgc cggtcaaagt ccattggacg tcggctactc actggcgacc 1440
   actcgatccg cgctaccgca ccgagccgcg ctggtcgcgg acgacgtacc gaaactgctc 1500
   gccgggttga aggccctcgc tgacggcgac gacgcgccca cgctgtgcac gggcacgact 1560
   tccggcgagc gggcaacagt cttcgtcttt cccggacagg gcagccagtg gatcgggatg 1620
   ggtaggcagc tgctccaaac ctccgaggtt ttcgcggcgt ccatggcgga ctgcgcggat 1680
   gcgttggcgc cgcacctgga ttggtccctg ctggatgtgc tgcgtaacgc ggccggcgct 1740
   tcgcagcttg atcgcgacga tgtcgtccag cccgcactgt tcgccgtcat ggtctcgctg 1800
   gcagagctct ggcgttcgtg gggcgtgcgt ccggaggcgg tcgtcgggca ctcgcagggg 1860
   gagatcgcgg cggcctgcgt cgccggggcc ctctccgtcc gcgatgccgc aagggtagtg 1920
   gcggtgcgca gcaggcttct ggcggcgctg gcgggcagag gcgcgatggc gtcgttgcag 1980
   catcccgttg aagaggtgcg acaaatcctg ttgccatggc gcgatcggat cggcgtggcg 2040
   ggggtgaacg gaccgtcgtc gactctggtg tcgggggacc gggaggcgat ggcggaactg 2100
   ctggccgagt gcgcgcgccg agagctccgg atgcgccgga ttccagttga atacgcctcc 2160
   cattcgccgc acatcgagga tgtccgcgac gagctgctgg cgctgttggc gtcgatcgaa 2220
   cccaggacag ggaacatccc ggtctattcg acgacgaccg gggaactgct ggaccggccg 2280
   atggacgccg actactggta ccgcaacctt cgtcaaccgg tgctgttcga agcggcggtc 2340
   gaggccctgt tgaagcgggg gcacaacgca ttcatcgaga tcagcccgca cccggtgctg 2400
   actgcgagca tccaggaaac cgccgcgcga gcggggcggg aggtagtggc gctcgggaca 2460
   ctccgccgcg gcgaaggtgg cctgcggcag gcgctgacgt cgctggccaa agcacacgtc 2520
   cacggagtgg ccgcgaactg gcacgcggtc ttcgccggca ccggggcgca gcgggtcgac 2580
   ctgccgacgt acgcctttca acgacagcgc tactggctgg acacgaaacc ttccgacctc 2640
   gccatgcccg agggcgatgt gtcgacagcg ttgcgggaaa aactgcgctc ctcgccgggg 2700
   gcggacgtgg actcagcgac cctcacaatt atccgggcac aggcagccgt ggtactcggc 2760
   cactccgatc cgaaagagat ggactcggat cggacattca aagacctggg cttcgattcc 2820
   tcgaccgtgg tcgagctgtg cgaccgcctc aacgccgcca ccggactgcg cctcgcgccg 2880
   agcgtggttt tcgactgtcc gacgccctac aagctcgccc gccaggtacg gacgttgttg 2940
   ttggacgagc cagtccccac gacgtcaccc cgaacggaga ccgaagcgga cgagcctatc 3000
   gccgtgatcg ggatgggctg tcggtttccg ggtggcgtgt cctcgcccga ggagttgtgg 3060
   cagctggtcg ctgctggacg ggacgtcgtg tcagagttcc cggctgaccg aggttgggac 3120
   ccggagcgtg cggggacttc gcacgtgcgc gccggcggat tcctgcatgg cgccacggat 3180
   ttcgatcccg ggttcttcgg gatttccccg cgcgaggcgt tggcgatgga tccgcagcag 3240
   cgcttgctgc tggaaatcgc ctgggaggcg atcgaacgag gcgggatcaa cccgcagacc 3300
   ctgcacggaa gtcaaaccgg cgtcttcgtc ggcgcaacct ccctggatta cgggccacgc 3360
   ctgcacgaag cgtccgacga ggcggccggc tacgtgctca ccggcagcac cacgagtgtg 3420
   gcgtcgggtc gggttgcgta ttcgtttggt cttgagggtc ctgcggtgac ggtggatacg 3480
   gcgtgttcat cgtcgttggt ggcgttgcat ctggcgtgcc agtcgttgcg ttcgggtgag 3540
   tgtgatttgg cgttggccgg tggtgtgacg gtgatggcca cgccggggat gttcgtggag 3600
   ttttcgcgtc agcggggctt ggcacccgac ggtcgctgca agtcgttcgc ggaggccgcg 3660
   gatggcaccg gctggtccga gggtgccggc ctggttctac tggagcggtt gtcggatgcc 3720
   cggcggaatg ggcatgacgt tttggcggtg gttcgtggca gcgcggttaa ccaggacggc 3780
   gcgtcgaacg gactgactgc tccgaatggc ccgtcgcagc ggcgggtgat cacccaagca 3840
   ctcgccaacg cgaagttgtc ggtgtccgat gtggacgcag tggaggcgca cgggacgggc 3900
   acccggcttg gtgatccgat cgaggcgcag gcgctgatcg ccacttacgg gcagggacgg 3960
   ggtccggaac ggccgttgtg gttggggtcg gtcaagtcca acatcggtca tacgcaagcg 4020
   gcggccggtg ttgccggtgt catcaagatg gtcatggcga tgcggtatgg ggagctgccc 4080
   gccacgttgc acgtggacga gccctcctcg caggtggact ggtctgctgg gatggttcag 4140
   gttctgaccg agcacgtgcc ttggcccgac aacagccgtc ctcgtcgggt gggggtgtcg 4200
   tcgttcggga tcagcggcac caatgcgcac gtcatcctcg aacagtctcc gacagcgtca 4260
   agtgagttcg tggagcacag cggacctgat tcggaatctg ctgtggatgt tccggtggtt 4320
   ccgtgggtgg tgtcgggcaa aacgccggaa gcgctcagtg ctcaggcgga caacttggtg 4380
   tcctatctgg atgatcgccc taatgtttcc gcgctgaatg tggcatattc gctggcttcc 4440
   gaacgagccg cactggatga gcgggcggtg gtgctggggg cggatcgtga agcgttgttg 4500
   tctggactga aagcactggc tgccggtcac gaggatcctg gtgtggcgtc gggatccctg 4560
   gtttctggtg gggttgggtt tgtgttctcc ggtcagggtg gtcagtggtc ggggatgggc 4620
   cgggggcttt accgggcgtt tccggtgttc gctgctgcct ttgacgaagc ttgtgccgaa 4680
   ctggatgcac atctgggcca ggaagtgggg gttcgggatg tggcgttcgg ttccgatgcg 4740
   cagttgctgg agcggacgtt gtgggcgcag tcgggtttgt tcgcgctgca ggtaggtttg 4800
   ctgaggctgt tgggttcatg gggtgttcgg ccgggtgcgg tgctggggca ttcggtgggc 4860
   gagttggcag cggcgcacgc ggcgggtgtg ttgtcgttgc cggatgcagc tcggttggtg 4920
   gcgggtcgtg cccggttgat gcaggcgatg ccggatggcg gtggcatgct cgcggtggct 4980
   acaagtgaga cccaggtcga acctatgctg gatggagtgc gggaccggat cgggatcgcg 5040
   gcgatcaacg ctccggaatc ggtcgtgctc tccggtgacc gcgaactact cgccgaagtc 5100
   gctgatcagc tgaacgatca agggtgccgg acacgatggt tgcaggtgtc tcacgctttc 5160
   cattcgtatc ggatggaacc gatgctcgac gagttcgccc agatcgcagg cagcgtggat 5220
   ttccggcgtt gcgaactgcc tatcatctcg accctgacag gaaacctcga tgacgtcggc 5280
   gtgatggcta cgccggagta ttgggtgcgt caggtgcgtg agcccgtccg cttcgccgat 5340
   ggtgtccagt cgctcgtcga gcaagatgtg gctactgttg tcgagcttgg ccctgatgcg 5400
   attctgtcgg ctctgattcc tgattgtcat tcctggggtg atcagactgt gccgattccg 5460
   ttgctgcgca aggaccgcgc tgaacccgaa actgtggtcg ccgcggtggc gcgggcgcac 5520
   acgcgtggtg ttcaggtcga ttggtcggcg tttttcgctg gtaccggggc tgggcgggtc 5580
   gagttgccga cgtatgcctt ccagcggcag cggtattggc tggagtcatc ggtttccggt 5640
   gatgtgacag gtatcggtct ggctggggcg gagcatccgt tgctgggggc cgtggttgtg 5700
   ttggccgacg gtgatgggat ggtgttgacc ggtcggttgt cggtggggac gcatcggtgg 5760
   ctggccgagc atcgtgtgct gggggaggtc gtggttcccg gcacggctat cctggagatg 5820
   gtcttgcatg cgggggcgcg ggttggttgt ggccgggtgg aggagctcac cctggaagca 5880
   ccgctggtgg tgcccgaacg cgatgccatc gaaatccagc tgctggtgaa cgcgcccgac 5940
   gacaagggtc ggcggtccgt gtcgctgcat tcccgcccgg ccggtgggtc tgggggtggg 6000
   ggttggacgc ggcacgccac gggcgaactc gtcgtcgccg gcacgggtgg tggggcggtt 6060
   actggttggt cgactgaggg tgccgagccg gttgctctcg gtgagtttta tgtcgttcag 6120
   gcggggaacg ggttcgagta tgggccgttg ttccaggggc ttcgggcggc gtggcgtcgt 6180
   ggtggcgagg ttctcgcgga ggtcgccctg ccggcagcgg ctggtgcgat ggcggggttc 6240
   ttgatcaatc cggcgttgct ggatgccgcc ttgcaggcgt ccgcgctggg tgaccgtccg 6300
   gcggagggtg gtgcgtggct gccgttctct tttaccgggg tagaactttc cggtcagggt 6360
   gggacgatca gcagggcacg ggtggagtct acgcgacccg atgcggtgtc ggtggctgtg 6420
   atggatgagg gtgggcggtt gctcgcctcg atcgattctc tccggttgcg gccggtgtcg 6480
   tcggtgcggt tggcgaatcg ggacgttgtc ggtgacgcgc tgttcgaggt gacttgggag 6540
   ccggtggcga cgcggtcgac ggtatcgggt cgctgggcgt tgcttggtga tgctgtcggc 6600
   ggcatggccg gtctcattgg gctcgcacca ggttccgtcg atcgttgtgc gggtctggct 6660
   gagctcgcgg ggaaccttga ttccggtgcg ctggttgctg atgtcgtggt ttattgcgcc 6720
   ggtgaacagg cggatcccga cgccggcgtg gcggcactcg cggagacccg ggagatgctg 6780
   gccctggtcc agtcgtggtt ggccgaggag cggttggccg ggtcacgtct ggtggtggtg 6840
   acgtgtggcg cggtgacgac ggctgcgggt gacggcgcat caaagctggc gcatgcgccg 6900
   ttgtgggggt tgttgcgttc agcgcagtcg gagaacccgg gccggtttgt gctggtcgat 6960
   gtggacggta ccgccgagtc gtggcgcgcg ttgccgagtg cggtggggtc gatgcaaccg 7020
   cagttggccg tgcgtaaggg tgtggtgaca gtgccgcgtg tggcgtcggt tccggggccg 7080
   gtcgaggtgc ccgcggtggt ggccggtccc gaccggacgg tgctgatttc cggtggcacg 7140
   ggtctgttgg gtggcgtggt ggcacgccac ctggtggccg agcgcggtgt tcgtcgagtg 7200
   gtgttgacgg gccgtcgtgg ctgggatgct cccggaatca ccgagttggt gggtgagctg 7260
   gagggtttcg gtgcggtggt cgatgtggtg gcgtgcgacg ttgcggatcg tgctggtctg 7320
   gaggggttgc tggcggcggt cccggcggag tttccgctgt gtggtgtggt gcatgccgcg 7380
   ggtgtgctgg ctgacggggt gatcgagtcg ttgacaccgg aggacgtggg ggcggtgttc 7440
   ggtccgaagg cggcgggggc gtggaacctg cacgagctga ctcgggatat ggacttgtcg 7500
   tttttcgcgt tgttctcctc gctgtccggg gtgaccggcg ccgcgggtca gggtaattat 7560
   gcggcggcga acacgttcct ggacgcattg gcgcattacc ggcgggcgca gggattgcct 7620
   gcggtgtcgt tggcgtgggg cttgtgggag cagtcgagcg ggatgaccgg gcggctcagt 7680
   gatgtcgacc ggagcaggat cgcccgctcc agtccaccgt tgtccaccaa ggatggtttg 7740
   cggctgttcg atgccgggct ggcgttggat cgggcagcgg tggttccggc gaggttggac 7800
   agggccttcc tggccgagca ggcccggtcg ggaacgctac ccgcgatgct gacggcactg 7860
   gtacctacca tcacctctat caggcgcagt agtggcaccg acctcgcgga cgaggacgcc 7920
   ttgcttgggg tggtgcggga gcacgccgcg agggtgctgg ggtattcggg tgcggccgag 7980
   gtcggggtcg agcgtgcttt ccgggatctg ggctttgatt cgttgtctgg tgtggagttg 8040
   cgtaatcggc tggccggggt gctgggagcc cggctgccgg caaccgccgt attcgactac 8100
   ccgacgccgc gggcgttggc ccggttcctg caccaggaac tggcaggcga ggtcgggacg 8160
   acgccggcgc cggtgacgac cacgaccgcg agcgtcgaag acgatctcgt cgcgatagtc 8220
   gggatggggt gtcgttatcc gggtggggtg tcctcaccgg aggagctttg gcgtttggtg 8280
   gccgggggcg tggatgcggt cgcggacttc ccggacgatc gcggctggga tctggccgga 8340
   ttgttcgatc cagatcccga tcgtttcggg acttcgtatg tgcgtgaggg cgggttcctg 8400
   cgggacgcgg cggagttcga tgccgcgttt ttcgggattt ctccgcgtga ggcactggcg 8460
   atggacccgc agcaacggtt gctgctggag ctgtcctggg aggccgttga acgcgctggg 8520
   atcgatccgg ggtcgctgcg cgggagccgg acgggtgtgt tcgcggggct gatgtatcac 8580
   gactacgccg gacggttcgc ggccggagtg ccggagggct tcgaaggcta tctcggtaat 8640
   ggcagcgcgg gcagtgtggc ctcgggccgg gtcgcgtatt cgttcggttt cgagggtcct 8700
   gcggtgacgg tggacacggc gtgttcgtca tcgctggtgg cgttgcacct ggcaggtcaa 8760
   tcactgcgtt ccggtgagtg tgatctcgcc cttgccggtg gcgtgacggt gatggccacc 8820
   ccggcgacgt ttgtggagtt ctcccgtcag cggggtctgg caccggatgg gcgctgcaag 8880
   tcgttcgcgg aggccgcgga cgggaccggc tggggcgagg gtgctggcct agtgctgttg 8940
   gagaggttgt cggatgcccg tcgtaatggg catcgggtgt tggcggtggt tcgtgggtcg 9000
   gcggtgaatc aggacggcgc gtcaaacgga ctgaccgcgc cgaatggtcc ctcgcagcaa 9060
   agggtgatca cccaagcact cacgagtgcg gggttgtccg tgtccgatgt ggatgctgtg 9120
   gaggcgcacg ggaccgggac caggcttggt gatccgatcg aggcacaggc attgatcgcc 9180
   acctatggcc gtgatcgtga tcctgaccgg ccgttgtggt tggggtcgat gaagtccaac 9240
   atcggtcaca cacaggcagc ggcgggtgtt gccggtgtga tcaagatggt gatggcgatg 9300
   cgccacgggg agctgccgcg cacattgcac gtcggcgagc ccacgtcgga ggtggattgg 9360
   tcggcaggtt cggtccagct cctcacggag aacacgccct ggcccgacag cggccatcct 9420
   cgtcgggcgg gagtgtcgtc gttcgggatc agcggcacca acgcacacgt catcctcgaa 9480
   cagtctccga cagcgtcaag tgagttcgtg gagcacagcg gacctgattc ggaatctgct 9540
   gtgaatgtcc ctgtggttcc gtgggtggtg tcgggcaaaa cacccgaagc gctcagtgct 9600
   caggcggaca ccttggtgtc ctatctggac gatcgatctg atgtctcctc gcgggatgtt 9660
   gggtattcgc tggcgatgac gcgttcggcg ctggatgagc gggcggtggt gctggggtcg 9720
   gaccgtgaaa cgttgttgtc cgggttgaaa gcactggctg ccggtcatga ggccactggg 9780
   gtggttacgg gatctgtggg ttctggcggc cggcccggtt ttgtgttcgc cggtcagggt 9840
   ggtcagtggt tggggatggg ccgggggctt taccgggcgt ttccggtgtt cgctgatgcc 9900
   tttgacgaag cttgtgccgg actggatgcg catctggggc agaaagtggg ggttcgggat 9960
   gtggtgttcg gttccgacgc gcagttgctc gatcggacgt tgtgggcgca gtcgggtttg 10020
   ttcgcgttgc aggttggttt gctgaagttg ttgggttcgt ggggtgttcg gcctgttgta 10080
   gtgctgggcc attcggtcgg ggagctagca gcggcgttcg ccgccggtgt gctgtcgatg 10140
   gcggaggcgg ctcggttggt ggccggtcgt gcccggttga tgcaggcgtt gccgtctggc 10200
   ggtgccatgc tcgcggtggc gacaagtgag acccaggtcg aacctttgct ggatggagtg 10260
   cgagaccgga tcgatatcgc ggcgatcaac gctccggaat cgatcgtgct ctccggtgac 10320
   cgcgaactac tcaccgaagc cgctgatcag ctgcacgatc aagggtgccg gacacggtgg 10380
   ttgcaggtgt cacacgcctt ccattcgccc cagatggatc cgatgctgga cgagttcgcc 10440
   gacatcgcac gaaccgtgga tttccggggt tccgaactgc cggtcgtgtc gacgctgact 10500
   ggtgcgctcg atgacagcgg cctgatggct acaccagagt attgggtgcg tcaggtgcga 10560
   gagcccgtcc gcttcgccga cggggttcgg gcgctcgtcg agcacgatgt ggccactgtt 10620
   gtcgagctcg gcccggacgg ggcgttgtca gcgctgatcc aggaatgtgc agccgaattc 10680
   gatcagtcca gaagggtggc cgcggttccg gcgatgcgcc ggagccagga cgaggcgcag 10740
   aaggtgatga cggccctggc gcaggtccat gtgcgtggtg gtgcggtgga ctggcggtca 10800
   gttttcgctg gtacggggtc gaagcaggtc gagctgccga cgtatgcctt ccaacgacag 10860
   cggtactggc tgaatgcggt gcatgaatct tctgccggcg acatgggtcg gcgtattgaa 10920
   acggaattct ggagcgctgt cgagcacgaa gatgtgacat cgcttgcaaa catattgggt 10980
   attgtggacg acggcgctgc cgtggattcc ttgcgaaacg cccttccggt gttggccggc 11040
   tggcagagaa cccgtaatga cgagtcgatt atggatcggc agtgttaccg aatcggctgg 11100
   aggcaggtag ccggactccc gccaagggga accgtcttcg gcacttggct ggtcttcgca 11160
   ccccatggct ggtccggcga accgcaggtg gcgaactgcg ttgcggcatt gcgggcaagc 11220
   ggtgcctcgg tggtgttggt ggaagctgat cccgacccgg tcgtcttcgg cgaccgggta 11280
   cggaccctgt gttcggactc tccggatctt gttggcgttt tgtcaatgct gtgcttggaa 11340
   gaatcggcga ttccgggatt ttctgcggtg tcacggggtt ttgcgttgac cgtggagttg 11400
   gtgcgggctt tggcggccgc tggtgcggat gcccggttgt ggttgctgac gtgtggtggc 11460
   gtgtcggtgg gggatgtacc ggttcgtcca gagcaggcat tggtgtgggg gttggggcgt 11520
   gttgcggggt tggagcatcc ggactggtgg ggcggcttga tcgatattcc ggtcttgttc 11580
   gacgaagatg ctcaagagcg cttgtcgatt gtgctggcag gtctcggtga ggaagaggtc 11640
   gcgatccgtt ctgacggcgt gttcgcacgt cggttggtac gccatggtgt ctcggctggt 11700
   gtgaagaagg cgtggcgccc ccggggatct gtactggtga cgggcggcac gggtggtttg 11760
   ggggcgcacg ctgctcgctg gttggccgac gccggagccg aacatgtggt gatggtgagt 11820
   cgacgcggag agcaggcacc gagtgcggag aaattgcgga cggaactgga ggatctgggt 11880
   actcgggtgt cgatcctgtc atgcgatgtg accgatcgcg aagcactggc cgaagtgttg 11940
   aaagcccttc cggctgaata tccattgact gcggtagtgc atacggcagg cgtgatcgag 12000
   actggtgatg cggcgtcaat gagcttggct gatttcgatg acgtgttgtc cgcaaaggtg 12060
   gctggtgccg cgaatctgga tgccttgctg gccgatgtgg aattggacgc gttcgtcttg 12120
   ttctcatcgg tgtcgggagt ttggggcgct gggggacagg gggcttacgc ggcggcgaat 12180
   gcctatctgg atgcgctggc ggaagagcgt cggtcgcgag ggttggtcgc gaccgcggtg 12240
   gcgtgggggc cgtgggccgg cgaggggatg gccgccggcg aaacaggaga ccagctgcgt 12300
   cgatacggcc tttccccaat gggtccgcag tacgccatcg ccggaattcg gcgggcagtg 12360
   gaacaggacg aaatttccct ggtagtggcc gacgtcgatt gggcacgttt cagcgcggga 12420
   ttcctggcgg ctaggccgcg gccactgctg aacgaactga ccgaggtcaa ggaactcctc 12480
   gtcaatgctc agtccgaggt gggagtcgtt gccgaggcgt cggtggcatg gcggcagcga 12540
   ttggccgcag caccgaggcc ggcacaggaa cagctgatcc tggagctggt acgcggcgaa 12600
   acggctctgg tactgggaca tcccggagca gaggccgttg caccggaacg agctttcaag 12660
   gacagcggat tcgactcgca ggccgcggtc gaactccgcg ttcggctcaa tcgagccacc 12720
   ggcctccagt tgccatcgac aattatcttc agccatccca cgcctgcaga actggctgcg 12780
   gagctgcggg cgaggctcct ccccgagtcc gcaggagtag acatttccga ggaggacgag 12840
   gcgcgaatca gagcggcact gacgtcgatc ccgttcgcgg ccttgcgcga ggcagacttg 12900
   gtgaatcgcc tgctcgccct tgccggacac ccagtcgact ccggcagctc cccggacgat 12960
   gcggtcgcga cctcgatcga tgcgatggat gtagccgacc tcgtcgaagc agcgctgggc 13020
   gaacgcgagt cctgagaccg cagacctggg agatcacggt gaccaccagt tacgaagaag 13080
   ttgtcgaggc actgcgagca tcgctcaagg agaacgaacg cctccggcgc ggccgggatc 13140
   gattcgccgc ggagaagggc gatcccatcg cgatcgtggc gatgagttgc cgttaccccg 13200
   gtcaggtctc ctcgccggag gacttgtggc aactggccgc cggcggtgtg gacgcgatct 13260
   ccgaagtccc gggggatcgc ggttgggacc tagccggcgt gttcgatccg gactccgatc 13320
   gtcctggcac atcgtatgcc tgtgcgggcg gtttccttca gggcgtgtcg gagttcgatg 13380
   cgggcttctt cgggatttct ccgcgtgagg cgttggcgat ggacccgcag cagcggttgc 13440
   tgctggaagt cgcgtgggag gtcttcgaga gggctgggct ggagcagcgg tcgacacgtg 13500
   gttcccgcgt tggcgtgttc gtcggtacca atggccagga ctacgcgtcg tggttgcgga 13560
   cgccgccgtc tgaggtggca ggtcatgtgc tgacgggcgg cgcggcagcg attctttcgg 13620
   gtcgggttgc gtattcgttc gggttcgagg gtcctgccgt gacggtggat acggcgtgtt 13680
   cgtcgtcgtt ggtggcgttg cacctggcgg gtcaagcact gcgcgctggt gagtgcgacc 13740
   tcgcccttgc cggtggcgta acggtgatgt cgacgccgaa ggcgttcctg gagttctccc 13800
   gccaacgtgg tctcgcggct gacgggcggt gcaagtcgtt cgcggcggcg gcggatggta 13860
   ctgggtgggg cgagggtgcc ggactgttgt tgctggagcg gctgtccgac gctcgtcgaa 13920
   acggacaccg ggtgttggca gtggtgcgag gtagcgctgt gaaccaggac ggtgcctcca 13980
   acgggctgac cgcaccgaac ggttcttccc aggcgcgggt gatcacccag gcgttggcaa 14040
   gtgcggggtt gtcggtgtct gatgtggacg cagtggaggc gcatggcacg ggcacgcggc 14100
   ttggtgatcc gattgaggcg caggctctga tcgccaccta tggccgtgat cgtgatcctg 14160
   ctcggccgtt atggttgggt tcggtcaagt cgaacatcgg tcatacgcag gcggcggcgg 14220
   gtgtggccgg cgtgatcaaa atggtgatgg cgatgcggca cgggcagctg ccgcgcacgt 14280
   tgcacgtgga cgcgccgtcg ccggaggtgg attggtcggc agggacggtc caactcctta 14340
   cggagaacat gctttggccc gagagcggtc gtgttcgccg ggcgggggtg tcgtcgttcg 14400
   ggatcagcgg caccaacgcg cacgtcatcc ttgaacagcc cacgggcgag acgcgtcagt 14460
   cagcggggcc ggattcgggc tctgtcgtgg atgttccggt ggtgccgtgg atggtatcgg 14520
   gcaaaacacc ggatgcgctc ggcgcccagg cggacacatt gatgtcctat ctggatgatc 14580
   gtgttgacgt cccttcgctg gatattgcgt attcgctggc gatgacgcgt tcggcgctgg 14640
   atgagcgggc ggtggtcctg ggtccggacc gcgaaacgtt gttgtccggg ttgaaagcgc 14700
   tgtctgccgg gcatgaggct tctggggtgg ttacgggatc tgtggggact gggggacgca 14760
   tcgggtttgt gttttccggt cagggtggtc agtggctggg gatgggccgg gggctctata 14820
   gggcttttcc ggtgttcgct gctgcctttg acgaagcttg tgccgagctg gaggcacatc 14880
   tgggccagga ggttggggtt cgggatgtcg tgttcggttc ggatgcgcag ttgctgaatc 14940
   ggacgttgtg ggcgcagtcg ggtttgttcg cgttgcaggt cggtttgctg aagttgctgg 15000
   attcgtgggg tgttcggccg agtgcggtgc tgggccattc ggtgggtgag ttggcggcgg 15060
   cgttcgcggc gggtgtgttg tcgttgtcgg atgcggctcg gttggtggcg ggtcgtgccc 15120
   ggttgatgca ggcgttgccg tcaggcggtg ggatgttggc ggtggctgct ggcgaggagc 15180
   aactgcggcc gttgttggcc gatcacggtg atcgtgtggg gctcgctgcg gtcaacgttg 15240
   cggagtcggt ggtgctctcc ggtgatcggg atgtgctcga tgacattgcc gggcggctgg 15300
   acgggcaagg ggttcggaca cggtggttgc gggtttcgca tgcgtttcat tcgtatcgga 15360
   tggacccgat gctggacgag ttcgccgaaa tcgcacgagc cgtggactac cggcgttgcg 15420
   aactgccgat cgtgtcgacc ctgacgggaa aactcgatga cgctggcagg atgagcggtc 15480
   ccgactactg ggtgcgtcag gtgcgcgagc ccgtccgctt cgccgacggt gcccaggcac 15540
   tcgtcgagca cgacgtggcc accatagtcg agatcggtcc ggacggggcg ttgtcggcgc 15600
   tgatccagga atgtgtggcc gcatccgatc agtccagaag ggtggccgcg gtcccggcga 15660
   tgcgcaggaa ccgggacgaa gcacagaact tgacaacagc cctggcgcag gtccatgtgc 15720
   gtggtggtgc ggtggactgg cggtcgtttt tcgccggtac gggggcgaag caagtcgagc 15780
   tgcccaccta tgccttccag cggcagcggt actggctgga gccatcggat tccggtgatg 15840
   tgacaggtgc cggtctggcc ggggcggagc atccactgtt gggtgctgtg gtgccggtcg 15900
   cgggtggtga tgaggtgttg ctgaccggca ggatttcggt ggggacccat ccgtggctgg 15960
   ccgaacaccg ggtgctgggc gaagtgatcg tcccgggcac cgcgttgctg gagatcgcct 16020
   tgcatgcggg ggaacgtctt ggttgtgaac gggtggaaga actcaccctg gaagcaccgc 16080
   tggtccttcc ggagcgcggg gcgatgcagg ttcagctgcg agtgggtgcg cccgagaatt 16140
   ccggacgcag gccgatggtg ctctactcgc gccccgaagg ggcggcggac catgactgga 16200
   cacggcacgc cacgggccgg ttggcgccag gcggcggaga ggcggccgga gacctggccg 16260
   actggccggc tcctggtgcg ctgccggtcg acctcgacga gttctatcgg gacctcgctg 16320
   agcatggcct ggagtacggc ccgatcttcc aagggctcaa ggcggcctgg cggcaagggg 16380
   acgaggtgta cgccgaagcc gcgctgccag gaacagaaga ctccggtttc ggggtgcatc 16440
   cggcattgct ggacgcggct ctgcacgcaa cggctgtccg ggacatggat ggcgcatggt 16500
   tgccattcca gtgggaaggt gtgtgcctgc acgccagggc cgcgtcggct ttgcgggtcc 16560
   gcgtggtccc ggctggtgac gatgccaaat ccctgctggt gtgcgatggc accggtcgac 16620
   cggtgatctc ggtggaccgg ctcgtgtttc ggtcggctgc ggccgggcgg accggtgcgc 16680
   gccgacaggc ccatcgagct cggttgtacc ggttgggctg gccaacggtt caactgccga 16740
   catccgctca gcccccgtcc tgcgtgcttc tcggcacctc ggaagtgtcc tctgacatgc 16800
   aggtgtatcc ggacctccgg tcgttgacgg ccgcgttgga tgccggtgcc gaaccacccg 16860
   gcgtcgtcat cgcacccacg ccccccggcg gtggacaaac agcggatgtc cgggagtcga 16920
   ctcggcatgc actcgacctg gtacaaggct ggcttgccga tcagcgactc aacgattccc 16980
   gattgttcct ggtgacacgg ggagcagtgg ccgtggagcc cggcgaaccc gtgaccgatc 17040
   tggcgcaggc cgcgctctgg ggactgttgc gttcgacgca gaccgaacac cctgatcgct 17100
   tcgtcctcgt cgatgtggct gagcccgcgc aactcctccc cgccctgccg ggggtgctgg 17160
   cctgcggcga gcctcagctc gcactgcgac gtggcggcgc acacgcgccc agactggctg 17220
   gactgggcgg cgatgacgtc ctgcccgtgc cggacagcat ggggtggcga ttggaggcca 17280
   cgagcccagg aactctggat ggcttggcat tgctggacga accggcggcc acggcatcgc 17340
   tgggtgacgg gcaggtcagg attgcgatgc gcgctgccgg ggtgaacttc cgggatgcgc 17400
   tcatcgcgct cggcatgtat cccggtgcgg cttcgctggg cggtgagggg gccggggtcg 17460
   tggtggagac cggccccggc gtcaccggcc tggcacccgg cgaccgggtg atggggatga 17520
   tcccgaaggc gttcgggccg ctcgcagtcg ccgaccatcg catggtgacg aggattcccg 17580
   ctggttggag cttcgcgcag gccgcatcgg tgccgatcgt ctttctcacc gcctactacg 17640
   cgctggttga tctcgccggg ttgagaccag gggagtcgct gctggttcat tcggccgccg 17700
   gtggggtggg catggccgcg atccaactcg ccaggcacct cggtgcagag gtgtacgcca 17760
   ccgcaagcga ggacaagtgg caagccgtgg agctgacacg agaacgcctc gcttcgtcgc 17820
   ggacgtgcga tttcgagaag cagttcctcg gggcgaccgg cggacgcggc gtcgacgtcg 17880
   tgctcaactc cctcgccggg gacttcgccg atgcgtccct gcgaatgctg ccgcgcggtg 17940
   gccgtttcct ggagttgggg aagacggatg ttcgtgaccc cgtcgaggtc gccgatgcgc 18000
   atccgggcgt gtcctaccag gcgttcgaca ccgtagaggc cggcccgcag cgaatcggcg 18060
   agatgcttga cgagctggtg gagctgttcg agggaggcgt gctggagccc ctgcctgtca 18120
   cggcttggga cgttcggcag gcgcccgagg cgctacgaca cctgagccaa gcgcggcatg 18180
   tgggcaagct ggtgctcacc atgcctccgg cgtgggacac cgccggcacg gttctggtta 18240
   ccggcggaac gggagcactt ggagcagagg tcgcccggca cctcgtgatc gagcacgggg 18300
   tgcgcaacct ggtgctcgtc agcagacgcg gtcccgcagc cagtggcgct gctgagctcg 18360
   tggcgcaact gacggcctac ggtgccgagg tttccctgca ggcgtgtgat gtcgccgatc 18420
   gtgagacctt ggcgaaggtg cttgccggca tcccggacga gcatacgttg accgccgtgg 18480
   tgcacgcggc tggtgttctc gacgacggag tggccgaatc gctcacagcg caacggctgg 18540
   accacgttct gcgcccgaag gtcgatggcg cgcgcaatct gcacgagctg atcgcacccg 18600
   acgtggccct cgtgctgttc tcgtcggtgt cgggcgtgct cggcagcggt gggcagggta 18660
   attacgcggc ggccaactcc ttcctcgacg cattggcgca gcaaaggcag tcgcgcggcc 18720
   tacctacgag atcgttggcc tggggtccct gggcggaaca tggcatggca agcaccttgc 18780
   gcgaagccga gcaggataga ttggcgctat ctgggctgct gccgatctcg accgaggagg 18840
   ggttgtccca gttcgacgcc gcgtgcggcg gcgcgcatac cgtggtggct ccggttcgaa 18900
   tcggccgctc gtccgacggg aacccgatca agtttcccgt cctgcgaggc ttggtcgagc 18960
   cgcatcgcgt caacaaggcg accgcggatg atgccgagag catccggaaa cggttgggac 19020
   gcttgccgga tgcagaacaa caccggattc tgctggacct cgtccgcacg cacgtggcgg 19080
   cagtgctcgg attcgccggt ccccaggaga tcaccgcgga cggcacgttc aaggcgctgg 19140
   gcttcgactc gttgaccgtg gtcgagttgc gcaaccggat caacggggca accgggctgc 19200
   gactgcccgc caccctggtg ttcaactacc cgacgccgga tgcgctcgcc gcacacctcg 19260
   tcaccgcgct ttccgcagac cgccttgccg ggacgttcga ggaactcgac aggtgggcgg 19320
   cgaacctgcc cgcgctggcc agggatgagg ccacgcgggc gcagatcacc acccggctgc 19380
   aggcgatctt gcagagcctg gcggacgtgt ctggcggaac cggcggcggc tccgtgccgg 19440
   accggctcag atcggccacg gacgaagagc ttttccaact cctcgacaac gatctcgaac 19500
   ttccctgatg cctcagccgg tgccttcgca gcttcctgga gggaaacgcc ccatgtcgaa 19560
   cgaagagaag ctccgggagt acttgcggcg tgcgctcgtg gatctgcacc aggcgcgcga 19620
   gcggcttgac gaggcggagt cgggggagca ggaacccatc gcgatcgtgg cgatgggctg 19680
   tcggtacccg ggtggggtgc acgacccgga aggtctgtgg aaactggtcg cctccggtgg 19740
   cgacgccatc ggtgaatttc ccgctgaccg tggttggcac ctcgacgagc tctacgatcc 19800
   cgacccggat cagcccggaa cctgctacac ccggcacggc ggcttcctcc acgaggccgg 19860
   cgagttcgac gcggggttct tcgacatcag cccccgtgag gcgctcgcca tggacccgca 19920
   gcagcggctg ctgttggaaa tctcctggga gaccgtcgaa tccgctggga tggacccgag 19980
   gtccttgcgg gggagccgca ccggggtgtt cgcgggattg atgtacgagg gctatgacac 20040
   cggcgcccac ccggaaggtg tcgaaggcta tctcggaacc ggcaatgcgg ggagcgtcgc 20100
   ctctggtcgg gttgcgtatt cgttcgggtt cgagggccca gcggtgacgg tggacacggc 20160
   gtgctcgtcg tcgttggtgg ccctgcattt ggcgtgtcag tcgttgcggc agggcgagtg 20220
   tgatttggcg ctggccggtg gagtgacggt gatggccacg ccggcgacgt tcgtggagtt 20280
   ctcccgtcag cgtggtctcg caccggatgg gcggtgcaag tcgttcgcgg ctgctgcgga 20340
   tggaaccggt tggggtgagg gtgccggctt ggtgttgctg gagcggctgt cggacgccag 20400
   gcgcaacggg catcgggtac tggcggttgt tcgtggtagc gcggtgaatc aggacggtgc 20460
   gtcgaacgga ttgacggccc ccaacgggct ggcccaggag cgggtcattc agcaggcgct 20520
   cacgagtgcg gggctgtcgg tgtccgatgt ggacgttgtg gaggcgcatg ggacgggtac 20580
   gcggcttggt gatccgatcg aggcgcaggc tctgatcgcc acctatggac aggatcggga 20640
   ccgggatcgg ccgctgtggt tggggtcggt caagtccaac atcggtcata cgcaggcggc 20700
   cgcgggcgtt gctggtgtga tcaagatggt catggcgatg cggcgcgggg agctgccgcg 20760
   cacgttgcac gtggacgagc cgaattcgca cgtggactgg tcggctggtg cggtccggct 20820
   cctcaccgag aacatccggt ggccagggac gggtacgcgc cgagttggcg tgtcgtcgtt 20880
   cggggtaagc ggtaccaacg cacacgtcat cctcgaacac gacccgctcg ccttgaccga 20940
   gaacgagaac gcagcggtgt ccccagcacc tgggatcgtg ccttgggcgt tgtccgggcg 21000
   gtcgtcgacg gcgctgcgag cccaggccga acggctgagc gagctgtgcg agcagaccga 21060
   tcccgacccc gtcgacgtcg gtttctcact ggccaccacg cgcacggcct gggagcaccg 21120
   agcggtggtg cttggtggtg atagcgctac attgcgttcc ggacttggcg ttgtcgctag 21180
   cggcgaaccg gcagtcgatg tcgttcaggg gagcgtcctg ggcggcgagg tcgtcttcgt 21240
   ctttcccggt cagggttggc aatgggccgg tatggcagtc gacttgctgg acgcttcgcc 21300
   gacgttcgcg cggcacatgg acgagtgcgc caccgcgctg cggaagtacg tggactggtc 21360
   gttggtcgac gtgctgcgcg gagcggagaa cgccccaccg ctcgaccggg tggacgtcct 21420
   gcaaccggtg tccttcgcgg tgatggtgtc gctcgccgag gtgtggcgtt cctacggggt 21480
   gcggccggcg gccgtcgtcg gccacagtca aggcgagatc gccgcggcct gcgcagccgg 21540
   ggtgctgcca ctggaggatg cggccaggct tgtcgccttg cgcagcagag cgttgaaggc 21600
   actatcgggg cgaggtggca tggcgtcgct ggcttgctct gcggatgagg ccgcggcgtt 21660
   gttcgcggga ttgggcggtc gtctggaaat tgcggcgatc aacggcccgc gatcggtcgt 21720
   ggtgtccggc gatctggaag cggtggaaga actgctggca gagtgcgctg aaagggacat 21780
   gcgtgcacgc cgtatccccg tcgactacgc ctcgcattcg gcacacgtgg aggtggttcg 21840
   gagcccggtg ctggcggctg cggccggcgt gcggcaccgg gacggccagg tgccgtggtg 21900
   gtcgacggtg atcggcgact ggttggatcc ggccgggctg gacggcgagt actggtaccg 21960
   gaacctccgg cagccggtcc gattcgaaca cgccgtgcag ggcctggttg agcggggatt 22020
   cggcctgttc atcgaaatga gtgcgcatcc ggtgctgacc atggcggtcg aggaaaccag 22080
   tgccgagtcg gagtccgccg tggccgcggt aggtaccttg cgacgtgact cgggcggccg 22140
   ccggaggttg ttacagtcgc tggccgaggc gtacgtgcgc ggcgccaccg tggactgggc 22200
   cgtggcgttc gggggcgtgg gtcgacggct ggacctgccg acctacccgt tccagcgccg 22260
   gcggtactgg ctggacaggg gagctgcctc cgaggaggct cgtgcgtttt cggacccggc 22320
   ggcggactgg ttctggcaag ccgtggagcg ccaagacctg aaaggcgtgg ccgacgccct 22380
   cgatctcgac gccgacgcac cgctgagcgc aacacttccg gccctgtccg tctggcaccg 22440
   tcaggaacga gaaaaggtct tggtggacgg ttggcggtac cgagtcgact gggtaccggt 22500
   ggccccgcag ccgatccgga gaacacggga aacctggctc ctggtcgttc ccgcgggcgg 22560
   cattgaagaa gcgctggtcg aacggttgac ggatgcgttg aacacgcgag ggatcagcac 22620
   cctgcgcctc gacgtgccac cgacggcgac aagtggggaa ctcgcgaccg gcctccgcgc 22680
   cgcagttggc ggtgacccgg tgaagggaat cctgtcgctc actgcgttgg acgagcgaac 22740
   acaccccgaa cgcaaggccg tccccagcgg gattgccttg ctgttgaacc tggtcaaggc 22800
   gctcggtgaa ggcgacctca gagttcctct gtggacgatc acgcgtggtg cggtcaaggc 22860
   agaccccgca gatcggctgc tgcgcccgat gcaggctcaa gcatggggtc tggggcgagt 22920
   agccgcactc gaacaccccg agcgctgggg tgggctgatc gacctcccgg aatcgctgga 22980
   cggcgacgtc ctcacaaggt tgggcgaagc gctcatcaac ggcttggcgg aggaccaact 23040
   ggcgattcgc cagtcgggcg tgctggcccg gcgcctggta ccggccccgg cgaatcagcc 23100
   cgctggacgt aagtggcgac cccgaggtag cgcgctgatc acgggcgggc tcggcgcggt 23160
   gggcgcgcag gtggcgaggt ggttggccga aagcggagcc gagcgaatcg tgctcaccag 23220
   tcgacggggc aaagaagcgc cgggcgccgc agagctggaa gccgaactcc gggcccttgg 23280
   agcgcaagtg tccatcgtgg cttgtgacgt gaccgatcgt gccgagatgt ccgcactgct 23340
   ggccgagttc ggcgtcaccg cggtgttcca cgcggccgga gtcggccggc tgctgccgct 23400
   ggcggaaacc gaacagaacg acctggccga aatatgcacg gccaaggttc acggcgctca 23460
   ggtgttggac gagctgtgcg acagcaccga tctcgatgcc ttcgtcctgt tctcctcggg 23520
   tgccggggtc tggggcggtg gcggtcaggg cgcctatggc gcggcgaacg cattcttgga 23580
   cacactcgcc gaacaacgcc gagcacgcgg tctgccggca accgcgatct cctggggcag 23640
   ctggggcggc ggcatggccg acggcgcagc gggcgaactc ctgcggcgac ggggaatacg 23700
   tccgatgccg gcggcgtcgg ccatcctggc tctgcaggaa gtactcgacc aggatgagac 23760
   gtgcgtgtcg atcgctgatg tggactggga ccgattcgtg cccacgttcg ccgcgacccg 23820
   cgccacccgg ttgctggacg aactgcccgc ggtgagaaag gcgatgtccg cgaacggccc 23880
   ggcagaacca ggcggctcgc cgttcgcccg caatctcgcg gagctgccgg aagcccaacg 23940
   acgccacgaa ctggtagatc tggtcagcgc ccaggtggca gccgtgctcg ggcacggcag 24000
   tcgcgaggaa gtccagcctg aacgggcgtt ccgcgcgctc gggttcgact ccctcatggc 24060
   ggtggacctg cgcaatcgtt tgaccaccgc caccgggttg cgcctgccga ccacaactgt 24120
   cttcgactac ccgaatccgg ccgcattggc cgctcacctg ctcgaggagc tggtgggcga 24180
   tgtcgcgtcg gccgcggtga ccactgccat cgcgccgtcg actgacgaac cggtcgcgat 24240
   cgtcgcgatg agctgccggt tccctggcgg cgcgcactcg ccggaagacc tgtggcggct 24300
   ggtcgcctcc ggcgcggagg tgatcggcga gttcccctcc gaccggggtt gggatgcgga 24360
   aagcctctac gatccggacg cttccaaacc tggaaccacg tatgcgcgga tggcgggatt 24420
   cctttacgac gccggtgagt tcgatgccgg cttgttcggc atcagcccac gcgaggcgtt 24480
   ggcgatggat ccgcagcagc ggttggtgct cgaaatcgcc tgggaagccc tcgaacgggc 24540
   cggaatcgat ccgttgtcct tgaagggcag tggggtcggc acgtacatcg gtgctggaag 24600
   ccgcgggtac gcgacggatg tgcggcagtt tcccgaggag gcggagggct acctcctgac 24660
   gggtacctcc gccagtgtgc tgtcgggtcg ggtcgcgtac tcgtttggtt tcgagggtcc 24720
   tgcggtgacg gtggacacgg cttgttcgtc gtcgttggtg gcgttgcact tggcgtgcca 24780
   gtcgttgcgt tcgggtgagt gtgatctggc gttggccggt ggtgtgaccg taatgtcgac 24840
   gccggagatg ttcgtggagt tctcccgtca gcgtggtttg gcgccggatg gccggtgcaa 24900
   gtcgttcgcg gagagcgcgg acggcaccgg ctggggcgaa ggcgcgggcc tgttgttgct 24960
   ggagcggttg tcggacgccc accggaatgg gcatcgggtg ttggcggtgg ttcgtgggtc 25020
   tgcggtgaat caggacggcg catcgaacgg actggcggcg ccgaatggtc cgtcgcagca 25080
   gcgggtgatc aaacaggcac tcgcgaatgc ggggctttcg gcgtctgatg tggacgccgt 25140
   ggaggcccat ggaaccggga ccaggctggg tgatccgatc gaggcgcagg ccttgatcgc 25200
   aacgtatggg cagggccggg agcgggatcg gccgttgtgg ttggggtcgg tcaagtcgaa 25260
   catcggtcac acgcaggcgg cggcgggtgt tgccggtgtg atcaagatgg tgatgtccat 25320
   gcggaacgac gagctgcccg ccacgctgca cgtgggtgcg cccacgtcgc aggtcgactg 25380
   gtcggcgggg gcggtccggc tccttaccga acaggtacct tggccggagt ctgatcgcgt 25440
   tcgtcgggtg ggggtgtctt cgttcgggat cagcggcacc aatgcacacg tgatcctcga 25500
   acaatctacg aatgcgccag atagtcccgc ggccacggac aaatcaggat ccggatctac 25560
   cgtggatatt ccggttgttc cctggttggt gtcgggacag acatcggatt ccctgcgggg 25620
   acaggctgaa cgagtcttgt cccaggttga gtcccggccg gagcagcgtc cgctggatgt 25680
   ggcctactcg cttgcttctg gccgagccgc gctggatgaa cgcgctgtcg tgctgggtgc 25740
   ggaccggaat gagctggtag ctggattggt ggcgttggcc gccggtcatg aggcttccgg 25800
   ggtgatcacc ggaactcgtg cttctgctcg gttcgggttc gtgttctcgg ggcagggcgg 25860
   tcagtggttg gggatgggcc gggagctcta ctcgaagttt ccggtgttcg ctgctgcgtt 25920
   tgatgaggct tgcgccgagt tggacgcaca tctgagtgaa gacctccggg tccgagatgt 25980
   ggtcttcggt tccgatgcgc agctgctgga tcagacgttg tgggcgcagt cgggactgtt 26040
   cgcgctgcaa gtcggcctct tggggctgct gggttcgtgg ggcgtccggc cggatgtggt 26100
   gatggggcat tcggtcgggg agttggccgc cgcgtttgcg gctggagtgt tgtcgttgcg 26160
   ggatgcggct cggttggtgg ccgcacgcgc ccggttgatg caagccctgc cctctgacgg 26220
   cgcgatgctc gcggtggccg ctggtgaaga cctgattcgg ccattgctgg ctggtcggga 26280
   ggcatccgtg aacgtcgccg cgctcaatgc ccccggttcg gtggtgttgt cgggtgatcg 26340
   ggatgtgctg gccgacatcg ccggccggct gaacgagctc ggagtccgga cgagacggtt 26400
   gcgggtctcc catgcttttc attcgcaccg gatggacccg atgttgggcg agttcgccca 26460
   gatcgcggag tctgcggagt tcggtaggcc aacgacaccg cttgtgtcga cgttgacggg 26520
   tgagctcgac agagctgggg aaatgagcac gccagggtat tgggtgcgtc aggtgcgtga 26580
   acccgtccgt ttcgccgacg gtgtccgggc cctggcagcg cagggcgtag acacggttgt 26640
   tgagctcggc ccggacggag cgctgtccgc actggttcag gagtgtgcca ccgggtttga 26700
   tcgggtcggg cggatttcgc ctgttcccct gatgcgcagg gagcgggacg agacccgttc 26760
   ggtgatgaca gccctggcgc atcttcacac ccgtggcggt gagttggact ggcaggcgtt 26820
   tttctccggc accggggcca ggcaggtcga gttgcccacg tatgccttcc aacgacggca 26880
   ctactggatc gaatccagtg cgcggacagc acgcgaccgc gcagacatcg gcgaggtggc 26940
   tgaacagttc tggaccgcgg ttgaacaagg cgatctggaa gcattggtct ccgcactgga 27000
   gcttggggcg gacgacgaca catgcgcatc tttgagcgat gtactgccgg cgctgtcatc 27060
   ctggcgaagc ggactccgca accgttcgct cgtcgattcc tgccggtacc gaatcaattg 27120
   gcattcctct cgggaagcac cggccccgaa gatttccggt acctggctgt tggtcgtgcc 27180
   cggcgatgcg gatgacggct tggccacggc tttgacgagt tcactggtcg aaggtggcgc 27240
   cgaggtcgtc cggatcgacc tgtccgaaga ggacctgcac cgcgaggacc tcgcacagcg 27300
   gctggccaat gcgctgacgg atgtcggtcg actcggtggc gtgctgtcgc tgttggggct 27360
   cgatgactcg gctgttggag aattctcctg cttgacaagg ggtttcgcgt tgactgtgca 27420
   gctggtgcgg gccttgcgca acgccgagct cgaggcgcct ttgtgggcgg tgacgcgcgg 27480
   cggcgtctcg ttggaagacg taagtgtgtc tcctgagcag gccttgattt gggggctgct 27540
   gcgtgttgcg ggcctggagc atccggagtt ctggggtggc ttgatcgacc tgccatcgga 27600
   ttgggacgac cgattgggtg cgcggttggt gggtgtgttg gcggatggtg gcgaggatca 27660
   agttgccatt cgtcgtggtg gtgtgttcgt gcggcggttg gaacgcgccg gtgcgtcggg 27720
   tgccgggtcg gtgtggcgtc ctcgggggac ggtgttggtg acgggtggta cgggcggttt 27780
   gggggcgcat gttgctcggt ggttggcggg tgccggggct gagcatgtgg tgttgaccag 27840
   ccgtcgtggc gcggaggctc cgggcgctgg ggaattgcga gcggagctgg aggcgctggg 27900
   tgctcgggtg tcgattgtgc cctgcgatgt ggctgatcgt gacgccgtgg ctggagtgtt 27960
   ggcagggatc ggcggggagt gtccgctgac tgcggtggtg cacgccgctg gggtcggcga 28020
   ggcgggcggc gtggtggaga tggccttggc ggactttgca gaggtgttgt cggcgaaggt 28080
   gcggggtgcg gcgaatctgg acgagttgct ggccgactcg gagttggatg cgtttgtgtt 28140
   gttctcctcg gtgtcgggtg tgtggggtgc cgggggacaa ggtgcgtatg cggctgcgaa 28200
   cgcctacttg gatgcgttgg ccgagcagcg tcgggcgagt gggttggccg ggaccgcggt 28260
   tgcgtggggg ccgtgggcgg gtgacggcat ggccgcgggc gaaaccggcg cacagctgca 28320
   tcgcatgggc ctggtgtcga tggaaccgag agcggctctg ctggcacttc agggcgcact 28380
   ggaccgcgat gagacctccc tcgtcgtggc cgatgtcgac tgggcacggt tcgccccagc 28440
   cttcacctcg gcacgtcggc gcccgctgct ggacaccatc gacgaggccc gagccgcatt 28500
   ggaaaccacc agcgaaaaag cgggaacagg caaacccgtt gagctcaagc atcgcctggc 28560
   cgggttgtca cggaaggaac gtgacgatgc ggtattggat ctggtgcggg cggaaacggc 28620
   agctgtgctg ggacgcgacg atgccacggc cctggcgccg tcgcggccgt tccaggaact 28680
   cggattcgac tccttgatgg cggtggagct gcgcaaccgg ctgaacaccg ccaccgggat 28740
   ccagctgccc gccagcacga tcttcgacta ccccaatgcc gagtcgctgt cgcgtcacct 28800
   ctgcgccggg cttttcccaa cagagacaac tgtggactcg gcccttgccg agctcgatcg 28860
   aatcgagcag cagctctcga tgttcaccga ggaagcgcgg gcacgggacc gaatcgcgac 28920
   acgactgcga gccctccacg cgaagtggaa cagcgcatct gaggcaccga ccggtgccga 28980
   tgtcctgaac acactcgatt cggcaacgca cgacgagatc ttcgagttca tcgacaacga 29040
   gctcgacctg tcctgagcag ttcctgcgga acgtccagtc gccgaaaccg ggtggaaatc 29100
   acaatggcca atgaagaaaa gctcttcggc tatctgaaga aggtaactgc cgacctgcat 29160
   cagacccggc agcgcctgct cgcagccgag agccggagtc aggagccgat cgtctccgcg 29220
   agctgccggc tgcccggcgg cgtcgactct cccgaagcgc tttggcaact cgtgcgcact 29280
   ggcactgacg ccatctcgga gttccccgcc gaccggggct gggatctcga ccggttgtac 29340
   gatcctgacc cggaccacca gggaacctcg tacacgcggg ccggcggttt cctcgcagat 29400
   gcgggcgatt tcgaccccgc catgttcggg atctcgccgc gtgaggcgtt ggcgatggac 29460
   ccgcagcaac ggctgttgct ggagctgacc tgggaggccc tcgaacgggc gggaatagac 29520
   ccgacatcgc tgcgcggcag caagaccggt gtcttcggcg gtgtcacgcc ccaggagtac 29580
   gggccgccct tgccggagat gagccggaac tctggcggtt ttggactcac cgggcggatg 29640
   gtgagtgtgg cgtcgggacg ggttgcgtat tcgtttggtt ttgagggtcc tgcggtgacg 29700
   gtggatacgg cgtgttcgtc gtcgttggtg gcactgcatt tggcgtgtca gtcgttgcgt 29760
   tccggcgaat gtgatctagc gttggccggc ggtgtgacgg tgatggccac gccggcgacg 29820
   ttcgtggagt tctcccgtca gcgtggtttg gcgccggatg ggcggtgtaa gtcgtttgcg 29880
   gctgctgcgg atggcaccgg gtggggtgag ggtgccggtc tagtgttgtt ggagcgcttg 29940
   tcggatgccc ggcgcaatgg gcacaaggtt ctggcggtgg tccgtggtag cgcggtgaac 30000
   caggacggcg cgtcgaatgg tttgacggcg ccgaatggtc cgtcgcagca gcgggtgatc 30060
   acccaggcgt tgtcaaatgc agggttgtcg gtgtccgatg tggatgcggt cgaggcgcat 30120
   gggacgggca cgcggcttgg tgatccgatc gaggcacagg ccctgatcgc cacgtacggg 30180
   cagggccggg agaaggatcg gccgttgtgg ttggggtcgg tcaagtccaa catcggtcac 30240
   acgcaggcgg ccgctggcgt tgccggcgtc atcaagatgg tcttggcgat gcggcacggg 30300
   cagcttcccg ccacgttgca tgtggatgat cccacgtcgg cggtggactg gtcggcgggt 30360
   tcggtccggc ttctcacgga gaacacgccc tggccggaca gtggtcgtcc ttgtcgggtg 30420
   ggagtgtcgt cgttcgggat cagcggcacc aatgcacatg tcattctcga acaatctcca 30480
   gtcgagcagg gcgaaccgac cgggccggtc gaaggcgagc gggaaccgga ggcagccatc 30540
   cccgtggtgc cgtggatggt gtcgggtaag acaccggagg ccgcgcgggc ccaggccgaa 30600
   cgggtgcttt cgcatatcga ggaccggccg gagctgtcgc cggtggatgt ggcgtattcg 30660
   ctaggcatga cgcgtgcggc gctggatgaa cgcgcagtga tgttgggctc ggaccgtgac 30720
   acgctcctga ccgggttgag ggcgttcgcc gacggttgcg acgtgcccga agtggtgtcg 30780
   ggatctgtgg ggaatggggg ccgcgtcggg tttgtgttcg ccggccaggg tgggcagtgg 30840
   ccggggatgg gccgggggct ctactcggtg tttccgggtt tcgccgatgc gtttgacgag 30900
   gcttgcgctg agttggatac acacctgggc caggaactgg gggttcggga tgtggtgttc 30960
   ggttcggatg cgcggctggt ggatcggacg gtgtgggcgc agtcggggtt gttcgcgttg 31020
   caggttggtt tgttgcggct gctgggttcg tggggtgttc ggcctgatgt ggtgttgggg 31080
   cattcggtgg gtgagctggc tgcggtgcac gcggcgggtg tgttgtcgtt gccggaggcg 31140
   gcgcggttgg tggcgggtcg tgcccggttg atgcaggcat tgccttctgg tggtgccatg 31200
   ttggcggtgg ccgcgagtga ggcccaggtc gaaccgttgc tggatcgggt gcggggccgg 31260
   gtcgagatcg cggcgatcaa cggtccggga tcggttgtgc tctctggcga ccgcgagctg 31320
   ctcaccgaga tcgccgatcg gttgcacgat caggggtgtc ggacgcgatg gttgcgggtg 31380
   tcgcacgctt tccattcgcc ccacatggag ccgatgctgg aagagttcgc ccagatcgcc 31440
   cgaagccgtg agtatcaagc acccgaactg ccgatcatct cgaccctgac cggtgagctg 31500
   gacggtggtc gagtgatggg cactcccgag tactgggtgc gtcaggtgcg tgagcccgtc 31560
   cgtttcgccg agggtgtcca ggcgcttgtc ggtcagggtg ccgacacgat tgtcgaattc 31620
   ggtccggacg gggcgttgtc gacgttggtc gaggagtgtt tggcggaatc cgggcgggtg 31680
   gccgggatcc cgctgatgcg caaggaccgc gacgaggcgc gaaccgtgct ggccgctttg 31740
   gcgcagatcc acacccgtgg tggtgaggtg gaatggcagt cgtttttcgc cggcaccggg 31800
   gcgaagcaag tcgagttgcc cacctacgct ttccagcggc agcgctactg gctggcatcc 31860
   accggcggtg cgggtgacgt gaccgccgcg ggattggccg aggcggacca tccgttgctc 31920
   ggtgcggtcg ttgcgttggc agacggcgaa ggtgtggtgc tgaccggtcg gctgacagcg 31980
   gattcgcatc cgtggttgtc cgatcaccgg gtgctgggcg aaatcgtcgt ccccggcacc 32040
   gcaatcgtcg agctggcgtg gcacgtcggc gagcgcctcg gttgtggccg ggtggaagaa 32100
   ctggctttgg aagcgcccct gatcctgccg gatcatggag cggtccaggt tcaggtgctg 32160
   gtgggaccgc ccggggaatc cggagcccgg tcggtggcgc tctactcccg ccctggagat 32220
   gcgaccgaat ccgagtggaa gaagcacgcg acgggggtgc tgctgccacc cgtggccgcc 32280
   gagaatcatg agctgcccgc ctggcccccg gagaatgcga ctgaaatcga tgccgacgag 32340
   gtctacgaat tcctcgaagg gcacggtttc gcgtacggac cggcctttag atgtctgcgc 32400
   ggtgcctggc gacgaggcgg ggaggtgttc gccgaagtcg cgttgccgga tggcatgcag 32460
   gtgggggtgg atcgattcgg cgtccacccc gcgttgttgg acgcggttct gcatgccgcc 32520
   gcggccgaga cgtccgtggt ccagagcgaa gcgcgggtgc cgttctcgtg gcgtggggtg 32580
   gaacttcgcg ctaccgaaac cgcggtggtg cgggcacgca tctcgttgac cgcggatgac 32640
   gagctgtcgt tggtcgcagt ggacccggtt ggcggattcg tggcctcggt cgattcgctg 32700
   gtgacacgac cgatctcccg gcagcaggtg aggtctggcg cgatcggtga ttgcctgttc 32760
   gaagtggagt ggcaccggag agcgttgttg gaaacagccg ccgacgacgg ccttgccatc 32820
   gtcggtgacg gtgccagttg gccggaatcg gtgcgcgcaa ccgcacggtt cgcgaccctg 32880
   gatgagctcc gttcggcggc ggactcggat gttcccgccc cgggtccggt gttggtcgca 32940
   gctatgtcgg ccgaagaggt cgaaagtgaa tccctgccgt cgcgcgccca ggagtcgacc 33000
   tccgatctgc tggctctcgt gcagtcgtgg cttgccgatg agcagttcgc cgaatcccag 33060
   ctcgtggttg tcacgcgtgc agcggtgtcg gccgactcgg atacggacgt cgccgacctg 33120
   gtgagtgcgt cgtcgtgggg gttgttgcgt tcagcccagt cggagaaccc gggtcgcttc 33180
   gtactggtgg acgtggacgg cacaccagag tcgtggcagg cgttgccgac cgccgtgcga 33240
   gcgggagaac cgcagctggc acttcggcgc ggcgtggcgc tggtgcctcg gttggcgcga 33300
   ctcaaggcgc acggggaggg ctcctccccg cgactcgaca cggacgggac agtcctcatc 33360
   accggtggca ctggtgcgtt gggtggagtg gttgcccgtc acctggtggc ggagcacggg 33420
   atccggcgtt tggttttggc aggccggcgt ggctggaacg cgcctggagt ccacgatttg 33480
   gtggatgagc tggcgcgctc gggcgctgtg gttgacgtgg tggcttgcga tgtgggtaac 33540
   cggacagatc tggagcaggc gctggccgcc attccggtcg accgcccgtt gcgggggatc 33600
   gtgcataccg ctggggtgtt ggccgacgga gtgctcgggt ccttgtcggc ggcggatgtg 33660
   gacacggtgt tcgccccgaa ggtggcgggg gcgtggcatc tgcatgagtt gacccgcgag 33720
   ctggatctgt cgttcttcgt tcttttctcg tccttctcgg ggattgcggg tgccgcgggg 33780
   caggccaact acgcggcggc gaacacgttc ctggatgcat tggcaggtta tcgccgcgcg 33840
   cgtggactac ccgggttgtc gttggcatgg ggactgtggg cgcaacccgg cggtatgacg 33900
   agtggcttgg acgcggcgtc ggtggagcgg ttggcgcgga cgggcatagc agaacattcc 33960
   acggaggatg gactccgcct gttcgatgcc gcgattgcga aggacagggc ttgcgtcgtt 34020
   cccgctcgat tggacagggc gctgctggtc gagcacgcac ggtcgcacgc gattccagca 34080
   ctgatgaccg cgttggctcc tgctcgtggc ggtgtggcga ggagagcaac caactctcag 34140
   gccgcggatg aggacgcgct gttgggtttg gtgcgggacc acgtctcggc ggtactgggc 34200
   tattcgggtg cggtcgaggt tgggggcgac cgtgctttcc gtgatctagg ttttgattcg 34260
   ttgtctggag tggagttgcg gaaccgcctg gccggggtgc tgggggtgcg gttgccggcg 34320
   actgcggtgt tcgattaccc gacgccgcgg gcgctggcgc gtttcttgca tcaggaattg 34380
   gcaggcgagg tcgggtcgat gtcgacgccg gtgaccaggg cagcgagcgt cgaagaggat 34440
   cttattgcga ttgtcgggat ggggtgtcgt tttccgggtg gggtgtcgtc gccggaggag 34500
   ctttggcggt tggtggccgg gggcgtggat gcggtggctg ggttcccgga cgatcgcggc 34560
   tgggatctgg cggggttgtt cgatccggat cccgatcatc tcggcacttc gtacgtatgt 34620
   gagggcgggt ttctgcggga cgcggcggag ttcgatgccg acatgttcgg cgtcagcccg 34680
   cgtgaggcgt tggcgatgga tccgcagcag cggttgctgc tggaggtcgc ttgggaaacc 34740
   ctggagcggg ctgggatcga tccgttctcg ttgcacggca gccggaccgg tgtgttcgcg 34800
   ggcttgatgt accacgacta cggggcccga ttcatcacca gagcaccgga gggcttcgaa 34860
   gggcacctcg ggacgggtaa tgcggggagc gtgctgtcgg gtcgggttgc gtactcgttt 34920
   ggttttgagg gtcctgcggt gacggtggat actgcgtgtt cgtcgtcgtt ggtggcgttg 34980
   cacctggcgg gtcaagcact gcgggccggt gagtgcgaac tcgcccttgc cggtggcgtc 35040
   acggtgatgt cgacgccgac gacgttcgtg gagttctccc gtcaacgggg actggctccg 35100
   gatgggcggt gcaagtcgtt cgcggcggcc gcggatggca ccggttgggg agaaggcgcg 35160
   ggcctggtgt tgctggagag gttgtcggat gcccggcgca acggacacaa ggtcctggcg 35220
   gtggttcgtg gtagcgcggt gaaccaggac ggcgcgtcga atggtttgac cgcgccaaat 35280
   ggcccgtcac agcaaagggt gatcacccag gcactcacga gtgccgggct gtccctgtcc 35340
   gacgtggatg ctgtggaggc gcatgggacg ggcacgcggc taggtgatcc gatcgaggca 35400
   caggcgttga tcgctacgta tggccgagat cgtgatcccg gtcggccgct gtggttgggg 35460
   tcggtgaagt cgaatattgg tcatacccag gcggcagcgg gtgtggctgg tgtgatcaag 35520
   atggtgatgg cgatgcggca tggggagctg ccgcgcacgt tgcacgtgga cgagccctcc 35580
   gcgcaggtgg actggtctgc gggcacggtc caactcctca cggagaacac gccctggccc 35640
   gacagcggtc gtcttcgtcg ggccggcgtg tcatcgttcg ggatcagcgg caccaacgcg 35700
   cacctgatcc ttgaacaacc tccgcgagag acgcatcgcg caacagagcc ggattcgagt 35760
   tctgtcctcg atgttccggt ggtgccgtgg atggtgtcgg gcaaaacacc cgaagcgcta 35820
   tccgcccagg cagatgcact gatgtcctac ttgaacaatc gcgttgatgt ttctccacga 35880
   gatatcgggt attcacttgc ggtgacccgt ccggcgttgg accaccgggc tgtcgtgctg 35940
   ggtgcggatc gtgaagcgtt gctgccgggg ttgaaagcgc tggctgccag tcatgacgcc 36000
   gctgaggtga tcacaggcac tcgtgccgct gggccggtcg gattcgtgtt ctccggtcaa 36060
   ggtggtcagt ggcccgggat gggaagcggg ctctactcgg cgtttccggt gttcgccgac 36120
   gcgtttgatg aagcctgcgg cgagctggat gcgcatctcg ggcagaaagc acgggttcga 36180
   gacgtgatgt ccggttcgga taagcaactt ctggatcaga ctttgtgggc gcagtcgggc 36240
   ctgtttgcgt tgcaagtcgg gctctgggag ttgttgggtt cgtggggtgt ccgacccggt 36300
   gtggtgctgg gccattcggt cggtgagctg gcggcggcgt ttgcggctgg agtgttggcg 36360
   ttgccggatg cggctcggtt ggtggcaggc cgtgcccggt tgatgcaagc cctgccacct 36420
   ggcggtgcca tgctcgcggc ggctgctgga gagaaggagc tgcggccgtt gttggccgac 36480
   cgggctgatc gtgtggggat cgccgcggtc aacgcacccg agtcggtggt gctctccggt 36540
   gatcgggatg cgctcgatga catcgccggc cgactggacg ggcaaggggt ccggtcgagg 36600
   tggttgcggg tttcgcatgc gtttcattcg catcggatgg atccgatgct ggaggagttc 36660
   gccgaaatcg cacggagcgt ggactaccgg tcgccagggc tgccggctgt gtcgacgttg 36720
   acgggtgagc tcgatgaggt cggcatgatg gctacgccgg agtattgggt gcgtcaggtg 36780
   cgagaacccg tccgcttcgc cgacggtgtt gctgctctcg cggctcacgg tgtgagcagc 36840
   atcgtcgagg tcggtccgga cggggtgttg tcggcgctgg tgcaggagtg tgcggccgga 36900
   tccgatcagg gcggacgggt ggccgcggtt ccactcatgc gcagcaattg cgacgaggcg 36960
   caaaaggtga taacggcctt ggcgcaggtc catgcgcgtg gtgctgaggt ggactggcgg 37020
   tcgtttttcg ccggtaccgg ggcaaagcag gtcgagctgc ccacgtatgc cttccaacga 37080
   cagcggtact ggcttgactc gccatccgaa ccggtcgggc aatccgccga tctcgcgccc 37140
   cagtcgggct tctgggaact cgtcgagcag gaagatgtca gcgcgcttag cgccgccctg 37200
   aatataaccg gcgatcccga cgtgcaggcg tccctggaat cggtggttcc ggtcctctcc 37260
   tcctggcatc gccggatccg caacgaatcc ctggtgcacc agtggcggta ccgcatttcc 37320
   tggcatgagc gggcagatct gccagaccgg tcgttgtcgg ggacatggct cgtcgtcgtg 37380
   ccggagggtt ggtctacgag tcagcaagtt ctgcgtttcc gcgagatgtt cgaggaacgg 37440
   ggttgcgcgg cggttttgtt cgagctcgcc gggcacgacg aggaagccct ggtgcaacga 37500
   ttccgctcgt tgcctgtcgc gtcaggggga ataagcggcg tgctgtcctt gctggcgctg 37560
   gatgaatcgc cgtcctcgtc gaacgctgcc ttgccgaatg gtgcgctgaa ctcattggta 37620
   ctgctgcgag ctctgcggac cgcggatgtg ccggcgccat tgtggttggc gacgtgtggt 37680
   ggggtggcgg taggggatgt gccggtgaat ccggggcagg cgctgatgtg gggactgggc 37740
   cgcgtcgtcg gcctggaaaa tccggactgg tggggcggcc tggtcgacgt gccggacttg 37800
   ctcgataagg acgctcaaga acgcttgtcg gtcgtgttgg ctggtcttgg cgaggacgag 37860
   atcgcggtgc gccccgatgg cgtgttcgtg cggcggttgg aacgcgctga tttgccggat 37920
   atggggtcgg catggcgtcc tcggggcacc gtgttggtga cgggtggtac gggcggtttg 37980
   ggggcgcatg ttgctcggtg gctggcgggt gccggggccg agcatgtggt gttgaccagc 38040
   cgtcgtggcg cggaggctcc gggcgctgga gatttgcgag cggagctgga ggcgctgggc 38100
   gctcgggtgt cgatcagatc ctgcgatgtg gcagatcgtg acgctttggc cgaagtgttg 38160
   gcgaccattc cggatgattg cccgctgacc gcggtgatgc atgcggcggg ggtcgttgaa 38220
   gtcggcgacg tggcgtcgat gtgtctgacc gacttcattg gggtgctgtc ggcgaaggtg 38280
   ggtggtgcgg cgaatctcga tgagttgctc gccgacgtcg agctggatgc cttcgtgctg 38340
   ttctcctcgg tatcgggtgt gtggggtgct ggggggcagg gcgcttatgc ggcggcgaac 38400
   gcctacttgg atgcgttggc gcagcagcgt cgggcaaggg gcttggccgg gactgcggtt 38460
   gcgtgggggc cgtgggccgg tgacggcatg gccgcaggtg aaggcggcgc acagctgcgc 38520
   cgtaccggcc tggtgccaat ggctgcggat cgcgcgttgc tggcacttca gggtgcattg 38580
   gatcgagacg agacatccct ggtcgtagcc gatatggcat gggagaggtt cgccccggtg 38640
   ttcgccatgt cccgtcggcg tccgctgctc gacgagctgc ccgaagcaca gcaggcgttg 38700
   gcggatgcgg agaacaccac gggtgcggcg gactcggccg gcccgctgca gcggatcgtg 38760
   ggcatggcag ccgccgaacg ccgccgggcg atgatggaac tggtgctggc ggagacctcg 38820
   attgtgttgg ggcacaacgg gtcggatgca gtgagtcccg accgggcgtt ccaggagctc 38880
   ggattcgatt cgctgatggc cgtcgaactg cgcaacaggc tgggcgaggc aacaggattg 38940
   agtctgccga ccacgttgat cttcgattat ccgagcccat ccgctctggc ggagcagctg 39000
   gtcggcgagc tggtgggagc gcagcccgcg accaccgtcg tggccggggc cgatccagtg 39060
   gatgatccgg ttgtcgtggt cgcgatggga tgccggtatc cgggcgatgt ctgctcgcct 39120
   gaggagctgt ggcagctggt ttccgcggga cgtgatgcgg tttcgacgtt ccccaccgat 39180
   cggggttggg actgcgacgc gttgttcgac ccggatccgg atcgggcagg ccgtacctac 39240
   gtgcgagaag gtgccttcct gaccggtgct gatcggttcg atgcggggtt cttcggcatc 39300
   agccctcgcg aggcgcgagc aatggatccg cagcagaggt tgttgctcga ggtggcgtgg 39360
   gaggttttcg aacgagcggg gatcgctccg ctgtcgttgc ggggcagcag gaccggtgtg 39420
   ttcgcgggca ccaatggaca ggaccacggt gcgaaagtgg ctgccgcgcc ggaggcggcg 39480
   ggtcacctcc tgaccggaaa cgccgcgagt gtcatggccg gccggatttc ctacacgttc 39540
   ggcctcgagg gtcctgcggt ggcggtggat accgcgtgtt cgtcgtcatt ggtggcgttg 39600
   catttggcgt gccagtcgct gcgttcgggt gagtgtgata tggcgttggc gggtggtgtg 39660
   acggtgatgt cgacacccct ggcgttcctc gaattctctc gtcagcgcgg tttggcgccc 39720
   gatggccggt gtaagtcgtt tgcggctgcg gcggatggca ccgggtgggg tgagggcgcc 39780
   ggcctggttc tgctggagcg gttgtcggat gcgcgtcgta atggtcaccg ggtgttagcc 39840
   gtggttcgcg ggtctgcggt gaatcaggat ggtgcgtcga atggcttgac ggcgccgaat 39900
   ggcccgtcgc agcagcgggt gatccggcag gccctcgcga atgcgggact gtcggcgtcc 39960
   gatgtggatg tcgtggaggc gcacgggacc ggcaccgggc tcggggatcc gatcgaggcg 40020
   caggcactga tcgcggcata tgggcaggga cgggatcctg aacgggccct gtggttgggg 40080
   tcgatcaagt ccaacatcgg ccacacgcag gcagcggccg gtgtggctgg ggtcatcaag 40140
   atggtgcagg ccatgcggca tggggagttg cctgccacgt tgcacgtgga caaacccact 40200
   ccgcaggtcg actggtctgc cggggccgtt cggctcctca ccgggaacac gccctggccc 40260
   gagagcggcc gtcctcgtcg agctggggtg tcgtcgttcg ggatcagcgg caccaacgca 40320
   cacctcatcc tcgaacaacc gccgtcggaa ccagcggaga tcgaccgttc gaatcggcgg 40380
   gtcactgcgc atccggcggt gatcccgtgg atgttgtcgg ccaggagtct cacagcgctg 40440
   caggcccagg cggctgcgct gcagggccgg ctggaccggg tgcctggcgc ttctccgctg 40500
   gatttggggt attcactcgc gaccactcgt tctgtgctgg acgagcgcgc cgtcgtgtgg 40560
   ggtgccgatc gggagaccct gttgtcgagg ctggcagcgc tggccgatgg ccggactgcg 40620
   ccgggggtgg tcaccggcgc tgcgaattcc ggtggccgca tcggattcgt tttttccggt 40680
   cagggcagtc agtggctggg gatgggaaag gcgttgtgcg cggctttccc ggcgttcgca 40740
   gacgccttcg aggaagcctg cgacgcgctg ggcgcgcact tgggcgcgca cttgggcgcg 40800
   gacttgggcg tggacgtccg gggcgtgctg ttcggtgctg atgagcaggt gctcgaccgg 40860
   acgttgtggg cgcagccggg gatcttcgcg gttcaggtcg gcctcctggg attgctgagg 40920
   tcgtggggcg tgcggccaga cgcggtgctg gggcactcgg tcggcgagtt ggctgcggcg 40980
   cacgcggctg gtgtgttgtc cttgccggac gcggcacggt tggttgcggc ccgggccagc 41040
   ctgatgcagg cattgcccac cggcggcgca atgctcgcgg tcgccaccag cgaggcggcg 41100
   gtcgaaccgc tgcttgccgg gatgtgcgat cgggtcagca tcgctgcgat caacggcccg 41160
   gagtcggtag tgctctccgg cgaccgcgac gtgctcgcag aggtcgccgg cgaactcgat 41220
   gcccgagggc ttaggaccaa atggttgcgg gtctcccacg ctttccactc gcaccggatg 41280
   caaccgattc tggacgagta cgccgaaacc gccgggtgcg tcgagttcgg tgaaccggtg 41340
   gtgccgatcg tctccgccgc gaccggtgcg ctggacaccg ccggactgat gtgcgcagcc 41400
   ggctactggg tgcgccaggt gcgtgatccc gtccgcttcg gagacggtgt ccaagcgctc 41460
   gtggaccaag gcgtggacac gatcgtcgag ttcggcccgg acggggcgtt gtcggccttg 41520
   gtccagcagt gcttggccgg gtccgaccag gccgggaggg tggcggcgat cccgctgatg 41580
   cgcagggacc gcgatgaggt cgagaccgcg gtggctgccc tggcgcacgt gcatgtccgc 41640
   ggcggtgcgg tggactggtc ggcttgcttc gccggcacgg gcgctcgcac cgtcgagttg 41700
   cccacctacg ccttccagcg gcagcggtac tggctggccg ggcaagcgga cgggcgtggc 41760
   ggcgatgtgg ttgccgaccc ggtcaacgcg cgcttctggg agttggtcga gcgcgccgat 41820
   ccggaaccgt tggtggatga gctctgcatc gaccgggacc agcccttcag ggaggtgctg 41880
   cccgtgctgg cttcctggcg cgagaaacaa cgccagaagg ccgtcacgga ttcttggcgc 41940
   taccaggtgc ggtggaggtc cgtcgaggtg cagtccgcag ccagcctccg gggcgtgtgg 42000
   ctggtggtgc ttccagctga cggactccga gatcaaccgg cggccgtcat cgacgcgctg 42060
   atcgcgcgcg gcgccgaggt cgcggtcctg gaattgaccg agcaggactt ccaacgcggt 42120
   gcgcttgtgg acaaggtgcg cgccgtcatt gccgaccgca ccgaggtgac gggtgtgctg 42180
   tctctgttgg caatggacgg aatgccctgc gcagagcatc cgcacctgtc ccgtggtgtc 42240
   gccgctaccg tgatcctgac gcaggtgttg ggcgatgcgg gcgtttccgc cccgctgtgg 42300
   ctggccacga ctggtggcgt cgaggtcggg accgaggacg gtccggccga tccggaccac 42360
   ggcttgatct gggggctcgg cagggtcgtc ggccttgaac atccgcagcg gtggggtggc 42420
   ctgatcgacc ttccggcgac actggacgag acgtcccgga acgggttggt ggccgcgctc 42480
   gccgggacgg cggccgaaga tcagctcgcc gtgcgttcat ccgggttgtt cgttcgcaga 42540
   gtggtgcgcg cagcgcagaa ttcccgttca gggacatggc gtagccgggg aacggtcctc 42600
   atcacgggcg gaacaggcgc gctcggtgcc gaggtcgcac gatggctggc ccggcggggt 42660
   gctgagcatc tggtgttgat cagtcgccgc ggtccggaag ctcccggcgc cgcggacctg 42720
   caggccgagc tgaccgagct cggcgtgaaa gtcacagtcg tggcctgtga tgtgacggac 42780
   ggcgacgaac tgagggcggt gctggcggcc gttccgacgg agcatccgct gtcggcggta 42840
   gtgcacaccg ccggcgtcgg gacgcctgcg aacctggccg agacgacctt ggcgcagttc 42900
   gccgacgtgt tgtcggccaa ggtcgtcggc gcggcgaacc tggaccggct gcttggtggg 42960
   caaccgttgg acgccttcgt gctgttctcc tcgatctcgg gggtttgggg agccggcggc 43020
   caaggagcct attcggccgc caatgcgtat ctcgatgccc ttgccgagcg ccgacgggct 43080
   tgcggtcggc cggcgacgtg cgtcgcctgg ggtccgtggg ccggtgcggg catggccgtt 43140
   caggaaggca acgaggcgca tctccgccga aggggcctgg taccgatgga accgcagtcg 43200
   gccctctccg cgctgcaaca ggccctgtcc cgacgagaaa ccgccatcac cgtcgcagat 43260
   gtggactggg aacgattcgc cgccactttc accgcggccc gcccgcggcc actattggat 43320
   gagatcgtgg atctacggcc caacaccgag actgcggaga agcacggtgc cggcgagctg 43380
   gggcagcagc tggccgcact gccggccgct gagcgcggac atctgctgct ggaggtggtg 43440
   ctggcggaaa ccgccaacac cctggggcac gattcggcgg aggctgtgca acccgatcgg 43500
   accttcgccg aactgggctt cgattcgctt accgcggtag agctgcgcaa caggttgaac 43560
   gcggtgaccg ggcttcgcct gccgccgacg ctggttttcg accacccgac accgctggcg 43620
   gtgtccgaac agttggttcc ggcgttggtc gcggagccgg gcgatggcat cgagtcgttg 43680
   ctcgcggagc tcgacaggct ggataccacg ttggcgcaac gaccttcgat cccaccggaa 43740
   gaccaggcca aggtggcgga gcgcttgcag gcactcatcg ccaagtggga cggggcgcgt 43800
   gatggcacgg ccaaagtgac gtcaccccaa tcgctgacgg cggccacgga cgacgaaatc 43860
   ttcgacctca tcgaccggaa gttccggcgc tgaccgcctt cttcctcgcc tcagctcccc 43920
   tgatcactgg aacggtgtat ttcgatggcc aatgaagaaa agctccgcga gtacctcaag 43980
   cgtgtcgtcg tcgaactgga ggaggcgcac gaacgcctgc acgagttgga gcgccaggag 44040
   cacgacccca tcgcgatcgt gtcgatggga tgccgttatc ccggtggcgt ctccactccg 44100
   gaggagctgt ggcgactggt cgtcgacgga ggagacgcga tcgcgaactt ccccgaagac 44160
   cgtggctgga acctgggcga gctgttcgat cctgatccgg gtcgagccgg gacctcctac 44220
   gtccgcgagg gtggtttcct gcgcggagtc gcggacttcg atgccgggct cttcgggatc 44280
   agtccgcgcg aggcgcaggc gatggacccg caacagcggt tgctgctgga gatctcgtgg 44340
   gaagtgctcg agcgcgccgg tatcgacccg ttttccttgc ggggcaccaa gaccagtgtg 44400
   tttgcgggcc tgatttacca cgactacgcg tcgcggttca gcaagacccc agccgagttc 44460
   gagggttact tcgccaccgg gaacgcgggc agcgtcgcat ccggccgggt ggcttacacc 44520
   ttcggattgg agggcccggc ggtcaccgtg gacaccgcct gctcgtcgtc cctggtggcg 44580
   ttgcacctgg cctgccagtc cctgcggctg ggcgaatgcg acctggccct ggccggtggc 44640
   atttcggtga tggccacgcc gggagccttc gtcgagttca gccggcaacg cgcactcgcc 44700
   tcggatggcc ggtgcaagcc cttcgcggat gccgcggacg gcacgggctg gggcgagggc 44760
   gccggaatgc tgctgctgga acggctgtcg gacgcacggc gaaacggcca cccggtgctg 44820
   gcggcggtag tcggttccgc gatcaaccag gacgggatgt ccaacggcct gaccgcgccc 44880
   agcggtcccg cacagcagcg agtgatccgc caggccctga cgaacgccgg gttgtcgccc 44940
   gccgaggtcg atgtggtcga ggcgcacggt acgggcacgg ccttgggcga cccgatcgag 45000
   gcgcgggccc tgatcgccac ctacggggcg aaccggtcgg cggatcaccc gctgctgctg 45060
   ggttccctca agtcgaacat cggccacacc caggctgccg ccggtgtggc cggggtgatc 45120
   aagtcggtca tggccatcag gcaccgggag atgccccgca gcctgcacat cgaccagccc 45180
   tcgcggcacg tggactggtc ggcgggcgcg gtgcggctgc tcacggacag cgttgactgg 45240
   gcggatcccg gccggccgcg ccgagcaggg gtgtcctcgt tcggcatgag cggtaccaac 45300
   gcacacctga tcgtcgagga agtatccgac gagccggtct cgggcagtac cgagccgacc 45360
   ggggcacttc cctggccgct gtccggcaag acggagaccg cattgcgcga gcaggctgcc 45420
   gagctgctct ccgccgtgac cgcgcacccg gagccgggtc tggggaacgt cgggtactcg 45480
   ctggccaccg gtcgcgctgc gatggagcac cgggctgtcg tggttgccga ggatcgggac 45540
   tccttcgtcg ccggactgac ggcgttggct gcgggcgttc cggcagccaa cgtggtgcaa 45600
   ggggcggccg actgcaaagg aaaggtcgcg ttcgtgttcc ccggccaggg ctcgcattgg 45660
   caggggatgg cgagggaact gttcgaatcc tcgccggtgt tccggcggaa gctggaggaa 45720
   tgcgcggcgg ctacggcccc ctacgtggac tggtcgctgc tcggcgtcct tcgcggtgat 45780
   cccgatgcac ccgcactgga tcgcgacgac gtgattcagt tcgcgctgtt cgccatgatg 45840
   gtgtcgctgg cagaactgtg gcgttcgtgc ggagtggagc ccgccgcggt ggtcggtcac 45900
   tcccagggcg agatcgccgc cgcccatgtg gcgggggctt tgtccttgac ggatgcggtg 45960
   cgcatcgtcg ctgcccgctg caatgcggtg tcggtgcttg cagggaaagg aggcatgctc 46020
   gcgatcgcct tgccggaaag cgcagtggtg aagcgaatcg caggcctgcc agagttgacc 46080
   gttgcagcgg tcaacggacc cggctccact gtcgtttccg gcgaaccgtc cgctctggag 46140
   cgtttgcaga ccgaactgtc cgcggagaac gtgcaggctc ggcgggtgcg aattgattac 46200
   gcctcgcact cggcgcagat cgcacaggtc cagggccggc ttctggaccg gctgggcgag 46260
   gtcgggtccg aacctgctga gatcgctttc tactcgacgg tgaccggcga gcggacggac 46320
   accggccggc ttgacgcgga ctactggtac cagaaccttc ggcagcccgt ccggttccag 46380
   cagaccgtcg cccggatggc agatcagggc tatcggttct tcgtcgaggt gagcccgcac 46440
   ccgctgctca ccgcgggaat ccaggaaacg ctggaagccg cggacgcgga cgcgggcggg 46500
   gtggtggtcg gttcgctgcg gggtggcgag ggcggctccc ggcgctggct gacttcgctg 46560
   gccgagtgcc aggtgcgcgg actaccggtg aattgggaac aggtattcct cgacaccgga 46620
   gcccgacgcg tgccgctgcc gacatacccg ttccagcggc agcggtactg gttggagtcc 46680
   gccgagtacg acgcgggcga tctcggttcg gtgggcttgc gctccgcgga gcatcccctg 46740
   ctcggggctg cggtgacact ggccgatgcg ggcgggttcc tgctgaccgg caagctgtcg 46800
   gtcaagaccc agccctggtt ggccgaccac gcggtccgtg gggcgatcct gctgcccggc 46860
   accgccttcg tggaaatgct gatacgcgcc gcggaccagg tcgggtgcga tctgatcgag 46920
   gagttgtccc tgacgactcc gctggttctg cccgcgaccg gtgcggtgca ggtgcagatc 46980
   gcggttggcg gtccggacga ggccgggcgc cgctcggtcc gcgtgcattc ctgtcgggac 47040
   gactccgtgc cgcaggactc gtggacctgc cacgcgaccg gcacgttgac caccagtgag 47100
   caccgggacg ccggccaggc ccgcgatggg atttggccgc cgaacgatgc tgtcgcggtt 47160
   ccactggaca gcttttacgc gcgcgcagct gagcggggct tcgatttcgg tccggcgttc 47220
   caggggttgc aggcggtttg gaaacgcgga gacgagatct tcgccgaggt cggcctgccc 47280
   gcagcacagc gcgaggacgc cggcaggttc ggagtccacc cggctctgct ggatgcggca 47340
   ctgcaggcgc tgggcgcagc cgaggaggat ccggacgagg gatggctccc cttcgcgtgg 47400
   caaggtgtgt ccctcaaggc gaccggcgcg ctttcgcttc gggtgcacat cgtcccggcg 47460
   ggtgcgaacg cggtgtcggt gttcacgacc gacgcgacgg gccaagccgt gctctccatc 47520
   gactcgctgg tgctgcgcaa gatttcggac gagcagttgg cagcggtccg tgcgatggac 47580
   cacgagtccc tgttccgggt cgactggagg cgaatctcgc ccggcgctgc caagccggtc 47640
   tcctgggcag tgatcggcaa tgacgaactc gctcgagcct gcggctcggc acttggcacg 47700
   gaactccacc ccgacctgac cgggttggct gacccgcccc cggacgtggt ggtggtgcca 47760
   tgcggtgcgt ttcaccagga cttggaggtt gcttccgagg cacgtgccgc aacgcaacgc 47820
   gtgcttgacc tgatccaggg ttggttggcg gcggagcgat tcgccggatc tcgcctggtg 47880
   gtggtgacgt gtggtgcggt gtcgaccggg cccgccgagg gtgtttccga cctggtgcat 47940
   gctgcgtcgt ggggcttgtt gcgttcagcg cagtcggaga acccgaatcg attcgtgttg 48000
   gtcgatgtgg acgcaaccgc cgagtcatgg cgcgcgctcg cggcggcggt gcgttccgga 48060
   gaaccgcagc tagcgctgcg cgccggcgaa gtccgagtgc ctcgcctgac acgatgtgtt 48120
   gccgccgagg acagccggat cccagtgcct ggtgcggatg ggacggtgtt gatttccggc 48180
   ggtacgggcc tgctgggcgg gttggtagcc cggcatttgg tggcggagcg cggtgtccgc 48240
   cgcctggtgc tggcagggcg acgcggctgg agcgcccccg gggtcaccga attggtggat 48300
   gagctggtgg gcctgggagc tgtggtcgag gtggcgagct gtgatgtcgg ggaccgggcc 48360
   cagctggacc ggctgctgac gacgatctcg gcagagttcc cgctgcgcgg agtggtgcat 48420
   gcggccgggg cactggccga cggggtcgtc gagtcgttga caccagagca cgtggcaaag 48480
   gtgttcgggc cgaaggtcgc cggtgcgtgg cacctgcacg agctgacccg tgaactggat 48540
   ctctcgttct tcgtgctctt ctcctcgttc tccggggtgg tgggggctgc gggtcaagga 48600
   aactacgcgg cggcgaacgc gttcctggac ggcctggctc agcaccggcg gacggcggga 48660
   ctgcctgcgg tgtcgctggc ttggggcttg tgggagccga ccagcgggat gaccggagcg 48720
   ctcgatgcgg cggaccgcag ccgcatttcg cgcaccaatc cgccgatgtc cgcggaggac 48780
   gggttgcggc tgttcgagat ggcgtttcat gttccgggcg aatcgcttct ggtcccggtc 48840
   cacatcgacc tgaacgccct gcgcgccgat gcggccgacg gcggtgtgcc tgcgttgttg 48900
   cacgacctgg tgcccgcgcc cgtgcggcgg agcgcggtca acgagtcgga ggatgtcacc 48960
   ggtctggtcg gtcggctgcg gaggcttccg gacctggatc aggaaaccct gctgttgggt 49020
   ttggtgcggg agcatgtttc ggctgtgctg gggtattcgg gtgcggtcga ggttggggtc 49080
   gagcgtgctt tccgggattt gggttttgat tcgttgtccg gtgtggagtt gcggaaccgg 49140
   cttggcgggg tgctgggcgt tcggttgccg gctactgcgg tgttcgacta tccgacaccg 49200
   cgggccttgg ttcggttctt gcgcgacaaa ctgattggtg gcgtggaggc acgcaattcg 49260
   gcaccggcgg ttgtggaggc ggccagtggt gacgacccgg ttgtgatcgt ggggatgggg 49320
   tgtcgttttc cgggtggggt gtcctcgccg gaggagcttt ggcgtttggt ggccgggggc 49380
   ttggatgcgg tggcggagtt ccccgacgat cgtggttggg atcaggcggg gttgttcgat 49440
   ccggatcccg atcgtctcgg gacttcgtat gtgtgtgagg gcggcttcct gcgagatgcg 49500
   gcggagttcg atgccggttt cttcgggatt tccccgcgtg aggcgttggc gatggatccg 49560
   cagcagcggt tgttgctgga gatcgcttgg gagaccttgg agcgggcggg gattgatccg 49620
   ctttcgttgc gagggagtcg gaccggcgtg ttcgcggggc tgatgcacca cgactacggc 49680
   gcgcggttcg tcaccagggc gccggagggt ttcgagggtt atctaggtaa tggcagcgcg 49740
   ggcggcgtct tttcgggtcg ggtcgcgtat tcgtttggtt tcgagggtcc tgcggtgacg 49800
   gtggatacgg cgtgttcgtc gtcgttggtg tccatgcacc tggcgggtca agcactgcgg 49860
   tctggtgagt gtgatctggc tcttgcgggt ggtgtgacgg tgatggccac gccggggatg 49920
   ttcgtggagt tttcgcgcca gaggggtttg gcggcggacg ggcggtgtaa gtcgtttgcg 49980
   gctgctgcgg atggcaccgg ctggggcgaa ggcgcgggcc tggtgttgtt ggagcggctg 50040
   tcggatgccc ggcgcaacgg gcacgcggtt ctggcggtcg tgcggggtag cgcggtgaat 50100
   caggatggtg cgtcgaatgg tttgacggcg ccgaatggtc cgtcgcagca gcgggtgatc 50160
   acgcaggcgt tggcgagtgc gggtctgtcg gtgtctgatg tggacgctgt ggaggcgcat 50220
   gggactggga ccaggcttgg tgatccgatt gaggcgcagg cactgattgc cacttacggg 50280
   caggagcggg atagggatcg gccgttgtgg ttggggtcgg tgaagtcgaa tattggtcat 50340
   acgcaggcgg cagcgggtgt tgctggtgtg atcaagatgg tgatggcgat gcggcacgag 50400
   cagctgcccg ccacgttgca tgtggatgaa cctacgccgg aagtggattg gtcggcgggg 50460
   gaggtccagc tccttacgga gaacacgccc tggcccgaca gcggccatcc tcgtcgggcg 50520
   ggagtgtcgt cgttcggcat cagcggcacc aacgcacatg tcatcctcga acaagcctcg 50580
   aatacaccag acgagattgc gcagagcaac ggtcccgaat cggaatctac cgtggacatc 50640
   ccagcggtcc cgttgatcgt gtcgggcaga acaccggaag cgctcagcgc tcaggcgagc 50700
   gcattgatgt cctatttgga taatcgtccc gatatttcat cccttgatgc cgcgttttcg 50760
   ttggcttctt cccgggccgc gttggaggag cgggcggtcg tgctgggagc ggaccgtgaa 50820
   gcgctgttgt ctgggttgga agcgctggct gccggtcgcg acgcttctgg ggtggtgtcg 50880
   ggatccctga tctctggcgg ggttgggttt gtgttttccg gtcagggtgg tcagtggctg 50940
   gggatgggaa gagggctcta ctcggcgttt ccggtgttcg ctgacgcgtt tgacgaagct 51000
   tgtgccggac tggatgcgca tctggggcag caggtggggg ttcgggatgt ggtgttcggt 51060
   tccgacgggt ccttgctgga tcggacgttg tgggcgcagt cgggtttgtt cgcgttgcag 51120
   gttggtttgc tgaggctgct gggctcgtgg ggtgttcggc ctggtgtggt gatggggcat 51180
   tcggtgggtg agtttgcggc ggcgtttgcg gcgggtgtgt tgtcgttgcc ggatgcggct 51240
   cggttggtgg cgggtcgtgc ccggttgatg caggcgttgc cggatggcgg tgccatgttg 51300
   gcggtggctg ctggcgagga gcagctgcgg ccgttgttgg ccgctcgggg tgaaggggtg 51360
   gggatcgccg cggtcaacgc ttctgagtcg gtggtgctct ccggcgatcg ggaggtgctt 51420
   gaggacattg ccggcgggct ggatgggcaa ggggttcggt ggcggtggtt gcgggtttcg 51480
   catgcgtttc attcgtatcg gatggacccg atgctgcagg agttcacaga tatcgcaggc 51540
   agcgtggact accggcgttg cgacctgccg gtcgtgtcga cgttgacggg tgagctcgac 51600
   accgctggca tgctggctac accagggtat tgggtgcgtc aggtgcgtga gcccgtccgc 51660
   ttcgccgacg gggttcgggc gctcgcgcag cagggggtcg gcacgatctt cgagcttggc 51720
   cctgatgcga ttctgtcggc tctgattcct gattgtcatt cctggggtga tcagactgtg 51780
   ccgattccgt tgctgcgcaa ggaccgcgct gaacccgaaa ctgtggtcgc cgcggtggcg 51840
   cgggcgcaca cgcgtggtgt tcaggtcgat tggtcggcgt ttttcgctgg taccggggct 51900
   gggcgggtcg agttgccgac gtatgccttc cagcggcagc ggtattggct ggagtcatcg 51960
   gtttccggtg atgtgacagg tatcggtctg gctggggcgg agcatccgtt gctgggggcc 52020
   gtggttgtgt tggccgacgg tgatgggatg gtgttgaccg gtcggttgtc ggtggggacg 52080
   catcggtggc tggccgagca tcgtgtgctg ggggaggtcg tggttcccgg cacggctatc 52140
   ctggagatgg tcttgcatgc gggggcgcgg gttggttgtg gccgggtgga ggagctcacc 52200
   ctggaagcac cgctggtggt gcccgaacgc gatgccatcg aaatccagct gctggtgaac 52260
   gcgcccgacg acaagggtcg gcggtccgtg tcgctgcatt cccgcccggc cggtgggtct 52320
   gggggtgggg gttggacgcg gcacgccacg ggcgaactcg tcgtcgccgg cacgggtggt 52380
   ggggcggtta ctggttggtc gactgagggt gccgagccgg ttgctctcgg tgagttttat 52440
   gtcgttcagg cggggaacgg gttcgagtat gggccgttgt tccaggggct tcgggcggcg 52500
   tggcgtcgtg gtggcgaggt tctcgcggag gtcgccctgc cggcagcggc tggtgcgatg 52560
   gcggggttct tgatcaatcc ggcgttgctg gatgccgcct tgcaggcgtc cgcgctgggt 52620
   gaccgtccgg cggagggtgg tgcgtggctg ccgttctctt ttaccggggt agaactttcc 52680
   ggtcagggtg ggacgatcag cagggcacgg gtggagtcta cgcgacccga tgcggtgtcg 52740
   gtggctgtga tggatgaggg tgggcggttg ctcgcctcga tcgattctct ccggttgcgg 52800
   ccggtgtcgt cggtgcggtt ggcgaatcgg gacgttgtcg gtgacgcgct gttcgaggtg 52860
   acttgggagc cggtggcgac gcggtcgacg gtatcgggtc gctgggcgtt gcttggtgat 52920
   gctgtcggcg gcatggccgg tctcattggg ctcgcaccag gttccgtcga tcgttgtgcg 52980
   ggtctggctg agctcgcggg gaaccttgat tccggtgcgc tggttgctga tgtcgtggtt 53040
   tattgcgccg gtgaacaggc ggatcccgac gccggcgtgg cggcactcgc ggagacccgg 53100
   gagatgctgg ccctggtcca gtcgtggttg gccgaggagc ggttggccgg gtcacgtctg 53160
   gtggtggtga cgtgtggcgc ggtgacgacg gctgcgggtg acggcgcatc aaagctggcg 53220
   catgcgccgt tgtgggggtt gttgcgttca gcgcagtcgg agaacccggg ccggtttgtg 53280
   ctggtcgatg tggacggtac cgccgagtcg tggcgcgcgt tgccgagtgc ggtggggtcg 53340
   atgcaaccgc agttggccgt gcgtaagggt gtggtgacag tgccgcgtgt ggcgtcggtt 53400
   ccggggccgg tcgaggtgcc cgcggtggtg gccggtcccg accggacggt gctgatttcc 53460
   ggtggcacgg gtctgttggg tggcgtggtg gcacgccacc tggtggccga gcgcggtgtt 53520
   cgtcgagtgg tgttgacggg ccgtcgtggc tgggatgctc ccggaatcac cgagttggtg 53580
   ggtgagctgg agggtttcgg tgcggtggtc gatgtggtgg cgtgcgacgt tgcggatcgt 53640
   gctggtctgg aggggttgct ggcggcggtc ccggcggagt ttccgctgtg tggtgtggtg 53700
   catgccgcgg gtgtgctggc tgacggggtg atcgagtcgt tgacaccgga ggacgtgggg 53760
   gcggtgttcg gtccgaaggc ggcgggggcg tggaacctgc acgagctgac tcgggatatg 53820
   gacttgtcgt ttttcgcgtt gttctcctcg ctgtccgggg tgaccggcgc cgcgggtcag 53880
   ggtaattatg cggcggcgaa cacgttcctg gacgcattgg cgcattaccg gcgggcgcag 53940
   ggattgcctg cggtgtcgtt ggcgtggggc ttgtgggagc agtcgagcgg gatgaccggg 54000
   cggctcagtg atgtcgaccg gagcaggatc gcccgctcca gtccaccgtt gtccaccaag 54060
   gatggtttgc ggctgttcga tgccgggctg gcgttggatc gggcagcggt ggttccggcg 54120
   aggttggaca gggccttcct ggccgagcag gcccggtcgg gaacgctacc cgcgatgctg 54180
   acggcactgg tacctaccat cacctctatc aggcgcagta gtggcaccga cctcgcggac 54240
   gaggacgcct tgcttggggt ggtgcgggag cacgccgcga gggtgctggg gtattcgggt 54300
   gcggccgagg tcggggtcga gcgtgctttc cgggatctgg gctttgattc gttgtctggt 54360
   gtggagttgc gtaatcggct ggccggggtg ctgggagccc ggctgccggc aaccgccgta 54420
   ttcgactacc cgacgccgcg ggcgttggcc cggttcctgc accaggaact ggcaggcgag 54480
   gtcgggacga cgccggcgcc ggtgacgacc acgaccgcga gcgtcgaaga cgatctcgtc 54540
   gcgatagtcg ggatggggtg tcgttatccg ggtggggtgt cctcaccgga ggagctttgg 54600
   cgtttggtgg ccgggggcgt ggatgcggtc gcggacttcc cggacgatcg cggctgggat 54660
   ctggccggat tgttcgatcc agatcccgat cgtttcggga cttcgtatgt gcgtgagggc 54720
   gggttcctgc gggacgcggc ggagttcgat gccgcgtttt tcgggatttc tccgcgtgag 54780
   gcactggcga tggacccgca gcaacggttg ctgctggagc tgtcctggga ggccgttgaa 54840
   cgcgctggga tcgatccggg gtcgctgcgc gggagccgga cgggtgtgtt cgcggggctg 54900
   atgtatcacg actacgccgg acggttcgcg gccggagtgc cggagggctt cgaaggctat 54960
   ctcggtaatg gcagcgcggg cagtgtggcc tcgggccggg tcgcgtattc gttcggtttc 55020
   gagggtcctg cggtgacggt ggacacggcg tgttcgtcat cgctggtggc gttgcacctg 55080
   gcaggtcaat cactgcgttc cggtgagtgt gatctcgccc ttgccggtgg cgtgacggtg 55140
   atggccaccc cggcgacgtt tgtggagttc tcccgtcagc ggggtctggc accggatggg 55200
   cgctgcaagt cgttcgcgga ggccgcggac gggaccggct ggggcgaggg tgctggccta 55260
   gtgctgttgg agaggttgtc ggatgcccgt cgtaatgggc atcgggtgtt ggcggtggtt 55320
   cgtgggtcgg cggtgaatca ggacggcgcg tcaaacggac tgaccgcgcc gaatggtccc 55380
   tcgcagcaaa gggtgatcac ccaagcactc acgagtgcgg ggttgtccgt gtccgatgtg 55440
   gatgctgtgg aggcgcacgg gaccgggacc aggcttggtg atccgatcga ggcacaggca 55500
   ttgatcgcca cctatggccg tgatcgtgat cctgaccggc cgttgtggtt ggggtcgatg 55560
   aagtccaaca tcggtcacac acaggcagcg gcgggtgttg ccggtgtgat caagatggtg 55620
   atggcgatgc gccacgggga gctgccgcgc acattgcacg tcggcgagcc cacgtcggag 55680
   gtggattggt cggcaggttc ggtccagctc ctcacggaga acacgccctg gcccgacagc 55740
   ggccatcctc gtcgggcggg agtgtcgtcg ttcgggatca gcggcaccaa cgcacacgtc 55800
   atcctcgaac agtctccgac agcgtcaagt gagttcgtgg agcacagcgg acctgattcg 55860
   gaatctgctg tgaatgtccc tgtggttccg tgggtggtgt cgggcaaaac acccgaagcg 55920
   ctcagtgctc aggcggacac cttggtgtcc tatctggacg atcgatctga tgtctcctcg 55980
   cgggatgttg ggtattcgct ggcgatgacg cgttcggcgc tggatgagcg ggcggtggtg 56040
   ctggggtcgg accgtgaaac gttgttgtcc gggttgaaag cactggctgc cggtcatgag 56100
   gccactgggg tggttacggg atctgtgggt tctggcggcc ggcccggttt tgtgttcgcc 56160
   ggtcagggtg gtcagtggtt ggggatgggc cgggggcttt accgggcgtt tccggtgttc 56220
   gctgatgcct ttgacgaagc ttgtgccgga ctggatgcgc atctggggca ggaagtgggg 56280
   gttcgggatg tggtgttcgg ttccgacgcg cagttgctcg atcggacgtt gtgggcgcag 56340
   tcgggtttgt tcgcgttgca ggttggtttg ctgaagttgt tgggttcgtg gggtgttcgg 56400
   cctgttgtag tgctgggcca ttcggtcggg gagctagcag cggcgttcgc cgccggtgtg 56460
   ctgtcgatgg cggaggcggc tcggttggtg gccggtcgtg cccggttgat gcaggcgttg 56520
   ccgtctggcg gtgccatgtt ggcggtggcc gcgaccgagg accgaatcag cccgctgctg 56580
   gatggggtgc gggatcgtgt tggtgtcgca gcggttaatg ctccggggtc ggcggtgctt 56640
   tccggtcacc gggatgtgct tgaggacgtt gttggccggt tggatgggct gggtgttcgg 56700
   tggcgatggt tgcgggtttc gcatgcgttc cattcgtatc ggatggatcc gatgctggat 56760
   gagttcgccg acatcgcacg gagcgtggat taccggtctc cagggctgcc gattgtctcg 56820
   acgctgaccg gaaacctcga tgacgtgggc gtgatggcta cgccggagta ttgggtgcgt 56880
   caggtgcgag agcccgttcg cttcgccgac ggtgtccagg cgcttgtgaa ccagggcgtc 56940
   gacacgattg tggaactcgg tccggacggg gtgttgtcga gcttggttca tgagtgtgtg 57000
   tcggagtccg ggcgggtgac ggggattccg ttggtgcgga aggaccgtga tgaggtccca 57060
   acggtgctgg ccgctttggc gcagatccac actcgtggtg gcgcggtgga ctgggggtcg 57120
   tttttcgctg gtacgggggc aaagcaggtc gaactgccca cgtatgcctt tcagcgacga 57180
   cggtactggc tggagccatc ggattccggc gatgtgacag gtgctggcct taccggggcg 57240
   gagcatccgc tgttgggggc cgtggtgccg gtcgcgggtg cggatgaggt gctgctgacc 57300
   ggcaggctgt cggtggggac gcatccgtgg ctggccgacc atcgcgtgct gggcgaagtc 57360
   gtcgtccccg gcaccgcgtt gctggagatg gcgtggcggg ccggtagcca ggtcggttgt 57420
   gaacgtgtgg aggagctcac cttggaagca ccgctggttc tgccggagcg gggtgctgcg 57480
   gcggtgcagt tggcggtggg ggctccggac gaggccggcc ggcgcagttt gcagctctat 57540
   tcccgaggcg ctgacgaaga cggcgactgg cggcggattg cctccgggct gttggcccag 57600
   gccagtgtgg tgccgccagc ggattcgact gcatggccgc cggacggtgc tgtgcaggtc 57660
   gatctggcgg agttctacga gcgcctcgcc gagcgcggct tgacttatgg cccggtgttc 57720
   caagggctcc gcgccgcatg gcggtacggc gacgatatct tcgccgagct tgccgtgtca 57780
   ccagacgccg ctggtttcgg catccacccg gcgctgctgg acgctgcact gcacgcgatg 57840
   gcgcttggtg cttcgcccga ctcggaagct cgtctaccgt tttcctggag tggcgcccag 57900
   ttgtaccgcg ctggaggagc agcgcttcgg gtacggctct cgccgctggg caccggtgca 57960
   gtctcattga cgctgatgga tgccgcaggg ggacaagtcg ctgcggtgga atcgctttcg 58020
   acgcgaccgg tctccgccga ccagatcggt gccggtcgcg gcgatcacga gcggctgctg 58080
   cacgtcgagt gggtaaggcc ggctgaatcg gcggggatgt ccctgacctc ctgcgcggtg 58140
   gtcggtttgg acgaaccgga gtggcacgct gccctgaagg ccactggtgt ccaggtcgag 58200
   tcccatgcgg atctggcttc gttggccacc gaggttgcca agcggggatc ggctcctggt 58260
   gcggtgatcg tcccgtgccc gcgaccccag gcgatggagg agctgccgac cgccgcgcga 58320
   agggcgacgc aacaggcgat ggcgttgctg caggaatggc ttgccgatga ccggttcgtc 58380
   agtacgcgcc tgatcctgct gacgcatcgg gcggtcgccg cagttgctgg agaagacgtg 58440
   ttcgacctgg tacacgcgcc gctgtggggc ttggtccgca gcgcgcaggc ggagcacccg 58500
   gaccgattcg ccttgatcga tgtggacgag gcggaagcat cgcgggcagc actcgccgaa 58560
   gcgctgactg caggagaagc gcagctcgcg gtgcggtcgg gagttgtgct ggtgccccgc 58620
   ctcggccagg tgaaggcgag cggaggtgaa gcgttcaggt gggatgaagg caccgtgttg 58680
   gttaccggcg gaaccggcgg gctaggggcc ctgctcgcac gccatctggt cagcgcacac 58740
   ggtgtgcggc acctgttgct cgcaagtcgt cgcggtctgg cggcgccagg agcggatgag 58800
   ctggtggccg agctggagca gtccggcgcc gatgtcgcgg tcgtcgcgtg cgacgcggca 58860
   gatcgggact cgcttgcgcg gctggtggcg tcggtgcccg cggaaaaccc gttgagggcg 58920
   gtggtgcacg cggccggtgt gctggatgac ggtgtgctga tgtcgatgtc gccggagcgc 58980
   ttggacgcgg tgttgcggtc caaagtggat gccgcgtggt acctgcacga gctgactcgg 59040
   gaactcggtc tgtcggcgtt cgtgttgttc tcctcggtcg cgggcctgct cggtggtgcg 59100
   gggcagagta attacgctgc cggcaacgcg ttcctggatg ccttggcgca ttgccggcag 59160
   gctcaggggc tgcccgcgct gtcgctggcc tccgggctgt gggcgagtat cgatggaatg 59220
   gcgggtgacc tcgctgcggc ggacgtggag cggctgtcgc gggcaggcat tgccccgctt 59280
   tcggcaccgg gagggctggc cctgttcgac gctgccattc gctcggacga accgttgctg 59340
   gcgccggtgc gattggatgt cgaagcactg cgtgtgcagg cccgatccgc ggagacccgg 59400
   attccggaaa tgctgcatgg catggcaatg gggccaagcc gccgcacttc gttcagctcc 59460
   agggttgagc cgttgcaaga acggttggcc ggtttgtcag aggacgaacg tcggcagcaa 59520
   gtgctccagc gcgtccgcgc cgatatcgcg gtggtactgg ggcacggcaa gtcgaacgac 59580
   gtggacaccg agaagccctt ggccgagctg ggtttcgact cgctgacggc catcgaactc 59640
   cgcaaccgcc tcgctaccgc caccggactg cggctacccg caacgctggc cttcgaccac 59700
   ggcaccgcgg cagcactcgc ctggcacgtg tgcgcgcagc tgggtaccgc gaccgtgccg 59760
   gcaccgaggc gaactgacga caacgactcc gcggagcccg tgaggtcgct cttccaacag 59820
   gcgtatgcgg ctggtcggat acttgacggg atggatttgg tgaaggtcgc tgcccagttg 59880
   cgaccggtgt tcggttcgcc tggcgagctg gaatccctgc cgaaacctgt ccagctttcc 59940
   cgtggcccca aagagcctgc cttggtgtgc atgccggcgc tgatcgggat gccgcccgcg 60000
   cagcagtacg cgcggatcgc cgccggcttc cgcgatgtgc gggacgtttc ggtggtcccg 60060
   atgcctggat tcgttgcggg agaaccgctg ccgtccgcca tcgaagtggc ggttcggacg 60120
   caggcggagg cggtgctgca ggagttcgcc ggtgactcgt tcgtgctggt cgggcattcc 60180
   tctgggggct ggctggcgca tgaggtagcc ggtgtgctgg agcgtcgcgg ggtcctcccg 60240
   gccggggtcg tactgctgga cacctacatc ccgggtgaga tcacgccgag gttctccgcg 60300
   gcgatggccc accggacgta tgagaagctc gcgaccttca cggacatgca ggacatcgct 60360
   atcaccgcga tgggcgggta cttccggatg ttcaccgagt ggaccccaac accgatcggt 60420
   actccgacgc tgttcgtgcg gaccgaagac tgcgtcgcag accctgaagg gcggccgtgg 60480
   accgatgact cctggcggcc ggggtggact ctcgcggatg ccacggtcca ggtgccgggc 60540
   gaccacttct cgatgatgga cgagcactcc gggtccaccg cacaggcagt cgcgagttgg 60600
   cttgagaaac tcagccagcg caccgctcgg caacgttgac gtacaccgtt cagggtgtcg 60660
   gttccgtgtc catgttggct tgcgggagca ggagcaattc tgaagcgagg gatgtagcgt 60720
   aggagcagca tggaacgggc tgtgaacgga agcgttttgc cctgcttttt tcggtttcgc 60780
   aggtcactgt ctcttgtgga cttgactggg ggtcaagggg tcgcaggttc aaatcctgtc 60840
   agcccgacgt tggcggaagc ccgctgaccg gcacgtaagt gcaggtcagc gggcttttta 60900
   tgctgttgtg gatcttgggt gatcgtcccg aagtgtgccg tctcgaattt cggcgatttg 60960
   tgcgaggatt tcgggagttg ttttctggaa gcggaatcgc gaggcggaaa cgctgctgat 61020
   cgtgctgacg ggtggccgat ccgcatccgg aacaaggaga tcgactggtc ggggacaacg 61080
   gcgctgccgg gaggcctcga agactggctc accgaggcgc cgaactatgt ctacctgcgg 61140
   cgggccaagg cgcggccgac cgtcagggac aaggcattcc ggagcaaggt agcggatctc 61200
   gtctccggtg tgaccgggaa ggatcccgag cggattgacg gccagtaccg gcgccagcgg 61260
   cgccagcggg gggcctggcc gcggccgcgg tgcttcggcg ttgctggtgc tcgtcgtcat 61320
   ggcgactgcg ctcattgtcc ggcaacgcga gcagactgac cagcaacggc gcgtcgccgt 61380
   cggccgggag ctgatcaccg cagccgagga cctgcgggac aatgatccga ggctctcgtt 61440
   gctgtccagc ctcgggagac ctgagctcgc cccaccccgg aagcccgtgc cgggctcgtg 61500
   aataccctga tgcggacccg tttcgcaggt accccggcga gtttcaacgc gtacgaactc 61560
   aacctcgtca cggcggccac cggcaaccgg gccgccacca gggcgcggag ccgctcggtc 61620
   gagaccagcc cagatttcat ccacgaggtg gtcctgtggg gacaccggcg gtggtggggc 61680
   gtggcgacgg ctcggggcgc tgcccgagtt cgtcggtgac ggcatctcgc tggcgctgag 61740
   cgcggacggg aacaccctgg ccatcgggga caccgacgaa ggggtggtgc tatgggacat 61800
   caccgacccg ggaaatcccc gcaggctggc cagcgcaccg ggggaggtac gtgtcctcgc 61860
   tcgtgttcgg ccgaaacggg gaggcgcttg ccgtctcggg catcgacggg gtggcgctgt 61920
   gggacatccg cggtgtacgg gaacgggaaa ccttggagcg ccgcgccacc ttgcccgggt 61980
   tggcgcaggc gggcggggtg ctcctcagcc cagatggcct gcagctcgcc accacccggg 62040
   acacgcgccg cgagaccgct ccggacgtta acgaccacca gacgaccctg tgggatgtct 62100
   ccgatctcac gcggcctacc cggccagcgc cgatcagggg gcacttctcc gcgaccctgt 62160
   tcgacgcggc gttcagcccg gacgggcaca cgatcgccac cgccggcgta cacggcgaca 62220
   tcgtgctggt cgacattacc gacccggcca atcccaagga gctaaccgtc ctctagggac 62280
   actccggcaa gtggatgacc tcggtggcgt tcagcccgga cgggggaaag ttggtcacca 62340
   gcggcgaaga cgacaccgct gtcctgtggt acctcggcga ccggcagcac ccacagcagc 62400
   tggcgacgct ggacggccac gccggcaggg tgtccgcagc ggcgttcagc tcgaacgggg 62460
   cggcggtctt caccgcggat tcctccgcaa ccggcggtcc cgcggcgcgg tgcgccaatg 62520
   gcgggcggcc gaccgtgcgc ggccggtggc gaccggcctc ctgaacggcc acgaactgag 62580
   actgaccgcg gtgtcgacgg ttgacgactc cgcgctcgcc atcgcgagta agccccggtc 62640
   tcctatccct gacccacggt gcaaagtagc caccacgtct tctgcctcat ccccgttgcc 62700
   cgggctcgga ccggtcgtta ggtcgaattt cggcccgacc cggctcgggt tcgtgctgat 62760
   gctgaagttc ttcgagttgg agggccggtt tcctcagttc gtggaggagt tcccgcaggc 62820
   tgcggtcgac tacgtggccg gcgtggtcaa ggtgcccgcg gaggacttgg cgaaatacgg 62880
   yctgtcgtcc cgctcggcga aggggcaccg tacacagatc cgcgagaccc tcgggtacyg 62940
   gcccgcgacc cgcgccgacg aggaacggct gaccgcctgg ctcgccgatg aggtctgccc 63000
   ggtcgagatg gtggaggacc ggctgcgcga ggccctgctt gtgcagtgtc gcagcgacca 63060
   tgtcgagccc ccgggccgcg tcgagcggat cgtggccgca gcacgggcgc gggcggaccg 63120
   cgtcttctgc gcgcagaccg tcgcgcgcct gggcgaggcg tgcgctggcc gcctgctgac 63180
   cctggtggcg gagggcaacg aggagggtac ggcgctgctg gcctcgctga agcgggaccc 63240
   gggcgcggtg gggctggact cgctgctggc ggagatcacg aagctgactg ccgtgcggcg 63300
   gttgggtctg ccggaagggc tgttcgcgga ctgctcggag aagctggtgg ccgcgtgggc 63360
   gggcgcgggc gatcaagatg tatccctcgg acttccggga cgctggcaag gatgtgcgga 63420
   ccatgctgct ggcggcgctg tgcgcgtccc ggcaggcgga gatcaccgat gccctggtgg 63480
   agctgctggt cgctctggtt cacataagat caatgctcgt gccgagcggc gggtggagcg 63540
   gcagctgacg gcggagctga agaaggtacg gggcaaggag ggcatcctct tccagctcgc 63600
   tgatgcgtcg gtcgggcagc ctgaggggac cgtgcgcagg gtgctgtttc cggtggtcgg 63660
   ggagaagacg ctgcgcgacc tggtcgcgga ggcgaacgag aaggcgttca aggccagggt 63720
   ccgtaccacg ctccggtcgt cgtacagctc gtactacccg gcagatgctg ccgtcactgc 63780
   tgcggacgct cggcttcagg tgcaacaaca ccgcctaccg gccggtgatg gacgcgctcg 63840
   tgctgctgga gaagtacgcc gacgtcgacg gcaagacccg cttctacgac gtcggcgacg 63900
   tggtgccgat ggacggccta gtccgcaagg actggcgtga ggcggtcgtc gatgacaagg 63960
   gcaggaccga gcgcatcccc tatgagctgt gcgtgctggt ggccctgcgg gatgcgatcc 64020
   gccgccgcga gatctatgtc gggggcggga cgcggtggcg caacccggag gacgacctgc 64080
   ccggcgactt cgagtcggcc ggcaccgtgc actacgccgc gattcgccag cccgaggacc 64140
   cggggggagt tcgtcgccgg cctgaagcgg cggatgacgc agggcctgga ccggctgtct 64200
   gcggcgctcg cggacggctc ggagggtggg gtgaaggtca ccacccgcaa gggcgagccc 64260
   tggatcaggg tgccgaagct ggagccgctg gacgagccca cctgcctggc ggccctcaag 64320
   gacgaggttg tacggcggtg gggcgtgctc gacctcctgg atgtgctgaa gaacgccgac 64380
   ttcctcaccg gcttcaccga tgagttctcc tcggtcgccg cctatgagcg catcgaccgt 64440
   gccaccctcc agcggcgtct gctgctcgcg ctgttcgcct gggcaccaac atgggcatcc 64500
   gcgcgatcgt ggcgaccggc gagcacggcg agagcgaggc cgcgctgcgg cacgtgcgtc 64560
   ggcacttcat caccgtcgac aacctgcgcg ccgcggtgac gaagctggtg aacgccacct 64620
   tcgccgctcg ggacgcggca tggtgggggc agggcaccgc gtgcgcgtcg gattcgaaga 64680
   agttcgggtc ctggtcctcg aacttcatga ccgagtacca cgcccgctac ggcgcaacgg 64740
   cgtgatgttt tactggcacg tcgagaagaa gaacgtctgc atctattccc agctcaagag 64800
   ctgttcctcg tccgcaggca tcccgcaggc agggttctcg acgagatcct caaccgcgcc 64860
   accgcctggg ccacccgcgc cgcgtgcggc gaggataccg gcgttccgct ggtcgtgctc 64920
   cacaaccagg tcaaggacag gactgaccga cgataacgat actgtggaca cgagcgcggc 64980
   tcctgacacg atccttcggc gtaaccacct gaaagatcaa cagatagctg cgctcacccc 65040
   ttcaccgggg ccaccaagac taaaccggac aaaccggagc ttccgcccaa aagatcaaac 65100
   cgccggggtc ctgcggcatg accaagttcc tttctgagac aaaggacagt gtccattcgt 65160
   gcagagggct tgtttgatct ctgggcggaa ccaagtatag gaattagcta gacgacgaac 65220
   cgattttact gcgggaatgg gatcccgcgt tctgcgcgcg gctcgcgggc gcatattcgg 65280
   aaacgctgca gcggagaatc gagagcgtcc agatttgcgt acaagattcg ccgtaattgt 65340
   ctaagagtgc tggaagcaac agtttccaaa caaaaggtgc acacaacgtg aatacaccca 65400
   tgacggctgg aagcgtgaac gtggatcccc agcttatgat gcggcatgaa cctgcgaccg 65460
   aggagagcgc ccggatcttc cagcggcgtc gacacgccct tgttggcaca gctcttttca 65520
   acagctactt caaacaagca cgactggacg agctcctgcg gtggacggtg accgagttcg 65580
   agtcgctgca tgtgttcctc cctgacacga ccaccgcttt cacgatgcag gcccgcggct 65640
   atccggcggt ggaagcagag cggaagacgc gacgcgaggc acgaaggctg cgcgggaaga 65700
   tactcgggtc gcttgccatg ctcggcgtcg cgtcccccga gcagatgatc atcgacttcg 65760
   cctggctcaa cgagaacgag gagtacctgc gcacgaggga cgaggttgcg gaggtcttcg 65820
   ggtcggatga agacttcagg aaggcctgtc tggccgagtc tgaatcggtc gcgaggagca 65880
   ggcggagaac cgacggcagt ctcacggagg aagagctgaa catggcggca cggtacttgc 65940
   tggccgagat cccgcttttc gtaaacgctc ccgccatact tggcatgccc gagactgtgt 66000
   tctgctacca ccgcatcgaa ccgttcgaac gtgacctgta ccgcggcgcg ttcgcggtga 66060
   aagcagtacc tcggcaaggc tgggtcgtgg tcgagtccgc ttcttcggaa atggtggacg 66120
   ccggtgccgc ggccgggtcc ctgccgcgac cacgtaccgc aggtgtgctg tgaaccgcgc 66180
   cgatacggcc gattgcgaac cccggcggtc cggcgagcaa gtacgccgga agtccagcct 66240
   gtggtaggcg cggcttcggc tggtctgcac ggcgaccaaa ccggctggtc ctcctggaat 66300
   ctcgatcgtc ccgccgaccg gcagcgggac gatcgagggg agcagcaaga cccgtagtgc 66360
   ttcaagaagg gttcgctggc tgtgagctgg ggtttcgtcg ggccgtcagg ctgcttggcg 66420
   gtactcgttg atgagtcctg ccacggcttg tcggcgttcg atccgagctg tcggcagcgg 66480
   tatgacgcgc gcgccacgta gggtgcctgc tggtcgtgtg cttggtgggg ccgatgggtg 66540
   ttgaagtggc aggcgtattc ctggcggatc ttctccgcgt ggcctctgtc gaagatcagc 66600
   actcggttgg tgcactcctc gcgaacggag cgtatgaatc gttgcgcgtg cgggttgcgg 66660
   ttggggctgc gcggcgggat cttcgtgacg gtgatgcctt cgctggcgaa gattgtgtcg 66720
   aaggcggcgg tgaacttcgc gtcgcggtca cggatgaggt gcgtaaatgt ggcggcatgt 66780
   caccgagctg ccacagcagc tgccgggcgt gttgagtggc ccaggcggcg gtggggtggg 66840
   cggtgaccct gcggatattc caggccgccg agtgagtgac gacgcgcttg atcgccgttc 66900
   ttgtccagca cctgactccg tcctgagcct cactcggcga atgctcgcga tccattgagc 66960
   gcgagggttt ggcggagtcg gtcggggttc atgagcccgg cggtgggtcg cccttcgcgt 67020
   ttcgtatcca ggagagtggc ttccacgtcg tcgagggccc gccacgaagc tacggcggct 67080
   gctcgaacac gccgacctca ccgatgcgca gcaacgcgcc catcctcggt gctggcgagg 67140
   cgtggccatc gtgccgctca gagtgttaag ccgccgacat cgctgtcgtt tcggatgact 67200
   ttgatgaacc cagtcagggc cccgttggca tcgcgctgcg ctgtgaccac cacgtgcgcc 67260
   cagtaccggg tgccgttctt acgtacccgc cagccctcat cgacagaaaa gcccgcctcg 67320
   gctgcctgat ccagctcctt ctgcggatac ccggctgcga cacgatccgg cgggtagaag 67380
   accgaaacgt gccgaccgat gatctctctg gccgtgtagc ccttcatgcg ttcggcgtcg 67440
   gtgttccagc tcctgacaac gccgtccgca tccagggcga agatcgcata ccggcggttt 67500
   gctgacgtgg cagtcccctc aatgtcaaga cccactgcac aggcccgccc gtcgccggat 67560
   acgaaggtcg cgataaaccc gaaagtcccg gcggcgtgcg ggacgctcca tgttgagact 67620
   gcacccggga acgcgaccgc ttcaccgggc tttcgtcctg atgtcgcttg tccgtaagcc 67680
   gtcccccacg tcaatcccag cgcctcgcct ttggccgatg ctcaagccga cccgaccacg 67740
   caccgaacac gattacctcg ctctgactcg cccacgcggt gacataatgg acatcaggtg 67800
   attggcggta acgccatgca aagtcgagac acctccgacc agaagcagcc ttggggtagc 67860
   cccgggacgc gcgatagctt caccaatctt gacgcggcac ttcagggcca tccagcgatc 67920
   gagcaggcca agggcatgtt gaagctggcc tacgggatcg atgaccacga cgcactcgaa 67980
   ctgctcgcaa gcatgtcgca cgacacccgt acggacatgc gtgaggtggc ccagacgctg 68040
   gtggagcggg tcacgggcgt agcgaacgcg ccgaccgtga agaccgtgag cgcccgtgtc 68100
   cttgaggagt gggagcgact caaagagcgc taacccagcc ttgcaaaggc gatcaagggg 68160
   catcgtctac ctcggccgct gatcgaacta cgttggtcga cgtgccgcgt ggtttcggtc 68220
   aattgccgtt gttcttcgtt ggtgttggtc ctcgtgcgct ccggatgtgc tccatgcgcg 68280
   tccggggcgt ctgcacagct tcgacctggc cgaaatccat acgatctggt ttcgaacctg 68340
   gcctcccatt caggctccag cgcgttacgg acccgcgcct ggtggcatcg atccatcatc 68400
   gcgagcacag tcgacgatcg tcactggctt cgccaggcgc agctgctcga acaccttctt 68460
   gacgccgcat tccttcgcca caacatctcg caccgtgcca agcagtgccg actgcacggc 68520
   gggaagaatg gcgacactcg cgttcagtgc tggacagtcc ccggccccaa cggcgccgag 68580
   atcctcgacg ccctggggcc ggccgcgacg gtcctccttg atcaccgcac tcgcctgatc 68640
   accgactacc cgtgaaccat catcaccgtc agaccgttgt gggagacggt cgaggatttc 68700
   gcggaggagc tggcggaggc ggcttgagcc caagtccccc tgctcaccct cttcggcgtc 68760
   acccgccggg agcgatcatg accacctggt ggtctccggg ccgtccggtg agggcgtcca 68820
   tcatcgctga gaccggcccg gcgtccaggg ggacgacgtc ggtgatgatc cgctcgaccg 68880
   ggatacggcc gcctccgaga agctccaagg ccgcctcgaa ggcgcctctg ttctgggcga 68940
   agctgcccac gatccggatc tccttggtga tgagggcgac cgagtccagt tctgagggtc 69000
   gctcgttcac tccgagtgcg acgatcgttc cccgtgtccg cacgcgtcgg gccgcgtccg 69060
   tgagcgcggt caccgagccc acgcactcga acaccacgtc tgcgtccggg ccccgctcat 69120
   cgacgtccac ggtggtgatc ccggtcgcgg tcagacgtgc gcgccgtacg gggtgcggtt 69180
   ccaccacccg cacgtcctcg actccccgta gccgcagcac gtgtgccagc aggaagccga 69240
   ccgtgccgcc tccgaggacc gtgacagtat ccgctgccga aattccggag cggtcgaccg 69300
   cgtggaccgc gcaggacagc ggttccgtca gcgccgcgtg ctccagcgaa acaccgtccg 69360
   gcaccgcgta caccgtgtgc tcggggacga cgacggcttc ggcgtagccg ccggggcgtg 69420
   tgcccaggcc gagggaactc agccgccagg gctgtacagc gcacaggtgg ttgtcgccta 69480
   cccggcagtc gtcgcagtcc ccgcagcccg ctttcggcca caccaccacg gcctgcccag 69540
   ccgtcagccg ctccccgccc ggtgcggcga cgtgcccgga gatttcgtgt ccgagaaccg 69600
   cgtcggccgg gacgaggtgc ggcattgccc gcaggtgcag gtcggaaccg cagatggcgc 69660
   agaacgctac gtccacgcgt acccagccgg gctccggctc gcgttcctcg gcctccgcga 69720
   gccgcagccc gcgctcctcg gtgatgacca gtgccctcaa ttttcctcca cagttttctc 69780
   agcggccagg aacgcccgga tccgggcgac caggatgtcc ggctgctcct cggccgggaa 69840
   atgcccgcag gaggcgatct gcgccccctc aagccgggcg gcgtagtccc gccacaggtc 69900
   gagtacgggc agtgtcccca ccagcccttc ggcgccccac gccacgtgca cgggcattgt 69960
   caggcggcgg cccgcagccg cgtcggcctc gtcagcttcc agatcctcat ggcgagcggc 70020
   gcggtagtcg tcgaagctcg cgcgaagcgc cccgggctgt gcgtacgctt ccgcgtagtg 70080
   ccgcaggtcc gcctcagtga acgcctcgcg ccggaatgct ccggcggcca gcatgaaccg 70140
   gacgtactcc ccggcttttc cttcggtgag gaactcgggg aggtcgggga cgaggtggaa 70200
   gagccagtgc cagtacgcgg ccccgacccg tgcgtccatg tgccgccaca tttcccgcgt 70260
   cggtacgacg ctgagcagta tcaggcggct gatctcgtgt ggccggtcca gtccccaccg 70320
   gtgggccacg cgtgcgcccc ggtcgtgccc gaccacaaca gcctgcgtgt atcccagctc 70380
   gtgcatgagt ccgctcatgt ccgcggccat ggtccgcttg tcgtagccgc cccggggccg 70440
   gctggacgcc ccgtatccgc gcagatccgg cgcgaccacc gtatggtccc gcgcgaggac 70500
   gggcagaacg cggcgccagg cgtagctggt ctgcggccat ccgtggagga acaggatcag 70560
   cggaccctct ccagcgcgcc gtacatggaa ccgcagaccg cccaccgtcc gcgtttcttc 70620
   ccgcgcatgc atcgccacgg cggtttacct cccctgcgct gggaggggaa ttcctacggc 70680
   cccctgcgct gggaggggaa ttcctacggc ccgctgcgct gggaggggaa tttctacggc 70740
   ccgcgctggg gacgtgaatt ccgtcaggta gccgtcgtcg accatggggg cggtgacgag 70800
   ttcgggcagg ggcgcgagca actcggccag gaggccgatc ttttccctgt cggccgacac 70860
   aggccggtgc ggttcctgca gccggtgtgc ggcgaaatcg gctaccaggt cgtccatgat 70920
   ctgctgcggc acctgcgggt cggcgtcgtg ctcgctcagg atgaggtcga gcatgcgggc 70980
   gtgaacgagc gtgacggcct cggcatgggt gaacgatccg cccggacggc tgcgccacgc 71040
   ctcggtcagt tccttctcga cccgctcggg cagtgtccca ggcgggcgtg aggcgaaccg 71100
   gtctgaacgc agagcagtgc gcgccacttg gtaggccatc acggagttgt cgcccgcgaa 71160
   ggtcacattg acctcgaagt cggtccgcag ggtgacgatc tggttgtggc agtggaaccc 71220
   ctgcgacccg cacatctcgc ggcacgcggc caggacgtcc aggaccagcc aggagccgca 71280
   ttttccggtc gcggtgagca ggtgcatgtt tttacggccg gggtcggtgt gccagtcgcg 71340
   ttcgacgccc ctgacgacgg cgcgttccag caggcgcagc gcgaggcagc gcagttgaac 71400
   cggatacagg cggtccagga acagcggctc gtccaggagg accctgcggg taggcgtggg 71460
   gcgggtttcg cggtgaccgg cgaaccgcca ggtgaggtga gctgcgagtt cggaggctcg 71520
   tgctccggcg ctgagcggga agatacgttc ctggacgaag gtctcgatgg atttcacgaa 71580
   gcgggcgccg gcatcgggaa gcgtgctgga aaaacgtccg cccgcgtcga tgcgcgagta 71640
   ccgccccatc agcgcctcgc gggggagccg gaccccggtg aagcgggcac cgccgacctg 71700
   gttggcctgg atacctccct tcgggtcaca cgacaggatc gtgatacccg gcaggggagg 71760
   accgttctcc tcgccgcgca gcgggacacg gaaccagtgg tggccgacgt cttcgccgtc 71820
   gatcacaagg cgggccagga ccatgccgac ggtcgcggcg tgtttgatgt ttccgatcca 71880
   gaacttgcag gcggcgtccg tgggggtgtc gagcgtgaaa ctctgctcct cacgattcca 71940
   ggagaccgtc gtgcggatct cccgcaggtt ggtaccgccg ccgatctccg tgcagcagaa 72000
   cgcgtagacc tggtgcatcc gcgtgatttc gtcgtggtac cgggcgacct gctcaggggt 72060
   gccgtggttg aacagcgcgc tccccgcgat gaggtgatcg gtgatggtcg aggtgagcgc 72120
   gaaatcgaag gcgcccgtca ggcccatcat ctcgcacatg gaccggaacg cgtgctcccg 72180
   ggactggccc atccacatgt cgttgtcgac gagcccctcc cggaagatcg ctttcatccg 72240
   gtcgatggtc aggtccatgt actcccgcgg ggacagatcg tcacgcagtc gcggatcgaa 72300
   caactcctgc cgcagcaggt cacgcatcgt ccgccggtag gcggaggcgt cctgttccac 72360
   gtccagggca gtcaaccgct ccgcctccgt ggcatgggct tgggtaacga gatgttgccc 72420
   ataagggact gcagttgcgg ccatgtgatc gaaatcctct cgattcgtgg gcatcgcgca 72480
   ttttcaaagg ggaccgtgcc agctgggcag ggcccatcca gcggccgttc acccacggtg 72540
   cgggaaaggc atacaggccg gaagggcaga tattgtcgac tacgagtatc ggggtgccct 72600
   ccgggagaat tccctgcacc gcccgctcgg cctgtttgta cggaaggaac ttctcgtgcg 72660
   gcgaggccac gcacgggtcc gcctccggtc gcagttgccg gatctcgctc cgcgcttcag 72720
   cgcggccagc gggcagacgg tccaccatgc cgtcgaggac catgtcggcg gcgagggccg 72780
   cgtccagttc gtgattggct tcggtagatg cagcgaggaa gagcgcggac cgggaatgcg 72840
   gtgccggctc cgtcgtcgta ctgaggcatt gcatgagaca acgacgcagc cagaacacct 72900
   gaagatcgcc gcgttcaatt gaggtttgcc cattaatcgg gccggtcgcc gaagtggcgg 72960
   gcacttcccc gcggtaggcg gcgcagaccg catcgatctc ttcggcgagt tccgcggcga 73020
   tacactgtcc tcgcgaatag tgcgcgaagg gatccgtaaa ataggcccct gcgccgggaa 73080
   ccatcagggc ggtgttcatg atcgcctttc atgcgccgca tccaaccaga tatagcgttt 73140
   gtgtctaagg tttctcgccg tccatgctgc gctggttcgc gatctcatgt cgatatgaag 73200
   gcaagcaggt atcgcagtgg gtgtcgcagg tcattgcgcc tggtgtccgt gatggcattt 73260
   gccggtattg ataatgactt ctcgagtatc cctcaagctc atacgatcgg cggatggttt 73320
   gcccgtctac ccatctcgtg ttgcccgtct gttccctggt tcgatgagta ccggcgctgc 73380
   tgctcgaccc ggccaagcgg gacgccggtc gtggactgtg aactcagcga gatcgaagat 73440
   gtgctgcgcg aacaccaccg ggttgatgat gctctggtcc tgcagggtgc gaagatcgaa 73500
   gcgcacctcg tggtgaccga accagtgcag ccagccaact ctgtcgacct ggtgcaccct 73560
   cctcctcggc acgtgcgaac cgtatgttta cgaggcggat ccgcagttga cgcgcgatga 73620
   gagcgatgct ttggtgaatc acctattcgc ggtttccgag agtccgcagc gcgtttgaag 73680
   gtaccggtgg gttccgggcg cggtcgaggt gtgggacaat cgggcgactc aacgcaataa 73740
   cacactggcc gcaccacgca gcagttgtct accgggctgg ccattgctgt tgccacggtg 73800
   gcactgcggc tcggtgaacc gttgggtgag ctggtgttcg gtgcgcgtag cggggctacc 73860
   tacaccgtag ccttcatcct gcttgggctg actgcgctga tcgccactgc cggggcgttg 73920
   agcctgcacc cgaatgccgg caacgccgtg cgcacgctgg gcacgcgggc gagcgggcga 73980
   gcgcgacctc caccacggaa ccgggctgag actgccagca gttggacgtt gcaggccagc 74040
   atgcaacgtc atgacaccaa cgacataagc aggtcacagc tgcagaggcg atcctctcgt 74100
   tagcaacgag agattcgggc aacccccgag ggcactttcg ggtagaccga gagattcgat 74160
   cttcttatcc ttgactcccg ccgcatagct ttccactgcg accgccccga cggtgcggga 74220
   aatcatgtcg atcaacatca acggcggaat ccacagtgga cgttgtttct gccgcgtctc 74280
   ggatgccacg ccgcgggtgg gaacgtgcag gtacgtgccg gagaagagga gaacgaccgt 74340
   ggctgaggta gtggaatcac cctcacccaa ctcgttggaa gacgtacgcc cgctaccgca 74400
   tcctcgatcc acgcgccagg gtcaagtcga acaactcccc gagttgactc cggcgttgtt 74460
   gctggagtta actacggggc tctggagttt caaggcttcg ccgccgctgt cgagctggag 74520
   ctgttcacca agctctccga cctcggctcg gccacagtcg aaacagcgtc cgaggccctc 74580
   ggaccgcccg atcggcccac cgatctgcta ctggccgcgt gtgcgtctcc caactgaggc 74640
   cctgcgggct ggtcgtacat aagaaggggc tcccgcagcc cagggagtga ctgtaactcc 74700
   cgcagggggg ccgccacccg caccgccacg caaaacgagt tccgctaaca ggatctaagc 74760
   gttgtgtcca gccgagagcg aaggaagaga catgccggca atccgattgg cagcgctggg 74820
   tgacagcttc gtcgagggcc gtggtgatcc tttaaaacct cagcaatccc aggaggccga 74880
   cctgaaccgc gaagatcccc ggctgcgccc acaacgtccg gtcgagcacc tgctcatcag 74940
   caccgaacag cacgccccgg acgtccacgc ccaagtccgc gcccaagtgc gcgcccaagt 75000
   gcgcgcccag cgcgtcgcag gcttcctcga aggcgtctgc gaacgccggg aaagccgcgc 75060
   acaacgcctt tcccatcccc agccactgac tgccctgacc ggaaaaaacg aatccgatgc 75120
   ggccaccgga attcgcagcg ccggtgacca cccccggcgc agtccggcca tcggccagcg 75180
   ctgccagcct cgacaacagg gtctgatgaa ctacttgcag ttgacctcga atctct 75236
<210> 2
   <211> 36538
   <212> DNA
   <213> Saccharopolyspora sp. NRRL30141
<400> 2
   tcggtactgc ccctccgatt cctggacaca ccatcagcaa gttcggcgtc ttccggcgcc     60
   tccaccggcg attccggagc gcctgcatat catcgagttg cggcacacct tcagccgacc    120
   ggcttccgcg ccgtcaaaat cgcatagccc atgtcgtcgg cgtatttctc gtaatcgcag    180
   accgcggcgg cccagtcggc gacagccggc ccgtaccttt ccgcgatccc gtgctggtgc    240
   gcagcgagcg cttcggcgaa ctgcggcatg aagtaccggg tccgcgacga cacgtcgtca    300
   caagcgagga tttcgaaacc cgctgcacac agcgattcca gaagttgctc agccaggcag    360
   atccggaggc cggtcggcca catgtcccag gacaccggga tcccgctgcc tatttctcgt    420
   ttgacgacct cggtgactcc gaggatgcca ccgggtttga gcactcgaac gatttcccgg    480
   atggcacggt ccggttccga catctccaac agcgactgga tggcccaggc agcgtcgaac    540
   gcattgtccg ggtacggcag ggacatggcg tcgacgcacg agaagtccac ctggtggctt    600
   agtccgcgtt cgcgggcgca atcaacggcg atggcagctt gcacctggct gaccgtgatg    660
   ccggtgatcc ggatcgcgtt gtcgcgcgcg acgcgcagcg ctggttgtcc ggtgccgcac    720
   cccacatcca gcagtcgatt gccgccatcg agcgcggtcc gttcggcgac aagatcggtg    780
   agccggtcgg cggcctgctg ccaggaagtc cgcccgtcgt tctcccagta gccgtggtgg    840
   atggcgcagg ggccgcccgc gaccgaattc agcaacgggg tgaccaggtc atacatctgc    900
   ccgacctgct gcgatgttgg cacgccacct ggcaacaccg gtatgcctga tccctgcaac    960
   gttcaccttc tcgaaatttt cttcaccgag cagacacaca gagagaaaag aaggcaaact   1020
   agccgtcagt taattcgcgg ttaccgccgc atgcggcggt aacaactaat aaccatccgg   1080
   ccaggaggcg aacagccagc gcaaattttc cccatgaatc cccgcagagc caacggtgat 1140
   cagcgtatag gtctgtccca ccctgatcga gcgaagctca agcatttcac tcagacgggt 1200
   gaaaccaggt gcggcaagct aaagatcctc gatgggggcg tgacactcct gcggcgacac 1260
   actattattg ccgccggaag tccgttggac gtcaacggcc atcgacatac gcagcgcatt 1320
   ttcctactcc tgaccaacag gagtaggggt tgctgcgcca atcgggggaa gagaccgggg 1380
   tccgaagtat gcgcgtactc gtcgttccct tgccctatcc gacgcatctc atggcaatgg 1440
   tgccgctgtg ctgggcgctg cgagcatccg ggcacgaggt tctggtcgcc gcgccaccgg 1500
   agctgcaggc gaccgcgcat ggcgccggtc tcaccacggc cgagatccgc gggaacgaca 1560
   agacccgcga cacgggtagc accacgcggc tgcgctttcc caatccggcg ttcggtcagc 1620
   gcgacaccga gaccggccgg caactgtggg aacagaccgc gtcctatgtc gtgcagagct 1680
   cgctcgatca gctccccgaa taccttcgac tggccgaggc ctggcgaccg tcagtgctgt 1740
   tggtcgacgt ctgcgcgctg atcggccggg tgctcggcgg attgctcgac ctgccggtcg 1800
   tgctgcaccg ctggggagtc gaccccaccg caggcccctt cagcgatcga gcccacgagt 1860
   tgctcgaccc ggtgtgccgc caccacggac tggccggact gccgactcca gagctcatac 1920
   tcgatccctg cccgcctagc ctgcaagcaa gcgacgcgcc gcgaggcgtt ccggtccagt 1980
   acgtgccgta caacgggagc ggcgaactcc cggcctgggg cgcggcgcgc acctcagcac 2040
   ggcgggtctg catctgcatg ggccgcatgg tgctgaacgc caccggaccg gctccgctgc 2100
   tgcgcgcggt agcggctgcc accgggctgc ccggcgtcga ggctgtgatc gccgttcccc 2160
   ctgagcaccg ggcacttctc accgacctac cggacaacgc acggatcgcc gaatcggtcc 2220
   cgctcaacct gttcctgcgt acctgcgagc tggtcatctg cgcgggcggc tcgggaacgg 2280
   cgttcaccgc gacccgactc ggcatcccgc aactcgtgct tccccagtac ttcgaccagt 2340
   tcgactacgc gcgcaacctc accgctgccg gggcgggcat ctgcttgccg gatgagcagg 2400
   cccagtccga ccacgaacag ttcaccggct ccatcgcaac agtgctcggc gacaccggct 2460
   tcgcggctgc cgcaaccaaa ctcagcgacg agatcacggc catgcccaat cccgccgagc 2520
   tggtgcggac gctggagagc tccgcggcca tcggtgcctg acgaactgct cacccgagaa 2580
   cagacggatc cggagaaccg atgccctccc agaacgcgtt gtacctggac ctgctcaaga 2640
   aggtactcac caacacgatc tacggtgatc ggccgcatac gaacgtctgg caggacaaca 2700
   ccgactacag gcaggccgct cgggccaaag gcacggactg gccgactgtc gcgcacacga 2760
   tgatcggtct ggagcggctg gacaacctcc agcactgcgt ggaagccgtg ctcgcagacg 2820
   gtgttcccgg ggatttcgcc gagaccggtg tctggcgggg cggcgcatgc atcttcatgc 2880
   gcgcggttct ccaggcattc ggagataccg gacgtaccgt ctgggtggtg gattctttcc 2940
   agggaatgcc ggaaagctct gcgcaagacc acgagtcgga ccaggctatg gcgctgcacg 3000
   agtacaacga cgtgcttggc gtcccgcttg agaccgtccg gcagaacttc gcccgctacg 3060
   ggctgctcga cgaacaggtc aggttcctcc ctggctggtt ccgggacacc ttgcccaccg 3120
   cccccatcca ggaactcgct gtgctgcgac tcgacggcga cctctacgaa tccacaatgg 3180
   actctttgcg gaacctgtac ccgaagctct cgccgggcgg attcgtcatc atcgacgact 3240
   acgtcctgcc gtcctgccag gacgcggtga aggggttccg cgcggaactc gggatcacgg 3300
   aacccatcca cgacatcgac ggcacgggcg cctactggcg ccgcagctgg tgaacggctc 3360
   agctgtcctc gacgccgagc gcttgccggg gcacgaaccc cggcgcggca ggctcagcgt 3420
   tgagcccttt ttccacgaat accaggttgt ggtagaagtg cagggccgcc acgttccgtt 3480
   ccgtgtagca gggctcggtc ccgcgccgcg attcgcgctc ctgataatgc aggccgtcga 3540
   tcagttcttt gagcatgtcg atcgaggtgc gctgggccgc gggttccgta tcgcggccgc 3600
   cgtagccggg ccagtacgac gtctggagat cttcgatgac gtacaaacca cccgggcgga 3660
   cgtgcggaaa cagggcatgg aaggacttct tgacgtggtc gttgacatgg ctgccgtcgt 3720
   cgatgacgat gtcgaacggg ccgatcttcc ccgccatgtc tgccaggaat tccgcatcgc 3780
   tctggtcacc tcgcagcttt cgcactcggt gtccttcgtt cccggctttc tcgaaaatgt 3840
   ccaggccgta cacgagacct cgccggaagt accgctgcca catgcgcagc gaagcgccac 3900
   cgagttcggg tgcgtggtaa ccaccgattc ctatttccag cacgcgcacc gggacatcct 3960
   ggaatcggga gaagtggtgc tcgtagtgtt cggtgtacca gtgcaggtcc gcccatttgt 4020
   cggatccgta gcggaccgcc agctcaccga ggtccgagca cctggtggcg gctgcggcca 4080
   gcaccgcgtg caccgcggtg gacaccttgt tccggaacgc cagcagtcgc tgcgcgccgg 4140
   ccaggccctg gtcgggggag aactgcgtca tgctgtccga ccaacggact tcacggctgt 4200
   tgtgccgcct gccgtcaacc gggccgaaga gcccttcgag cagatccacc gcgtcgaacc 4260
   ggagaacagc aggtgcttcg gcgaccgccg cgagccgcag gcctgcgtga tcgagctcaa 4320
   cggttctacg gaccagctga gcgccagaag tgatctccag gccgattcgc accgactcgt 4380
   ccaacgacag cggatcgcaa cgcacgacga gttcgtcgac gatggcgtcg gccaccgcct 4440
   ccagtccagc cacctgcact gcttcctgga gcctctccgt gcccgcaccc gccgcgagca 4500
   gcaagtgctc caccaccgac cagggggcaa ctgcgatctc acccatggaa agtcatcacc 4560
   ttttcggttc ctgcgcatag gacgccatgc gcaccgcgat accgatccac aaagtagagc 4620
   cggcggaggc gaccagcttt cacgtatgag ccgaaacaca cagataatca cccggacgcg 4680
   tggatgatct cggccgaggg cgaacaaagt ggaccagtca gcaaaggagg ggcggtgccc 4740
   gatttccatg acccagcaac catgaatcgc cgaaccccag gaacagagat caccgtcgag 4800
   cccggcgatc ctcgttatcc ggacctcgtc gtcgggcaca acccccgttt caccggaaaa 4860
   cccgaacgca tccacatcgc cggctccacc gaagacgtcg tgcacgctgt cgccgaagcc 4920
   gtgcgcaccg gcaggcgggt cggggtgcgc agcggcgggc actgcttcga gaatctcgtt 4980
   gcggacccgg cgatccgggt gctcgtcgac ctctccgagc tcaaccgcgt gtacttcgac 5040
   agcacgcgcg gggcattcgc gatcgaggcg ggcgccgcgc tcgggcaggt ataccgaacc 5100
   ctgttcaaga actggggcgt gacgatcccg accggcgcat gtcccggggt gggcgcaggc 5160
   gggcacatcc ccggcggggg atacggcccg ctgtcgcgcc gattcggttc ggtcgtcgac 5220
   taccttcaag gcgtcgaggt cgtcgtggtc gaccgggccg gtgaagtgca cattgtcgag 5280
   gtcgaccgga attccattgg tgccggtcac gacttgtggt gggcgcacac cggtggtggt 5340
   ggcggcaact tcggggtcgt caccaggttc tggctccgag cgccggacgt ggtcagcacc 5400
   gacccctcgg agctcctgcc acggccgccc gcgacggtgc tgctccgatc gttccactgg 5460
   ccgtggtgcg aactgacaga gcagtcattc gccctcctgc tacggaactt cggcacttgg 5520
   tacgagcagc acagcgcgcc ggaatccacg caactcgggt tgttcagcac gctcgtctgc 5580
   gcacaccgcc aagccggcta cgtcacgctg aacatccatc tggacggcac ggatccgaac 5640
   gcggaacgca ccttggccga acacctatcg gcgatcaacg accaggtcgg cgtgactcca 5700
   gccgaagggc tgcgggaaac cctgccgtgg ttgcgatcga cccaggtgtc cggatcgctc 5760
   gccgaaggcg gcgagccgag cgggcagcgg accaaggtca aggccgccta cttgcgcacc 5820
   gggctgtccg aagcgcaact agccacggtt taccggcggc tgaccgactc cggatacgac 5880
   aaccccgcag cagcgctgtt gctgctcggt tacggcggta gggcgaatgc cgtggcgccg 5940
   tcggccacag cgctcgctca gcgcgactcg gttctcaaag cgctgttcgt cacgaactgg 6000
   tcggagcccg ccgaggacga gcggcatctg acctggattc gtggtttcta ccgcgagatg 6060
   tacgccgaaa ccggcggagt tccggtgcca ggtacccgtg tcgacggctc ctacatcaac 6120
   tacccggaca ccgacctggc cgatccattg tggaacacct ccggagttgc ctggcacgac 6180
   ctgtactaca aggacaacta cccgcggctg caacgggcca aagcgcggtg ggacccacag 6240
   aacatcttcc agcacggcct gtcgatcaaa ccgccggaac ggctttcacc cggtcagcca 6300
   tgaggagtcc gtcacgatgt ccgcaacgca cgagatcgaa accgtggaac gcatcatcct 6360
   cgccgccgga tccagtgcgg cgagtctggc cgaactgacc accgaactcg gactggccag 6420
   gatcgcaccc gtgctgatcg aggagatcct cttccgcgcg gaaccggccc ccgacatcga 6480
   accgaccgag gtcgcggtcc agatcaccca cggggtcgag accgttgact tcgtcctgaa 6540
   gctacagtcc ggtgagctca tcaaggccga gcaacgaccg gtcggagacg tcccgctgcg 6600
   gatcggttac gagctcaccg atctcatcgc cgagttgttc ggcccaggag ctccgagggc 6660
   cgtcggtgcc aggagcacca acttcctccg aaccaccaca tccggttcga tacccggccc 6720
   gtccgaactg tccgatggct tccaagccat ctccgcagtg gtcgccggct gcgggcaccg 6780
   acgtcccgac ctcgaccagc tcgcctccca ctaccgcacg gacaagtggg gcggtctgca 6840
   ctggttcacc ccgctgtacg agcgacatct cggcgagttt cgtgatcgcc cggtgcgcat 6900
   cctggagatc ggtgtcggtg gctacaactt cgacggtggc ggcggcgagt ccctgaaaat 6960
   gtggaagcgc tacttccacc gcggcctcgt gttcgggatg gacgtcttcg acaagtcctt 7020
   cctcgaccag cagcggctat acaccgtccg cgccgaccag agcaagcccg aggagttggc 7080
   cgccgtcgac gacgagtacg gaccgttcga catcatcatc gacgacggca gccacatcaa 7140
   cggacatgtg cgcacgtccc tggaaacgct gtttccccgg ttgcgcagcg gtggcgtata 7200
   cgtgatcgag gatctgtgga cgacctatgc tcccggattc ggcgggcagg cgcagtcccc 7260
   ggccgcgccc ggcaccacgg tcagcctgct caagaacctg ctggaaggcg ttcaacacga 7320
   ggagcagccg catgcgggct cgtacgagcc gagctacctg gaacgcaatg tggtcggcct 7380
   ccacgtctac cacaacatcg cgttcctgga gaaaggcgtc aacgccgagg gcgccgttcc 7440
   tgcttgggtg ccgaggagtc tagacgacat tttgcacctg gccgacgtga acagcgcgga 7500
   ggacaagtga acagcaaagg gtcgaacgca caggcctttc caagcgcgga tcaggtggag 7560
   tccatcttcg acgcgttggc gcaagggcgt gccctgcacc acggatactg ggcgggcggg 7620
   tatcgggagg atgccggggc cacaccttgg tcggacgctg ccgaccacct gaccgacctg 7680
   ttcatcgaca aggccgcgct ccgccccgga gcgcacctgt tcgacctggg ctgtggcaat 7740
   gggcagcccg tagtccgcgc ggcacgcacc aaaggcgttc gagtcaccgg aatcaccgtg 7800
   aacgccgaac atctcgccgc cgctaccagg ctcgccaacg agaccggact ggccgacagt 7860
   cttcggttcg atctagtcga cggcgcccgg ctgccctacc cggaaggttc ctttcacgcc 7920
   gcatgggcga tgcagtccgt ggtacagatc gtcgaccagg ctgccgcgat ccgcgaggtc 7980
   caccgaatcc tggaacccgg cggccagttc gtcctcgggg acatcatcac tcgtgctcga 8040
   ctcccggaag agtacgcggc ggtttggacc ggcacgaccg cccatacctt gaacagcctc 8100
   accgcgctgg taagcgaagc cgggttcgag attctcgaag tcaccgacct cacggcgcag 8160
   accagatgca tggtctcctg gtatgtcgac gagttgctcc gggaactcga tgagctcgcc 8220
   ggcgtcgagc ctgcggctgt cggcacctac cagcaacgct acttgggaga catcgcggcg 8280
   aagcacggac cgggaccagc gcagctgatc gccgcggtcg cggaataccg gaaacatccg 8340
   gattacgcca gaaacgagga aagcatgggt ttcatgctcc tgcaggcgcg aaagaagcag 8400
   tcctgatggc ctccgagcac gccagcctgg tcggcgacga tctgcgggca cccgcggacg 8460
   atcccttcta ccgaccgccg acgccgctgc cgccgggtgc cccgggcacg ctcatcaggg 8520
   cccggcccgt cacggcactg cgcagcacgg gcgaacccgt cgcggccaag gtctggcaaa 8580
   tcctctaccg gtccaactcc gccattggca ggccgaacgc cgtctccggc accgttctgg 8640
   tgccgaacat cccgtggccg ggcgaagatc gccccatcat cactttcgca gtgggcaccc 8700
   acggcctcgg cagccaagtt gccccgtcct acctgctccg aaccggaacc gagccggaga 8760
   ccgagctgat cgccgtggcg ctcgaccgcg ggtgggccgt ggtcatcacc gactacgagg 8820
   gcctcggtac tccgggaacg cacacctaca ccgtcggcag gccgcaggga cacgccatgc 8880
   tcgatgccgc ccgcgctgcg cagcggctac cgggctcggg cctggggacc gactgcccgg 8940
   tcggcatctg gggctatgcg cagggtgggc aagcgtcggc cttcgccggc gaactgcacc 9000
   ccacctacgc ccctgaattg ccaatccgcg ctgcggccgc aggtgcggtg ccgatcgatc 9060
   tgctggacat cctccaccga aatgacgggg tgttcaccgg gccagtgctg gccggcctgg 9120
   tcgggcatgc cgccgcctac cccgatctgc cattcgacga gctgctcacc gacgcgggtc 9180
   gtatcgccgt tgatcaagtg cgcgagctcg gcgcaccgga gctcgtcacc cgcttcctcg 9240
   gccgcgagct gagcgatttc cttgatactt ccggcctttt cgagcaccct cgatggcgag 9300
   cacgactggt ggagagcgtc gcaggtagga acggcggccc ggtggtcccc acgctcgtct 9360
   accacagtac ggacgacgag atcgttccgt tcgcattcgg cgagcgactc cgggacagct 9420
   accgcgcagc gggtacgccg gtgcggtggc atccgctctc cggattggct cacttccccg 9480
   ctgccctggc cagctcgcga gtggtcgtct cctggttcga cgagcacttc tccgggccgt 9540
   ccgcgatcag cggtccgcga gatgacgggt gagcggatgg cggtgagcct ctccagcggc 9600
   ttcaccgccg gcttccggat gcccggtcag gcttcgaggc gaactactac ccgcggatgc 9660
   cggacggcta cgtggacccg caccggcagg cctgaccgat cgcctccacc agcgcggcct 9720
   gctggatcat cgattcgccc gaatctccgg ccaccgcagg ttcgtccacg cctgcctctg 9780
   ctctgatgtc gcgtgcaaag gcggtgaccg ccttgcgaac ctgatcttcc gctggcaagg 9840
   acaactcgtc gacaacgccc ttccgctcga ttcggatcac ggcctgccac tcggcgggcg 9900
   gagtgaacgc ccgatcgatg acgattcgtc cacgactccc ccacagctcg tacgcgctgc 9960
   ggtagtggtg cacgaaaccg tatccgaggt gggcaacggc gccaccttcc gattggagca 10020
   gcacgctgcc cgacaagtcg acgcccgact catgagcctc gtgcgagctt gcgccggcaa 10080
   ccgtgagcgg accgaggaga aagagccgag cggcacgggc gggatagaca ccgatgtcca 10140
   gcaacgcccc gccaccgagt tcggtgcgat agcggatgtc cgtgtcggaa agcggcggaa 10200
   tcccgaacac ggcggtgaac tcccggagct caccgatctc ctcggattgc agcaggtcgc 10260
   ggaccacgtc gtgccggccg tggtggagga acaggtaatt ctcccgcagc agcaggtgct 10320
   tccgccgggc cagcccgacc aggcgagcgg tttcggacgc cgtcgtcgtc agcggtttct 10380
   cggcaagcac gtgtttgcct gcctcaagcg ccttgccgat ccactctgca tgcatgccag 10440
   gaggcaacgg cacgtagacg gcatcgatgt ccggccgctc caggagccgc tggtaaccca 10500
   gcaccgcctc gcattcgaat cgtgctgcga accgttcggc cttcgccgga tgacggctcg 10560
   ccaccgccac cacctctgtt tcggccacgt cgcacatcgc gggcagcatc cgtcgccaag 10620
   cgaaggaagc acacccgagc acaccgatgc gcaccggctt tcgcatcgag ctggtcatcg 10680
   ccccaacgcc cacaagctat gcagggaggc aaccaagctg cgcgcctgga tgttcaagga 10740
   gtgggtgctc cggagcagct cgcccaactg gcccaaggtc atccaccgga agtcgctcgg 10800
   aggtcgtgcc gcgaagtcct catgcacctc gatgatccgg tacctgttct gcgcctggta 10860
   gaaccgaccg ccttcttcag acaggatcga ttcgtaccgc acggtttcgg gatcggcggt 10920
   gagcacgtcg tccacgaacg gcggccagtc gttgcgcgga gtgctttggt agttggccac 10980
   actgcactgg accgtgggag cgatttccgc agtcgacttg taaccagcct ctacccgagc 11040
   gcggaccaaa ccgtgcagca ctcctccgat ccgtttgacc aacagtgcga tctcacctgg 11100
   ttctcgcggt tcgatcatcg gctgagtcca gctggagacc tcacgattgg tcgcggacac 11160
   cgacactgcg atcaccgaga agtacttgcc gtcctggtgg gcgatctcgg tgtcggtgcg 11220
   ataccacttg tcgaccctac tgagcggaac gcgcgttgcc cgcaagctgt agcgggcctt 11280
   ggcttcctcg aaccaaccga ccgcctcggt aatactcgcc gaatcgatgc cgtgcgagag 11340
   cgacctggcc accgcctgcc ggaagggctc cgccgaggcg gctagtccgg gcccggtggc 11400
   ggaatcgtgg aacgggatgc aagacagcac cgtccgggtg tccatgttga cgatgttgtc 11460
   ctgacgaagg agatccagca cctggccgag ggtcaaccag cagaagtcgg gcaggactgg 11520
   cacttcctcg tcgacttcca ccaccatgtt acggttacgt ttccggtaga accaggcccc 11580
   ctgttcagac tggagcacgt ctaccagcac gcggctgcgg ccccgcccga ggaagtagtc 11640
   cacatagggt ggaacgctgc cacgatgtgc ctgcgtgtag ttgctccgag ttgcctggac 11700
   cgtcggcgag agctgcagga cgttgacgtt gccgggttcc atcttggctg acatgaggca 11760
   gtgcagcacg ccgtcgatct ccttgacgag aatgccgagg atacctactt cagcctggtt 11820
   gatgatcggt tgatgccagc aggtcgccgc gccatagttg gtctcgacct gcaggccttc 11880
   taccgtgaaa aatctgccat cagcatgaac caggttctca gtgctggcat cgaatttcca 11940
   tttcgacagg cggtcgaacg ggatgcgagt ggtctcgaag ctgttctcgc ccaaccggtc 12000
   ggccagccag cagtggaaac gcgtggtcgg aaacctgcca ttgcaagcgc tcagcgcaga 12060
   gtcgacgaac cgccgcgtgt tgttgctgct gagcggcgca gcagcacttg cctcagcttc 12120
   ggcaaaactg ctcataccca atccctcact gccgtgacga tgtgcacgcc gactcccagg 12180
   ggcatcgcgg tctggtccag cgacctggcg aggatcaccg aagcgatccg cccgtaatga 12240
   ccgcgagcac ctcgtcctgg cgttcggtca ttgaagaaat gcccgccgga gaagacacgg 12300
   acatcggcct cagcctcggt gtgctctcgc caggcttccg cctcctccag ggtgaccttc 12360
   gggtcggcat ctcccaccag cacatcgaac cgcggcgcga gcgctcggga cagcgggaag 12420
   tagaagctgg ccgaaccacc tgcgtgcgga gcacgaccaa acgtgttcca gccccgggcg 12480
   ccgggtggaa gcgacggatc caaatctcgc tgtgggttgc caagtcggtc acaattctcc 12540
   ctcggacgac ggccgaactg gcgtgaacac ccaccccgcc ctggtagcgc accgactttc 12600
   agagtcatca atcggtgttc tcgacaccag cggagcagga agtgagtggc atggcgatgc 12660
   aagcggtcag gacagccgct gcggacgcgg catgacgtac ctttccaact tgtagtgcgc 12720
   tgttgcttag cgggcgtcac gatcttcgtc ggaaagcggt tggagcgcaa acatctgggg 12780
   agatcaacac tccattgggg gattccggac gagaatctga tttcgggact cgttcggcgt 12840
   ccatgttgcc ggccatacgt tgctcacaga tggccgtcag acttcccagc gcggcggttc 12900
   ttaccttcgg caaccagctt ctccagacgt gcaacgatgt cgtgcgggct gggattttcc 12960
   tcgatttcac cgcggatcag cgccgcgttg gcggcgaacg acggctcgtc gagcaggcgg 13020
   gccagctgac gtcgcacgtc gtcttcggta aacgtcgcgc ggtcgaggac cagaccggct 13080
   ccccggtcgg cgaggagctc tgccctacga gattcgtccc agaaggtccc aggaagaatc 13140
   aactgcggta cgccgttgac cgtggcggtt tcctgcgtcg tcgttgagcc atggtggatg 13200
   atcgctgaac acgactccag cagttcgttg agcggtacgt attcgtggac ccggacgttc 13260
   gggggcaact cccccatctc ccgtacttca ccgccagaca aggtggcgat cacctcgacg 13320
   tcgagcccgg ccgcgccgcg caacaacgtt tccaccattg cctgttcctg ggcttctccc 13380
   tcccactgct cggccaccct gctctgctgc cgcttggtca gcccgcgggt gacgcagaca 13440
   cgcggcttgg tcggtggttc gcgcaaccac tccggcacca ccgccggacc gttgtacggc 13500
   acgaagcgca tcgagatgta gtccaagtcc acaggcagtc gcatccagga tgaaaccgga 13560
   tctatggtcg cttggcccgt cacgatctct tcatcgaacg tggcaccgaa cttggagagc 13620
   ttcgctccga gccacgcccc gagcgggtcg acgcgctgct caggcggctt cgattccagg 13680
   tattcgagga aaccggaccg cagccacccc gacacatcga gggcgacgag catccgtacg 13740
   tgtcgtacgc cgagcgcttg cgccacaact ggccccgaac acaccatggc gtcccacaca 13800
   acgagatccg gctgccattt ctcggcgaac cccatgagat cgtccagcga tcggtcatcc 13860
   acaaggtgga gttgctccac ggcgtccatg tctctgccag agttgattga cagcagctcg 13920
   tcgaagagtt ccggacgccg gccctcgtcg aacgcgaccc cgttgccgag aacgagtttg 13980
   ttcctggccg ccaaggagat gaggtcgagc tcgtcgccga cgggaaccgc ggtgagtccc 14040
   gctccggtga ccatcgacac catattcggg cagatggcga cacggacctc gtgccccgcc 14100
   gcacgcaacg cccacgccaa cggcaccagg ttgaagaagt gcgaactcgc cggtagcggg 14160
   gtgaacagaa cacgcatgga ctctccgatc gcaattgaac acccgggaaa acatggcaag 14220
   aatcacagaa acatgtgata tacccccggg aaacgccgct cccctaagct acatcttcct 14280
   cggtgcatcc aggcttaggc cttccaggtg atggtagcga tcttgacaag cgcaagcagg 14340
   tcgttcccgc tagcctggac tctgtcgagt cgggtgtgcc gggtagatcg aggagcactg 14400
   agtcaatgag cgcttctctg tgctccgctg tcctcatgtc ccgcaccgtg tcgaaccagg 14460
   acaggaagga gtcatgctcc gggacagcac cattgtccca ctgggacccc ataacgcgat 14520
   tcgccacggg aatcgctcac ctcctgaagg tcaaggcgcg aggactgatc gtcgcctgca 14580
   tgcagagccg gaaaaagccg gagcgttggg gaaagggcgc gccagagtga cttcgtgtga 14640
   cgacacttgc gctaccgcta ctgagatgac gccggatgcc aaggaccgga tattggcatc 14700
   cgtgcgcgat taccaccgcg agcagaaatc ttcgatcttc gtagctggat cgacaccgat 14760
   ccgaccatcg ggcgccgtgc tcgacgagga cgaccgggtg gcgctggtgg aagccgcgct 14820
   ggagcttcgg atcgccgcag gcgggaatgc tcggcgattc gagagcgagt tcgcccgctt 14880
   cttcggcctc cgcaaggctc acctcaccaa ctccggttcg tcggcaaatc ttctggcgtt 14940
   gagttcgctt acctccccca acctcggcga ggcacgacta cggcccggcg acgaagtgat 15000
   cactgcggcg gtcgggttcc ccacgaccat caatccagcg gtccaaaacg gactcgtccc 15060
   ggtattcgtc gacgtggaac tgggcaccta caacgcaacg ccggaccgca tcaaggccgc 15120
   cgtctcggaa cggacgcgag ccatcatgct ggcgcacacc ttgggcaacc cctttgccgc 15180
   tgacgaaatc gcagagatcg cacgagaaca cgagctgttc ctcatcgaag acaactgcga 15240
   tgcggtggga tccacctacc ggggacggct gaccggaacc ttcggcgacc tgacaacggt 15300
   cagcttctat cctgcccatc acatcaccag cggtgagggt ggctgcgtgt tgaccggcag 15360
   tctggagttg gctcgcatca tcgagtcgct gcgtgactgg ggacgggatt gctggtgcga 15420
   gcccggcgtg gacaacacct gccgcaagag gttcgattac cagctcggta ctctcccagc 15480
   cggctacgac cacaagtaca cgttctccca cgtcggttac aacctcaaga ccaccgacct 15540
   gcaggccgcg cttgcgctga gccagctgag caagatttcc gaattcggat cggcacgccg 15600
   ccgtaactgg cgacggttgc gcgaaggtct gtccggggtg ccgggcctgc tgctgccggt 15660
   gcccacgccg cacagcgacc cgagctggtt cgggtttgcg atcactgtca gtgcagacgc 15720
   cgggttcacc cgtgccgccc tggtgaactt cctggaatcc cgcaacatcg gcacccgact 15780
   gctgttcggc ggtaacatca cccggcaccc ggccttccag catgtgcggt accggattgc 15840
   cgacgcgctc accaacagcg acatcgtcac cgaccgaacc ttctgggtcg gcgtataccc 15900
   aggcataacc gaccaaatga tcgactacgt cgccgaatcg atcgctgaat tcgtggccaa 15960
   gaattcctag catccagcat ggctgcatct cggaggattt cagcaacgtg atcaacctgc 16020
   accagccgac cctcggcgcc gaagaactcg acgcgatcgc ggaggtgttc gccagcaact 16080
   ggatcgggct cgggccgcgc acccggacgt tcgaggccga cttcgcccac cacctgggcg 16140
   tggatcccga ccagatcgtg ttcgtcaact cggggactgc cgcgctgttc cttaccgtgc 16200
   aggtgctcga cctcggccca ggcgacgacg tggtacttcc ttcgataagc ttcgtagcgg 16260
   cggccaacgc catcgcatcc tccggtgccc gcccggtgtt ctgcgacgtc gacccccgga 16320
   cgttgaaccc cactctggat gatgtggcga aggccataac gccaacgacc aaggccgtgt 16380
   tgttgctcca ctatggaggt tcgccgggcg aagtcaccga gatcgccggt ttctgccgtg 16440
   aaaagggcct cgtgctcatc gaggacaccg cctgcgcggt ggcatcgtcc gtgcacggca 16500
   ccgcctgcgg aacctttggt gacctggcca cttggagttt cgatgcgatg aagatcctgg 16560
   tcaccgggga tgggggcatg ttctacgcgg cggaccgcga gctggcgcac cgtgcaagac 16620
   gactcgccta ccacggtctt gagcagatga gcggattcga ttcggccaag tcttccaacc 16680
   gctggtggga tatttgcgtc gaagacatcg gccaccggct gatcgggaac gacatgacgg 16740
   cagcgcttgg cagcgtgcag ctgcgcaaac tgccagattt cgtcagcagg cgccgggaaa 16800
   tcgctacgca gtacgaccgg ttgctttccg atgtgccggg tgtccacctg ccgccgacgc 16860
   taccggatgg gcacgtctcg tcacactact tctactgggt ccagctcgct ccggagatcc 16920
   gcgaccgggt agcgcaacaa atgctggaac gcggcatcta cacgagcttc cgctacccgc 16980
   ccctgcacaa ggtccccatc taccgcgccg actgcaagct gccttctgcg gagcacgcct 17040
   gccgcagaac actcctgcta ccactgcacc cgagccttga cgacgccgag gtgcgcacgg 17100
   tggctgacga gttccgcaag gccgtcgagc aacacatcag ctgaagatca ccacgtcgaa 17160
   agtgaggatg tcgcgcgtga gcggcacatt cgaagaactc tcctcggtat acagcccaga 17220
   ccatgccgac atctacgacg cgatccactc cgcgcgtggc cgggactggg caaccgaggc 17280
   cgaggaaata atccagctca tacgcaccag gctgcccgaa gcacagtccc tactcgacat 17340
   cgcctgtggg accggggcgc acctagagcg gttccgtacc gaatacgcga aggtcgcggg 17400
   gcttgaactg tccgatgcga tgcgggagat cgcgatcaga cgagtccctg aggtaccgat 17460
   tcacactggt gacatccgcg atttcgacct cggcgagcca ttcgacgtcg tcacctgcct 17520
   gtgctttacc gcagcttaca tgcggaccgt tgacgaactg cgacgcgtga cgcggaacat 17580
   ggcccggcac ctggcccctg gcggagtcgc ggtcatcgaa ccctggtggt ttcccgacaa 17640
   gttcatcgac gggttcgtca ccggagccgt cgctcaccac ggcgagcggg tgatcagccg 17700
   gctatcgcac tcggtcctgg agggccgtac gagccgaatg accgttcgct acacagtcgc 17760
   cgaacccgcc gggatccggg atttcacaga gttcgaaatc ctctcgctgt tcaccgagga 17820
   cgagtacacc gccgcgctcg aggacgcagg aatccgcgcg gaataccttc ctggagggcc 17880
   gaacggccga ggcctgttcg tcggaacccg caactgagcc cggaacaaag acgcaaggcc 17940
   ctggctgggc aggccccaga actcactcga gcggtgagcc gacgtgaccc cgggatcact 18000
   tcgatcgtcc ccgatgccgg caagttactg accgccgtga tctaattacc aacaccgtcg 18060
   gacgagttct ggtacctgtt tcggctggtg cagcgaaccg ggaacggggg cgtgttcgcc 18120
   aggggcatca gtgcgattca tagatcgagg agggccgcac cgccgtcgat caagtgcgca 18180
   acctcggtcc accgggactc gtcacgcact tcctcgacag agagctgagc gagtttccca 18240
   ccgttgcgga cctcttcgag caacctcgat ggcgggcacg actcgaggaa agttccgccg 18300
   gcaggagtgg cccggtagcc ccaacgctcg tctaccacag cacggacgac gagatcgttc 18360
   cgttcgcttt tcgagaacgg ctccgggaca gctaccgcgc ggcgggaacc ccggtgcggt 18420
   ggcatccact gtccgggctg gcacacttcc ccgccgccct ggccggctcg caagtcgtca 18480
   tcgcctggtt cgacgagcac ttctccgagc cgcccgcgat cagcgggagg cgatgacagg 18540
   tcgacgagtg gtggcgagcc tttccaacaa cttcacggcc gtgccgcatg cgtcgtactc 18600
   cgcatactca accgccagcc gctgcgcggc ctgccgatac cgaggatctg tcgacatcac 18660
   cttcaccgcc ccagcaacct gctgcggcga cggcctccga gtccgcaaat caacccccgc 18720
   gccactccac gcaacccgag cacacacatc cgttttgtcc tcactacggc cggcaacgac 18780
   caacggcaga ccgtgcgaca acgcctgctg aacggtcccg aacccaccgt tggtcaccac 18840
   cgccgccaac ttcggcatca actctcggta aggcaagaaa gacgccaccc gcgcattgtc 18900
   cggaacataa cccagatcaa cgccttcgcg accggtcgtc gccaccacca gaacttgatc 18960
   gccggccaga cctcgcagcg ctggacggat cagatcatcc gcatccacag ccatcgttcc 19020
   ctgtgtcacc aacaccaccg gacgatcgcc gtccaactcc ccccaccagg acggcaatcc 19080
   cacccccatc ggcgaatcag gttccaaccg tccaatgaag tgcatctgct gtggcagggc 19140
   tcgcggatac tccaaggatc gcgttccagc ctgcatgaac agatacgggg attcgctgac 19200
   ctctcggctg accggaaccc cgatggaatt ccagaacgcg ttgatcttct tcatccccgg 19260
   atcgtgcacg agcgcattta tcaaccggtt cccgattctg ttccgcagcc ggtggaaggg 19320
   gctggtgccg aacttccatc cagtaccgat gggcggcacc gctggatcgg gcagcagaat 19380
   cggcatctgc gagatcgtgg cccacagaac gccggtaaca gcgtggacca gtttagctgg 19440
   tccccatgac gcatcggcaa gcagcacatc agcccgggtt cggtccacta ctgccacgag 19500
   atcgcgatac tggccttcgt acgccggaac ccagtggttg tccatcaacc agcgtgcccg 19560
   gcgacgcgcg gacatctgga tgctttccgg aaacttctgc tccagctcgc ggccgtcgat 19620
   gaagcgcccc tcgaccggcg cggcgaaatc cgctccggag cgttcaaccg cagcacggta 19680
   gttctcgccc gtgtaccacg tgacctgatg gtcgcgctcc accaaggctc gggaaacagg 19740
   aaccagcggg ccgatatggg cgtgatccgc ataagtagca aacacgaagt gcgccatgat 19800
   ccagtcccaa ctccccaacc accgaggccc gcaatacacg ccaccgtaac agaattgcac 19860
   gaccgccggg tgccgagcga ctcgggggta aagattcagt tggattgccg tgagtggtct 19920
   gcgtgatggc atggcgtgat cgcccgatgg gttcggtgcc ggtcggggtt ggcgcgggtc 19980
   aggctggggt ggggtccggt ggcatccagg ggtgtccgcg aatggcctcg cggagggctt 20040
   cgatcatgtt gcggccgtgc ttgccggcgg tggagaggga tccgcggatt cggtagcggt 20100
   ctttggtgcg tttctcgatg gtcagccgcc cggagatgtt ctgttgcacc ttggcgggcc 20160
   gtaggtcgcg ttcggcttgg ttgggtgtgc gcggccccac tcgcagcggg gtttcgtcgc 20220
   agcagacagc gtaggccagg gtgtggatcc gcttgtcgac ctcggtcaac acgcccgcgg 20280
   cgcgggtaag cacgccgtgc acgaaaccca cgctgggcac ggcgccggtc agcgatgcca 20340
   acagctcgac gcagcggtgt acgggaatga agtgcacgac catgaggtac acagcgaaag 20400
   cctgcaggtt cgggccgtag cccaccgcgc cgggacgggc gccttccggg cgggcagcgg 20460
   tgtgcaccct gccgcagccg cagcgcaccg cgtgctagtc gtactgggtg acctttcacc 20520
   gacaccgggg agatctcatg ctgctggtag cgatccacca cccccagatc ccgtgccccg 20580
   ggccaggtca ctgccgcact cgcatacgcc cccgggaaac cgatccttgt gatctcccgg 20640
   gagatcggtc caggccagat tcgcccccgg cgccccgggc tgcttgccct tccgcttcac 20700
   cgcgccgccg cgtttcgcct tcgccggagg cggtgtcctg cccgggccgt cgtctttgga 20760
   cggagcagat gacgaattct tgctgttacg agacagcgca tgctccagct tcgccagtct 20820
   ctcgcccagt gcctcgttga cctccgccaa ctcggccatc tgcgcggcca tcgctgtgat 20880
   ctgccggtct cgcaccgcaa tctgctcgcc cagcaccgcg atccgcgcgc cctgctcacc 20940
   cacgagctcg atcaactcgg cgacaccgac acagaaacca caccagctcc agcagacgca 21000
   gagtgccacg ccagcacccc tgtcactaca cacagtgcac cggctgaatg tttacactcg 21060
   gggagttagg accgcgggcg aattcgcaca tagcccagca ccgatgtcag tcgatcaagt 21120
   tatgttccgg gccgcctccg ctctgcccga acccggtcca gtaccgtaac aacacatcat 21180
   ggagataatc ggcagaggat tcatcgcccg caacttgctg cggatctccg ggcggcacgc 21240
   agacgcggtc gcattggcgg ctggcgtgtc gaacaccagc tgccgctccg aggacgagta 21300
   tcagcgggaa gccgccctcg tgtaccggac catcgaacgc tgccacgcta tcggccgcaa 21360
   actactgttc ttctccaccg cgtcagcatc gatgtacgga gcgctcacct caccagggtt 21420
   tgaggacggt ccggtgtacc cgccgaccac ctatggccgt cacaagctgg ccatggaagc 21480
   ggtgatcaag gcatccggag tggactttct catcctgcgg ctggcctacg tcattggagc 21540
   ccaccagcgc ggacaccaac tgctcccgtc cttggtgacc cagctcaggt ccggctcggt 21600
   cacggtgcac cgaggcgcgc atcgcgatgt aatcgcggcg gacgacgtgg tgaccatcgt 21660
   cgacgacctg ctcaccaagg cggtcgcggg gacggtggtc aacatcggct cggggttccc 21720
   cgtcccggcc gagaagatcg tggcacattt ggagtatcgg ctgggaacgg cagctgcacg 21780
   gcagtggatc gaccatccta ccgaatacca gatctcgttg acccggctga acacgctggt 21840
   cccacgaatc gccgagttgg gcttcgggcc ggactattac cggcaggtgc tggaccacta 21900
   cttggacctg tacccacagg cctgatcgat cgtcgtgacg agcacggcct gccggatcac 21960
   cgactcatcc gaatctccag ccaccgcagg gtcatccacg cctgtcccgg ctctgatgtc 22020
   gcgggcggaa gcggtgaccg cgttgcggtc ctgatcttcc gccggcaggg acaactcgtc 22080
   gacgacgccc ttgcgctaga tccggatcac gggttgccac gtggcgggcg gattgaatgc 22140
   ccgatcgatg acaatgccca ccgccgtatt ggccaccgcg cacatcgcgg gcagcatccg 22200
   ccgccaggcg aaggaagccc acccgagaac gccgatgcac cggctttcgc accgaactga 22260
   tcatctcccc gtcgttgacg ttgcccggtt gcatcttggc tgacatgagg cagtgcagca 22320
   ccccgtcgat ctccttgacg agaatgccca cttcgacctg attgtgatcg gttgatgcca 22380
   gcaggtcgcc gcgccgtagt cggtctcgac ctgaagacct tcgaccgtga agaatcgacc 22440
   ttcggcgtga accaggttct cggtggccgc ttcggatttc cagttcacta gggcgctgaa 22500
   cgggatgcgc gtggtctcga agctgttctc gcccaggcga tcggtcagcc agcagttgaa 22560
   ctcagcattc ggcatcagcc gattgcaggt actcagcgct gagttgatga accgctttcc 22620
   gccgagcagc gcatcggtac tcgccccagc ttcaacagag ctgcccctgt ctagtccctc 22680
   actgccacaa cgatgtgcac gctggctccc aggaatattg gggactgcga gacttccacc 22740
   tggtaatcaa gcttgcggag ccgatcgagc acctggcgca gccgaccgca gatgtcgtgc 22800
   acttccacga ctatccgacg aatacggggc cacatctcgt catcgatccc gttcagcacg 22860
   tcgagctccc cgcgttcgac atcgatcttg agcagatcga gcacatcaag ccggtgctgc 22920
   cttgcgatct ctgtcaacgt ggtcacccgc acgtcgagtt cttccttggt gcggaccagc 22980
   ccctgcatcg attcccccgc cagctcgggg ctgccgacgt tcgacatcac cgtgtcgatg 23040
   ttgcgccgct catccgccgc atcgaggtgc agcgtcgaca aagatggccc tgcggggtaa 23100
   tagacgaagc gggacgttcc gggctccgca cccaccgcca ggtcgaacgt cacacctcgc 23160
   ggtacgtggc gggcgaagtt ttcccgcagg caggcgaagg tcgttggtgc cggttcgtaa 23220
   gcgagtattc gtgctgctgg aattcgatcg gcgaagtaca tcgacgcaag gccgacatgc 23280
   gcgccgacat ccacgatcac cgaatcagca cccaagccgc gcagaccgcg ggcgtaagcc 23340
   gaatcgttcg cgatgtcctg ccaaatggca agtacttcaa gcgtgttggc gcatgcgact 23400
   agacgcccat caggaagggc ctgcgtcgcc ctgtcgtcaa ccgtgtcgaa catcggtaac 23460
   gccccgtcct cagcgattcg gaactcaacg gagttccctt cccagaacaa ttcccgagct 23520
   atattcccga tatctcacgc ggccggcaat ccgaagatca cccaatcacc cttcgcaaca 23580
   atcgaagggg tgagacggaa gtttccgacg gatgacccgt ggtgtagcag caccccgcgt 23640
   gctgcagtcc actcgacgca gtcgtccccg acgggatgat ccgccatcac cacgtgatcg 23700
   ggagggcctc cggcccgcgg gtcattcggc ctcgtttcca gacgacctca tccaccggaa 23760
   ccgcgaagga cagctgcgga aaccgtttga tcagcgtgcc gatcgctacc tgcagctcca 23820
   tcctcgccaa ttgggcgccg atgcagtagt gcgggccgtg ccccagcgcc atgtgcgagt 23880
   tgtgctcccg agccaggtcg agttcgtccg gcccgtcgaa gacggcactg tcacgattcg 23940
   ccgaggctat ctcgaagaac actgcgtcac cccggcgaat cgacactccg cccagttcga 24000
   gatcctcagt cgcgatgcgc gggaacccag gtgtggcacc gagcggcgtg tagcgcagca 24060
   actcctccac cgcgcgcggc accagctctg gatcggcgat cagcttgtca agctggtccg 24120
   gatgggtgag caggttgaag gtgaagtttg cgatgtggtt agcggtggtc tcgaacccgg 24180
   cgatcagcag acccgcgccg gtgacgacaa tctcctcctc gctcagctgg gctccttccg 24240
   ctctggcctg gaccagcacg ctcagcaggt cctcagtcgg catcttcttg cgctgctgga 24300
   ccagttctcc gatatacgcg cgaatttgat cgcggctttc ccgaatctcc tcaggactgt 24360
   tcgatgtgat cgccaacgca atgtccgacc agacccggaa gcgctcgcgg tcggccaccg 24420
   gaatgcccag caagtcgcag atcaccttga tgggcagcgg cagggccagg gctgacacca 24480
   ggtcgccagg cggcccgtcc gcggccatcc gatcaagtag gtggtcaacg agctgctggg 24540
   tgcgggggcg aagctgttcg actcgacgtg cggtgaacgc cttaccgacc agtttgcgca 24600
   gccgcgtatg ctccggcggg tccatcgttc caagcgaatg ttctcgcaag atcagcggga 24660
   acccccgcgg tacatccctg ttcaggatcg ccgcagcgct gaatcgcgga tcgccgagca 24720
   ctgtcttgat gtccgcatac ctggtgacga gccaaccgtc accaccgtat ggaagccgga 24780
   tcttgctcac cggttcgccc tcacgcaaaa cggcgtagcg atcatccagc aacagccggt 24840
   cgatctcgcc gaagggatag gccattggct catcaccatt ggtcatcaac ggtcccctct 24900
   ctcagacctg gccccgttcc ggggacgcgc ttcgcggagc cagcggtgga ctccacctcg 24960
   attgcgctac ttcctcttgc tagtcagcag tttcccgatc acgaggatcc aacgactcgc 25020
   tccgcggaac tggctcccag tgacgatcac ggaagcaatc cactggtgat gaccgcgagc 25080
   acctcatacc ggggttcggc gaggtagaaa tgcccacccg ggaacacgtg gaggtccatc 25140
   tcagcttcgg tgtgctcgcg ccaggattcc gcgtcatccg gagtgacctt cgggtcggca 25200
   tcccccacga gcacggtgat cgaacaactc accttcgatc ccagcggaca acggtaggtt 25260
   tcaaccgctc ggtaatcatt gcggacggcc gtcaggacca tgcgcagaag gtcctcgtca 25320
   ccgagcaccc aggaggtggt cccgcacagc tgagcgctcg ggatagtggg aagtagaagc 25380
   tagccgagcc gcccgcgtac ggcagcacga ccaaagagac cctggcccgg ggtgcgggat 25440
   ggaaacgttg gatccacaga tcgtcgccgg ttgctaggtc ggtcacacga ttctccctag 25500
   gacggcagcg gaactagcgt cgatcgctga gccagctgcc ggtagcgaac caactttcgg 25560
   agccatccat caccattcgc tgatgtcacg atcttcttgg gaggccggtc ggcgcgcaaa 25620
   cctgctgcga gatcagcact ccatcgggcg acttccgatg ggaaaatcca atctcggaac 25680
   tcaaccgttg actgccgtga ttcaagctgg gcctcttgct ggaagtaact gttgcaggaa 25740
   atacggaagc atagagcatc cggagcatcc ggagcatccg gagcatccgg agcatccgga 25800
   gcatccggag catccggagc atccggccag atctcgaatt gctcgcggtc gacctccgga 25860
   actcccgaca gcagcgagtt gatcaccttt atgggcaaat gtagggccag atcaaggaat 25920
   tgtcaacacc tgtggatcaa gattgttcag gcgacggtgg gtggggcgtg gtcgtcgggg 25980
   cgttcgacga gtttgccgcc ctcgaagcgt gctctggcgc gcaccagcgc gacgagttcg 26040
   ggtgcgttga ccgcgggcca gcgagcctgg gctgactcga tcagcttgaa cgccacggcc 26100
   agcaactcgt cgacatcgtc ggtgatcttg gcgactgcct tggggaactt cgccccgtag 26160
   gcggcctcaa acgccctcac cgtgtccaac acgtgccggc tgtcctcggc attccagatc 26220
   tcggccaggg ccttcttcgc gccgggatgc gccgatttcg gcagcgcggc gagcacattg 26280
   ccgatcttgt ggaaccagtg gcgctgctcg cgcgtatcag ggaaagcctc gcggagcgcg 26340
   ccccagaacc ccagtgcacc gtcgccgatg gccagtaccg gggcacgcat accgcggcgc 26400
   ttgcagtcac gcagtaggtc agcccagccc aggactcggt ggattcccga tagccgtcgg 26460
   ccagcgcgac gagctccttg cggccgtcgg cacggactcc gatcacgacc agcagagata 26520
   gtttgtgttc ttccaggcgg atgttgacgt gaatgccgtc tgcccgcaga tacacaaagt 26580
   ctacttcgga caaaccacgt tcgttgaaag cacggtgttc ggtcctccac tgctcggtca 26640
   gtttcgtgat caacgtagcg gataacccct tgctgctgcc aaggaattgg cccagcgcgg 26700
   gcacgaagtc tccgctggag agcccgtgta ggtacagcag cggcagcatt tcggtgatct 26760
   tcggggtctt gcgcgcccac ggcggcagga tcgccgagga aaaccgccga cgcgcgccag 26820
   tgtccgggtc ggtgcgcttg tcgttgactc gtggcgcagt gacctccacc gcgccagcgc 26880
   tggtcaacac ctcacgaggc tggtcatggc cgttgcggac caccaggcga tggccgcact 26940
   catcgcgctg atcagcgaac tacgcgatgt aggcatccac ctccgcctgc aacgcctcgg 27000
   ccagcatccg gcgggcaccc tcacggacaa tctcatcgat caacgacgcc aaagctgcgg 27060
   caggacggcc gtcgtcacgg gaatcagggt cggggactac gctgagcatc gggtcgtacc 27120
   ttcccgaccg acgcggcaac gtcggccatg cttggaacct tgcatccgat cactgggaag 27180
   gtacgcccct cctcagccga tccaaggttc cgagcattcc tcgaccagat ccgaaagaag 27240
   gtcgacttgc gcaacggcgg cacgcaggac caactcggac tccgcctcaa tcctgaccag 27300
   tgcacgggca ctcgatcgga tggactgctc ctgtggctgc gagattttgg atatctttga 27360
   cagaagagca ctttcgcgga tataggctga tccgatggct cgatcgaaac ccccacacgt 27420
   cccgacagga aggcttggcc tttgaagact cacgacgccg cgtccggaac gacggccacc 27480
   gtacaactcc agcgaatcat cggagggcac ttggcctacc acgtccttgg cgccgccgct 27540
   cgattggaca tagccgacca tctgcgcgaa ggcccgctca cggcttccga gttgagcgac 27600
   ctcatcggcg gcgacgatcc cgaaatagtt gacaaattcc tgcgagtggc cgaaacgatc 27660
   ggtctggtgc gcaggaccag ctccggtcag ctggcggaga ccgaactgct cgctctgctg 27720
   cggcgcgacg gggggcggta tcggagtacc gtcctggccc tcacagcccc gggtttcaac 27780
   cgccccagcg agatgatgca ccgcgcagtg ctcagcggca gggcgcatac cgcgcaggtg 27840
   ctgggcactg atctgtgggg ttactacggc accaaccccg aagaagccaa atggttcggc 27900
   ggcgccatga ccgacctgac caacttggtc gcagacctgg tgctggcccg gtacgaattc 27960
   tccggacgcg gcacgatcat ggatgtcggc ggcagccatg gcatattcct gtcccggatc 28020
   ctgcacgccc aaccggacgc gaagggtgtg ctgttcgacc gcatggaggt ggtcgaagaa 28080
   gcccgcaatc acctagatca ggacatccga acccgcatcc agatcgtcgg cggaaacttc 28140
   ttcgagggag ttcccgaagg cggggatctc tacatcctga agagcgtgct gtgtgactgg 28200
   gacgaccaga gctgcctgca aatactctcc cgcatccgga acgccgccat gcccggtgct 28260
   tcgctactga tcgtcgactg gttgtaccct gacgagtccg accccggctt ggacgcgatc 28320
   tacctccagc aggcgatctc ggtcaacggc cgggtccgca accaggaaca gttcgaatct 28380
   ctcctaaagg caacaggttt cgcggtcacg agggtcgaac gaaccactcc ggagaactgg 28440
   atcccggcga caatcatcga agcgatccgc cggtgatgac cgcgagcacc tcctcctggt 28500
   gttcggcagg cagaagtggc ctgcgggcaa gaccgggagg tgcactccat tgtctcgtct 28560
   atgaaccttg atcgggttat tgatcattag ctgttgggtg tcgggcaggc ttgctcggga 28620
   aggtcaagac tggtggaggt ggtggatgcc tgtcggggct tcctcccacg gtagttgatc 28680
   gctggcttcg gttcctcgct tcgtgctgat gagagtgcac gggtcgatgt ccggggtgca 28740
   ggcggttcca gcgtgtcggt ctaccgatgc tctggtgttg gccgtttcgg gtagagccgg 28800
   gtcatcgcgt ggatcgtgtc ggtcatgcgt gcgaccagtt cggcgggagc gagcacctgg 28860
   acgtccgggc cgaggcgggg gtgtcaggtg ggttgtgtac aagatccgat agttgatgga 28920
   gtacgcagat ggccacgact gatcagcagc aggaagggaa tccgttcggg cggccgccgg 28980
   gcagccggga tcggcgcggg acgcggtcaa cgagatggtc gacgcgggcc tgcttgacgg 29040
   gatgatggac gccatcgatc gggatgggct ggcgttgacc gggcagggtg ggttcctgcc 29100
   tgagctggtc aaggccgtgc tggagcgcgg tctacggacc gagatctcgg aacatctcgg 29160
   ctatgacaag ggcgatccgg ccgggcgggg cagcccgaac tcccgcaacg gcaccagcgc 29220
   caagaccctc tcgaccgagg tcggcgacgt ggacttggac gtgccccgcg accgcaacgg 29280
   cagtttcgag ccgcggctgg tcccgaaggg ctttcgccgg gctggcggcc tggacgagat 29340
   catcatcatc tcgctgtacg cgggcgggat gaccgtccgc gacatccagc accacgtgca 29400
   gcgcacctac ggaaccgagc tctcgcacga gacgatctga accgtcgcgg tttcggtaga 29460
   ggcgttgatg tgccccggcg ccggcggagt caggttactc gatcatcgtc atctcagtgc 29520
   cccgactgga ggttgcctcg catgccccgg agatacccac cggagttccg ccgtgggaat 29580
   ggatcaccgg cggcgaaagg ctgatgacca acggactgtc ctttcccctt gctggaaacc 29640
   ctcaacgcct acctctcgaa ccaacgctca tggcagcgca ccagcgcgac actccacgtc 29700
   caccggcaaa ccgtcctcta ccgcatacgc aagatcgaag agctcaccca ccacgacctc 29760
   agcgaaacaa gcgacattgc cgaactgtgg ctggcgctgc gcgcactcga actcatctcc 29820
   cagtgagtgc ctgctgatct cggggaagcg aacgcggtat ttagccgaag tcgcgtctgc 29880
   tgaccgcgca aaccccgtgc cccgcggagc ggtgaacgtg cgaggtctcg gctactcttc 29940
   tggagttccc aaagccgctg cgggcaagtt ccgcgacatg accggagctt tcgtacccgc 30000
   tgcccgtgcg ttacggctgg gccaacgccg cccaggtcct gcaccggacg acccgcgtcc 30060
   acgcccaggc cacccgtcgg gtcgtggaga ccacccagtt cctgcaggac gtacacgtac 30120
   aggccgacgg tgggctgcca acccccgagg gccacgggat gcggtcggcg cagaagatcc 30180
   ggctgctcca cgccacccat ccgatacttc ctgcggacca gcgacgactg ggactcggca 30240
   gtgctcggcc tggccgcgaa ccaggaagat ctggccgccg ccatgggcac cttctccgtg 30300
   tgcctgcccc gggggctggt ggcgctcggc gtggacctgc ccgaccacga ccgcgacgac 30360
   tgcttccacg tctggtcggt cgtcggccac ctactcggcg tcgacccgca acttatgccc 30420
   gccgggatcg acgaaggggt cgctctgatg gagcggatct ggggccgcca gaccgcggaa 30480
   tccgacgccg ggaaggtctt caccgccgca ctggtgtagt ccgtgcgtaa tgtgctcggt 30540
   cccgcgctgc acggcacccc atcgaggcga tgatcccgcg cttctgcggc gacgagttcg 30600
   ccgacctgct cgccgtcgac cccgcggacc ggaccgccct cgctgccggc cgcgcctcaa 30660
   ccgtcaacac ggtctacggc aagaccggcg accacagcga actcgccgcg tcgatcgcca 30720
   gcaggattgg cgagcttctc ctcgacgccc gccctccaca cggccaaccg cagcaaccgc 30780
   tacgactgga ccattcccaa ctgcgaacaa cagcaccgcg aaggctcgcg atgaacacca 30840
   cagctattga tcgggaactt ataggtgtga tcttcgttgt tctggcatga tcgctggtgt 30900
   gcgggacgcg cggaggttgt cgcctgaggc gcaggaggat ttgcggcgca gggtggtcgc 30960
   tgctgttcat ggtgggatga gtcaggtcga ggcggcccgg gtgttcgcgg tggccccgca 31020
   gtcggtgtcc agatgggtgc aggcgtggcg gaaacgtggc tcgaagggtc tcaccgggcg 31080
   tcgccggggt cgcaagcccg gcgagcagaa agcgttgagt gcccgccggc agcgcaagct 31140
   gcggtatgcg gtggccgagc acaccccggc cacgttcggg ctgaccggcc tggtgtggac 31200
   ccgcaagaca gtggccgagc tgatccgggt gcgccacggc atcgtgttga acctgcgcac 31260
   cgtcggcaac tacctgcgtt cctggggatt gtcgccgcag aaaccgatcc gcaaggccta 31320
   cgaacaggac cccgagtccg tacgccgatg gctggaggag gactacctgg ccatcgccgc 31380
   ccgcgcccgc cgcgagggcg cactgatcct gtggctggac cagaccggga tccgctccga 31440
   cgccaccgta gcccgcacct gggcaccggc gggccagaca ccggtggtgg gcaaaacggg 31500
   caaacgattc agcgtgaacg cgatgtgcgc gatcgggaac aaaggcgagc tgtacttcac 31560
   cgtctacacc ggctcgttca acggcaaggt gttcctgtcg ttcctggacc ggctgacccg 31620
   ccatctggac cgcaaggtcc acctgatcgt tgacggacac cccgtccacc gccgcaagac 31680
   catccagcaa tggatcacca agcacgctga ggcgatcgcg atgcacttcc tgccgggata 31740
   cagccccgaa ctcaaccccg acgagctact caatgccgac ctcaaacgca ccgtttccac 31800
   cagcacagcc cccaaaaccc gcgccgagtt gaaacaagcg gtccgctcct tcctccaccg 31860
   gctccagaag ctgcccgacc gagttcgctc ctacttcggc aaacccgaag ttcgctacgc 31920
   cgcctaacat cacacatttg ccacccggat caataacatc ccttttccag gtcgtagtgg 31980
   accgaccccg tcaggcaagt gcgagttcct tcggccctag gcacacgtcc aactctgcca 32040
   gcgtctcgtc gttcacgtcg accgggcgca gcgtgtgcgg cagcgagcgg acggcgtcag 32100
   cggcgatgaa cgcgtcctgg gtagtggtcg gcgatccgtc gcatggccga accggtgccc 32160
   gcaggcccgg gcaaccattc gacgcctcac cataaaccgt ctggtgacgc tcaaaggggc 32220
   gacgcgacag aacccagatc gcaccgccgc aacaccgcgg tccgtgccag cagccgcatg 32280
   ttcgcccggg accagccgta tccaggccgt gcagcgtcgg gtggtcagga actctgaccg 32340
   cggagtactc ctcccgtgtt gtcaacggaa gagctgtcgc ctggtcagcg cggatcgttc 32400
   acttagatgg cggtttctgt taccacaagt gaagatttgc aggcttcgag gatgcgcgat 32460
   gccggcagcg gatcacactt cttgacctga ccgcctcgag atcgtttctc tattctcagg 32520
   ccgtgcggct gagccgctga ccgggcatgt agatcgaaag gcgggctatg cacgcttact 32580
   actgtgcgaa gtgcaagaac gaacaaacag atcctgcgga tgcgctgaag ttcgcggcct 32640
   cgatcggctt ggaattgtgg ctgccgaagg atgaggtgac gttcgacttt ccccacggcg 32700
   cgcagcagtg ccagacggcg atcgagaaag cggaatgtgt gatctgccag cctccgatag 32760
   gcaacgactg ctcctgggaa ctcggatacg ccatcgggat cggcaaacca gtctacgtca 32820
   tcggaacgct ggccgagcag gactggatga ccaagctcgg cgtcactcac gtggacccgg 32880
   cgtcgttggc tgccgagaaa gaatgaggtc ctgccgtgac tcagcacagt cggcggccca 32940
   ccggcaaagc agtggggcgc cgcagagtag cggcgatcga ctgcgggacg aactcgatcc 33000
   ggatgctggt tgccgacctc ggtgccgacg ggcatttgac ggaagtcacg aaacggttcg 33060
   acatcgtccg tttgggtcag agagtcgacg aacacggctc gatctcccgc gaatccttcg 33120
   aacgagcgcg cgccgtgttg gccgaatacg caggaaccat caccacggcc agtgtggagc 33180
   gcgtgcgcat gtgcaccaca tggatctccc gacgagcgtc caacaacgac gaattccgca 33240
   cactggtcca agagacactc ggctgcgcac cggaagacat caccagcgac gaggaagcac 33300
   ggctcgcttt cgccggggct accagcggtc ttccgcaggc gagctacctg gtcgccgaca 33360
   tcggtggcgg gtccacacag ctcgcgctgg gaacagcagg tttcgtggac agatccgtct 33420
   ccgtcggcct gggctgcgta cgcctgaccg aacgccacct gcggtcggat cctccagcct 33480
   cgggcgaact cgcggcggta caggacgaga tcacggcact ggccgaccag gcactggccg 33540
   aacttcccga tttgtcgggc actcggcttc tcggtgtctc cgaagcagtc ggcacagttg 33600
   ccgccatcgc gcttcctgga cgaaggctcc atcacgcacg aatgacctac gaccaggtcg 33660
   attacgtgac agagcgggtg ctgggaatgt ccttcgccca gcgacaagcg ctgcccggaa 33720
   tccacacagg tatggccgat gtactacccg tcagcgcgct cagcgtgcgt acggtgatgc 33780
   aatgtgcccg ggccaaggag ctgatcatca gcgagcacga catcctgcat ggaatcgctt 33840
   actccctgag ataagcggga tcagatccca atgccgaaca gcatcacggc gagcgggcaa 33900
   tcgaatccac cgacgcaccg agtcccgaac accctgccag cccggcaggg tgttcggcgg 33960
   cgggtgtcag gtctgccgcc acgggcttcc ggcgcctctg atggacgcct agccgcaaac 34020
   ctttgcgtag tcggtttcct cgaccgcttc catcctcata caccacgaca ggcgcgaaaa 34080
   cgtatgccgc gaaggtctcg catgcctcgc gtatgtcggc ctcggcgtgg agttaggcaa 34140
   acgagttgcc aaggcagagg atcacgtcag aggtgacacc atgcgaaggt gcccagacat 34200
   ggtgccgaag atgccgcgct actcgcgagc atcttcggca ccttcgcctt ccagcaccca 34260
   ctcccacgcg ccgctggcga aaccggcttt tcatcgaacc agcacgaaca ccagcaggct 34320
   cagaccacag cccagcacga acacggcata gaggatgtcg acaccgtgcg ggggatgctc 34380
   ggctatgccg gccacgatcg ccgccgccgg caaccaggcg agcagcagca cacatgtcag 34440
   cctgttgcgg cggacccgca tcccggagcg cgacgaccac ggtcgcggtg accacccacc 34500
   ggtcagccag ggcggcagcc gccggacgaa caccaggacc agcgtgcagg cgaagcccag 34560
   agccagaccg atcacggtcg tcgacgtcca gccaccagcg aggtggatcg cggcgagtac 34620
   agcgaagacc ccaaggaacc accagcgcgg acgccaggca acgtcctcgg ccgccacctc 34680
   ggccggctcg ggaacaggcg acttttcggc cgctgattca gctccgtcgg aaaccaccac 34740
   accacccttg cccaaacaga cctgccgata gcgtatcgag cgcgatctga gcactctcgc 34800
   gccgaagggc accacagcag ggagtccccg cgccccactg cgctggctgg ccggcgactt 34860
   cgggttgcgt gatctcgtcg ccgacagcgg aaacacagca gccatcgccg catatgaaca 34920
   ggccactgct caggcagcag aggcaaaact agatggcaac gctgcgcgac gcagcgacgt 34980
   catgtcccgg gacaccgtca caagcagcga cacgctgtcg agtgcgggat caaccgcctc 35040
   aaacggaacc gagccgtggc caccagatac gacaagcttg ccgtccgcta cgagccaccg 35100
   tcaccatcgc agggatcaac gagtggctcc catgactttc gaaacaggcc caagcgcccg 35160
   ccgcctggtc caagccccgg cgcgcgacag ccacacccga actcgcgttg tcgcggttgg 35220
   gaatacccac aaccggctct ccgcgcgcgg ggcgccccag caacggcgcc ccgacacggt 35280
   gctgcgtgga acggacgaga gtgcgctccg acgccagccc cgagcacctg tgcggttcga 35340
   gcccgcggac gaactgcggc tctccgtcct gtcctcgctg atccccgccg ccgctgggcg 35400
   caggtcttcc cagacacgcc gagcacactg atggcctggc accgcacgct tgtggcccgc 35460
   cggtgggact actccgatcg gcgccgacct ggacgaccgc ccacaaggcc ggccatcaag 35520
   aagctcgtgc tacgcctcgc ccgcgaaaac agtcagtggg gacaccgccg gatccggggc 35580
   gaactggccc ggctcggaca cccgatcgcc gcctccaccg tctgggaaat cctgcacgta 35640
   ggcggcatcg atccggcccc gcgccacagc ggcccgacct ggcgtgagtt cctgtccgca 35700
   caagccagcc gtctgatcgc ctgcgacgtc ctgagcatcg acaccaccgg cctacaacgc 35760
   ccatactccc tggtcttcct agaacaccga acgcggcgcc tgcacatcac cggcgtcacc 35820
   gcacacccga cggcgcctgg gttactcagc aagcgcgcga cgtcgccacc gacctcggca 35880
   cgcgtatgga ctcgctgcgt ttccgcaccg gagaccgcaa acagcaagta caccgacgat 35940
   atcgagatca tcaagacacc ggcgcgggcg ccacgagcga atgtgcactg cgagagagcg 36000
   atcggcagcc tccatcgaga agtcctcgac cactcccctc atcatcggta agacccatgc 36060
   acgccgcgtt ctcaccgagt accaagagca ctacaacaaa caccgccccc accgggcccg 36120
   caactagaac tagtgtcctg cgccggagat tcgttgacaa tatgatcgga gtgactccaa 36180
   gatctcgtca gcgctcttcg tccagatgaa cggccgggga tcgttgttcc actcgtcgat 36240
   ccagttgcgg atatcggcct cgagtgcctg gacgctggtg tgcacgccgc gttgcaggag 36300
   tttggtggtc aattcgccga accagcgttc gacctggttg atccaggacg atccggcggt 36360
   gcgatagcgc gccgatcggg tccttcgcgg ggaactccgg ggattccgtg ggccaccacg 36420
   ccacgaccgg cgcgtccggc agcagcagcg gcaccaccga gccggcgccc tcgtcggcca 36480
   gcggcccgta cagccgcagc acgatgacct ccgaggcccc agcgtcgccg ccgatgcg 36538
<210> 3
   <211> 4344
   <212> PRT
   <213> Saccharopolyspora sp. NRRL30141
<400> 3
   Met Ser Glu Ala Gly Asn Leu Ile Ala Val Val Gly Phe Ser Cys Arg
   1               5                   10                  15
   Leu Pro Gln Ala Pro Asp Pro Ala Ser Phe Trp Arg Leu Leu Arg Thr
               20                  25                  30
   Gly Thr Asp Ala Ile Thr Thr Val Pro Glu Gly Arg Trp Gly Asp Pro
           35                  40                  45
   Leu Pro Gly Arg Asp Ala Pro Lys Gly Pro Glu Trp Gly Gly Phe Leu
   50 55 60
   Ala Asp Val Asp Cys Phe Asp Pro Glu Phe Phe Gly Ile Ser Pro Arg
   65 70 75 80
   Glu Ala Ala Ala Met Asp Pro Gln Gln Arg Leu Ala Leu Glu Leu Ala
   85 90 95
   Trp Glu Ala Leu Glu Asp Ala Gly Ile Pro Ala Gly Glu Leu Arg Gly
   100 105 110
   Thr Ala Ala Gly Val Phe Met Gly Ala Ile Ser Asp Asp Tyr Ala Ala
   115 120 125
   Leu Leu Arg Lys Ser Pro Pro Glu Val Ala Ala Gln Tyr Arg Leu Thr
   130 135 140
   Gly Thr His Arg Ser Leu Ile Ala Asn Arg Val Ser Tyr Val Leu Gly
   145 150 155 160
   Leu Arg Gly Pro Ser Leu Thr Val Asp Ser Gly Gln Ser Ser Ser Leu
   165 170 175
   Val Gly Val His Leu Ala Ser Glu Ser Leu Arg Arg Gly Glu Cys Ala
   180 185 190
   Ile Ala Leu Ala Gly Gly Val Asn Leu Asn Leu Ala Ala Glu Ser Asn
   195 200 205
   Arg Ala Leu Met Asp Phe Gly Ala Leu Ser Pro Asp Gly Arg Cys Phe
   210 215 220
   Thr Phe Asp Ala Arg Ala Asn Gly Tyr Val Arg Gly Glu Gly Gly Gly
   225 230 235 240
   Leu Val Val Leu Lys Lys Ala Asp Gln Ala Arg Ala Asp Gly Asp Arg
   245 250 255
   Ile Tyr Cys Leu Ile Arg Gly Ser Ala Val Asn Asn Asp Gly Gly Gly
   260 265 270
   Ala Gly Leu Thr Ala Pro Ala Ala Asp Ala Gln Ala Glu Leu Leu Arg
   275 280 285
   Gln Ala Tyr Arg Asn Ala Gly Val Asp Pro Ala Ala Val Gln Tyr Val
   290 295 300
   Glu Leu His Gly Ser Ala Thr Arg Val Gly Asp Pro Val Glu Ala Ala
   305 310 315 320
   Ala Leu Gly Ser Val Leu Gly Val Ala Arg Arg Pro Gly Asp Lys Leu
   325 330 335
   Arg Val Gly Ser Ala Lys Thr Asn Val Gly His Leu Glu Ala Ala Ala
   340 345 350
   Gly Val Thr Gly Leu Leu Lys Thr Ala Leu Ser Ile Trp His Arg Glu
   355 360 365
   Leu Pro Pro Ser Leu His Phe Thr Ala Pro Asn Pro Glu Ile Pro Leu
   370 375 380
   Asp Glu Leu Asn Leu Arg Val Gln Arg Asp Leu Arg Pro Trp Pro Glu
   385 390 395 400
   Ser Glu Gly Pro Leu Leu Ala Gly Val Ser Ala Phe Gly Met Gly Gly
   405 410 415
   Thr Asn Cys His Leu Val Leu Ser Asp Ser Ser Gln Val Glu Arg Arg
   420 425 430
   Arg Ser Gly Pro Ala Glu Ala Thr Met Pro Trp Val Leu Ser Ala Arg
   435 440 445
   Thr Pro Val Ala Leu Arg Ala Gln Ala Ala Arg Leu His Thr His Leu
   450 455 460
   Asn Thr Ala Gly Gln Ser Pro Leu Asp Val Gly Tyr Ser Leu Ala Thr
   465 470 475 480
   Thr Arg Ser Ala Leu Pro His Arg Ala Ala Leu Val Ala Asp Asp Val
   485 490 495
   Pro Lys Leu Leu Ala Gly Leu Lys Ala Leu Ala Asp Gly Asp Asp Ala
   500 505 510
   Pro Thr Leu Cys Thr Gly Thr Thr Ser Gly Glu Arg Ala Thr Val Phe
   515 520 525
   Val Phe Pro Gly Gln Gly Ser Gln Trp Ile Gly Met Gly Arg Gln Leu
   530 535 540
   Leu Gln Thr Ser Glu Val Phe Ala Ala Ser Met Ala Asp Cys Ala Asp
   545 550 555 560
   Ala Leu Ala Pro His Leu Asp Trp Ser Leu Leu Asp Val Leu Arg Asn
   565 570 575
   Ala Ala Gly Ala Ser Gln Leu Asp Arg Asp Asp Val Val Gln Pro Ala
   580 585 590
   Leu Phe Ala Val Met Val Ser Leu Ala Glu Leu Trp Arg Ser Trp Gly
   595 600 605
   Val Arg Pro Glu Ala Val Val Gly His Ser Gln Gly Glu Ile Ala Ala
   610 615 620
   Ala Cys Val Ala Gly Ala Leu Ser Val Arg Asp Ala Ala Arg Val Val
   625 630 635 640
   Ala Val Arg Ser Arg Leu Leu Ala Ala Leu Ala Gly Arg Gly Ala Met
   645 650 655
   Ala Ser Leu Gln His Pro Val Glu Glu Val Arg Gln Ile Leu Leu Pro
   660 665 670
   Trp Arg Asp Arg Ile Gly Val Ala Gly Val Asn Gly Pro Ser Ser Thr
   675 680 685
   Leu Val Ser Gly Asp Arg Glu Ala Met Ala Glu Leu Leu Ala Glu Cys
       690                 695                 700
   Ala Arg Arg Glu Leu Arg Met Arg Arg Ile Pro Val Glu Tyr Ala Ser
   705                 710                 715                 720
   His Ser Pro His Ile Glu Asp Val Arg Asp Glu Leu Leu Ala Leu Leu
                   725                 730                 735
   Ala Ser Ile Glu Pro Arg Thr Gly Asn Ile Pro Val Tyr Ser Thr Thr
               740                 745                 750
   Thr Gly Glu Leu Leu Asp Arg Pro Met Asp Ala Asp Tyr Trp Tyr Arg
           755                 760                 765
   Asn Leu Arg Gln Pro Val Leu Phe Glu Ala Ala Val Glu Ala Leu Leu
       770                 775                 780
   Lys Arg Gly His Asn Ala Phe Ile Glu Ile Ser Pro His Pro Val Leu
   785                 790                 795                 800
   Thr Ala Ser Ile Gln Glu Thr Ala Ala Arg Ala Gly Arg Glu Val Val
                   805                 810                 815
   Ala Leu Gly Thr Leu Arg Arg Gly Glu Gly Gly Leu Arg Gln Ala Leu
               820                 825                 830
   Thr Ser Leu Ala Lys Ala His Val His Gly Val Ala Ala Asn Trp His
           835                 840                 845
   Ala Val Phe Ala Gly Thr Gly Ala Gln Arg Val Asp Leu Pro Thr Tyr
       850                 855                 860
   Ala Phe Gln Arg Gln Arg Tyr Trp Leu Asp Thr Lys Pro Ser Asp Leu
   865                 870                 875                 880
   Ala Met Pro Glu Gly Asp Val Ser Thr Ala Leu Arg Glu Lys Leu Arg
                   885                 890                 895
   Ser Ser Pro Gly Ala Asp Val Asp Ser Ala Thr Leu Thr Ile Ile Arg
               900                 905                 910
   Ala Gln Ala Ala Val Val Leu Gly His Ser Asp Pro Lys Glu Met Asp
           915                 920                 925
   Ser Asp Arg Thr Phe Lys Asp Leu Gly Phe Asp Ser Ser Thr Val Val
       930                 935                 940
   Glu Leu Cys Asp Arg Leu Asn Ala Ala Thr Gly Leu Arg Leu Ala Pro
   945                 950                 955                 960
   Ser Val Val Phe Asp Cys Pro Thr Pro Tyr Lys Leu Ala Arg Gln Val
                   965                 970                 975
   Arg Thr Leu Leu Leu Asp Glu Pro Val Pro Thr Thr Ser Pro Arg Thr
               980                 985                 990
   Glu Thr Glu Ala Asp Glu Pro Ile  Ala Val Ile Gly Met  Gly Cys Arg
           995                 1000                 1005
   Phe Pro  Gly Gly Val Ser Ser  Pro Glu Glu Leu Trp  Gln Leu Val
   1010 1015 1020
   Ala Ala Gly Arg Asp Val Val Ser Glu Phe Pro Ala Asp Arg Gly
   1025 1030 1035
   Trp Asp Pro Glu Arg Ala Gly Thr Ser His Val Arg Ala Gly Gly
   1040 1045 1050
   Phe Leu His Gly Ala Thr Asp Phe Asp Pro Gly Phe Phe Gly Ile
   1055 1060 1065
   Ser Pro Arg Glu Ala Leu Ala Met Asp Pro Gln Gln Arg Leu Leu
   1070 1075 1080
   Leu Glu Ile Ala Trp Glu Ala Ile Glu Arg Gly Gly Ile Asn Pro
   1085 1090 1095
   Gln Thr Leu His Gly Ser Gln Thr Gly Val Phe Val Gly Ala Thr
   1100 1105 1110
   Ser Leu Asp Tyr Gly Pro Arg Leu His Glu Ala Ser Asp Glu Ala
   1115 1120 1125
   Ala Gly Tyr Val Leu Thr Gly Ser Thr Thr Ser Val Ala Ser Gly
   1130 1135 1140
   Arg Val Ala Tyr Ser Phe Gly Leu Glu Gly Pro Ala Val Thr Val
   1145 1150 1155
   Asp Thr Ala Cys Ser Ser Ser Leu Val Ala Leu His Leu Ala Cys
   1160 1165 1170
   Gln Ser Leu Arg Ser Gly Glu Cys Asp Leu Ala Leu Ala Gly Gly
   1175 1180 1185
   Val Thr Val Met Ala Thr Pro Gly Met Phe Val Glu Phe Ser Arg
   1190 1195 1200
   Gln Arg Gly Leu Ala Pro Asp Gly Arg Cys Lys Ser Phe Ala Glu
   1205 1210 1215
   Ala Ala Asp Gly Thr Gly Trp Ser Glu Gly Ala Gly Leu Val Leu
   1220 1225 1230
   Leu Glu Arg Leu Ser Asp Ala Arg Arg Asn Gly His Asp Val Leu
   1235 1240 1245
   Ala Val Val Arg Gly Ser Ala Val Asn Gln Asp Gly Ala Ser Asn
   1250 1255 1260
   Gly Leu Thr Ala Pro Asn Gly Pro Ser Gln Arg Arg Val Ile Thr
   1265 1270 1275
   Gln Ala Leu Ala Asn Ala Lys Leu Ser Val Ser Asp Val Asp Ala
   1280 1285 1290
   Val Glu Ala His Gly Thr Gly Thr Arg Leu Gly Asp Pro Ile Glu
   1295 1300 1305
   Ala Gln Ala Leu Ile Ala Thr Tyr Gly Gln Gly Arg Gly Pro Glu
   1310 1315 1320
   Arg Pro Leu Trp Leu Gly Ser Val Lys Ser Asn Ile Gly His Thr
   1325 1330 1335
   Gln Ala Ala Ala Gly Val Ala Gly Val Ile Lys Met Val Met Ala
   1340 1345 1350
   Met Arg Tyr Gly Glu Leu Pro Ala Thr Leu His Val Asp Glu Pro
   1355 1360 1365
   Ser Ser Gln Val Asp Trp Ser Ala Gly Met Val Gln Val Leu Thr
   1370 1375 1380
   Glu His Val Pro Trp Pro Asp Asn Ser Arg Pro Arg Arg Val Gly
   1385 1390 1395
   Val Ser Ser Phe Gly Ile Ser Gly Thr Asn Ala His Val Ile Leu
   1400 1405 1410
   Glu Gln Ser Pro Thr Ala Ser Ser Glu Phe Val Glu His Ser Gly
   1415 1420 1425
   Pro Asp Ser Glu Ser Ala Val Asp Val Pro Val Val Pro Trp Val
   1430 1435 1440
   Val Ser Gly Lys Thr Pro Glu Ala Leu Ser Ala Gln Ala Asp Asn
   1445 1450 1455
   Leu Val Ser Tyr Leu Asp Asp Arg Pro Asn Val Ser Ala Leu Asn
   1460 1465 1470
   Val Ala Tyr Ser Leu Ala Ser Glu Arg Ala Ala Leu Asp Glu Arg
   1475 1480 1485
   Ala Val Val Leu Gly Ala Asp Arg Glu Ala Leu Leu Ser Gly Leu
   1490 1495 1500
   Lys Ala Leu Ala Ala Gly His Glu Asp Pro Gly Val Ala Ser Gly
   1505 1510 1515
   Ser Leu Val Ser Gly Gly Val Gly Phe Val Phe Ser Gly Gln Gly
   1520 1525 1530
   Gly Gln Trp Ser Gly Met Gly Arg Gly Leu Tyr Arg Ala Phe Pro
   1535 1540 1545
   Val Phe Ala Ala Ala Phe Asp Glu Ala Cys Ala Glu Leu Asp Ala
   1550 1555 1560
   His Leu Gly Gln Glu Val Gly Val Arg Asp Val Ala Phe Gly Ser
   1565 1570 1575
   Asp Ala Gln Leu Leu Glu Arg Thr Leu Trp Ala Gln Ser Gly Leu
   1580 1585 1590
   Phe Ala Leu Gln Val Gly Leu Leu Arg Leu Leu Gly Ser Trp Gly
   1595 1600 1605
   Val Arg Pro Gly Ala Val Leu Gly His Ser Val Gly Glu Leu Ala
   1610 1615 1620
   Ala Ala His Ala Ala Gly Val Leu Ser Leu Pro Asp Ala Ala Arg
   1625 1630 1635
   Leu Val Ala Gly Arg Ala Arg Leu Met Gln Ala Met Pro Asp Gly
   1640 1645 1650
   Gly Gly Met Leu Ala Val Ala Thr Ser Glu Thr Gln Val Glu Pro
   1655 1660 1665
   Met Leu Asp Gly Val Arg Asp Arg Ile Gly Ile Ala Ala Ile Asn
   1670 1675 1680
   Ala Pro Glu Ser Val Val Leu Ser Gly Asp Arg Glu Leu Leu Ala
   1685 1690 1695
   Glu Val Ala Asp Gln Leu Asn Asp Gln Gly Cys Arg Thr Arg Trp
   1700 1705 1710
   Leu Gln Val Ser His Ala Phe His Ser Tyr Arg Met Glu Pro Met
   1715 1720 1725
   Leu Asp Glu Phe Ala Gln Ile Ala Gly Ser Val Asp Phe Arg Arg
   1730 1735 1740
   Cys Glu Leu Pro Ile Ile Ser Thr Leu Thr Gly Asn Leu Asp Asp
   1745 1750 1755
   Val Gly Val Met Ala Thr Pro Glu Tyr Trp Val Arg Gln Val Arg
   1760 1765 1770
   Glu Pro Val Arg Phe Ala Asp Gly Val Gln Ser Leu Val Glu Gln
   1775 1780 1785
   Asp Val Ala Thr Val Val Glu Leu Gly Pro Asp Ala Ile Leu Ser
   1790 1795 1800
   Ala Leu Ile Pro Asp Cys His Ser Trp Gly Asp Gln Thr Val Pro
   1805 1810 1815
   Ile Pro Leu Leu Arg Lys Asp Arg Ala Glu Pro Glu Thr Val Val
   1820 1825 1830
   Ala Ala Val Ala Arg Ala His Thr Arg Gly Val Gln Val Asp Trp
   1835 1840 1845
   Ser Ala Phe Phe Ala Gly Thr Gly Ala Gly Arg Val Glu Leu Pro
   1850 1855 1860
   Thr Tyr Ala Phe Gln Arg Gln Arg Tyr Trp Leu Glu Ser Ser Val
   1865 1870 1875
   Ser Gly Asp Val Thr Gly Ile Gly Leu Ala Gly Ala Glu His Pro
   1880 1885 1890
   Leu Leu Gly Ala Val Val Val Leu Ala Asp Gly Asp Gly Met Val
   1895 1900 1905
   Leu Thr Gly Arg Leu Ser Val Gly Thr His Arg Trp Leu Ala Glu
   1910 1915 1920
   His Arg Val Leu Gly Glu Val Val Val Pro Gly Thr Ala Ile Leu
   1925 1930 1935
   Glu Met Val Leu His Ala Gly Ala Arg Val Gly Cys Gly Arg Val
   1940 1945 1950
   Glu Glu Leu Thr Leu Glu Ala Pro Leu Val Val Pro Glu Arg Asp
   1955 1960 1965
   Ala Ile Glu Ile Gln Leu Leu Val Asn Ala Pro Asp Asp Lys Gly
   1970 1975 1980
   Arg Arg Ser Val Ser Leu His Ser Arg Pro Ala Gly Gly Ser Gly
   1985 1990 1995
   Gly Gly Gly Trp Thr Arg His Ala Thr Gly Glu Leu Val Val Ala
   2000 2005 2010
   Gly Thr Gly Gly Gly Ala Val Thr Gly Trp Ser Thr Glu Gly Ala
   2015 2020 2025
   Glu Pro Val Ala Leu Gly Glu Phe Tyr Val Val Gln Ala Gly Asn
   2030 2035 2040
   Gly Phe Glu Tyr Gly Pro Leu Phe Gln Gly Leu Arg Ala Ala Trp
   2045 2050 2055
   Arg Arg Gly Gly Glu Val Leu Ala Glu Val Ala Leu Pro Ala Ala
   2060 2065 2070
   Ala Gly Ala Met Ala Gly Phe Leu Ile Asn Pro Ala Leu Leu Asp
   2075 2080 2085
   Ala Ala Leu Gln Ala Ser Ala Leu Gly Asp Arg Pro Ala Glu Gly
   2090 2095 2100
   Gly Ala Trp Leu Pro Phe Ser Phe Thr Gly Val Glu Leu Ser Gly
   2105 2110 2115
   Gln Gly Gly Thr Ile Ser Arg Ala Arg Val Glu Ser Thr Arg Pro
   2120 2125 2130
   Asp Ala Val Ser Val Ala Val Met Asp Glu Gly Gly Arg Leu Leu
   2135 2140 2145
   Ala Ser Ile Asp Ser Leu Arg Leu Arg Pro Val Ser Ser Val Arg
   2150 2155 2160
   Leu Ala Asn Arg Asp Val Val Gly Asp Ala Leu Phe Glu Val Thr
   2165 2170 2175
   Trp Glu Pro Val Ala Thr Arg Ser Thr Val Ser Gly Arg Trp Ala
   2180 2185 2190
   Leu Leu Gly Asp Ala Val Gly Gly Met Ala Gly Leu Ile Gly Leu
   2195 2200 2205
   Ala Pro Gly Ser Val Asp Arg Cys Ala Gly Leu Ala Glu Leu Ala
   2210 2215 2220
   Gly Asn Leu Asp Ser Gly Ala Leu Val Ala Asp Val Val Val Tyr
   2225 2230 2235
   Cys Ala Gly Glu Gln Ala Asp Pro Asp Ala Gly Val Ala Ala Leu
   2240 2245 2250
   Ala Glu Thr Arg Glu Met Leu Ala Leu Val Gln Ser Trp Leu Ala
   2255 2260 2265
   Glu Glu Arg Leu Ala Gly Ser Arg Leu Val Val Val Thr Cys Gly
   2270 2275 2280
   Ala Val Thr Thr Ala Ala Gly Asp Gly Ala Ser Lys Leu Ala His
   2285 2290 2295
   Ala Pro Leu Trp Gly Leu Leu Arg Ser Ala Gln Ser Glu Asn Pro
   2300 2305 2310
   Gly Arg Phe Val Leu Val Asp Val Asp Gly Thr Ala Glu Ser Trp
   2315 2320 2325
   Arg Ala Leu Pro Ser Ala Val Gly Ser Met Gln Pro Gln Leu Ala
   2330 2335 2340
   Val Arg Lys Gly Val Val Thr Val Pro Arg Val Ala Ser Val Pro
   2345 2350 2355
   Gly Pro Val Glu Val Pro Ala Val Val Ala Gly Pro Asp Arg Thr
   2360 2365 2370
   Val Leu Ile Ser Gly Gly Thr Gly Leu Leu Gly Gly Val Val Ala
   2375 2380 2385
   Arg His Leu Val Ala Glu Arg Gly Val Arg Arg Val Val Leu Thr
   2390 2395 2400
   Gly Arg Arg Gly Trp Asp Ala Pro Gly Ile Thr Glu Leu Val Gly
   2405 2410 2415
   Glu Leu Glu Gly Phe Gly Ala Val Val Asp Val Val Ala Cys Asp
   2420 2425 2430
   Val Ala Asp Arg Ala Gly Leu Glu Gly Leu Leu Ala Ala Val Pro
   2435 2440 2445
   Ala Glu Phe Pro Leu Cys Gly Val Val His Ala Ala Gly Val Leu
   2450 2455 2460
   Ala Asp Gly Val Ile Glu Ser Leu Thr Pro Glu Asp Val Gly Ala
   2465 2470 2475
   Val Phe Gly Pro Lys Ala Ala Gly Ala Trp Asn Leu His Glu Leu
   2480 2485 2490
   Thr Arg Asp Met Asp Leu Ser Phe Phe Ala Leu Phe Ser Ser Leu
   2495 2500 2505
   Ser Gly Val Thr Gly Ala Ala Gly Gln Gly Asn Tyr Ala Ala Ala
   2510 2515 2520
   Asn Thr Phe Leu Asp Ala Leu Ala His Tyr Arg Arg Ala Gln Gly
   2525 2530 2535
   Leu Pro Ala Val Ser Leu Ala Trp Gly Leu Trp Glu Gln Ser Ser
   2540 2545 2550
   Gly Met Thr Gly Arg Leu Ser Asp Val Asp Arg Ser Arg Ile Ala
   2555 2560 2565
   Arg Ser Ser Pro Pro Leu Ser Thr Lys Asp Gly Leu Arg Leu Phe
   2570 2575 2580
   Asp Ala Gly Leu Ala Leu Asp Arg Ala Ala Val Val Pro Ala Arg
   2585 2590 2595
   Leu Asp Arg Ala Phe Leu Ala Glu Gln Ala Arg Ser Gly Thr Leu
   2600 2605 2610
   Pro Ala Met Leu Thr Ala Leu Val Pro Thr Ile Thr Ser Ile Arg
   2615 2620 2625
   Arg Ser Ser Gly Thr Asp Leu Ala Asp Glu Asp Ala Leu Leu Gly
   2630 2635 2640
   Val Val Arg Glu His Ala Ala Arg Val Leu Gly Tyr Ser Gly Ala
   2645 2650 2655
   Ala Glu Val Gly Val Glu Arg Ala Phe Arg Asp Leu Gly Phe Asp
   2660 2665 2670
   Ser Leu Ser Gly Val Glu Leu Arg Asn Arg Leu Ala Gly Val Leu
   2675 2680 2685
   Gly Ala Arg Leu Pro Ala Thr Ala Val Phe Asp Tyr Pro Thr Pro
   2690 2695 2700
   Arg Ala Leu Ala Arg Phe Leu His Gln Glu Leu Ala Gly Glu Val
   2705 2710 2715
   Gly Thr Thr Pro Ala Pro Val Thr Thr Thr Thr Ala Ser Val Glu
   2720 2725 2730
   Asp Asp Leu Val Ala Ile Val Gly Met Gly Cys Arg Tyr Pro Gly
   2735 2740 2745
   Gly Val Ser Ser Pro Glu Glu Leu Trp Arg Leu Val Ala Gly Gly
   2750 2755 2760
   Val Asp Ala Val Ala Asp Phe Pro Asp Asp Arg Gly Trp Asp Leu
   2765 2770 2775
   Ala Gly Leu Phe Asp Pro Asp Pro Asp Arg Phe Gly Thr Ser Tyr
   2780 2785 2790
   Val Arg Glu Gly Gly Phe Leu Arg Asp Ala Ala Glu Phe Asp Ala
   2795 2800 2805
   Ala Phe Phe Gly Ile Ser Pro Arg Glu Ala Leu Ala Met Asp Pro
   2810 2815 2820
   Gln Gln Arg Leu Leu Leu Glu Leu Ser Trp Glu Ala Val Glu Arg
   2825 2830 2835
   Ala Gly Ile Asp Pro Gly Ser Leu Arg Gly Ser Arg Thr Gly Val
   2840 2845 2850
   Phe Ala Gly Leu Met Tyr His Asp Tyr Ala Gly Arg Phe Ala Ala
   2855 2860 2865
   Gly Val Pro Glu Gly Phe Glu Gly Tyr Leu Gly Asn Gly Ser Ala
   2870 2875 2880
   Gly Ser Val Ala Ser Gly Arg Val Ala Tyr Ser Phe Gly Phe Glu
   2885 2890 2895
   Gly Pro Ala Val Thr Val Asp Thr Ala Cys Ser Ser Ser Leu Val
   2900 2905 2910
   Ala Leu His Leu Ala Gly Gln Ser Leu Arg Ser Gly Glu Cys Asp
   2915 2920 2925
   Leu Ala Leu Ala Gly Gly Val Thr Val Met Ala Thr Pro Ala Thr
   2930 2935 2940
   Phe Val Glu Phe Ser Arg Gln Arg Gly Leu Ala Pro Asp Gly Arg
   2945 2950 2955
   Cys Lys Ser Phe Ala Glu Ala Ala Asp Gly Thr Gly Trp Gly Glu
   2960 2965 2970
   Gly Ala Gly Leu Val Leu Leu Glu Arg Leu Ser Asp Ala Arg Arg
   2975 2980 2985
   Asn Gly His Arg Val Leu Ala Val Val Arg Gly Ser Ala Val Asn
   2990 2995 3000
   Gln Asp Gly Ala Ser Asn Gly Leu Thr Ala Pro Asn Gly Pro Ser
   3005 3010 3015
   Gln Gln Arg Val Ile Thr Gln Ala Leu Thr Ser Ala Gly Leu Ser
   3020 3025 3030
   Val Ser Asp Val Asp Ala Val Glu Ala His Gly Thr Gly Thr Arg
   3035 3040 3045
   Leu Gly Asp Pro Ile Glu Ala Gln Ala Leu Ile Ala Thr Tyr Gly
   3050 3055 3060
   Arg Asp Arg Asp Pro Asp Arg Pro Leu Trp Leu Gly Ser Met Lys
   3065 3070 3075
   Ser Asn Ile Gly His Thr Gln Ala Ala Ala Gly Val Ala Gly Val
   3080 3085 3090
   Ile Lys Met Val Met Ala Met Arg His Gly Glu Leu Pro Arg Thr
   3095 3100 3105
   Leu His Val Gly Glu Pro Thr Ser Glu Val Asp Trp Ser Ala Gly
   3110 3115 3120
   Ser Val Gln Leu Leu Thr Glu Asn Thr Pro Trp Pro Asp Ser Gly
   3125 3130 3135
   His Pro Arg Arg Ala Gly Val Ser Ser Phe Gly Ile Ser Gly Thr
   3140 3145 3150
   Asn Ala His Val Ile Leu Glu Gln Ser Pro Thr Ala Ser Ser Glu
   3155 3160 3165
   Phe Val Glu His Ser Gly Pro Asp Ser Glu Ser Ala Val Asn Val
   3170 3175 3180
   Pro Val Val Pro Trp Val Val Ser Gly Lys Thr Pro Glu Ala Leu
   3185 3190 3195
   Ser Ala Gln Ala Asp Thr Leu Val Ser Tyr Leu Asp Asp Arg Ser
   3200 3205 3210
   Asp Val Ser Ser Arg Asp Val Gly Tyr Ser Leu Ala Met Thr Arg
   3215 3220 3225
   Ser Ala Leu Asp Glu Arg Ala Val Val Leu Gly Ser Asp Arg Glu
   3230 3235 3240
   Thr Leu Leu Ser Gly Leu Lys Ala Leu Ala Ala Gly His Glu Ala
   3245 3250 3255
   Thr Gly Val Val Thr Gly Ser Val Gly Ser Gly Gly Arg Pro Gly
   3260 3265 3270
   Phe Val Phe Ala Gly Gln Gly Gly Gln Trp Leu Gly Met Gly Arg
   3275 3280 3285
   Gly Leu Tyr Arg Ala Phe Pro Val Phe Ala Asp Ala Phe Asp Glu
   3290 3295 3300
   Ala Cys Ala Gly Leu Asp Ala His Leu Gly Gln Lys Val Gly Val
   3305 3310 3315
   Arg Asp Val Val Phe Gly Ser Asp Ala Gln Leu Leu Asp Arg Thr
   3320 3325 3330
   Leu Trp Ala Gln Ser Gly Leu Phe Ala Leu Gln Val Gly Leu Leu
   3335 3340 3345
   Lys Leu Leu Gly Ser Trp Gly Val Arg Pro Val Val Val Leu Gly
   3350 3355 3360
   His Ser Val Gly Glu Leu Ala Ala Ala Phe Ala Ala Gly Val Leu
   3365 3370 3375
   Ser Met Ala Glu Ala Ala Arg Leu Val Ala Gly Arg Ala Arg Leu
   3380 3385 3390
   Met Gln Ala Leu Pro Ser Gly Gly Ala Met Leu Ala Val Ala Thr
   3395 3400 3405
   Ser Glu Thr Gln Val Glu Pro Leu Leu Asp Gly Val Arg Asp Arg
   3410 3415 3420
   Ile Asp Ile Ala Ala Ile Asn Ala Pro Glu Ser Ile Val Leu Ser
   3425 3430 3435
   Gly Asp Arg Glu Leu Leu Thr Glu Ala Ala Asp Gln Leu His Asp
   3440 3445 3450
   Gln Gly Cys Arg Thr Arg Trp Leu Gln Val Ser His Ala Phe His
   3455 3460 3465
   Ser Pro Gln Met Asp Pro Met Leu Asp Glu Phe Ala Asp Ile Ala
   3470 3475 3480
   Arg Thr Val Asp Phe Arg Gly Ser Glu Leu Pro Val Val Ser Thr
   3485 3490 3495
   Leu Thr Gly Ala Leu Asp Asp Ser Gly Leu Met Ala Thr Pro Glu
   3500 3505 3510
   Tyr Trp Val Arg Gln Val Arg Glu Pro Val Arg Phe Ala Asp Gly
   3515 3520 3525
   Val Arg Ala Leu Val Glu His Asp Val Ala Thr Val Val Glu Leu
   3530 3535 3540
   Gly Pro Asp Gly Ala Leu Ser Ala Leu Ile Gln Glu Cys Ala Ala
   3545 3550 3555
   Glu Phe Asp Gln Ser Arg Arg Val Ala Ala Val Pro Ala Met Arg
   3560 3565 3570
   Arg Ser Gln Asp Glu Ala Gln Lys Val Met Thr Ala Leu Ala Gln
   3575 3580 3585
   Val His Val Arg Gly Gly Ala Val Asp Trp Arg Ser Val Phe Ala
   3590 3595 3600
   Gly Thr Gly Ser Lys Gln Val Glu Leu Pro Thr Tyr Ala Phe Gln
   3605 3610 3615
   Arg Gln Arg Tyr Trp Leu Asn Ala Val His Glu Ser Ser Ala Gly
   3620 3625 3630
   Asp Met Gly Arg Arg Ile Glu Thr Glu Phe Trp Ser Ala Val Glu
   3635 3640 3645
   His Glu Asp Val Thr Ser Leu Ala Asn Ile Leu Gly Ile Val Asp
   3650 3655 3660
   Asp Gly Ala Ala Val Asp Ser Leu Arg Asn Ala Leu Pro Val Leu
   3665 3670 3675
   Ala Gly Trp Gln Arg Thr Arg Asn Asp Glu Ser Ile Met Asp Arg
   3680 3685 3690
   Gln Cys Tyr Arg Ile Gly Trp Arg Gln Val Ala Gly Leu Pro Pro
   3695 3700 3705
   Arg Gly Thr Val Phe Gly Thr Trp Leu Val Phe Ala Pro His Gly
   3710 3715 3720
   Trp Ser Gly Glu Pro Gln Val Ala Asn Cys Val Ala Ala Leu Arg
   3725 3730 3735
   Ala Ser Gly Ala Ser Val Val Leu Val Glu Ala Asp Pro Asp Pro
   3740 3745 3750
   Val Val Phe Gly Asp Arg Val Arg Thr Leu Cys Ser Asp Ser Pro
   3755 3760 3765
   Asp Leu Val Gly Val Leu Ser Met Leu Cys Leu Glu Glu Ser Ala
   3770 3775 3780
   Ile Pro Gly Phe Ser Ala Val Ser Arg Gly Phe Ala Leu Thr Val
   3785 3790 3795
   Glu Leu Val Arg Ala Leu Ala Ala Ala Gly Ala Asp Ala Arg Leu
   3800 3805 3810
   Trp Leu Leu Thr Cys Gly Gly Val Ser Val Gly Asp Val Pro Val
   3815 3820 3825
   Arg Pro Glu Gln Ala Leu Val Trp Gly Leu Gly Arg Val Ala Gly
   3830 3835 3840
   Leu Glu His Pro Asp Trp Trp Gly Gly Leu Ile Asp Ile Pro Val
   3845 3850 3855
   Leu Phe Asp Glu Asp Ala Gln Glu Arg Leu Ser Ile Val Leu Ala
   3860 3865 3870
   Gly Leu Gly Glu Glu Glu Val Ala Ile Arg Ser Asp Gly Val Phe
   3875 3880 3885
   Ala Arg Arg Leu Val Arg His Gly Val Ser Ala Gly Val Lys Lys
   3890 3895 3900
   Ala Trp Arg Pro Arg Gly Ser Val Leu Val Thr Gly Gly Thr Gly
   3905 3910 3915
   Gly Leu Gly Ala His Ala Ala Arg Trp Leu Ala Asp Ala Gly Ala
   3920 3925 3930
   Glu His Val Val Met Val Ser Arg Arg Gly Glu Gln Ala Pro Ser
   3935 3940 3945
   Ala Glu Lys Leu Arg Thr Glu Leu Glu Asp Leu Gly Thr Arg Val
   3950 3955 3960
   Ser Ile Leu Ser Cys Asp Val Thr Asp Arg Glu Ala Leu Ala Glu
   3965 3970 3975
   Val Leu Lys Ala Leu Pro Ala Glu Tyr Pro Leu Thr Ala Val Val
   3980 3985 3990
   His Thr Ala Gly Val Ile Glu Thr Gly Asp Ala Ala Ser Met Ser
   3995 4000 4005
   Leu Ala Asp Phe Asp Asp Val Leu Ser Ala Lys Val Ala Gly Ala
   4010 4015 4020
   Ala Asn Leu Asp Ala Leu Leu Ala Asp Val Glu Leu Asp Ala Phe
   4025 4030 4035
   Val Leu Phe Ser Ser Val Ser Gly Val Trp Gly Ala Gly Gly Gln
   4040 4045 4050
   Gly Ala Tyr Ala Ala Ala Asn Ala Tyr Leu Asp Ala Leu Ala Glu
   4055 4060 4065
   Glu Arg Arg Ser Arg Gly Leu Val Ala Thr Ala Val Ala Trp Gly
   4070 4075 4080
   Pro Trp Ala Gly Glu Gly Met Ala Ala Gly Glu Thr Gly Asp Gln
   4085 4090 4095
   Leu Arg Arg Tyr Gly Leu Ser Pro Met Gly Pro Gln Tyr Ala Ile
   4100 4105 4110
   Ala Gly Ile Arg Arg Ala Val Glu Gln Asp Glu Ile Ser Leu Val
   4115 4120 4125
   Val Ala Asp Val Asp Trp Ala Arg Phe Ser Ala Gly Phe Leu Ala
   4130 4135 4140
   Ala Arg Pro Arg Pro Leu Leu Asn Glu Leu Thr Glu Val Lys Glu
   4145 4150 4155
   Leu Leu Val Asn Ala Gln Ser Glu Val Gly Val Val Ala Glu Ala
   4160 4165 4170
   Ser Val Ala Trp Arg Gln Arg Leu Ala Ala Ala Pro Arg Pro Ala
   4175 4180 4185
   Gln Glu Gln Leu Ile Leu Glu Leu Val Arg Gly Glu Thr Ala Leu
   4190 4195 4200
   Val Leu Gly His Pro Gly Ala Glu Ala Val Ala Pro Glu Arg Ala
   4205 4210 4215
   Phe Lys Asp Ser Gly Phe Asp Ser Gln Ala Ala Val Glu Leu Arg
   4220 4225 4230
   Val Arg Leu Asn Arg Ala Thr Gly Leu Gln Leu Pro Ser Thr Ile
   4235 4240 4245
   Ile Phe Ser His Pro Thr Pro Ala Glu Leu Ala Ala Glu Leu Arg
   4250 4255 4260
   Ala Arg Leu Leu Pro Glu Ser Ala Gly Val Asp Ile Ser Glu Glu
   4265 4270 4275
   Asp Glu Ala Arg Ile Arg Ala Ala Leu Thr Ser Ile Pro Phe Ala
   4280 4285 4290
   Ala Leu Arg Glu Ala Asp Leu Val Asn Arg Leu Leu Ala Leu Ala
   4295 4300 4305
   Gly His Pro Val Asp Ser Gly Ser Ser Pro Asp Asp Ala Val Ala
   4310 4315 4320
   Thr Ser Ile Asp Ala Met Asp Val Ala Asp Leu Val Glu Ala Ala
   4325 4330 4335
   Leu Gly Glu Arg Glu Ser
   4340
<210> 4
   <211> 2149
   <212> PRT
   <213> Saccharopolyspora sp. NRRL30141
<400> 4
   Val Thr Thr Ser Tyr Glu Glu Val Val Glu Ala Leu Arg Ala Ser Leu
   1               5                   10                  15
   Lys Glu Asn Glu Arg Leu Arg Arg Gly Arg Asp Arg Phe Ala Ala Glu
               20                  25                  30
   Lys Gly Asp Pro Ile Ala Ile Val Ala Met Ser Cys Arg Tyr Pro Gly
           35                  40                  45
   Gln Val Ser Ser Pro Glu Asp Leu Trp Gln Leu Ala Ala Gly Gly Val
       50                  55                  60
   Asp Ala Ile Ser Glu Val Pro Gly Asp Arg Gly Trp Asp Leu Ala Gly
   65                  70                  75                  80
   Val Phe Asp Pro Asp Ser Asp Arg Pro Gly Thr Ser Tyr Ala Cys Ala
                   85                  90                  95
   Gly Gly Phe Leu Gln Gly Val Ser Glu Phe Asp Ala Gly Phe Phe Gly
               100                 105                 110
   Ile Ser Pro Arg Glu Ala Leu Ala Met Asp Pro Gln Gln Arg Leu Leu
           115                 120                 125
   Leu Glu Val Ala Trp Glu Val Phe Glu Arg Ala Gly Leu Glu Gln Arg
       130                 135                 140
   Ser Thr Arg Gly Ser Arg Val Gly Val Phe Val Gly Thr Asn Gly Gln
   145                 150                 155                 160
   Asp Tyr Ala Ser Trp Leu Arg Thr Pro Pro Ser Glu Val Ala Gly His
                   165                 170                 175
   Val Leu Thr Gly Gly Ala Ala Ala Ile Leu Ser Gly Arg Val Ala Tyr
               180                 185                 190
   Ser Phe Gly Phe Glu Gly Pro Ala Val Thr Val Asp Thr Ala Cys Ser
           195                 200                 205
   Ser Ser Leu Val Ala Leu His Leu Ala Gly Gln Ala Leu Arg Ala Gly
       210                 215                 220
   Glu Cys Asp Leu Ala Leu Ala Gly Gly Val Thr Val Met Ser Thr Pro
   225                 230                 235                 240
   Lys Ala Phe Leu Glu Phe Ser Arg Gln Arg Gly Leu Ala Ala Asp Gly
   245 250 255
   Arg Cys Lys Ser Phe Ala Ala Ala Ala Asp Gly Thr Gly Trp Gly Glu
   260 265 270
   Gly Ala Gly Leu Leu Leu Leu Glu Arg Leu Ser Asp Ala Arg Arg Asn
   275 280 285
   Gly His Arg Val Leu Ala Val Val Arg Gly Ser Ala Val Asn Gln Asp
   290 295 300
   Gly Ala Ser Asn Gly Leu Thr Ala Pro Asn Gly Ser Ser Gln Ala Arg
   305 310 315 320
   Val Ile Thr Gln Ala Leu Ala Ser Ala Gly Leu Ser Val Ser Asp Val
   325 330 335
   Asp Ala Val Glu Ala His Gly Thr Gly Thr Arg Leu Gly Asp Pro Ile
   340 345 350
   Glu Ala Gln Ala Leu Ile Ala Thr Tyr Gly Arg Asp Arg Asp Pro Ala
   355 360 365
   Arg Pro Leu Trp Leu Gly Ser Val Lys Ser Asn Ile Gly His Thr Gln
   370 375 380
   Ala Ala Ala Gly Val Ala Gly Val Ile Lys Met Val Met Ala Met Arg
   385 390 395 400
   His Gly Gln Leu Pro Arg Thr Leu His Val Asp Ala Pro Ser Pro Glu
   405 410 415
   Val Asp Trp Ser Ala Gly Thr Val Gln Leu Leu Thr Glu Asn Met Leu
   420 425 430
   Trp Pro Glu Ser Gly Arg Val Arg Arg Ala Gly Val Ser Ser Phe Gly
   435 440 445
   Ile Ser Gly Thr Asn Ala His Val Ile Leu Glu Gln Pro Thr Gly Glu
   450 455 460
   Thr Arg Gln Ser Ala Gly Pro Asp Ser Gly Ser Val Val Asp Val Pro
   465 470 475 480
   Val Val Pro Trp Met Val Ser Gly Lys Thr Pro Asp Ala Leu Gly Ala
   485 490 495
   Gln Ala Asp Thr Leu Met Ser Tyr Leu Asp Asp Arg Val Asp Val Pro
   500 505 510
   Ser Leu Asp Ile Ala Tyr Ser Leu Ala Met Thr Arg Ser Ala Leu Asp
   515 520 525
   Glu Arg Ala Val Val Leu Gly Pro Asp Arg Glu Thr Leu Leu Ser Gly
   530 535 540
   Leu Lys Ala Leu Ser Ala Gly His Glu Ala Ser Gly Val Val Thr Gly
   545 550 555 560
   Ser Val Gly Thr Gly Gly Arg Ile Gly Phe Val Phe Ser Gly Gln Gly
   565 570 575
   Gly Gln Trp Leu Gly Met Gly Arg Gly Leu Tyr Arg Ala Phe Pro Val
   580 585 590
   Phe Ala Ala Ala Phe Asp Glu Ala Cys Ala Glu Leu Glu Ala His Leu
   595 600 605
   Gly Gln Glu Val Gly Val Arg Asp Val Val Phe Gly Ser Asp Ala Gln
   610 615 620
   Leu Leu Asn Arg Thr Leu Trp Ala Gln Ser Gly Leu Phe Ala Leu Gln
   625 630 635 640
   Val Gly Leu Leu Lys Leu Leu Asp Ser Trp Gly Val Arg Pro Ser Ala
   645 650 655
   Val Leu Gly His Ser Val Gly Glu Leu Ala Ala Ala Phe Ala Ala Gly
   660 665 670
   Val Leu Ser Leu Ser Asp Ala Ala Arg Leu Val Ala Gly Arg Ala Arg
   675 680 685
   Leu Met Gln Ala Leu Pro Ser Gly Gly Gly Met Leu Ala Val Ala Ala
   690 695 700
   Gly Glu Glu Gln Leu Arg Pro Leu Leu Ala Asp His Gly Asp Arg Val
   705 710 715 720
   Gly Leu Ala Ala Val Asn Val Ala Glu Ser Val Val Leu Ser Gly Asp
   725 730 735
   Arg Asp Val Leu Asp Asp Ile Ala Gly Arg Leu Asp Gly Gln Gly Val
   740 745 750
   Arg Thr Arg Trp Leu Arg Val Ser His Ala Phe His Ser Tyr Arg Met
   755 760 765
   Asp Pro Met Leu Asp Glu Phe Ala Glu Ile Ala Arg Ala Val Asp Tyr
   770 775 780
   Arg Arg Cys Glu Leu Pro Ile Val Ser Thr Leu Thr Gly Lys Leu Asp
   785 790 795 800
   Asp Ala Gly Arg Met Ser Gly Pro Asp Tyr Trp Val Arg Gln Val Arg
   805 810 815
   Glu Pro Val Arg Phe Ala Asp Gly Ala Gln Ala Leu Val Glu His Asp
   820 825 830
   Val Ala Thr Ile Val Glu Ile Gly Pro Asp Gly Ala Leu Ser Ala Leu
   835 840 845
   Ile Gln Glu Cys Val Ala Ala Ser Asp Gln Ser Arg Arg Val Ala Ala
   850 855 860
   Val Pro Ala Met Arg Arg Asn Arg Asp Glu Ala Gln Asn Leu Thr Thr
   865 870 875 880
   Ala Leu Ala Gln Val His Val Arg Gly Gly Ala Val Asp Trp Arg Ser
   885 890 895
   Phe Phe Ala Gly Thr Gly Ala Lys Gln Val Glu Leu Pro Thr Tyr Ala
   900 905 910
   Phe Gln Arg Gln Arg Tyr Trp Leu Glu Pro Ser Asp Ser Gly Asp Val
   915 920 925
   Thr Gly Ala Gly Leu Ala Gly Ala Glu His Pro Leu Leu Gly Ala Val
   930 935 940
   Val Pro Val Ala Gly Gly Asp Glu Val Leu Leu Thr Gly Arg Ile Ser
   945 950 955 960
   Val Gly Thr His Pro Trp Leu Ala Glu His Arg Val Leu Gly Glu Val
   965 970 975
   Ile Val Pro Gly Thr Ala Leu Leu Glu Ile Ala Leu His Ala Gly Glu
   980 985 990
   Arg Leu Gly Cys Glu Arg Val Glu Glu Leu Thr Leu Glu Ala Pro Leu
   995 1000 1005
   Val Leu Pro Glu Arg Gly Ala Met Gln Val Gln Leu Arg Val Gly
   1010 1015 1020
   Ala Pro Glu Asn Ser Gly Arg Arg Pro Met Val Leu Tyr Ser Arg
   1025 1030 1035
   Pro Glu Gly Ala Ala Asp His Asp Trp Thr Arg His Ala Thr Gly
   1040 1045 1050
   Arg Leu Ala Pro Gly Gly Gly Glu Ala Ala Gly Asp Leu Ala Asp
   1055 1060 1065
   Trp Pro Ala Pro Gly Ala Leu Pro Val Asp Leu Asp Glu Phe Tyr
   1070 1075 1080
   Arg Asp Leu Ala Glu His Gly Leu Glu Tyr Gly Pro Ile Phe Gln
   1085 1090 1095
   Gly Leu Lys Ala Ala Trp Arg Gln Gly Asp Glu Val Tyr Ala Glu
   1100 1105 1110
   Ala Ala Leu Pro Gly Thr Glu Asp Ser Gly Phe Gly Val His Pro
   1115 1120 1125
   Ala Leu Leu Asp Ala Ala Leu His Ala Thr Ala Val Arg Asp Met
   1130 1135 1140
   Asp Gly Ala Trp Leu Pro Phe Gln Trp Glu Gly Val Cys Leu His
   1145 1150 1155
   Ala Arg Ala Ala Ser Ala Leu Arg Val Arg Val Val Pro Ala Gly
   1160 1165 1170
   Asp Asp Ala Lys Ser Leu Leu Val Cys Asp Gly Thr Gly Arg Pro
   1175 1180 1185
   Val Ile Ser Val Asp Arg Leu Val Phe Arg Ser Ala Ala Ala Gly
   1190 1195 1200
   Arg Thr Gly Ala Arg Arg Gln Ala His Arg Ala Arg Leu Tyr Arg
   1205 1210 1215
   Leu Gly Trp Pro Thr Val Gln Leu Pro Thr Ser Ala Gln Pro Pro
   1220 1225 1230
   Ser Cys Val Leu Leu Gly Thr Ser Glu Val Ser Ser Asp Met Gln
   1235 1240 1245
   Val Tyr Pro Asp Leu Arg Ser Leu Thr Ala Ala Leu Asp Ala Gly
   1250 1255 1260
   Ala Glu Pro Pro Gly Val Val Ile Ala Pro Thr Pro Pro Gly Gly
   1265 1270 1275
   Gly Gln Thr Ala Asp Val Arg Glu Ser Thr Arg His Ala Leu Asp
   1280 1285 1290
   Leu Val Gln Gly Trp Leu Ala Asp Gln Arg Leu Asn Asp Ser Arg
   1295 1300 1305
   Leu Phe Leu Val Thr Arg Gly Ala Val Ala Val Glu Pro Gly Glu
   1310 1315 1320
   Pro Val Thr Asp Leu Ala Gln Ala Ala Leu Trp Gly Leu Leu Arg
   1325 1330 1335
   Ser Thr Gln Thr Glu His Pro Asp Arg Phe Val Leu Val Asp Val
   1340 1345 1350
   Ala Glu Pro Ala Gln Leu Leu Pro Ala Leu Pro Gly Val Leu Ala
   1355 1360 1365
   Cys Gly Glu Pro Gln Leu Ala Leu Arg Arg Gly Gly Ala His Ala
   1370 1375 1380
   Pro Arg Leu Ala Gly Leu Gly Gly Asp Asp Val Leu Pro Val Pro
   1385 1390 1395
   Asp Ser Met Gly Trp Arg Leu Glu Ala Thr Ser Pro Gly Thr Leu
   1400 1405 1410
   Asp Gly Leu Ala Leu Leu Asp Glu Pro Ala Ala Thr Ala Ser Leu
   1415 1420 1425
   Gly Asp Gly Gln Val Arg Ile Ala Met Arg Ala Ala Gly Val Asn
   1430 1435 1440
   Phe Arg Asp Ala Leu Ile Ala Leu Gly Met Tyr Pro Gly Ala Ala
   1445 1450 1455
   Ser Leu Gly Gly Glu Gly Ala Gly Val Val Val Glu Thr Gly Pro
   1460 1465 1470
   Gly Val Thr Gly Leu Ala Pro Gly Asp Arg Val Met Gly Met Ile
   1475 1480 1485
   Pro Lys Ala Phe Gly Pro Leu Ala Val Ala Asp His Arg Met Val
   1490 1495 1500
   Thr Arg Ile Pro Ala Gly Trp Ser Phe Ala Gln Ala Ala Ser Val
   1505 1510 1515
   Pro Ile Val Phe Leu Thr Ala Tyr Tyr Ala Leu Val Asp Leu Ala
   1520 1525 1530
   Gly Leu Arg Pro Gly Glu Ser Leu Leu Val His Ser Ala Ala Gly
   1535 1540 1545
   Gly Val Gly Met Ala Ala Ile Gln Leu Ala Arg His Leu Gly Ala
   1550 1555 1560
   Glu Val Tyr Ala Thr Ala Ser Glu Asp Lys Trp Gln Ala Val Glu
   1565 1570 1575
   Leu Thr Arg Glu Arg Leu Ala Ser Ser Arg Thr Cys Asp Phe Glu
   1580 1585 1590
   Lys Gln Phe Leu Gly Ala Thr Gly Gly Arg Gly Val Asp Val Val
   1595 1600 1605
   Leu Asn Ser Leu Ala Gly Asp Phe Ala Asp Ala Ser Leu Arg Met
   1610 1615 1620
   Leu Pro Arg Gly Gly Arg Phe Leu Glu Leu Gly Lys Thr Asp Val
   1625 1630 1635
   Arg Asp Pro Val Glu Val Ala Asp Ala His Pro Gly Val Ser Tyr
   1640 1645 1650
   Gln Ala Phe Asp Thr Val Glu Ala Gly Pro Gln Arg Ile Gly Glu
   1655 1660 1665
   Met Leu Asp Glu Leu Val Glu Leu Phe Glu Gly Gly Val Leu Glu
   1670 1675 1680
   Pro Leu Pro Val Thr Ala Trp Asp Val Arg Gln Ala Pro Glu Ala
   1685 1690 1695
   Leu Arg His Leu Ser Gln Ala Arg His Val Gly Lys Leu Val Leu
   1700 1705 1710
   Thr Met Pro Pro Ala Trp Asp Thr Ala Gly Thr Val Leu Val Thr
   1715 1720 1725
   Gly Gly Thr Gly Ala Leu Gly Ala Glu Val Ala Arg His Leu Val
   1730 1735 1740
   Ile Glu His Gly Val Arg Asn Leu Val Leu Val Ser Arg Arg Gly
   1745 1750 1755
   Pro Ala Ala Ser Gly Ala Ala Glu Leu Val Ala Gln Leu Thr Ala
   1760 1765 1770
   Tyr Gly Ala Glu Val Ser Leu Gln Ala Cys Asp Val Ala Asp Arg
   1775 1780 1785
   Glu Thr Leu Ala Lys Val Leu Ala Gly Ile Pro Asp Glu His Thr
   1790 1795 1800
   Leu Thr Ala Val Val His Ala Ala Gly Val Leu Asp Asp Gly Val
   1805 1810 1815
   Ala Glu Ser Leu Thr Ala Gln Arg Leu Asp His Val Leu Arg Pro
   1820 1825 1830
   Lys Val Asp Gly Ala Arg Asn Leu His Glu Leu Ile Ala Pro Asp
   1835 1840 1845
   Val Ala Leu Val Leu Phe Ser Ser Val Ser Gly Val Leu Gly Ser
   1850 1855 1860
   Gly Gly Gln Gly Asn Tyr Ala Ala Ala Asn Ser Phe Leu Asp Ala
   1865 1870 1875
   Leu Ala Gln Gln Arg Gln Ser Arg Gly Leu Pro Thr Arg Ser Leu
   1880 1885 1890
   Ala Trp Gly Pro Trp Ala Glu His Gly Met Ala Ser Thr Leu Arg
   1895 1900 1905
   Glu Ala Glu Gln Asp Arg Leu Ala Leu Ser Gly Leu Leu Pro Ile
   1910 1915 1920
   Ser Thr Glu Glu Gly Leu Ser Gln Phe Asp Ala Ala Cys Gly Gly
   1925 1930 1935
   Ala His Thr Val Val Ala Pro Val Arg Ile Gly Arg Ser Ser Asp
   1940 1945 1950
   Gly Asn Pro Ile Lys Phe Pro Val Leu Arg Gly Leu Val Glu Pro
   1955 1960 1965
   His Arg Val Asn Lys Ala Thr Ala Asp Asp Ala Glu Ser Ile Arg
   1970 1975 1980
   Lys Arg Leu Gly Arg Leu Pro Asp Ala Glu Gln His Arg Ile Leu
   1985 1990 1995
   Leu Asp Leu Val Arg Thr His Val Ala Ala Val Leu Gly Phe Ala
   2000 2005 2010
   Gly Pro Gln Glu Ile Thr Ala Asp Gly Thr Phe Lys Ala Leu Gly
   2015 2020 2025
   Phe Asp Ser Leu Thr Val Val Glu Leu Arg Asn Arg Ile Asn Gly
   2030 2035 2040
   Ala Thr Gly Leu Arg Leu Pro Ala Thr Leu Val Phe Asn Tyr Pro
   2045 2050 2055
   Thr Pro Asp Ala Leu Ala Ala His Leu Val Thr Ala Leu Ser Ala
   2060 2065 2070
   Asp Arg Leu Ala Gly Thr Phe Glu Glu Leu Asp Arg Trp Ala Ala
   2075 2080 2085
   Asn Leu Pro Ala Leu Ala Arg Asp Glu Ala Thr Arg Ala Gln Ile
   2090 2095 2100
   Thr Thr Arg Leu Gln Ala Ile Leu Gln Ser Leu Ala Asp Val Ser
   2105 2110 2115
   Gly Gly Thr Gly Gly Gly Ser Val Pro Asp Arg Leu Arg Ser Ala
   2120 2125 2130
   Thr Asp Glu Glu Leu Phe Gln Leu Leu Asp Asn Asp Leu Glu Leu
   2135 2140 2145
   Pro
<210> 5
   <211> 3167
   <212> PRT
   <213> Saccharopolyspora sp. NRRL30141
<400> 5
   Met Ser Asn Glu Glu Lys Leu Arg Glu Tyr Leu Arg Arg Ala Leu Val
   1               5                   10                  15
   Asp Leu His Gln Ala Arg Glu Arg Leu Asp Glu Ala Glu Ser Gly Glu
               20                  25                  30
   Gln Glu Pro Ile Ala Ile Val Ala Met Gly Cys Arg Tyr Pro Gly Gly
           35                  40                  45
   Val His Asp Pro Glu Gly Leu Trp Lys Leu Val Ala Ser Gly Gly Asp
       50                  55                  60
   Ala Ile Gly Glu Phe Pro Ala Asp Arg Gly Trp His Leu Asp Glu Leu
   65                  70                  75                  80
   Tyr Asp Pro Asp Pro Asp Gln Pro Gly Thr Cys Tyr Thr Arg His Gly
                   85                  90                  95
   Gly Phe Leu His Glu Ala Gly Glu Phe Asp Ala Gly Phe Phe Asp Ile
               100                 105                 110
   Ser Pro Arg Glu Ala Leu Ala Met Asp Pro Gln Gln Arg Leu Leu Leu
           115                 120                 125
   Glu Ile Ser Trp Glu Thr Val Glu Ser Ala Gly Met Asp Pro Arg Ser
       130                 135                 140
   Leu Arg Gly Ser Arg Thr Gly Val Phe Ala Gly Leu Met Tyr Glu Gly
   145                 150                 155                 160
   Tyr Asp Thr Gly Ala His Pro Glu Gly Val Glu Gly Tyr Leu Gly Thr
                   165                 170                 175
   Gly Asn Ala Gly Ser Val Ala Ser Gly Arg Val Ala Tyr Ser Phe Gly
               180                 185                 190
   Phe Glu Gly Pro Ala Val Thr Val Asp Thr Ala Cys Ser Ser Ser Leu
   195 200 205
   Val Ala Leu His Leu Ala Cys Gln Ser Leu Arg Gln Gly Glu Cys Asp
   210 215 220
   Leu Ala Leu Ala Gly Gly Val Thr Val Met Ala Thr Pro Ala Thr Phe
   225 230 235 240
   Val Glu Phe Ser Arg Gln Arg Gly Leu Ala Pro Asp Gly Arg Cys Lys
   245 250 255
   Ser Phe Ala Ala Ala Ala Asp Gly Thr Gly Trp Gly Glu Gly Ala Gly
   260 265 270
   Leu Val Leu Leu Glu Arg Leu Ser Asp Ala Arg Arg Asn Gly His Arg
   275 280 285
   Val Leu Ala Val Val Arg Gly Ser Ala Val Asn Gln Asp Gly Ala Ser
   290 295 300
   Asn Gly Leu Thr Ala Pro Asn Gly Leu Ala Gln Glu Arg Val Ile Gln
   305 310 315 320
   Gln Ala Leu Thr Ser Ala Gly Leu Ser Val Ser Asp Val Asp Val Val
   325 330 335
   Glu Ala His Gly Thr Gly Thr Arg Leu Gly Asp Pro Ile Glu Ala Gln
   340 345 350
   Ala Leu Ile Ala Thr Tyr Gly Gln Asp Arg Asp Arg Asp Arg Pro Leu
   355 360 365
   Trp Leu Gly Ser Val Lys Ser Asn Ile Gly His Thr Gln Ala Ala Ala
   370 375 380
   Gly Val Ala Gly Val Ile Lys Met Val Met Ala Met Arg Arg Gly Glu
   385 390 395 400
   Leu Pro Arg Thr Leu His Val Asp Glu Pro Asn Ser His Val Asp Trp
   405 410 415
   Ser Ala Gly Ala Val Arg Leu Leu Thr Glu Asn Ile Arg Trp Pro Gly
   420 425 430
   Thr Gly Thr Arg Arg Val Gly Val Ser Ser Phe Gly Val Ser Gly Thr
   435 440 445
   Asn Ala His Val Ile Leu Glu His Asp Pro Leu Ala Leu Thr Glu Asn
   450 455 460
   Glu Asn Ala Ala Val Ser Pro Ala Pro Gly Ile Val Pro Trp Ala Leu
   465 470 475 480
   Ser Gly Arg Ser Ser Thr Ala Leu Arg Ala Gln Ala Glu Arg Leu Ser
   485 490 495
   Glu Leu Cys Glu Gln Thr Asp Pro Asp Pro Val Asp Val Gly Phe Ser
   500 505 510
   Leu Ala Thr Thr Arg Thr Ala Trp Glu His Arg Ala Val Val Leu Gly
   515 520 525
   Gly Asp Ser Ala Thr Leu Arg Ser Gly Leu Gly Val Val Ala Ser Gly
   530 535 540
   Glu Pro Ala Val Asp Val Val Gln Gly Ser Val Leu Gly Gly Glu Val
   545 550 555 560
   Val Phe Val Phe Pro Gly Gln Gly Trp Gln Trp Ala Gly Met Ala Val
   565 570 575
   Asp Leu Leu Asp Ala Ser Pro Thr Phe Ala Arg His Met Asp Glu Cys
   580 585 590
   Ala Thr Ala Leu Arg Lys Tyr Val Asp Trp Ser Leu Val Asp Val Leu
   595 600 605
   Arg Gly Ala Glu Asn Ala Pro Pro Leu Asp Arg Val Asp Val Leu Gln
   610 615 620
   Pro Val Ser Phe Ala Val Met Val Ser Leu Ala Glu Val Trp Arg Ser
   625 630 635 640
   Tyr Gly Val Arg Pro Ala Ala Val Val Gly His Ser Gln Gly Glu Ile
   645 650 655
   Ala Ala Ala Cys Ala Ala Gly Val Leu Pro Leu Glu Asp Ala Ala Arg
   660 665 670
   Leu Val Ala Leu Arg Ser Arg Ala Leu Lys Ala Leu Ser Gly Arg Gly
   675 680 685
   Gly Met Ala Ser Leu Ala Cys Ser Ala Asp Glu Ala Ala Ala Leu Phe
   690 695 700
   Ala Gly Leu Gly Gly Arg Leu Glu Ile Ala Ala Ile Asn Gly Pro Arg
   705 710 715 720
   Ser Val Val Val Ser Gly Asp Leu Glu Ala Val Glu Glu Leu Leu Ala
   725 730 735
   Glu Cys Ala Glu Arg Asp Met Arg Ala Arg Arg Ile Pro Val Asp Tyr
   740 745 750
   Ala Ser His Ser Ala His Val Glu Val Val Arg Ser Pro Val Leu Ala
   755 760 765
   Ala Ala Ala Gly Val Arg His Arg Asp Gly Gln Val Pro Trp Trp Ser
   770 775 780
   Thr Val Ile Gly Asp Trp Leu Asp Pro Ala Gly Leu Asp Gly Glu Tyr
   785 790 795 800
   Trp Tyr Arg Asn Leu Arg Gln Pro Val Arg Phe Glu His Ala Val Gln
   805 810 815
   Gly Leu Val Glu Arg Gly Phe Gly Leu Phe Ile Glu Met Ser Ala His
   820 825 830
   Pro Val Leu Thr Met Ala Val Glu Glu Thr Ser Ala Glu Ser Glu Ser
   835 840 845
   Ala Val Ala Ala Val Gly Thr Leu Arg Arg Asp Ser Gly Gly Arg Arg
   850 855 860
   Arg Leu Leu Gln Ser Leu Ala Glu Ala Tyr Val Arg Gly Ala Thr Val
   865 870 875 880
   Asp Trp Ala Val Ala Phe Gly Gly Val Gly Arg Arg Leu Asp Leu Pro
   885 890 895
   Thr Tyr Pro Phe Gln Arg Arg Arg Tyr Trp Leu Asp Arg Gly Ala Ala
   900 905 910
   Ser Glu Glu Ala Arg Ala Phe Ser Asp Pro Ala Ala Asp Trp Phe Trp
   915 920 925
   Gln Ala Val Glu Arg Gln Asp Leu Lys Gly Val Ala Asp Ala Leu Asp
   930 935 940
   Leu Asp Ala Asp Ala Pro Leu Ser Ala Thr Leu Pro Ala Leu Ser Val
   945 950 955 960
   Trp His Arg Gln Glu Arg Glu Lys Val Leu Val Asp Gly Trp Arg Tyr
   965 970 975
   Arg Val Asp Trp Val Pro Val Ala Pro Gln Pro Ile Arg Arg Thr Arg
   980 985 990
   Glu Thr Trp Leu Leu Val Val Pro Ala Gly Gly Ile Glu Glu Ala Leu
   995 1000 1005
   Val Glu Arg Leu Thr Asp Ala Leu Asn Thr Arg Gly Ile Ser Thr
   1010 1015 1020
   Leu Arg Leu Asp Val Pro Pro Thr Ala Thr Ser Gly Glu Leu Ala
   1025 1030 1035
   Thr Gly Leu Arg Ala Ala Val Gly Gly Asp Pro Val Lys Gly Ile
   1040 1045 1050
   Leu Ser Leu Thr Ala Leu Asp Glu Arg Thr His Pro Glu Arg Lys
   1055 1060 1065
   Ala Val Pro Ser Gly Ile Ala Leu Leu Leu Asn Leu Val Lys Ala
   1070 1075 1080
   Leu Gly Glu Gly Asp Leu Arg Val Pro Leu Trp Thr Ile Thr Arg
   1085 1090 1095
   Gly Ala Val Lys Ala Asp Pro Ala Asp Arg Leu Leu Arg Pro Met
   1100 1105 1110
   Gln Ala Gln Ala Trp Gly Leu Gly Arg Val Ala Ala Leu Glu His
   1115 1120 1125
   Pro Glu Arg Trp Gly Gly Leu Ile Asp Leu Pro Glu Ser Leu Asp
   1130 1135 1140
   Gly Asp Val Leu Thr Arg Leu Gly Glu Ala Leu Ile Asn Gly Leu
   1145 1150 1155
   Ala Glu Asp Gln Leu Ala Ile Arg Gln Ser Gly Val Leu Ala Arg
   1160 1165 1170
   Arg Leu Val Pro Ala Pro Ala Asn Gln Pro Ala Gly Arg Lys Trp
   1175 1180 1185
   Arg Pro Arg Gly Ser Ala Leu Ile Thr Gly Gly Leu Gly Ala Val
   1190 1195 1200
   Gly Ala Gln Val Ala Arg Trp Leu Ala Glu Ser Gly Ala Glu Arg
   1205 1210 1215
   Ile Val Leu Thr Ser Arg Arg Gly Lys Glu Ala Pro Gly Ala Ala
   1220 1225 1230
   Glu Leu Glu Ala Glu Leu Arg Ala Leu Gly Ala Gln Val Ser Ile
   1235 1240 1245
   Val Ala Cys Asp Val Thr Asp Arg Ala Glu Met Ser Ala Leu Leu
   1250 1255 1260
   Ala Glu Phe Gly Val Thr Ala Val Phe His Ala Ala Gly Val Gly
   1265 1270 1275
   Arg Leu Leu Pro Leu Ala Glu Thr Glu Gln Asn Asp Leu Ala Glu
   1280 1285 1290
   Ile Cys Thr Ala Lys Val His Gly Ala Gln Val Leu Asp Glu Leu
   1295 1300 1305
   Cys Asp Ser Thr Asp Leu Asp Ala Phe Val Leu Phe Ser Ser Gly
   1310 1315 1320
   Ala Gly Val Trp Gly Gly Gly Gly Gln Gly Ala Tyr Gly Ala Ala
   1325 1330 1335
   Asn Ala Phe Leu Asp Thr Leu Ala Glu Gln Arg Arg Ala Arg Gly
   1340 1345 1350
   Leu Pro Ala Thr Ala Ile Ser Trp Gly Ser Trp Gly Gly Gly Met
   1355 1360 1365
   Ala Asp Gly Ala Ala Gly Glu Leu Leu Arg Arg Arg Gly Ile Arg
   1370 1375 1380
   Pro Met Pro Ala Ala Ser Ala Ile Leu Ala Leu Gln Glu Val Leu
   1385 1390 1395
   Asp Gln Asp Glu Thr Cys Val Ser Ile Ala Asp Val Asp Trp Asp
   1400 1405 1410
   Arg Phe Val Pro Thr Phe Ala Ala Thr Arg Ala Thr Arg Leu Leu
   1415 1420 1425
   Asp Glu Leu Pro Ala Val Arg Lys Ala Met Ser Ala Asn Gly Pro
   1430 1435 1440
   Ala Glu Pro Gly Gly Ser Pro Phe Ala Arg Asn Leu Ala Glu Leu
           1445                1450                1455
   Pro Glu  Ala Gln Arg Arg His  Glu Leu Val Asp Leu  Val Ser Ala
       1460                1465                1470
   Gln Val  Ala Ala Val Leu Gly  His Gly Ser Arg Glu  Glu Val Gln
       1475                1480                1485
   Pro Glu  Arg Ala Phe Arg Ala  Leu Gly Phe Asp Ser  Leu Met Ala
       1490                1495                1500
   Val Asp  Leu Arg Asn Arg Leu  Thr Thr Ala Thr Gly  Leu Arg Leu
       1505                1510                1515
   Pro Thr  Thr Thr Val Phe Asp  Tyr Pro Asn Pro Ala  Ala Leu Ala
       1520                1525                1530
   Ala His  Leu Leu Glu Glu Leu  Val Gly Asp Val Ala  Ser Ala Ala
       1535                1540                1545
   Val Thr  Thr Ala Ile Ala Pro  Ser Thr Asp Glu Pro  Val Ala Ile
       1550                1555                1560
   Val Ala  Met Ser Cys Arg Phe  Pro Gly Gly Ala His  Ser Pro Glu
       1565                1570                1575
   Asp Leu  Trp Arg Leu Val Ala  Ser Gly Ala Glu Val  Ile Gly Glu
       1580                1585                1590
   Phe Pro  Ser Asp Arg Gly Trp  Asp Ala Glu Ser Leu  Tyr Asp Pro
       1595                1600                1605
   Asp Ala  Ser Lys Pro Gly Thr  Thr Tyr Ala Arg Met  Ala Gly Phe
       1610                1615                1620
   Leu Tyr  Asp Ala Gly Glu Phe  Asp Ala Gly Leu Phe  Gly Ile Ser
       1625                1630                1635
   Pro Arg  Glu Ala Leu Ala Met  Asp Pro Gln Gln Arg  Leu Val Leu
       1640                1645                1650
   Glu Ile  Ala Trp Glu Ala Leu  Glu Arg Ala Gly Ile  Asp Pro Leu
       1655                1660                1665
   Ser Leu  Lys Gly Ser Gly Val  Gly Thr Tyr Ile Gly  Ala Gly Ser
       1670                1675                1680
   Arg Gly  Tyr Ala Thr Asp Val  Arg Gln Phe Pro Glu  Glu Ala Glu
       1685                1690                1695
   Gly Tyr  Leu Leu Thr Gly Thr  Ser Ala Ser Val Leu  Ser Gly Arg
       1700                1705                1710
   Val Ala  Tyr Ser Phe Gly Phe  Glu Gly Pro Ala Val  Thr Val Asp
       1715                1720                1725
   Thr Ala  Cys Ser Ser Ser Leu  Val Ala Leu His Leu  Ala Cys Gln
       1730                1735                1740
   Ser Leu  Arg Ser Gly Glu Cys  Asp Leu Ala Leu Ala  Gly Gly Val
   1745 1750 1755
   Thr Val Met Ser Thr Pro Glu Met Phe Val Glu Phe Ser Arg Gln
   1760 1765 1770
   Arg Gly Leu Ala Pro Asp Gly Arg Cys Lys Ser Phe Ala Glu Ser
   1775 1780 1785
   Ala Asp Gly Thr Gly Trp Gly Glu Gly Ala Gly Leu Leu Leu Leu
   1790 1795 1800
   Glu Arg Leu Ser Asp Ala His Arg Asn Gly His Arg Val Leu Ala
   1805 1810 1815
   Val Val Arg Gly Ser Ala Val Asn Gln Asp Gly Ala Ser Asn Gly
   1820 1825 1830
   Leu Ala Ala Pro Asn Gly Pro Ser Gln Gln Arg Val Ile Lys Gln
   1835 1840 1845
   Ala Leu Ala Asn Ala Gly Leu Ser Ala Ser Asp Val Asp Ala Val
   1850 1855 1860
   Glu Ala His Gly Thr Gly Thr Arg Leu Gly Asp Pro Ile Glu Ala
   1865 1870 1875
   Gln Ala Leu Ile Ala Thr Tyr Gly Gln Gly Arg Glu Arg Asp Arg
   1880 1885 1890
   Pro Leu Trp Leu Gly Ser Val Lys Ser Asn Ile Gly His Thr Gln
   1895 1900 1905
   Ala Ala Ala Gly Val Ala Gly Val Ile Lys Met Val Met Ser Met
   1910 1915 1920
   Arg Asn Asp Glu Leu Pro Ala Thr Leu His Val Gly Ala Pro Thr
   1925 1930 1935
   Ser Gln Val Asp Trp Ser Ala Gly Ala Val Arg Leu Leu Thr Glu
   1940 1945 1950
   Gln Val Pro Trp Pro Glu Ser Asp Arg Val Arg Arg Val Gly Val
   1955 1960 1965
   Ser Ser Phe Gly Ile Ser Gly Thr Asn Ala His Val Ile Leu Glu
   1970 1975 1980
   Gln Ser Thr Asn Ala Pro Asp Ser Pro Ala Ala Thr Asp Lys Ser
   1985 1990 1995
   Gly Ser Gly Ser Thr Val Asp Ile Pro Val Val Pro Trp Leu Val
   2000 2005 2010
   Ser Gly Gln Thr Ser Asp Ser Leu Arg Gly Gln Ala Glu Arg Val
   2015 2020 2025
   Leu Ser Gln Val Glu Ser Arg Pro Glu Gln Arg Pro Leu Asp Val
   2030 2035 2040
   Ala Tyr Ser Leu Ala Ser Gly Arg Ala Ala Leu Asp Glu Arg Ala
   2045 2050 2055
   Val Val Leu Gly Ala Asp Arg Asn Glu Leu Val Ala Gly Leu Val
   2060 2065 2070
   Ala Leu Ala Ala Gly His Glu Ala Ser Gly Val Ile Thr Gly Thr
   2075 2080 2085
   Arg Ala Ser Ala Arg Phe Gly Phe Val Phe Ser Gly Gln Gly Gly
   2090 2095 2100
   Gln Trp Leu Gly Met Gly Arg Glu Leu Tyr Ser Lys Phe Pro Val
   2105 2110 2115
   Phe Ala Ala Ala Phe Asp Glu Ala Cys Ala Glu Leu Asp Ala His
   2120 2125 2130
   Leu Ser Glu Asp Leu Arg Val Arg Asp Val Val Phe Gly Ser Asp
   2135 2140 2145
   Ala Gln Leu Leu Asp Gln Thr Leu Trp Ala Gln Ser Gly Leu Phe
   2150 2155 2160
   Ala Leu Gln Val Gly Leu Leu Gly Leu Leu Gly Ser Trp Gly Val
   2165 2170 2175
   Arg Pro Asp Val Val Met Gly His Ser Val Gly Glu Leu Ala Ala
   2180 2185 2190
   Ala Phe Ala Ala Gly Val Leu Ser Leu Arg Asp Ala Ala Arg Leu
   2195 2200 2205
   Val Ala Ala Arg Ala Arg Leu Met Gln Ala Leu Pro Ser Asp Gly
   2210 2215 2220
   Ala Met Leu Ala Val Ala Ala Gly Glu Asp Leu Ile Arg Pro Leu
   2225 2230 2235
   Leu Ala Gly Arg Glu Ala Ser Val Asn Val Ala Ala Leu Asn Ala
   2240 2245 2250
   Pro Gly Ser Val Val Leu Ser Gly Asp Arg Asp Val Leu Ala Asp
   2255 2260 2265
   Ile Ala Gly Arg Leu Asn Glu Leu Gly Val Arg Thr Arg Arg Leu
   2270 2275 2280
   Arg Val Ser His Ala Phe His Ser His Arg Met Asp Pro Met Leu
   2285 2290 2295
   Gly Glu Phe Ala Gln Ile Ala Glu Ser Ala Glu Phe Gly Arg Pro
   2300 2305 2310
   Thr Thr Pro Leu Val Ser Thr Leu Thr Gly Glu Leu Asp Arg Ala
   2315 2320 2325
   Gly Glu Met Ser Thr Pro Gly Tyr Trp Val Arg Gln Val Arg Glu
   2330 2335 2340
   Pro Val Arg Phe Ala Asp Gly Val Arg Ala Leu Ala Ala Gln Gly
   2345 2350 2355
   Val Asp Thr Val Val Glu Leu Gly Pro Asp Gly Ala Leu Ser Ala
   2360 2365 2370
   Leu Val Gln Glu Cys Ala Thr Gly Phe Asp Arg Val Gly Arg Ile
   2375 2380 2385
   Ser Pro Val Pro Leu Met Arg Arg Glu Arg Asp Glu Thr Arg Ser
   2390 2395 2400
   Val Met Thr Ala Leu Ala His Leu His Thr Arg Gly Gly Glu Leu
   2405 2410 2415
   Asp Trp Gln Ala Phe Phe Ser Gly Thr Gly Ala Arg Gln Val Glu
   2420 2425 2430
   Leu Pro Thr Tyr Ala Phe Gln Arg Arg His Tyr Trp Ile Glu Ser
   2435 2440 2445
   Ser Ala Arg Thr Ala Arg Asp Arg Ala Asp Ile Gly Glu Val Ala
   2450 2455 2460
   Glu Gln Phe Trp Thr Ala Val Glu Gln Gly Asp Leu Glu Ala Leu
   2465 2470 2475
   Val Ser Ala Leu Glu Leu Gly Ala Asp Asp Asp Thr Cys Ala Ser
   2480 2485 2490
   Leu Ser Asp Val Leu Pro Ala Leu Ser Ser Trp Arg Ser Gly Leu
   2495 2500 2505
   Arg Asn Arg Ser Leu Val Asp Ser Cys Arg Tyr Arg Ile Asn Trp
   2510 2515 2520
   His Ser Ser Arg Glu Ala Pro Ala Pro Lys Ile Ser Gly Thr Trp
   2525 2530 2535
   Leu Leu Val Val Pro Gly Asp Ala Asp Asp Gly Leu Ala Thr Ala
   2540 2545 2550
   Leu Thr Ser Ser Leu Val Glu Gly Gly Ala Glu Val Val Arg Ile
   2555 2560 2565
   Asp Leu Ser Glu Glu Asp Leu His Arg Glu Asp Leu Ala Gln Arg
   2570 2575 2580
   Leu Ala Asn Ala Leu Thr Asp Val Gly Arg Leu Gly Gly Val Leu
   2585 2590 2595
   Ser Leu Leu Gly Leu Asp Asp Ser Ala Val Gly Glu Phe Ser Cys
   2600 2605 2610
   Leu Thr Arg Gly Phe Ala Leu Thr Val Gln Leu Val Arg Ala Leu
   2615 2620 2625
   Arg Asn Ala Glu Leu Glu Ala Pro Leu Trp Ala Val Thr Arg Gly
   2630 2635 2640
   Gly Val Ser Leu Glu Asp Val Ser Val Ser Pro Glu Gln Ala Leu
   2645 2650 2655
   Ile Trp Gly Leu Leu Arg Val Ala Gly Leu Glu His Pro Glu Phe
   2660 2665 2670
   Trp Gly Gly Leu Ile Asp Leu Pro Ser Asp Trp Asp Asp Arg Leu
   2675 2680 2685
   Gly Ala Arg Leu Val Gly Val Leu Ala Asp Gly Gly Glu Asp Gln
   2690 2695 2700
   Val Ala Ile Arg Arg Gly Gly Val Phe Val Arg Arg Leu Glu Arg
   2705 2710 2715
   Ala Gly Ala Ser Gly Ala Gly Ser Val Trp Arg Pro Arg Gly Thr
   2720 2725 2730
   Val Leu Val Thr Gly Gly Thr Gly Gly Leu Gly Ala His Val Ala
   2735 2740 2745
   Arg Trp Leu Ala Gly Ala Gly Ala Glu His Val Val Leu Thr Ser
   2750 2755 2760
   Arg Arg Gly Ala Glu Ala Pro Gly Ala Gly Glu Leu Arg Ala Glu
   2765 2770 2775
   Leu Glu Ala Leu Gly Ala Arg Val Ser Ile Val Pro Cys Asp Val
   2780 2785 2790
   Ala Asp Arg Asp Ala Val Ala Gly Val Leu Ala Gly Ile Gly Gly
   2795 2800 2805
   Glu Cys Pro Leu Thr Ala Val Val His Ala Ala Gly Val Gly Glu
   2810 2815 2820
   Ala Gly Gly Val Val Glu Met Ala Leu Ala Asp Phe Ala Glu Val
   2825 2830 2835
   Leu Ser Ala Lys Val Arg Gly Ala Ala Asn Leu Asp Glu Leu Leu
   2840 2845 2850
   Ala Asp Ser Glu Leu Asp Ala Phe Val Leu Phe Ser Ser Val Ser
   2855 2860 2865
   Gly Val Trp Gly Ala Gly Gly Gln Gly Ala Tyr Ala Ala Ala Asn
   2870 2875 2880
   Ala Tyr Leu Asp Ala Leu Ala Glu Gln Arg Arg Ala Ser Gly Leu
   2885 2890 2895
   Ala Gly Thr Ala Val Ala Trp Gly Pro Trp Ala Gly Asp Gly Met
   2900 2905 2910
   Ala Ala Gly Glu Thr Gly Ala Gln Leu His Arg Met Gly Leu Val
   2915 2920 2925
   Ser Met Glu Pro Arg Ala Ala Leu Leu Ala Leu Gln Gly Ala Leu
   2930 2935 2940
   Asp Arg Asp Glu Thr Ser Leu Val Val Ala Asp Val Asp Trp Ala
   2945 2950 2955
   Arg Phe Ala Pro Ala Phe Thr Ser Ala Arg Arg Arg Pro Leu Leu
   2960 2965 2970
   Asp Thr Ile Asp Glu Ala Arg Ala Ala Leu Glu Thr Thr Ser Glu
   2975 2980 2985
   Lys Ala Gly Thr Gly Lys Pro Val Glu Leu Lys His Arg Leu Ala
   2990 2995 3000
   Gly Leu Ser Arg Lys Glu Arg Asp Asp Ala Val Leu Asp Leu Val
   3005 3010 3015
   Arg Ala Glu Thr Ala Ala Val Leu Gly Arg Asp Asp Ala Thr Ala
   3020 3025 3030
   Leu Ala Pro Ser Arg Pro Phe Gln Glu Leu Gly Phe Asp Ser Leu
   3035 3040 3045
   Met Ala Val Glu Leu Arg Asn Arg Leu Asn Thr Ala Thr Gly Ile
   3050 3055 3060
   Gln Leu Pro Ala Ser Thr Ile Phe Asp Tyr Pro Asn Ala Glu Ser
   3065 3070 3075
   Leu Ser Arg His Leu Cys Ala Gly Leu Phe Pro Thr Glu Thr Thr
   3080 3085 3090
   Val Asp Ser Ala Leu Ala Glu Leu Asp Arg Ile Glu Gln Gln Leu
   3095 3100 3105
   Ser Met Phe Thr Glu Glu Ala Arg Ala Arg Asp Arg Ile Ala Thr
   3110 3115 3120
   Arg Leu Arg Ala Leu His Ala Lys Trp Asn Ser Ala Ser Glu Ala
   3125 3130 3135
   Pro Thr Gly Ala Asp Val Leu Asn Thr Leu Asp Ser Ala Thr His
   3140 3145 3150
   Asp Glu Ile Phe Glu Phe Ile Asp Asn Glu Leu Asp Leu Ser
   3155 3160 3165
<210> 6
   <211> 4933
   <212> PRT
   <213> Saccharopolyspora sp. NRRL30141
<400> 6
   Val Glu Ile Thr Met Ala Asn Glu Glu Lys Leu Phe Gly Tyr Leu Lys
   1 5 10 15
   Lys Val Thr Ala Asp Leu His Gln Thr Arg Gln Arg Leu Leu Ala Ala
   20 25 30
   Glu Ser Arg Ser Gln Glu Pro Ile Val Ser Ala Ser Cys Arg Leu Pro
   35 40 45
   Gly Gly Val Asp Ser Pro Glu Ala Leu Trp Gln Leu Val Arg Thr Gly
   50 55 60
   Thr Asp Ala Ile Ser Glu Phe Pro Ala Asp Arg Gly Trp Asp Leu Asp
   65 70 75 80
   Arg Leu Tyr Asp Pro Asp Pro Asp His Gln Gly Thr Ser Tyr Thr Arg
   85 90 95
   Ala Gly Gly Phe Leu Ala Asp Ala Gly Asp Phe Asp Pro Ala Met Phe
   100 105 110
   Gly Ile Ser Pro Arg Glu Ala Leu Ala Met Asp Pro Gln Gln Arg Leu
   115 120 125
   Leu Leu Glu Leu Thr Trp Glu Ala Leu Glu Arg Ala Gly Ile Asp Pro
   130 135 140
   Thr Ser Leu Arg Gly Ser Lys Thr Gly Val Phe Gly Gly Val Thr Pro
   145 150 155 160
   Gln Glu Tyr Gly Pro Pro Leu Pro Glu Met Ser Arg Asn Ser Gly Gly
   165 170 175
   Phe Gly Leu Thr Gly Arg Met Val Ser Val Ala Ser Gly Arg Val Ala
   180 185 190
   Tyr Ser Phe Gly Phe Glu Gly Pro Ala Val Thr Val Asp Thr Ala Cys
   195 200 205
   Ser Ser Ser Leu Val Ala Leu His Leu Ala Cys Gln Ser Leu Arg Ser
   210 215 220
   Gly Glu Cys Asp Leu Ala Leu Ala Gly Gly Val Thr Val Met Ala Thr
   225 230 235 240
   Pro Ala Thr Phe Val Glu Phe Ser Arg Gln Arg Gly Leu Ala Pro Asp
   245 250 255
   Gly Arg Cys Lys Ser Phe Ala Ala Ala Ala Asp Gly Thr Gly Trp Gly
   260 265 270
   Glu Gly Ala Gly Leu Val Leu Leu Glu Arg Leu Ser Asp Ala Arg Arg
   275 280 285
   Asn Gly His Lys Val Leu Ala Val Val Arg Gly Ser Ala Val Asn Gln
   290 295 300
   Asp Gly Ala Ser Asn Gly Leu Thr Ala Pro Asn Gly Pro Ser Gln Gln
   305 310 315 320
   Arg Val Ile Thr Gln Ala Leu Ser Asn Ala Gly Leu Ser Val Ser Asp
   325 330 335
   Val Asp Ala Val Glu Ala His Gly Thr Gly Thr Arg Leu Gly Asp Pro
   340 345 350
   Ile Glu Ala Gln Ala Leu Ile Ala Thr Tyr Gly Gln Gly Arg Glu Lys
   355 360 365
   Asp Arg Pro Leu Trp Leu Gly Ser Val Lys Ser Asn Ile Gly His Thr
   370 375 380
   Gln Ala Ala Ala Gly Val Ala Gly Val Ile Lys Met Val Leu Ala Met
   385 390 395 400
   Arg His Gly Gln Leu Pro Ala Thr Leu His Val Asp Asp Pro Thr Ser
   405 410 415
   Ala Val Asp Trp Ser Ala Gly Ser Val Arg Leu Leu Thr Glu Asn Thr
   420 425 430
   Pro Trp Pro Asp Ser Gly Arg Pro Cys Arg Val Gly Val Ser Ser Phe
   435 440 445
   Gly Ile Ser Gly Thr Asn Ala His Val Ile Leu Glu Gln Ser Pro Val
   450 455 460
   Glu Gln Gly Glu Pro Thr Gly Pro Val Glu Gly Glu Arg Glu Pro Glu
   465 470 475 480
   Ala Ala Ile Pro Val Val Pro Trp Met Val Ser Gly Lys Thr Pro Glu
   485 490 495
   Ala Ala Arg Ala Gln Ala Glu Arg Val Leu Ser His Ile Glu Asp Arg
   500 505 510
   Pro Glu Leu Ser Pro Val Asp Val Ala Tyr Ser Leu Gly Met Thr Arg
   515 520 525
   Ala Ala Leu Asp Glu Arg Ala Val Met Leu Gly Ser Asp Arg Asp Thr
   530 535 540
   Leu Leu Thr Gly Leu Arg Ala Phe Ala Asp Gly Cys Asp Val Pro Glu
   545 550 555 560
   Val Val Ser Gly Ser Val Gly Asn Gly Gly Arg Val Gly Phe Val Phe
   565 570 575
   Ala Gly Gln Gly Gly Gln Trp Pro Gly Met Gly Arg Gly Leu Tyr Ser
   580 585 590
   Val Phe Pro Gly Phe Ala Asp Ala Phe Asp Glu Ala Cys Ala Glu Leu
   595 600 605
   Asp Thr His Leu Gly Gln Glu Leu Gly Val Arg Asp Val Val Phe Gly
   610 615 620
   Ser Asp Ala Arg Leu Val Asp Arg Thr Val Trp Ala Gln Ser Gly Leu
   625 630 635 640
   Phe Ala Leu Gln Val Gly Leu Leu Arg Leu Leu Gly Ser Trp Gly Val
   645 650 655
   Arg Pro Asp Val Val Leu Gly His Ser Val Gly Glu Leu Ala Ala Val
   660 665 670
   His Ala Ala Gly Val Leu Ser Leu Pro Glu Ala Ala Arg Leu Val Ala
   675 680 685
   Gly Arg Ala Arg Leu Met Gln Ala Leu Pro Ser Gly Gly Ala Met Leu
   690 695 700
   Ala Val Ala Ala Ser Glu Ala Gln Val Glu Pro Leu Leu Asp Arg Val
   705 710 715 720
   Arg Gly Arg Val Glu Ile Ala Ala Ile Asn Gly Pro Gly Ser Val Val
   725 730 735
   Leu Ser Gly Asp Arg Glu Leu Leu Thr Glu Ile Ala Asp Arg Leu His
   740 745 750
   Asp Gln Gly Cys Arg Thr Arg Trp Leu Arg Val Ser His Ala Phe His
   755 760 765
   Ser Pro His Met Glu Pro Met Leu Glu Glu Phe Ala Gln Ile Ala Arg
   770 775 780
   Ser Arg Glu Tyr Gln Ala Pro Glu Leu Pro Ile Ile Ser Thr Leu Thr
   785 790 795 800
   Gly Glu Leu Asp Gly Gly Arg Val Met Gly Thr Pro Glu Tyr Trp Val
   805 810 815
   Arg Gln Val Arg Glu Pro Val Arg Phe Ala Glu Gly Val Gln Ala Leu
   820 825 830
   Val Gly Gln Gly Ala Asp Thr Ile Val Glu Phe Gly Pro Asp Gly Ala
   835 840 845
   Leu Ser Thr Leu Val Glu Glu Cys Leu Ala Glu Ser Gly Arg Val Ala
   850 855 860
   Gly Ile Pro Leu Met Arg Lys Asp Arg Asp Glu Ala Arg Thr Val Leu
   865 870 875 880
   Ala Ala Leu Ala Gln Ile His Thr Arg Gly Gly Glu Val Glu Trp Gln
   885 890 895
   Ser Phe Phe Ala Gly Thr Gly Ala Lys Gln Val Glu Leu Pro Thr Tyr
   900 905 910
   Ala Phe Gln Arg Gln Arg Tyr Trp Leu Ala Ser Thr Gly Gly Ala Gly
   915 920 925
   Asp Val Thr Ala Ala Gly Leu Ala Glu Ala Asp His Pro Leu Leu Gly
   930 935 940
   Ala Val Val Ala Leu Ala Asp Gly Glu Gly Val Val Leu Thr Gly Arg
   945 950 955 960
   Leu Thr Ala Asp Ser His Pro Trp Leu Ser Asp His Arg Val Leu Gly
   965 970 975
   Glu Ile Val Val Pro Gly Thr Ala Ile Val Glu Leu Ala Trp His Val
   980 985 990
   Gly Glu Arg Leu Gly Cys Gly Arg Val Glu Glu Leu Ala Leu Glu Ala
   995 1000 1005
   Pro Leu Ile Leu Pro Asp His Gly Ala Val Gln Val Gln Val Leu
   1010 1015 1020
   Val Gly Pro Pro Gly Glu Ser Gly Ala Arg Ser Val Ala Leu Tyr
   1025 1030 1035
   Ser Arg Pro Gly Asp Ala Thr Glu Ser Glu Trp Lys Lys His Ala
   1040 1045 1050
   Thr Gly Val Leu Leu Pro Pro Val Ala Ala Glu Asn His Glu Leu
   1055 1060 1065
   Pro Ala Trp Pro Pro Glu Asn Ala Thr Glu Ile Asp Ala Asp Glu
   1070 1075 1080
   Val Tyr Glu Phe Leu Glu Gly His Gly Phe Ala Tyr Gly Pro Ala
   1085 1090 1095
   Phe Arg Cys Leu Arg Gly Ala Trp Arg Arg Gly Gly Glu Val Phe
   1100 1105 1110
   Ala Glu Val Ala Leu Pro Asp Gly Met Gln Val Gly Val Asp Arg
   1115 1120 1125
   Phe Gly Val His Pro Ala Leu Leu Asp Ala Val Leu His Ala Ala
   1130 1135 1140
   Ala Ala Glu Thr Ser Val Val Gln Ser Glu Ala Arg Val Pro Phe
   1145 1150 1155
   Ser Trp Arg Gly Val Glu Leu Arg Ala Thr Glu Thr Ala Val Val
   1160 1165 1170
   Arg Ala Arg Ile Ser Leu Thr Ala Asp Asp Glu Leu Ser Leu Val
   1175 1180 1185
   Ala Val Asp Pro Val Gly Gly Phe Val Ala Ser Val Asp Ser Leu
   1190 1195 1200
   Val Thr Arg Pro Ile Ser Arg Gln Gln Val Arg Ser Gly Ala Ile
   1205 1210 1215
   Gly Asp Cys Leu Phe Glu Val Glu Trp His Arg Arg Ala Leu Leu
   1220 1225 1230
   Glu Thr Ala Ala Asp Asp Gly Leu Ala Ile Val Gly Asp Gly Ala
   1235 1240 1245
   Ser Trp Pro Glu Ser Val Arg Ala Thr Ala Arg Phe Ala Thr Leu
   1250 1255 1260
   Asp Glu Leu Arg Ser Ala Ala Asp Ser Asp Val Pro Ala Pro Gly
   1265 1270 1275
   Pro Val Leu Val Ala Ala Met Ser Ala Glu Glu Val Glu Ser Glu
   1280 1285 1290
   Ser Leu Pro Ser Arg Ala Gln Glu Ser Thr Ser Asp Leu Leu Ala
   1295 1300 1305
   Leu Val Gln Ser Trp Leu Ala Asp Glu Gln Phe Ala Glu Ser Gln
   1310 1315 1320
   Leu Val Val Val Thr Arg Ala Ala Val Ser Ala Asp Ser Asp Thr
   1325 1330 1335
   Asp Val Ala Asp Leu Val Ser Ala Ser Ser Trp Gly Leu Leu Arg
   1340 1345 1350
   Ser Ala Gln Ser Glu Asn Pro Gly Arg Phe Val Leu Val Asp Val
   1355 1360 1365
   Asp Gly Thr Pro Glu Ser Trp Gln Ala Leu Pro Thr Ala Val Arg
   1370 1375 1380
   Ala Gly Glu Pro Gln Leu Ala Leu Arg Arg Gly Val Ala Leu Val
   1385 1390 1395
   Pro Arg Leu Ala Arg Leu Lys Ala His Gly Glu Gly Ser Ser Pro
   1400 1405 1410
   Arg Leu Asp Thr Asp Gly Thr Val Leu Ile Thr Gly Gly Thr Gly
   1415 1420 1425
   Ala Leu Gly Gly Val Val Ala Arg His Leu Val Ala Glu His Gly
   1430 1435 1440
   Ile Arg Arg Leu Val Leu Ala Gly Arg Arg Gly Trp Asn Ala Pro
   1445 1450 1455
   Gly Val His Asp Leu Val Asp Glu Leu Ala Arg Ser Gly Ala Val
   1460 1465 1470
   Val Asp Val Val Ala Cys Asp Val Gly Asn Arg Thr Asp Leu Glu
   1475 1480 1485
   Gln Ala Leu Ala Ala Ile Pro Val Asp Arg Pro Leu Arg Gly Ile
   1490 1495 1500
   Val His Thr Ala Gly Val Leu Ala Asp Gly Val Leu Gly Ser Leu
   1505 1510 1515
   Ser Ala Ala Asp Val Asp Thr Val Phe Ala Pro Lys Val Ala Gly
   1520 1525 1530
   Ala Trp His Leu His Glu Leu Thr Arg Glu Leu Asp Leu Ser Phe
   1535 1540 1545
   Phe Val Leu Phe Ser Ser Phe Ser Gly Ile Ala Gly Ala Ala Gly
   1550 1555 1560
   Gln Ala Asn Tyr Ala Ala Ala Asn Thr Phe Leu Asp Ala Leu Ala
   1565 1570 1575
   Gly Tyr Arg Arg Ala Arg Gly Leu Pro Gly Leu Ser Leu Ala Trp
   1580 1585 1590
   Gly Leu Trp Ala Gln Pro Gly Gly Met Thr Ser Gly Leu Asp Ala
   1595 1600 1605
   Ala Ser Val Glu Arg Leu Ala Arg Thr Gly Ile Ala Glu His Ser
   1610 1615 1620
   Thr Glu Asp Gly Leu Arg Leu Phe Asp Ala Ala Ile Ala Lys Asp
   1625 1630 1635
   Arg Ala Cys Val Val Pro Ala Arg Leu Asp Arg Ala Leu Leu Val
   1640 1645 1650
   Glu His Ala Arg Ser His Ala Ile Pro Ala Leu Met Thr Ala Leu
   1655 1660 1665
   Ala Pro Ala Arg Gly Gly Val Ala Arg Arg Ala Thr Asn Ser Gln
   1670 1675 1680
   Ala Ala Asp Glu Asp Ala Leu Leu Gly Leu Val Arg Asp His Val
   1685 1690 1695
   Ser Ala Val Leu Gly Tyr Ser Gly Ala Val Glu Val Gly Gly Asp
   1700 1705 1710
   Arg Ala Phe Arg Asp Leu Gly Phe Asp Ser Leu Ser Gly Val Glu
   1715 1720 1725
   Leu Arg Asn Arg Leu Ala Gly Val Leu Gly Val Arg Leu Pro Ala
   1730 1735 1740
   Thr Ala Val Phe Asp Tyr Pro Thr Pro Arg Ala Leu Ala Arg Phe
   1745 1750 1755
   Leu His Gln Glu Leu Ala Gly Glu Val Gly Ser Met Ser Thr Pro
   1760 1765 1770
   Val Thr Arg Ala Ala Ser Val Glu Glu Asp Leu Ile Ala Ile Val
   1775 1780 1785
   Gly Met Gly Cys Arg Phe Pro Gly Gly Val Ser Ser Pro Glu Glu
   1790 1795 1800
   Leu Trp Arg Leu Val Ala Gly Gly Val Asp Ala Val Ala Gly Phe
   1805 1810 1815
   Pro Asp Asp Arg Gly Trp Asp Leu Ala Gly Leu Phe Asp Pro Asp
   1820 1825 1830
   Pro Asp His Leu Gly Thr Ser Tyr Val Cys Glu Gly Gly Phe Leu
   1835 1840 1845
   Arg Asp Ala Ala Glu Phe Asp Ala Asp Met Phe Gly Val Ser Pro
   1850 1855 1860
   Arg Glu Ala Leu Ala Met Asp Pro Gln Gln Arg Leu Leu Leu Glu
   1865 1870 1875
   Val Ala Trp Glu Thr Leu Glu Arg Ala Gly Ile Asp Pro Phe Ser
   1880 1885 1890
   Leu His Gly Ser Arg Thr Gly Val Phe Ala Gly Leu Met Tyr His
   1895 1900 1905
   Asp Tyr Gly Ala Arg Phe Ile Thr Arg Ala Pro Glu Gly Phe Glu
   1910 1915 1920
   Gly His Leu Gly Thr Gly Asn Ala Gly Ser Val Leu Ser Gly Arg
   1925 1930 1935
   Val Ala Tyr Ser Phe Gly Phe Glu Gly Pro Ala Val Thr Val Asp
   1940 1945 1950
   Thr Ala Cys Ser Ser Ser Leu Val Ala Leu His Leu Ala Gly Gln
   1955 1960 1965
   Ala Leu Arg Ala Gly Glu Cys Glu Leu Ala Leu Ala Gly Gly Val
   1970 1975 1980
   Thr Val Met Ser Thr Pro Thr Thr Phe Val Glu Phe Ser Arg Gln
   1985 1990 1995
   Arg Gly Leu Ala Pro Asp Gly Arg Cys Lys Ser Phe Ala Ala Ala
   2000 2005 2010
   Ala Asp Gly Thr Gly Trp Gly Glu Gly Ala Gly Leu Val Leu Leu
   2015 2020 2025
   Glu Arg Leu Ser Asp Ala Arg Arg Asn Gly His Lys Val Leu Ala
   2030 2035 2040
   Val Val Arg Gly Ser Ala Val Asn Gln Asp Gly Ala Ser Asn Gly
   2045 2050 2055
   Leu Thr Ala Pro Asn Gly Pro Ser Gln Gln Arg Val Ile Thr Gln
   2060 2065 2070
   Ala Leu Thr Ser Ala Gly Leu Ser Leu Ser Asp Val Asp Ala Val
   2075 2080 2085
   Glu Ala His Gly Thr Gly Thr Arg Leu Gly Asp Pro Ile Glu Ala
   2090 2095 2100
   Gln Ala Leu Ile Ala Thr Tyr Gly Arg Asp Arg Asp Pro Gly Arg
   2105 2110 2115
   Pro Leu Trp Leu Gly Ser Val Lys Ser Asn Ile Gly His Thr Gln
   2120 2125 2130
   Ala Ala Ala Gly Val Ala Gly Val Ile Lys Met Val Met Ala Met
   2135 2140 2145
   Arg His Gly Glu Leu Pro Arg Thr Leu His Val Asp Glu Pro Ser
   2150 2155 2160
   Ala Gln Val Asp Trp Ser Ala Gly Thr Val Gln Leu Leu Thr Glu
   2165 2170 2175
   Asn Thr Pro Trp Pro Asp Ser Gly Arg Leu Arg Arg Ala Gly Val
   2180 2185 2190
   Ser Ser Phe Gly Ile Ser Gly Thr Asn Ala His Leu Ile Leu Glu
   2195 2200 2205
   Gln Pro Pro Arg Glu Thr His Arg Ala Thr Glu Pro Asp Ser Ser
   2210 2215 2220
   Ser Val Leu Asp Val Pro Val Val Pro Trp Met Val Ser Gly Lys
   2225 2230 2235
   Thr Pro Glu Ala Leu Ser Ala Gln Ala Asp Ala Leu Met Ser Tyr
   2240 2245 2250
   Leu Asn Asn Arg Val Asp Val Ser Pro Arg Asp Ile Gly Tyr Ser
   2255 2260 2265
   Leu Ala Val Thr Arg Pro Ala Leu Asp His Arg Ala Val Val Leu
   2270 2275 2280
   Gly Ala Asp Arg Glu Ala Leu Leu Pro Gly Leu Lys Ala Leu Ala
   2285 2290 2295
   Ala Ser His Asp Ala Ala Glu Val Ile Thr Gly Thr Arg Ala Ala
   2300 2305 2310
   Gly Pro Val Gly Phe Val Phe Ser Gly Gln Gly Gly Gln Trp Pro
   2315 2320 2325
   Gly Met Gly Ser Gly Leu Tyr Ser Ala Phe Pro Val Phe Ala Asp
   2330 2335 2340
   Ala Phe Asp Glu Ala Cys Gly Glu Leu Asp Ala His Leu Gly Gln
   2345 2350 2355
   Lys Ala Arg Val Arg Asp Val Met Ser Gly Ser Asp Lys Gln Leu
   2360 2365 2370
   Leu Asp Gln Thr Leu Trp Ala Gln Ser Gly Leu Phe Ala Leu Gln
   2375 2380 2385
   Val Gly Leu Trp Glu Leu Leu Gly Ser Trp Gly Val Arg Pro Gly
   2390 2395 2400
   Val Val Leu Gly His Ser Val Gly Glu Leu Ala Ala Ala Phe Ala
   2405 2410 2415
   Ala Gly Val Leu Ala Leu Pro Asp Ala Ala Arg Leu Val Ala Gly
   2420 2425 2430
   Arg Ala Arg Leu Met Gln Ala Leu Pro Pro Gly Gly Ala Met Leu
   2435 2440 2445
   Ala Ala Ala Ala Gly Glu Lys Glu Leu Arg Pro Leu Leu Ala Asp
   2450 2455 2460
   Arg Ala Asp Arg Val Gly Ile Ala Ala Val Asn Ala Pro Glu Ser
   2465 2470 2475
   Val Val Leu Ser Gly Asp Arg Asp Ala Leu Asp Asp Ile Ala Gly
   2480 2485 2490
   Arg Leu Asp Gly Gln Gly Val Arg Ser Arg Trp Leu Arg Val Ser
   2495 2500 2505
   His Ala Phe His Ser His Arg Met Asp Pro Met Leu Glu Glu Phe
   2510 2515 2520
   Ala Glu Ile Ala Arg Ser Val Asp Tyr Arg Ser Pro Gly Leu Pro
   2525 2530 2535
   Ala Val Ser Thr Leu Thr Gly Glu Leu Asp Glu Val Gly Met Met
   2540 2545 2550
   Ala Thr Pro Glu Tyr Trp Val Arg Gln Val Arg Glu Pro Val Arg
   2555 2560 2565
   Phe Ala Asp Gly Val Ala Ala Leu Ala Ala His Gly Val Ser Ser
   2570 2575 2580
   Ile Val Glu Val Gly Pro Asp Gly Val Leu Ser Ala Leu Val Gln
   2585 2590 2595
   Glu Cys Ala Ala Gly Ser Asp Gln Gly Gly Arg Val Ala Ala Val
   2600 2605 2610
   Pro Leu Met Arg Ser Asn Cys Asp Glu Ala Gln Lys Val Ile Thr
   2615 2620 2625
   Ala Leu Ala Gln Val His Ala Arg Gly Ala Glu Val Asp Trp Arg
   2630 2635 2640
   Ser Phe Phe Ala Gly Thr Gly Ala Lys Gln Val Glu Leu Pro Thr
   2645 2650 2655
   Tyr Ala Phe Gln Arg Gln Arg Tyr Trp Leu Asp Ser Pro Ser Glu
   2660 2665 2670
   Pro Val Gly Gln Ser Ala Asp Leu Ala Pro Gln Ser Gly Phe Trp
   2675 2680 2685
   Glu Leu Val Glu Gln Glu Asp Val Ser Ala Leu Ser Ala Ala Leu
   2690 2695 2700
   Asn Ile Thr Gly Asp Pro Asp Val Gln Ala Ser Leu Glu Ser Val
   2705 2710 2715
   Val Pro Val Leu Ser Ser Trp His Arg Arg Ile Arg Asn Glu Ser
   2720 2725 2730
   Leu Val His Gln Trp Arg Tyr Arg Ile Ser Trp His Glu Arg Ala
   2735 2740 2745
   Asp Leu Pro Asp Arg Ser Leu Ser Gly Thr Trp Leu Val Val Val
   2750 2755 2760
   Pro Glu Gly Trp Ser Thr Ser Gln Gln Val Leu Arg Phe Arg Glu
   2765 2770 2775
   Met Phe Glu Glu Arg Gly Cys Ala Ala Val Leu Phe Glu Leu Ala
   2780 2785 2790
   Gly His Asp Glu Glu Ala Leu Val Gln Arg Phe Arg Ser Leu Pro
   2795 2800 2805
   Val Ala Ser Gly Gly Ile Ser Gly Val Leu Ser Leu Leu Ala Leu
   2810 2815 2820
   Asp Glu Ser Pro Ser Ser Ser Asn Ala Ala Leu Pro Asn Gly Ala
   2825 2830 2835
   Leu Asn Ser Leu Val Leu Leu Arg Ala Leu Arg Thr Ala Asp Val
   2840 2845 2850
   Pro Ala Pro Leu Trp Leu Ala Thr Cys Gly Gly Val Ala Val Gly
   2855 2860 2865
   Asp Val Pro Val Asn Pro Gly Gln Ala Leu Met Trp Gly Leu Gly
   2870 2875 2880
   Arg Val Val Gly Leu Glu Asn Pro Asp Trp Trp Gly Gly Leu Val
   2885 2890 2895
   Asp Val Pro Asp Leu Leu Asp Lys Asp Ala Gln Glu Arg Leu Ser
   2900 2905 2910
   Val Val Leu Ala Gly Leu Gly Glu Asp Glu Ile Ala Val Arg Pro
   2915 2920 2925
   Asp Gly Val Phe Val Arg Arg Leu Glu Arg Ala Asp Leu Pro Asp
   2930 2935 2940
   Met Gly Ser Ala Trp Arg Pro Arg Gly Thr Val Leu Val Thr Gly
   2945 2950 2955
   Gly Thr Gly Gly Leu Gly Ala His Val Ala Arg Trp Leu Ala Gly
   2960 2965 2970
   Ala Gly Ala Glu His Val Val Leu Thr Ser Arg Arg Gly Ala Glu
   2975 2980 2985
   Ala Pro Gly Ala Gly Asp Leu Arg Ala Glu Leu Glu Ala Leu Gly
   2990 2995 3000
   Ala Arg Val Ser Ile Arg Ser Cys Asp Val Ala Asp Arg Asp Ala
   3005 3010 3015
   Leu Ala Glu Val Leu Ala Thr Ile Pro Asp Asp Cys Pro Leu Thr
   3020 3025 3030
   Ala Val Met His Ala Ala Gly Val Val Glu Val Gly Asp Val Ala
   3035 3040 3045
   Ser Met Cys Leu Thr Asp Phe Ile Gly Val Leu Ser Ala Lys Val
   3050 3055 3060
   Gly Gly Ala Ala Asn Leu Asp Glu Leu Leu Ala Asp Val Glu Leu
   3065 3070 3075
   Asp Ala Phe Val Leu Phe Ser Ser Val Ser Gly Val Trp Gly Ala
   3080 3085 3090
   Gly Gly Gln Gly Ala Tyr Ala Ala Ala Asn Ala Tyr Leu Asp Ala
   3095 3100 3105
   Leu Ala  Gln Gln Arg Arg Ala  Arg Gly Leu Ala Gly  Thr Ala Val
       3110                 3115                 3120
   Ala Trp  Gly Pro Trp Ala Gly  Asp Gly Met Ala Ala  Gly Glu Gly
       3125                 3130                 3135
   Gly Ala  Gln Leu Arg Arg Thr  Gly Leu Val Pro Met  Ala Ala Asp
       3140                 3145                 3150
   Arg Ala  Leu Leu Ala Leu Gln  Gly Ala Leu Asp Arg  Asp Glu Thr
       3155                 3160                 3165
   Ser Leu  Val Val Ala Asp Met  Ala Trp Glu Arg Phe  Ala Pro Val
       3170                 3175                 3180
   Phe Ala  Met Ser Arg Arg Arg  Pro Leu Leu Asp Glu  Leu Pro Glu
       3185                 3190                 3195
   Ala Gln  Gln Ala Leu Ala Asp  Ala Glu Asn Thr Thr  Gly Ala Ala
       3200                 3205                 3210
   Asp Ser  Ala Gly Pro Leu Gln  Arg Ile Val Gly Met  Ala Ala Ala
       3215                 3220                 3225
   Glu Arg  Arg Arg Ala Met Met  Glu Leu Val Leu Ala  Glu Thr Ser
       3230                 3235                 3240
   Ile Val  Leu Gly His Asn Gly  Ser Asp Ala Val Ser  Pro Asp Arg
       3245                 3250                 3255
   Ala Phe  Gln Glu Leu Gly Phe  Asp Ser Leu Met Ala  Val Glu Leu
       3260                 3265                 3270
   Arg Asn  Arg Leu Gly Glu Ala  Thr Gly Leu Ser Leu  Pro Thr Thr
       3275                 3280                 3285
   Leu Ile  Phe Asp Tyr Pro Ser  Pro Ser Ala Leu Ala  Glu Gln Leu
       3290                 3295                 3300
   Val Gly  Glu Leu Val Gly Ala  Gln Pro Ala Thr Thr  Val Val Ala
       3305                 3310                 3315
   Gly Ala  Asp Pro Val Asp Asp  Pro Val Val Val Val  Ala Met Gly
       3320                 3325                 3330
   Cys Arg  Tyr Pro Gly Asp Val  Cys Ser Pro Glu Glu  Leu Trp Gln
       3335                 3340                 3345
   Leu Val  Ser Ala Gly Arg Asp  Ala Val Ser Thr Phe  Pro Thr Asp
       3350                 3355                 3360
   Arg Gly  Trp Asp Cys Asp Ala  Leu Phe Asp Pro Asp  Pro Asp Arg
       3365                 3370                 3375
   Ala Gly  Arg Thr Tyr Val Arg  Glu Gly Ala Phe Leu  Thr Gly Ala
       3380                 3385                 3390
   Asp Arg  Phe Asp Ala Gly Phe  Phe Gly Ile Ser Pro  Arg Glu Ala
       3395                 3400                 3405
   Arg Ala Met Asp Pro Gln Gln Arg Leu Leu Leu Glu Val Ala Trp
   3410 3415 3420
   Glu Val Phe Glu Arg Ala Gly Ile Ala Pro Leu Ser Leu Arg Gly
   3425 3430 3435
   Ser Arg Thr Gly Val Phe Ala Gly Thr Asn Gly Gln Asp His Gly
   3440 3445 3450
   Ala Lys Val Ala Ala Ala Pro Glu Ala Ala Gly His Leu Leu Thr
   3455 3460 3465
   Gly Asn Ala Ala Ser Val Met Ala Gly Arg Ile Ser Tyr Thr Phe
   3470 3475 3480
   Gly Leu Glu Gly Pro Ala Val Ala Val Asp Thr Ala Cys Ser Ser
   3485 3490 3495
   Ser Leu Val Ala Leu His Leu Ala Cys Gln Ser Leu Arg Ser Gly
   3500 3505 3510
   Glu Cys Asp Met Ala Leu Ala Gly Gly Val Thr Val Met Ser Thr
   3515 3520 3525
   Pro Leu Ala Phe Leu Glu Phe Ser Arg Gln Arg Gly Leu Ala Pro
   3530 3535 3540
   Asp Gly Arg Cys Lys Ser Phe Ala Ala Ala Ala Asp Gly Thr Gly
   3545 3550 3555
   Trp Gly Glu Gly Ala Gly Leu Val Leu Leu Glu Arg Leu Ser Asp
   3560 3565 3570
   Ala Arg Arg Asn Gly His Arg Val Leu Ala Val Val Arg Gly Ser
   3575 3580 3585
   Ala Val Asn Gln Asp Gly Ala Ser Asn Gly Leu Thr Ala Pro Asn
   3590 3595 3600
   Gly Pro Ser Gln Gln Arg Val Ile Arg Gln Ala Leu Ala Asn Ala
   3605 3610 3615
   Gly Leu Ser Ala Ser Asp Val Asp Val Val Glu Ala His Gly Thr
   3620 3625 3630
   Gly Thr Gly Leu Gly Asp Pro Ile Glu Ala Gln Ala Leu Ile Ala
   3635 3640 3645
   Ala Tyr Gly Gln Gly Arg Asp Pro Glu Arg Ala Leu Trp Leu Gly
   3650 3655 3660
   Ser Ile Lys Ser Asn Ile Gly His Thr Gln Ala Ala Ala Gly Val
   3665 3670 3675
   Ala Gly Val Ile Lys Met Val Gln Ala Met Arg His Gly Glu Leu
   3680 3685 3690
   Pro Ala Thr Leu His Val Asp Lys Pro Thr Pro Gln Val Asp Trp
   3695 3700 3705
   Ser Ala Gly Ala Val Arg Leu Leu Thr Gly Asn Thr Pro Trp Pro
   3710 3715 3720
   Glu Ser Gly Arg Pro Arg Arg Ala Gly Val Ser Ser Phe Gly Ile
   3725 3730 3735
   Ser Gly Thr Asn Ala His Leu Ile Leu Glu Gln Pro Pro Ser Glu
   3740 3745 3750
   Pro Ala Glu Ile Asp Arg Ser Asn Arg Arg Val Thr Ala His Pro
   3755 3760 3765
   Ala Val Ile Pro Trp Met Leu Ser Ala Arg Ser Leu Thr Ala Leu
   3770 3775 3780
   Gln Ala Gln Ala Ala Ala Leu Gln Gly Arg Leu Asp Arg Val Pro
   3785 3790 3795
   Gly Ala Ser Pro Leu Asp Leu Gly Tyr Ser Leu Ala Thr Thr Arg
   3800 3805 3810
   Ser Val Leu Asp Glu Arg Ala Val Val Trp Gly Ala Asp Arg Glu
   3815 3820 3825
   Thr Leu Leu Ser Arg Leu Ala Ala Leu Ala Asp Gly Arg Thr Ala
   3830 3835 3840
   Pro Gly Val Val Thr Gly Ala Ala Asn Ser Gly Gly Arg Ile Gly
   3845 3850 3855
   Phe Val Phe Ser Gly Gln Gly Ser Gln Trp Leu Gly Met Gly Lys
   3860 3865 3870
   Ala Leu Cys Ala Ala Phe Pro Ala Phe Ala Asp Ala Phe Glu Glu
   3875 3880 3885
   Ala Cys Asp Ala Leu Gly Ala His Leu Gly Ala His Leu Gly Ala
   3890 3895 3900
   Asp Leu Gly Val Asp Val Arg Gly Val Leu Phe Gly Ala Asp Glu
   3905 3910 3915
   Gln Val Leu Asp Arg Thr Leu Trp Ala Gln Pro Gly Ile Phe Ala
   3920 3925 3930
   Val Gln Val Gly Leu Leu Gly Leu Leu Arg Ser Trp Gly Val Arg
   3935 3940 3945
   Pro Asp Ala Val Leu Gly His Ser Val Gly Glu Leu Ala Ala Ala
   3950 3955 3960
   His Ala Ala Gly Val Leu Ser Leu Pro Asp Ala Ala Arg Leu Val
   3965 3970 3975
   Ala Ala Arg Ala Ser Leu Met Gln Ala Leu Pro Thr Gly Gly Ala
   3980 3985 3990
   Met Leu Ala Val Ala Thr Ser Glu Ala Ala Val Glu Pro Leu Leu
   3995 4000 4005
   Ala Gly Met Cys Asp Arg Val Ser Ile Ala Ala Ile Asn Gly Pro
   4010 4015 4020
   Glu Ser Val Val Leu Ser Gly Asp Arg Asp Val Leu Ala Glu Val
   4025 4030 4035
   Ala Gly Glu Leu Asp Ala Arg Gly Leu Arg Thr Lys Trp Leu Arg
   4040 4045 4050
   Val Ser His Ala Phe His Ser His Arg Met Gln Pro Ile Leu Asp
   4055 4060 4065
   Glu Tyr Ala Glu Thr Ala Gly Cys Val Glu Phe Gly Glu Pro Val
   4070 4075 4080
   Val Pro Ile Val Ser Ala Ala Thr Gly Ala Leu Asp Thr Ala Gly
   4085 4090 4095
   Leu Met Cys Ala Ala Gly Tyr Trp Val Arg Gln Val Arg Asp Pro
   4100 4105 4110
   Val Arg Phe Gly Asp Gly Val Gln Ala Leu Val Asp Gln Gly Val
   4115 4120 4125
   Asp Thr Ile Val Glu Phe Gly Pro Asp Gly Ala Leu Ser Ala Leu
   4130 4135 4140
   Val Gln Gln Cys Leu Ala Gly Ser Asp Gln Ala Gly Arg Val Ala
   4145 4150 4155
   Ala Ile Pro Leu Met Arg Arg Asp Arg Asp Glu Val Glu Thr Ala
   4160 4165 4170
   Val Ala Ala Leu Ala His Val His Val Arg Gly Gly Ala Val Asp
   4175 4180 4185
   Trp Ser Ala Cys Phe Ala Gly Thr Gly Ala Arg Thr Val Glu Leu
   4190 4195 4200
   Pro Thr Tyr Ala Phe Gln Arg Gln Arg Tyr Trp Leu Ala Gly Gln
   4205 4210 4215
   Ala Asp Gly Arg Gly Gly Asp Val Val Ala Asp Pro Val Asn Ala
   4220 4225 4230
   Arg Phe Trp Glu Leu Val Glu Arg Ala Asp Pro Glu Pro Leu Val
   4235 4240 4245
   Asp Glu Leu Cys Ile Asp Arg Asp Gln Pro Phe Arg Glu Val Leu
   4250 4255 4260
   Pro Val Leu Ala Ser Trp Arg Glu Lys Gln Arg Gln Lys Ala Val
   4265 4270 4275
   Thr Asp Ser Trp Arg Tyr Gln Val Arg Trp Arg Ser Val Glu Val
   4280 4285 4290
   Gln Ser Ala Ala Ser Leu Arg Gly Val Trp Leu Val Val Leu Pro
   4295 4300 4305
   Ala Asp Gly Leu Arg Asp Gln Pro Ala Ala Val Ile Asp Ala Leu
   4310 4315 4320
   Ile Ala Arg Gly Ala Glu Val Ala Val Leu Glu Leu Thr Glu Gln
   4325 4330 4335
   Asp Phe Gln Arg Gly Ala Leu Val Asp Lys Val Arg Ala Val Ile
   4340 4345 4350
   Ala Asp Arg Thr Glu Val Thr Gly Val Leu Ser Leu Leu Ala Met
   4355 4360 4365
   Asp Gly Met Pro Cys Ala Glu His Pro His Leu Ser Arg Gly Val
   4370 4375 4380
   Ala Ala Thr Val Ile Leu Thr Gln Val Leu Gly Asp Ala Gly Val
   4385 4390 4395
   Ser Ala Pro Leu Trp Leu Ala Thr Thr Gly Gly Val Glu Val Gly
   4400 4405 4410
   Thr Glu Asp Gly Pro Ala Asp Pro Asp His Gly Leu Ile Trp Gly
   4415 4420 4425
   Leu Gly Arg Val Val Gly Leu Glu His Pro Gln Arg Trp Gly Gly
   4430 4435 4440
   Leu Ile Asp Leu Pro Ala Thr Leu Asp Glu Thr Ser Arg Asn Gly
   4445 4450 4455
   Leu Val Ala Ala Leu Ala Gly Thr Ala Ala Glu Asp Gln Leu Ala
   4460 4465 4470
   Val Arg Ser Ser Gly Leu Phe Val Arg Arg Val Val Arg Ala Ala
   4475 4480 4485
   Gln Asn Ser Arg Ser Gly Thr Trp Arg Ser Arg Gly Thr Val Leu
   4490 4495 4500
   Ile Thr Gly Gly Thr Gly Ala Leu Gly Ala Glu Val Ala Arg Trp
   4505 4510 4515
   Leu Ala Arg Arg Gly Ala Glu His Leu Val Leu Ile Ser Arg Arg
   4520 4525 4530
   Gly Pro Glu Ala Pro Gly Ala Ala Asp Leu Gln Ala Glu Leu Thr
   4535 4540 4545
   Glu Leu Gly Val Lys Val Thr Val Val Ala Cys Asp Val Thr Asp
   4550 4555 4560
   Gly Asp Glu Leu Arg Ala Val Leu Ala Ala Val Pro Thr Glu His
   4565 4570 4575
   Pro Leu Ser Ala Val Val His Thr Ala Gly Val Gly Thr Pro Ala
   4580 4585 4590
   Asn Leu Ala Glu Thr Thr Leu Ala Gln Phe Ala Asp Val Leu Ser
   4595 4600 4605
   Ala Lys Val Val Gly Ala Ala Asn Leu Asp Arg Leu Leu Gly Gly
   4610 4615 4620
   Gln Pro Leu Asp Ala Phe Val Leu Phe Ser Ser Ile Ser Gly Val
   4625 4630 4635
   Trp Gly Ala Gly Gly Gln Gly Ala Tyr Ser Ala Ala Asn Ala Tyr
   4640 4645 4650
   Leu Asp Ala Leu Ala Glu Arg Arg Arg Ala Cys Gly Arg Pro Ala
   4655 4660 4665
   Thr Cys Val Ala Trp Gly Pro Trp Ala Gly Ala Gly Met Ala Val
   4670 4675 4680
   Gln Glu Gly Asn Glu Ala His Leu Arg Arg Arg Gly Leu Val Pro
   4685 4690 4695
   Met Glu Pro Gln Ser Ala Leu Ser Ala Leu Gln Gln Ala Leu Ser
   4700 4705 4710
   Arg Arg Glu Thr Ala Ile Thr Val Ala Asp Val Asp Trp Glu Arg
   4715 4720 4725
   Phe Ala Ala Thr Phe Thr Ala Ala Arg Pro Arg Pro Leu Leu Asp
   4730 4735 4740
   Glu Ile Val Asp Leu Arg Pro Asn Thr Glu Thr Ala Glu Lys His
   4745 4750 4755
   Gly Ala Gly Glu Leu Gly Gln Gln Leu Ala Ala Leu Pro Ala Ala
   4760 4765 4770
   Glu Arg Gly His Leu Leu Leu Glu Val Val Leu Ala Glu Thr Ala
   4775 4780 4785
   Asn Thr Leu Gly His Asp Ser Ala Glu Ala Val Gln Pro Asp Arg
   4790 4795 4800
   Thr Phe Ala Glu Leu Gly Phe Asp Ser Leu Thr Ala Val Glu Leu
   4805 4810 4815
   Arg Asn Arg Leu Asn Ala Val Thr Gly Leu Arg Leu Pro Pro Thr
   4820 4825 4830
   Leu Val Phe Asp His Pro Thr Pro Leu Ala Val Ser Glu Gln Leu
   4835 4840 4845
   Val Pro Ala Leu Val Ala Glu Pro Gly Asp Gly Ile Glu Ser Leu
   4850 4855 4860
   Leu Ala Glu Leu Asp Arg Leu Asp Thr Thr Leu Ala Gln Arg Pro
   4865 4870 4875
   Ser Ile Pro Pro Glu Asp Gln Ala Lys Val Ala Glu Arg Leu Gln
   4880 4885 4890
   Ala Leu Ile Ala Lys Trp Asp Gly Ala Arg Asp Gly Thr Ala Lys
   4895 4900 4905
   Val Thr Ser Pro Gln Ser Leu Thr Ala Ala Thr Asp Asp Glu Ile
   4910 4915 4920
   Phe Asp Leu Ile Asp Arg Lys Phe Arg Arg
   4925 4930
<210> 7
   <211> 5564
   <212> PRT
   <213> Saccharopolyspora sp. NRRL30141
<400> 7
   Met Ala Asn Glu Glu Lys Leu Arg Glu Tyr Leu Lys Arg Val Val Val
   1 5 10 15
   Glu Leu Glu Glu Ala His Glu Arg Leu His Glu Leu Glu Arg Gln Glu
   20 25 30
   His Asp Pro Ile Ala Ile Val Ser Met Gly Cys Arg Tyr Pro Gly Gly
   35 40 45
   Val Ser Thr Pro Glu Glu Leu Trp Arg Leu Val Val Asp Gly Gly Asp
   50 55 60
   Ala Ile Ala Asn Phe Pro Glu Asp Arg Gly Trp Asn Leu Gly Glu Leu
   65 70 75 80
   Phe Asp Pro Asp Pro Gly Arg Ala Gly Thr Ser Tyr Val Arg Glu Gly
   85 90 95
   Gly Phe Leu Arg Gly Val Ala Asp Phe Asp Ala Gly Leu Phe Gly Ile
   100 105 110
   Ser Pro Arg Glu Ala Gln Ala Met Asp Pro Gln Gln Arg Leu Leu Leu
   115 120 125
   Glu Ile Ser Trp Glu Val Leu Glu Arg Ala Gly Ile Asp Pro Phe Ser
   130 135 140
   Leu Arg Gly Thr Lys Thr Ser Val Phe Ala Gly Leu Ile Tyr His Asp
   145 150 155 160
   Tyr Ala Ser Arg Phe Ser Lys Thr Pro Ala Glu Phe Glu Gly Tyr Phe
   165 170 175
   Ala Thr Gly Asn Ala Gly Ser Val Ala Ser Gly Arg Val Ala Tyr Thr
   180 185 190
   Phe Gly Leu Glu Gly Pro Ala Val Thr Val Asp Thr Ala Cys Ser Ser
   195 200 205
   Ser Leu Val Ala Leu His Leu Ala Cys Gln Ser Leu Arg Leu Gly Glu
   210 215 220
   Cys Asp Leu Ala Leu Ala Gly Gly Ile Ser Val Met Ala Thr Pro Gly
   225 230 235 240
   Ala Phe Val Glu Phe Ser Arg Gln Arg Ala Leu Ala Ser Asp Gly Arg
   245 250 255
   Cys Lys Pro Phe Ala Asp Ala Ala Asp Gly Thr Gly Trp Gly Glu Gly
   260 265 270
   Ala Gly Met Leu Leu Leu Glu Arg Leu Ser Asp Ala Arg Arg Asn Gly
   275 280 285
   His Pro Val Leu Ala Ala Val Val Gly Ser Ala Ile Asn Gln Asp Gly
   290 295 300
   Met Ser Asn Gly Leu Thr Ala Pro Ser Gly Pro Ala Gln Gln Arg Val
   305 310 315 320
   Ile Arg Gln Ala Leu Thr Asn Ala Gly Leu Ser Pro Ala Glu Val Asp
   325 330 335
   Val Val Glu Ala His Gly Thr Gly Thr Ala Leu Gly Asp Pro Ile Glu
   340 345 350
   Ala Arg Ala Leu Ile Ala Thr Tyr Gly Ala Asn Arg Ser Ala Asp His
   355 360 365
   Pro Leu Leu Leu Gly Ser Leu Lys Ser Asn Ile Gly His Thr Gln Ala
   370 375 380
   Ala Ala Gly Val Ala Gly Val Ile Lys Ser Val Met Ala Ile Arg His
   385 390 395 400
   Arg Glu Met Pro Arg Ser Leu His Ile Asp Gln Pro Ser Arg His Val
   405 410 415
   Asp Trp Ser Ala Gly Ala Val Arg Leu Leu Thr Asp Ser Val Asp Trp
   420 425 430
   Ala Asp Pro Gly Arg Pro Arg Arg Ala Gly Val Ser Ser Phe Gly Met
   435 440 445
   Ser Gly Thr Asn Ala His Leu Ile Val Glu Glu Val Ser Asp Glu Pro
   450 455 460
   Val Ser Gly Ser Thr Glu Pro Thr Gly Ala Leu Pro Trp Pro Leu Ser
   465 470 475 480
   Gly Lys Thr Glu Thr Ala Leu Arg Glu Gln Ala Ala Glu Leu Leu Ser
   485 490 495
   Ala Val Thr Ala His Pro Glu Pro Gly Leu Gly Asn Val Gly Tyr Ser
   500 505 510
   Leu Ala Thr Gly Arg Ala Ala Met Glu His Arg Ala Val Val Val Ala
   515 520 525
   Glu Asp Arg Asp Ser Phe Val Ala Gly Leu Thr Ala Leu Ala Ala Gly
   530 535 540
   Val Pro Ala Ala Asn Val Val Gln Gly Ala Ala Asp Cys Lys Gly Lys
   545 550 555 560
   Val Ala Phe Val Phe Pro Gly Gln Gly Ser His Trp Gln Gly Met Ala
   565 570 575
   Arg Glu Leu Phe Glu Ser Ser Pro Val Phe Arg Arg Lys Leu Glu Glu
   580 585 590
   Cys Ala Ala Ala Thr Ala Pro Tyr Val Asp Trp Ser Leu Leu Gly Val
   595 600 605
   Leu Arg Gly Asp Pro Asp Ala Pro Ala Leu Asp Arg Asp Asp Val Ile
   610 615 620
   Gln Phe Ala Leu Phe Ala Met Met Val Ser Leu Ala Glu Leu Trp Arg
   625 630 635 640
   Ser Cys Gly Val Glu Pro Ala Ala Val Val Gly His Ser Gln Gly Glu
   645 650 655
   Ile Ala Ala Ala His Val Ala Gly Ala Leu Ser Leu Thr Asp Ala Val
   660 665 670
   Arg Ile Val Ala Ala Arg Cys Asn Ala Val Ser Val Leu Ala Gly Lys
   675 680 685
   Gly Gly Met Leu Ala Ile Ala Leu Pro Glu Ser Ala Val Val Lys Arg
   690 695 700
   Ile Ala Gly Leu Pro Glu Leu Thr Val Ala Ala Val Asn Gly Pro Gly
   705 710 715 720
   Ser Thr Val Val Ser Gly Glu Pro Ser Ala Leu Glu Arg Leu Gln Thr
   725 730 735
   Glu Leu Ser Ala Glu Asn Val Gln Ala Arg Arg Val Arg Ile Asp Tyr
   740 745 750
   Ala Ser His Ser Ala Gln Ile Ala Gln Val Gln Gly Arg Leu Leu Asp
   755 760 765
   Arg Leu Gly Glu Val Gly Ser Glu Pro Ala Glu Ile Ala Phe Tyr Ser
   770 775 780
   Thr Val Thr Gly Glu Arg Thr Asp Thr Gly Arg Leu Asp Ala Asp Tyr
   785 790 795 800
   Trp Tyr Gln Asn Leu Arg Gln Pro Val Arg Phe Gln Gln Thr Val Ala
   805 810 815
   Arg Met Ala Asp Gln Gly Tyr Arg Phe Phe Val Glu Val Ser Pro His
   820 825 830
   Pro Leu Leu Thr Ala Gly Ile Gln Glu Thr Leu Glu Ala Ala Asp Ala
   835 840 845
   Asp Ala Gly Gly Val Val Val Gly Ser Leu Arg Gly Gly Glu Gly Gly
   850 855 860
   Ser Arg Arg Trp Leu Thr Ser Leu Ala Glu Cys Gln Val Arg Gly Leu
   865 870 875 880
   Pro Val Asn Trp Glu Gln Val Phe Leu Asp Thr Gly Ala Arg Arg Val
   885 890 895
   Pro Leu Pro Thr Tyr Pro Phe Gln Arg Gln Arg Tyr Trp Leu Glu Ser
   900 905 910
   Ala Glu Tyr Asp Ala Gly Asp Leu Gly Ser Val Gly Leu Arg Ser Ala
   915 920 925
   Glu His Pro Leu Leu Gly Ala Ala Val Thr Leu Ala Asp Ala Gly Gly
   930 935 940
   Phe Leu Leu Thr Gly Lys Leu Ser Val Lys Thr Gln Pro Trp Leu Ala
   945 950 955 960
   Asp His Ala Val Arg Gly Ala Ile Leu Leu Pro Gly Thr Ala Phe Val
   965 970 975
   Glu Met Leu Ile Arg Ala Ala Asp Gln Val Gly Cys Asp Leu Ile Glu
   980 985 990
   Glu Leu Ser Leu Thr Thr Pro Leu Val Leu Pro Ala Thr Gly Ala Val
   995 1000 1005
   Gln Val Gln Ile Ala Val Gly Gly Pro Asp Glu Ala Gly Arg Arg
   1010 1015 1020
   Ser Val Arg Val His Ser Cys Arg Asp Asp Ser Val Pro Gln Asp
   1025 1030 1035
   Ser Trp Thr Cys His Ala Thr Gly Thr Leu Thr Thr Ser Glu His
   1040 1045 1050
   Arg Asp Ala Gly Gln Ala Arg Asp Gly Ile Trp Pro Pro Asn Asp
   1055 1060 1065
   Ala Val Ala Val Pro Leu Asp Ser Phe Tyr Ala Arg Ala Ala Glu
   1070 1075 1080
   Arg Gly Phe Asp Phe Gly Pro Ala Phe Gln Gly Leu Gln Ala Val
   1085 1090 1095
   Trp Lys Arg Gly Asp Glu Ile Phe Ala Glu Val Gly Leu Pro Ala
   1100 1105 1110
   Ala Gln Arg Glu Asp Ala Gly Arg Phe Gly Val His Pro Ala Leu
   1115 1120 1125
   Leu Asp Ala Ala Leu Gln Ala Leu Gly Ala Ala Glu Glu Asp Pro
   1130 1135 1140
   Asp Glu Gly Trp Leu Pro Phe Ala Trp Gln Gly Val Ser Leu Lys
   1145 1150 1155
   Ala Thr Gly Ala Leu Ser Leu Arg Val His Ile Val Pro Ala Gly
   1160 1165 1170
   Ala Asn Ala Val Ser Val Phe Thr Thr Asp Ala Thr Gly Gln Ala
   1175 1180 1185
   Val Leu Ser Ile Asp Ser Leu Val Leu Arg Lys Ile Ser Asp Glu
   1190 1195 1200
   Gln Leu Ala Ala Val Arg Ala Met Asp His Glu Ser Leu Phe Arg
   1205 1210 1215
   Val Asp Trp Arg Arg Ile Ser Pro Gly Ala Ala Lys Pro Val Ser
   1220 1225 1230
   Trp Ala Val Ile Gly Asn Asp Glu Leu Ala Arg Ala Cys Gly Ser
   1235 1240 1245
   Ala Leu Gly Thr Glu Leu His Pro Asp Leu Thr Gly Leu Ala Asp
   1250 1255 1260
   Pro Pro Pro Asp Val Val Val Val Pro Cys Gly Ala Phe His Gln
   1265 1270 1275
   Asp Leu Glu Val Ala Ser Glu Ala Arg Ala Ala Thr Gln Arg Val
   1280 1285 1290
   Leu Asp Leu Ile Gln Gly Trp Leu Ala Ala Glu Arg Phe Ala Gly
   1295 1300 1305
   Ser Arg Leu Val Val Val Thr Cys Gly Ala Val Ser Thr Gly Pro
   1310 1315 1320
   Ala Glu Gly Val Ser Asp Leu Val His Ala Ala Ser Trp Gly Leu
   1325 1330 1335
   Leu Arg Ser Ala Gln Ser Glu Asn Pro Asn Arg Phe Val Leu Val
   1340 1345 1350
   Asp Val Asp Ala Thr Ala Glu Ser Trp Arg Ala Leu Ala Ala Ala
   1355 1360 1365
   Val Arg Ser Gly Glu Pro Gln Leu Ala Leu Arg Ala Gly Glu Val
   1370 1375 1380
   Arg Val Pro Arg Leu Thr Arg Cys Val Ala Ala Glu Asp Ser Arg
   1385 1390 1395
   Ile Pro Val Pro Gly Ala Asp Gly Thr Val Leu Ile Ser Gly Gly
   1400 1405 1410
   Thr Gly Leu Leu Gly Gly Leu Val Ala Arg His Leu Val Ala Glu
   1415 1420 1425
   Arg Gly Val Arg Arg Leu Val Leu Ala Gly Arg Arg Gly Trp Ser
   1430 1435 1440
   Ala Pro Gly Val Thr Glu Leu Val Asp Glu Leu Val Gly Leu Gly
   1445 1450 1455
   Ala Val Val Glu Val Ala Ser Cys Asp Val Gly Asp Arg Ala Gln
   1460 1465 1470
   Leu Asp Arg Leu Leu Thr Thr Ile Ser Ala Glu Phe Pro Leu Arg
   1475 1480 1485
   Gly Val Val His Ala Ala Gly Ala Leu Ala Asp Gly Val Val Glu
   1490 1495 1500
   Ser Leu Thr Pro Glu His Val Ala Lys Val Phe Gly Pro Lys Val
   1505 1510 1515
   Ala Gly Ala Trp His Leu His Glu Leu Thr Arg Glu Leu Asp Leu
   1520 1525 1530
   Ser Phe Phe Val Leu Phe Ser Ser Phe Ser Gly Val Val Gly Ala
   1535 1540 1545
   Ala Gly Gln Gly Asn Tyr Ala Ala Ala Asn Ala Phe Leu Asp Gly
   1550 1555 1560
   Leu Ala Gln His Arg Arg Thr Ala Gly Leu Pro Ala Val Ser Leu
   1565 1570 1575
   Ala Trp Gly Leu Trp Glu Pro Thr Ser Gly Met Thr Gly Ala Leu
   1580 1585 1590
   Asp Ala Ala Asp Arg Ser Arg Ile Ser Arg Thr Asn Pro Pro Met
   1595 1600 1605
   Ser Ala Glu Asp Gly Leu Arg Leu Phe Glu Met Ala Phe His Val
   1610 1615 1620
   Pro Gly Glu Ser Leu Leu Val Pro Val His Ile Asp Leu Asn Ala
   1625 1630 1635
   Leu Arg Ala Asp Ala Ala Asp Gly Gly Val Pro Ala Leu Leu His
   1640 1645 1650
   Asp Leu Val Pro Ala Pro Val Arg Arg Ser Ala Val Asn Glu Ser
   1655 1660 1665
   Glu Asp Val Thr Gly Leu Val Gly Arg Leu Arg Arg Leu Pro Asp
   1670 1675 1680
   Leu Asp Gln Glu Thr Leu Leu Leu Gly Leu Val Arg Glu His Val
   1685 1690 1695
   Ser Ala Val Leu Gly Tyr Ser Gly Ala Val Glu Val Gly Val Glu
   1700 1705 1710
   Arg Ala Phe Arg Asp Leu Gly Phe Asp Ser Leu Ser Gly Val Glu
   1715 1720 1725
   Leu Arg Asn Arg Leu Gly Gly Val Leu Gly Val Arg Leu Pro Ala
   1730 1735 1740
   Thr Ala Val Phe Asp Tyr Pro Thr Pro Arg Ala Leu Val Arg Phe
   1745 1750 1755
   Leu Arg Asp Lys Leu Ile Gly Gly Val Glu Ala Arg Asn Ser Ala
   1760 1765 1770
   Pro Ala Val Val Glu Ala Ala Ser Gly Asp Asp Pro Val Val Ile
   1775 1780 1785
   Val Gly Met Gly Cys Arg Phe Pro Gly Gly Val Ser Ser Pro Glu
   1790 1795 1800
   Glu Leu Trp Arg Leu Val Ala Gly Gly Leu Asp Ala Val Ala Glu
   1805 1810 1815
   Phe Pro Asp Asp Arg Gly Trp Asp Gln Ala Gly Leu Phe Asp Pro
   1820 1825 1830
   Asp Pro Asp Arg Leu Gly Thr Ser Tyr Val Cys Glu Gly Gly Phe
   1835 1840 1845
   Leu Arg Asp Ala Ala Glu Phe Asp Ala Gly Phe Phe Gly Ile Ser
   1850 1855 1860
   Pro Arg Glu Ala Leu Ala Met Asp Pro Gln Gln Arg Leu Leu Leu
   1865 1870 1875
   Glu Ile Ala Trp Glu Thr Leu Glu Arg Ala Gly Ile Asp Pro Leu
   1880 1885 1890
   Ser Leu Arg Gly Ser Arg Thr Gly Val Phe Ala Gly Leu Met His
   1895 1900 1905
   His Asp Tyr Gly Ala Arg Phe Val Thr Arg Ala Pro Glu Gly Phe
   1910 1915 1920
   Glu Gly Tyr Leu Gly Asn Gly Ser Ala Gly Gly Val Phe Ser Gly
   1925 1930 1935
   Arg Val Ala Tyr Ser Phe Gly Phe Glu Gly Pro Ala Val Thr Val
   1940 1945 1950
   Asp Thr Ala Cys Ser Ser Ser Leu Val Ser Met His Leu Ala Gly
   1955 1960 1965
   Gln Ala Leu Arg Ser Gly Glu Cys Asp Leu Ala Leu Ala Gly Gly
   1970 1975 1980
   Val Thr Val Met Ala Thr Pro Gly Met Phe Val Glu Phe Ser Arg
   1985 1990 1995
   Gln Arg Gly Leu Ala Ala Asp Gly Arg Cys Lys Ser Phe Ala Ala
   2000 2005 2010
   Ala Ala Asp Gly Thr Gly Trp Gly Glu Gly Ala Gly Leu Val Leu
   2015 2020 2025
   Leu Glu Arg Leu Ser Asp Ala Arg Arg Asn Gly His Ala Val Leu
   2030 2035 2040
   Ala Val Val Arg Gly Ser Ala Val Asn Gln Asp Gly Ala Ser Asn
   2045 2050 2055
   Gly Leu Thr Ala Pro Asn Gly Pro Ser Gln Gln Arg Val Ile Thr
   2060 2065 2070
   Gln Ala Leu Ala Ser Ala Gly Leu Ser Val Ser Asp Val Asp Ala
   2075 2080 2085
   Val Glu Ala His Gly Thr Gly Thr Arg Leu Gly Asp Pro Ile Glu
   2090 2095 2100
   Ala Gln Ala Leu Ile Ala Thr Tyr Gly Gln Glu Arg Asp Arg Asp
   2105 2110 2115
   Arg Pro Leu Trp Leu Gly Ser Val Lys Ser Asn Ile Gly His Thr
   2120 2125 2130
   Gln Ala Ala Ala Gly Val Ala Gly Val Ile Lys Met Val Met Ala
   2135 2140 2145
   Met Arg His Glu Gln Leu Pro Ala Thr Leu His Val Asp Glu Pro
   2150 2155 2160
   Thr Pro Glu Val Asp Trp Ser Ala Gly Glu Val Gln Leu Leu Thr
   2165 2170 2175
   Glu Asn Thr Pro Trp Pro Asp Ser Gly His Pro Arg Arg Ala Gly
   2180 2185 2190
   Val Ser Ser Phe Gly Ile Ser Gly Thr Asn Ala His Val Ile Leu
   2195 2200 2205
   Glu Gln Ala Ser Asn Thr Pro Asp Glu Ile Ala Gln Ser Asn Gly
   2210 2215 2220
   Pro Glu Ser Glu Ser Thr Val Asp Ile Pro Ala Val Pro Leu Ile
   2225 2230 2235
   Val Ser Gly Arg Thr Pro Glu Ala Leu Ser Ala Gln Ala Ser Ala
   2240 2245 2250
   Leu Met Ser Tyr Leu Asp Asn Arg Pro Asp Ile Ser Ser Leu Asp
   2255 2260 2265
   Ala Ala Phe Ser Leu Ala Ser Ser Arg Ala Ala Leu Glu Glu Arg
   2270 2275 2280
   Ala Val Val Leu Gly Ala Asp Arg Glu Ala Leu Leu Ser Gly Leu
   2285 2290 2295
   Glu Ala Leu Ala Ala Gly Arg Asp Ala Ser Gly Val Val Ser Gly
   2300 2305 2310
   Ser Leu Ile Ser Gly Gly Val Gly Phe Val Phe Ser Gly Gln Gly
   2315 2320 2325
   Gly Gln Trp Leu Gly Met Gly Arg Gly Leu Tyr Ser Ala Phe Pro
   2330 2335 2340
   Val Phe Ala Asp Ala Phe Asp Glu Ala Cys Ala Gly Leu Asp Ala
   2345 2350 2355
   His Leu Gly Gln Gln Val Gly Val Arg Asp Val Val Phe Gly Ser
   2360 2365 2370
   Asp Gly Ser Leu Leu Asp Arg Thr Leu Trp Ala Gln Ser Gly Leu
   2375 2380 2385
   Phe Ala Leu Gln Val Gly Leu Leu Arg Leu Leu Gly Ser Trp Gly
   2390 2395 2400
   Val Arg Pro Gly Val Val Met Gly His Ser Val Gly Glu Phe Ala
   2405 2410 2415
   Ala Ala Phe Ala Ala Gly Val Leu Ser Leu Pro Asp Ala Ala Arg
   2420 2425 2430
   Leu Val Ala Gly Arg Ala Arg Leu Met Gln Ala Leu Pro Asp Gly
   2435 2440 2445
   Gly Ala Met Leu Ala Val Ala Ala Gly Glu Glu Gln Leu Arg Pro
   2450 2455 2460
   Leu Leu Ala Ala Arg Gly Glu Gly Val Gly Ile Ala Ala Val Asn
   2465 2470 2475
   Ala Ser Glu Ser Val Val Leu Ser Gly Asp Arg Glu Val Leu Glu
   2480 2485 2490
   Asp Ile Ala Gly Gly Leu Asp Gly Gln Gly Val Arg Trp Arg Trp
   2495 2500 2505
   Leu Arg Val Ser His Ala Phe His Ser Tyr Arg Met Asp Pro Met
   2510 2515 2520
   Leu Gln Glu Phe Thr Asp Ile Ala Gly Ser Val Asp Tyr Arg Arg
   2525 2530 2535
   Cys Asp Leu Pro Val Val Ser Thr Leu Thr Gly Glu Leu Asp Thr
   2540 2545 2550
   Ala Gly Met Leu Ala Thr Pro Gly Tyr Trp Val Arg Gln Val Arg
   2555 2560 2565
   Glu Pro Val Arg Phe Ala Asp Gly Val Arg Ala Leu Ala Gln Gln
   2570 2575 2580
   Gly Val Gly Thr Ile Phe Glu Leu Gly Pro Asp Ala Ile Leu Ser
   2585 2590 2595
   Ala Leu Ile Pro Asp Cys His Ser Trp Gly Asp Gln Thr Val Pro
   2600 2605 2610
   Ile Pro Leu Leu Arg Lys Asp Arg Ala Glu Pro Glu Thr Val Val
   2615 2620 2625
   Ala Ala Val Ala Arg Ala His Thr Arg Gly Val Gln Val Asp Trp
   2630 2635 2640
   Ser Ala Phe Phe Ala Gly Thr Gly Ala Gly Arg Val Glu Leu Pro
   2645 2650 2655
   Thr Tyr Ala Phe Gln Arg Gln Arg Tyr Trp Leu Glu Ser Ser Val
   2660 2665 2670
   Ser Gly Asp Val Thr Gly Ile Gly Leu Ala Gly Ala Glu His Pro
   2675 2680 2685
   Leu Leu Gly Ala Val Val Val Leu Ala Asp Gly Asp Gly Met Val
   2690 2695 2700
   Leu Thr Gly Arg Leu Ser Val Gly Thr His Arg Trp Leu Ala Glu
   2705 2710 2715
   His Arg Val Leu Gly Glu Val Val Val Pro Gly Thr Ala Ile Leu
   2720 2725 2730
   Glu Met Val Leu His Ala Gly Ala Arg Val Gly Cys Gly Arg Val
   2735 2740 2745
   Glu Glu Leu Thr Leu Glu Ala Pro Leu Val Val Pro Glu Arg Asp
   2750 2755 2760
   Ala Ile Glu Ile Gln Leu Leu Val Asn Ala Pro Asp Asp Lys Gly
   2765 2770 2775
   Arg Arg Ser Val Ser Leu His Ser Arg Pro Ala Gly Gly Ser Gly
   2780 2785 2790
   Gly Gly Gly Trp Thr Arg His Ala Thr Gly Glu Leu Val Val Ala
   2795 2800 2805
   Gly Thr Gly Gly Gly Ala Val Thr Gly Trp Ser Thr Glu Gly Ala
   2810 2815 2820
   Glu Pro Val Ala Leu Gly Glu Phe Tyr Val Val Gln Ala Gly Asn
   2825 2830 2835
   Gly Phe Glu Tyr Gly Pro Leu Phe Gln Gly Leu Arg Ala Ala Trp
   2840 2845 2850
   Arg Arg Gly Gly Glu Val Leu Ala Glu Val Ala Leu Pro Ala Ala
   2855 2860 2865
   Ala Gly Ala Met Ala Gly Phe Leu Ile Asn Pro Ala Leu Leu Asp
   2870 2875 2880
   Ala Ala Leu Gln Ala Ser Ala Leu Gly Asp Arg Pro Ala Glu Gly
   2885 2890 2895
   Gly Ala Trp Leu Pro Phe Ser Phe Thr Gly Val Glu Leu Ser Gly
   2900 2905 2910
   Gln Gly Gly Thr Ile Ser Arg Ala Arg Val Glu Ser Thr Arg Pro
   2915 2920 2925
   Asp Ala Val Ser Val Ala Val Met Asp Glu Gly Gly Arg Leu Leu
   2930 2935 2940
   Ala Ser Ile Asp Ser Leu Arg Leu Arg Pro Val Ser Ser Val Arg
   2945 2950 2955
   Leu Ala Asn Arg Asp Val Val Gly Asp Ala Leu Phe Glu Val Thr
   2960 2965 2970
   Trp Glu Pro Val Ala Thr Arg Ser Thr Val Ser Gly Arg Trp Ala
   2975 2980 2985
   Leu Leu Gly Asp Ala Val Gly Gly Met Ala Gly Leu Ile Gly Leu
   2990 2995 3000
   Ala Pro Gly Ser Val Asp Arg Cys Ala Gly Leu Ala Glu Leu Ala
   3005 3010 3015
   Gly Asn Leu Asp Ser Gly Ala Leu Val Ala Asp Val Val Val Tyr
   3020 3025 3030
   Cys Ala Gly Glu Gln Ala Asp Pro Asp Ala Gly Val Ala Ala Leu
   3035 3040 3045
   Ala Glu Thr Arg Glu Met Leu Ala Leu Val Gln Ser Trp Leu Ala
   3050 3055 3060
   Glu Glu Arg Leu Ala Gly Ser Arg Leu Val Val Val Thr Cys Gly
   3065 3070 3075
   Ala Val Thr Thr Ala Ala Gly Asp Gly Ala Ser Lys Leu Ala His
   3080 3085 3090
   Ala Pro Leu Trp Gly Leu Leu Arg Ser Ala Gln Ser Glu Asn Pro
   3095 3100 3105
   Gly Arg Phe Val Leu Val Asp Val Asp Gly Thr Ala Glu Ser Trp
   3110 3115 3120
   Arg Ala Leu Pro Ser Ala Val Gly Ser Met Gln Pro Gln Leu Ala
   3125 3130 3135
   Val Arg Lys Gly Val Val Thr Val Pro Arg Val Ala Ser Val Pro
   3140 3145 3150
   Gly Pro Val Glu Val Pro Ala Val Val Ala Gly Pro Asp Arg Thr
   3155 3160 3165
   Val Leu Ile Ser Gly Gly Thr Gly Leu Leu Gly Gly Val Val Ala
   3170 3175 3180
   Arg His Leu Val Ala Glu Arg Gly Val Arg Arg Val Val Leu Thr
   3185 3190 3195
   Gly Arg Arg Gly Trp Asp Ala Pro Gly Ile Thr Glu Leu Val Gly
   3200 3205 3210
   Glu Leu Glu Gly Phe Gly Ala Val Val Asp Val Val Ala Cys Asp
   3215 3220 3225
   Val Ala Asp Arg Ala Gly Leu Glu Gly Leu Leu Ala Ala Val Pro
   3230 3235 3240
   Ala Glu Phe Pro Leu Cys Gly Val Val His Ala Ala Gly Val Leu
   3245 3250 3255
   Ala Asp Gly Val Ile Glu Ser Leu Thr Pro Glu Asp Val Gly Ala
   3260 3265 3270
   Val Phe Gly Pro Lys Ala Ala Gly Ala Trp Asn Leu His Glu Leu
   3275 3280 3285
   Thr Arg Asp Met Asp Leu Ser Phe Phe Ala Leu Phe Ser Ser Leu
   3290 3295 3300
   Ser Gly Val Thr Gly Ala Ala Gly Gln Gly Asn Tyr Ala Ala Ala
   3305 3310 3315
   Asn Thr Phe Leu Asp Ala Leu Ala His Tyr Arg Arg Ala Gln Gly
   3320 3325 3330
   Leu Pro Ala Val Ser Leu Ala Trp Gly Leu Trp Glu Gln Ser Ser
   3335 3340 3345
   Gly Met Thr Gly Arg Leu Ser Asp Val Asp Arg Ser Arg Ile Ala
   3350 3355 3360
   Arg Ser Ser Pro Pro Leu Ser Thr Lys Asp Gly Leu Arg Leu Phe
   3365 3370 3375
   Asp Ala Gly Leu Ala Leu Asp Arg Ala Ala Val Val Pro Ala Arg
   3380 3385 3390
   Leu Asp Arg Ala Phe Leu Ala Glu Gln Ala Arg Ser Gly Thr Leu
   3395 3400 3405
   Pro Ala Met Leu Thr Ala Leu Val Pro Thr Ile Thr Ser Ile Arg
   3410 3415 3420
   Arg Ser Ser Gly Thr Asp Leu Ala Asp Glu Asp Ala Leu Leu Gly
   3425 3430 3435
   Val Val Arg Glu His Ala Ala Arg Val Leu Gly Tyr Ser Gly Ala
   3440 3445 3450
   Ala Glu Val Gly Val Glu Arg Ala Phe Arg Asp Leu Gly Phe Asp
   3455 3460 3465
   Ser Leu Ser Gly Val Glu Leu Arg Asn Arg Leu Ala Gly Val Leu
   3470 3475 3480
   Gly Ala Arg Leu Pro Ala Thr Ala Val Phe Asp Tyr Pro Thr Pro
   3485 3490 3495
   Arg Ala Leu Ala Arg Phe Leu His Gln Glu Leu Ala Gly Glu Val
   3500 3505 3510
   Gly Thr Thr Pro Ala Pro Val Thr Thr Thr Thr Ala Ser Val Glu
   3515 3520 3525
   Asp Asp Leu Val Ala Ile Val Gly Met Gly Cys Arg Tyr Pro Gly
   3530 3535 3540
   Gly Val Ser Ser Pro Glu Glu Leu Trp Arg Leu Val Ala Gly Gly
   3545 3550 3555
   Val Asp Ala Val Ala Asp Phe Pro Asp Asp Arg Gly Trp Asp Leu
   3560 3565 3570
   Ala Gly Leu Phe Asp Pro Asp Pro Asp Arg Phe Gly Thr Ser Tyr
   3575 3580 3585
   Val Arg Glu Gly Gly Phe Leu Arg Asp Ala Ala Glu Phe Asp Ala
   3590 3595 3600
   Ala Phe Phe Gly Ile Ser Pro Arg Glu Ala Leu Ala Met Asp Pro
   3605 3610 3615
   Gln Gln Arg Leu Leu Leu Glu Leu Ser Trp Glu Ala Val Glu Arg
   3620 3625 3630
   Ala Gly Ile Asp Pro Gly Ser Leu Arg Gly Ser Arg Thr Gly Val
   3635 3640 3645
   Phe Ala Gly Leu Met Tyr His Asp Tyr Ala Gly Arg Phe Ala Ala
   3650 3655 3660
   Gly Val Pro Glu Gly Phe Glu Gly Tyr Leu Gly Asn Gly Ser Ala
   3665 3670 3675
   Gly Ser Val Ala Ser Gly Arg Val Ala Tyr Ser Phe Gly Phe Glu
   3680 3685 3690
   Gly Pro Ala Val Thr Val Asp Thr Ala Cys Ser Ser Ser Leu Val
   3695 3700 3705
   Ala Leu His Leu Ala Gly Gln Ser Leu Arg Ser Gly Glu Cys Asp
   3710 3715 3720
   Leu Ala Leu Ala Gly Gly Val Thr Val Met Ala Thr Pro Ala Thr
   3725 3730 3735
   Phe Val Glu Phe Ser Arg Gln Arg Gly Leu Ala Pro Asp Gly Arg
   3740 3745 3750
   Cys Lys Ser Phe Ala Glu Ala Ala Asp Gly Thr Gly Trp Gly Glu
   3755 3760 3765
   Gly Ala Gly Leu Val Leu Leu Glu Arg Leu Ser Asp Ala Arg Arg
   3770 3775 3780
   Asn Gly His Arg Val Leu Ala Val Val Arg Gly Ser Ala Val Asn
   3785 3790 3795
   Gln Asp Gly Ala Ser Asn Gly Leu Thr Ala Pro Asn Gly Pro Ser
   3800 3805 3810
   Gln Gln Arg Val Ile Thr Gln Ala Leu Thr Ser Ala Gly Leu Ser
   3815 3820 3825
   Val Ser Asp Val Asp Ala Val Glu Ala His Gly Thr Gly Thr Arg
   3830 3835 3840
   Leu Gly Asp Pro Ile Glu Ala Gln Ala Leu Ile Ala Thr Tyr Gly
   3845 3850 3855
   Arg Asp Arg Asp Pro Asp Arg Pro Leu Trp Leu Gly Ser Met Lys
   3860 3865 3870
   Ser Asn Ile Gly His Thr Gln Ala Ala Ala Gly Val Ala Gly Val
   3875 3880 3885
   Ile Lys Met Val Met Ala Met Arg His Gly Glu Leu Pro Arg Thr
   3890 3895 3900
   Leu His Val Gly Glu Pro Thr Ser Glu Val Asp Trp Ser Ala Gly
   3905 3910 3915
   Ser Val Gln Leu Leu Thr Glu Asn Thr Pro Trp Pro Asp Ser Gly
   3920 3925 3930
   His Pro Arg Arg Ala Gly Val Ser Ser Phe Gly Ile Ser Gly Thr
   3935 3940 3945
   Asn Ala His Val Ile Leu Glu Gln Ser Pro Thr Ala Ser Ser Glu
   3950 3955 3960
   Phe Val Glu His Ser Gly Pro Asp Ser Glu Ser Ala Val Asn Val
   3965 3970 3975
   Pro Val Val Pro Trp Val Val Ser Gly Lys Thr Pro Glu Ala Leu
   3980 3985 3990
   Ser Ala Gln Ala Asp Thr Leu Val Ser Tyr Leu Asp Asp Arg Ser
   3995 4000 4005
   Asp Val Ser Ser Arg Asp Val Gly Tyr Ser Leu Ala Met Thr Arg
   4010 4015 4020
   Ser Ala Leu Asp Glu Arg Ala Val Val Leu Gly Ser Asp Arg Glu
   4025 4030 4035
   Thr Leu Leu Ser Gly Leu Lys Ala Leu Ala Ala Gly His Glu Ala
   4040 4045 4050
   Thr Gly Val Val Thr Gly Ser Val Gly Ser Gly Gly Arg Pro Gly
   4055 4060 4065
   Phe Val Phe Ala Gly Gln Gly Gly Gln Trp Leu Gly Met Gly Arg
   4070 4075 4080
   Gly Leu Tyr Arg Ala Phe Pro Val Phe Ala Asp Ala Phe Asp Glu
   4085 4090 4095
   Ala Cys Ala Gly Leu Asp Ala His Leu Gly Gln Glu Val Gly Val
   4100 4105 4110
   Arg Asp Val Val Phe Gly Ser Asp Ala Gln Leu Leu Asp Arg Thr
   4115 4120 4125
   Leu Trp Ala Gln Ser Gly Leu Phe Ala Leu Gln Val Gly Leu Leu
   4130 4135 4140
   Lys Leu Leu Gly Ser Trp Gly Val Arg Pro Val Val Val Leu Gly
   4145 4150 4155
   His Ser Val Gly Glu Leu Ala Ala Ala Phe Ala Ala Gly Val Leu
   4160 4165 4170
   Ser Met Ala Glu Ala Ala Arg Leu Val Ala Gly Arg Ala Arg Leu
   4175 4180 4185
   Met Gln Ala Leu Pro Ser Gly Gly Ala Met Leu Ala Val Ala Ala
   4190 4195 4200
   Thr Glu Asp Arg Ile Ser Pro Leu Leu Asp Gly Val Arg Asp Arg
   4205 4210 4215
   Val Gly Val Ala Ala Val Asn Ala Pro Gly Ser Ala Val Leu Ser
   4220 4225 4230
   Gly His Arg Asp Val Leu Glu Asp Val Val Gly Arg Leu Asp Gly
   4235 4240 4245
   Leu Gly Val Arg Trp Arg Trp Leu Arg Val Ser His Ala Phe His
   4250 4255 4260
   Ser Tyr Arg Met Asp Pro Met Leu Asp Glu Phe Ala Asp Ile Ala
   4265 4270 4275
   Arg Ser Val Asp Tyr Arg Ser Pro Gly Leu Pro Ile Val Ser Thr
   4280 4285 4290
   Leu Thr Gly Asn Leu Asp Asp Val Gly Val Met Ala Thr Pro Glu
   4295 4300 4305
   Tyr Trp Val Arg Gln Val Arg Glu Pro Val Arg Phe Ala Asp Gly
   4310 4315 4320
   Val Gln Ala Leu Val Asn Gln Gly Val Asp Thr Ile Val Glu Leu
   4325 4330 4335
   Gly Pro Asp Gly Val Leu Ser Ser Leu Val His Glu Cys Val Ser
   4340 4345 4350
   Glu Ser Gly Arg Val Thr Gly Ile Pro Leu Val Arg Lys Asp Arg
   4355 4360 4365
   Asp Glu Val Pro Thr Val Leu Ala Ala Leu Ala Gln Ile His Thr
   4370 4375 4380
   Arg Gly Gly Ala Val Asp Trp Gly Ser Phe Phe Ala Gly Thr Gly
   4385 4390 4395
   Ala Lys Gln Val Glu Leu Pro Thr Tyr Ala Phe Gln Arg Arg Arg
   4400 4405 4410
   Tyr Trp Leu Glu Pro Ser Asp Ser Gly Asp Val Thr Gly Ala Gly
   4415 4420 4425
   Leu Thr Gly Ala Glu His Pro Leu Leu Gly Ala Val Val Pro Val
   4430 4435 4440
   Ala Gly Ala Asp Glu Val Leu Leu Thr Gly Arg Leu Ser Val Gly
   4445 4450 4455
   Thr His Pro Trp Leu Ala Asp His Arg Val Leu Gly Glu Val Val
   4460 4465 4470
   Val Pro Gly Thr Ala Leu Leu Glu Met Ala Trp Arg Ala Gly Ser
   4475 4480 4485
   Gln Val Gly Cys Glu Arg Val Glu Glu Leu Thr Leu Glu Ala Pro
   4490 4495 4500
   Leu Val Leu Pro Glu Arg Gly Ala Ala Ala Val Gln Leu Ala Val
   4505 4510 4515
   Gly Ala Pro Asp Glu Ala Gly Arg Arg Ser Leu Gln Leu Tyr Ser
   4520 4525 4530
   Arg Gly Ala Asp Glu Asp Gly Asp Trp Arg Arg Ile Ala Ser Gly
   4535 4540 4545
   Leu Leu Ala Gln Ala Ser Val Val Pro Pro Ala Asp Ser Thr Ala
   4550 4555 4560
   Trp Pro Pro Asp Gly Ala Val Gln Val Asp Leu Ala Glu Phe Tyr
   4565 4570 4575
   Glu Arg Leu Ala Glu Arg Gly Leu Thr Tyr Gly Pro Val Phe Gln
   4580 4585 4590
   Gly Leu Arg Ala Ala Trp Arg Tyr Gly Asp Asp Ile Phe Ala Glu
   4595 4600 4605
   Leu Ala Val Ser Pro Asp Ala Ala Gly Phe Gly Ile His Pro Ala
   4610 4615 4620
   Leu Leu Asp Ala Ala Leu His Ala Met Ala Leu Gly Ala Ser Pro
   4625 4630 4635
   Asp Ser Glu Ala Arg Leu Pro Phe Ser Trp Ser Gly Ala Gln Leu
   4640 4645 4650
   Tyr Arg Ala Gly Gly Ala Ala Leu Arg Val Arg Leu Ser Pro Leu
   4655 4660 4665
   Gly Thr Gly Ala Val Ser Leu Thr Leu Met Asp Ala Ala Gly Gly
   4670 4675 4680
   Gln Val Ala Ala Val Glu Ser Leu Ser Thr Arg Pro Val Ser Ala
   4685 4690 4695
   Asp Gln Ile Gly Ala Gly Arg Gly Asp His Glu Arg Leu Leu His
   4700 4705 4710
   Val Glu Trp Val Arg Pro Ala Glu Ser Ala Gly Met Ser Leu Thr
   4715 4720 4725
   Ser Cys Ala Val Val Gly Leu Asp Glu Pro Glu Trp His Ala Ala
   4730 4735 4740
   Leu Lys Ala Thr Gly Val Gln Val Glu Ser His Ala Asp Leu Ala
   4745 4750 4755
   Ser Leu Ala Thr Glu Val Ala Lys Arg Gly Ser Ala Pro Gly Ala
   4760 4765 4770
   Val Ile Val Pro Cys Pro Arg Pro Gln Ala Met Glu Glu Leu Pro
   4775 4780 4785
   Thr Ala Ala Arg Arg Ala Thr Gln Gln Ala Met Ala Leu Leu Gln
   4790 4795 4800
   Glu Trp Leu Ala Asp Asp Arg Phe Val Ser Thr Arg Leu Ile Leu
   4805 4810 4815
   Leu Thr His Arg Ala Val Ala Ala Val Ala Gly Glu Asp Val Phe
   4820 4825 4830
   Asp Leu Val His Ala Pro Leu Trp Gly Leu Val Arg Ser Ala Gln
   4835 4840 4845
   Ala Glu His Pro Asp Arg Phe Ala Leu Ile Asp Val Asp Glu Ala
   4850 4855 4860
   Glu Ala Ser Arg Ala Ala Leu Ala Glu Ala Leu Thr Ala Gly Glu
   4865 4870 4875
   Ala Gln Leu Ala Val Arg Ser Gly Val Val Leu Val Pro Arg Leu
   4880 4885 4890
   Gly Gln Val Lys Ala Ser Gly Gly Glu Ala Phe Arg Trp Asp Glu
   4895 4900 4905
   Gly Thr Val Leu Val Thr Gly Gly Thr Gly Gly Leu Gly Ala Leu
   4910 4915 4920
   Leu Ala Arg His Leu Val Ser Ala His Gly Val Arg His Leu Leu
   4925 4930 4935
   Leu Ala Ser Arg Arg Gly Leu Ala Ala Pro Gly Ala Asp Glu Leu
   4940 4945 4950
   Val Ala Glu Leu Glu Gln Ser Gly Ala Asp Val Ala Val Val Ala
   4955 4960 4965
   Cys Asp Ala Ala Asp Arg Asp Ser Leu Ala Arg Leu Val Ala Ser
   4970 4975 4980
   Val Pro Ala Glu Asn Pro Leu Arg Ala Val Val His Ala Ala Gly
   4985 4990 4995
   Val Leu Asp Asp Gly Val Leu Met Ser Met Ser Pro Glu Arg Leu
   5000 5005 5010
   Asp Ala Val Leu Arg Ser Lys Val Asp Ala Ala Trp Tyr Leu His
   5015 5020 5025
   Glu Leu Thr Arg Glu Leu Gly Leu Ser Ala Phe Val Leu Phe Ser
   5030 5035 5040
   Ser Val Ala Gly Leu Leu Gly Gly Ala Gly Gln Ser Asn Tyr Ala
   5045 5050 5055
   Ala Gly Asn Ala Phe Leu Asp Ala Leu Ala His Cys Arg Gln Ala
   5060 5065 5070
   Gln Gly Leu Pro Ala Leu Ser Leu Ala Ser Gly Leu Trp Ala Ser
   5075 5080 5085
   Ile Asp Gly Met Ala Gly Asp Leu Ala Ala Ala Asp Val Glu Arg
   5090 5095 5100
   Leu Ser Arg Ala Gly Ile Ala Pro Leu Ser Ala Pro Gly Gly Leu
   5105 5110 5115
   Ala Leu Phe Asp Ala Ala Ile Arg Ser Asp Glu Pro Leu Leu Ala
   5120 5125 5130
   Pro Val Arg Leu Asp Val Glu Ala Leu Arg Val Gln Ala Arg Ser
   5135 5140 5145
   Ala Glu Thr Arg Ile Pro Glu Met Leu His Gly Met Ala Met Gly
   5150 5155 5160
   Pro Ser Arg Arg Thr Ser Phe Ser Ser Arg Val Glu Pro Leu Gln
   5165 5170 5175
   Glu Arg Leu Ala Gly Leu Ser Glu Asp Glu Arg Arg Gln Gln Val
   5180 5185 5190
   Leu Gln Arg Val Arg Ala Asp Ile Ala Val Val Leu Gly His Gly
   5195 5200 5205
   Lys Ser Asn Asp Val Asp Thr Glu Lys Pro Leu Ala Glu Leu Gly
   5210 5215 5220
   Phe Asp Ser Leu Thr Ala Ile Glu Leu Arg Asn Arg Leu Ala Thr
   5225 5230 5235
   Ala Thr Gly Leu Arg Leu Pro Ala Thr Leu Ala Phe Asp His Gly
   5240 5245 5250
   Thr Ala Ala Ala Leu Ala Trp His Val Cys Ala Gln Leu Gly Thr
   5255 5260 5265
   Ala Thr Val Pro Ala Pro Arg Arg Thr Asp Asp Asn Asp Ser Ala
   5270 5275 5280
   Glu Pro Val Arg Ser Leu Phe Gln Gln Ala Tyr Ala Ala Gly Arg
   5285 5290 5295
   Ile Leu Asp Gly Met Asp Leu Val Lys Val Ala Ala Gln Leu Arg
   5300 5305 5310
   Pro Val Phe Gly Ser Pro Gly Glu Leu Glu Ser Leu Pro Lys Pro
   5315 5320 5325
   Val Gln Leu Ser Arg Gly Pro Lys Glu Pro Ala Leu Val Cys Met
   5330 5335 5340
   Pro Ala Leu Ile Gly Met Pro Pro Ala Gln Gln Tyr Ala Arg Ile
   5345 5350 5355
   Ala Ala Gly Phe Arg Asp Val Arg Asp Val Ser Val Val Pro Met
   5360 5365 5370
   Pro Gly Phe Val Ala Gly Glu Pro Leu Pro Ser Ala Ile Glu Val
   5375 5380 5385
   Ala Val Arg Thr Gln Ala Glu Ala Val Leu Gln Glu Phe Ala Gly
   5390 5395 5400
   Asp Ser Phe Val Leu Val Gly His Ser Ser Gly Gly Trp Leu Ala
   5405 5410 5415
   His Glu Val Ala Gly Val Leu Glu Arg Arg Gly Val Leu Pro Ala
   5420 5425 5430
   Gly Val Val Leu Leu Asp Thr Tyr Ile Pro Gly Glu Ile Thr Pro
   5435 5440 5445
   Arg Phe Ser Ala Ala Met Ala His Arg Thr Tyr Glu Lys Leu Ala
   5450 5455 5460
   Thr Phe Thr Asp Met Gln Asp Ile Ala Ile Thr Ala Met Gly Gly
   5465 5470 5475
   Tyr Phe Arg Met Phe Thr Glu Trp Thr Pro Thr Pro Ile Gly Thr
   5480 5485 5490
   Pro Thr Leu Phe Val Arg Thr Glu Asp Cys Val Ala Asp Pro Glu
   5495 5500 5505
   Gly Arg Pro Trp Thr Asp Asp Ser Trp Arg Pro Gly Trp Thr Leu
   5510 5515 5520
   Ala Asp Ala Thr Val Gln Val Pro Gly Asp His Phe Ser Met Met
   5525 5530 5535
   Asp Glu His Ser Gly Ser Thr Ala Gln Ala Val Ala Ser Trp Leu
   5540 5545 5550
   Glu Lys Leu Ser Gln Arg Thr Ala Arg Gln Arg
   5555 5560
<210> 8
   <211> 275
   <212> PRT
   <213> Saccharopolyspora sp. NRRL30141
<400> 8
   Val Leu Pro Gly Gly Val Pro Thr Ser Gln Gln Val Gly Gln Met Tyr
   1               5                   10                  15
   Asp Leu Val Thr Pro Leu Leu Asn Ser Val Ala Gly Gly Pro Cys Ala
               20                  25                  30
   Ile His His Gly Tyr Trp Glu Asn Asp Gly Arg Thr Ser Trp Gln Gln
           35                  40                  45
   Ala Ala Asp Arg Leu Thr Asp Leu Val Ala Glu Arg Thr Ala Leu Asp
       50                  55                  60
   Gly Gly Asn Arg Leu Leu Asp Val Gly Cys Gly Thr Gly Gln Pro Ala
   65                  70                  75                  80
   Leu Arg Val Ala Arg Asp Asn Ala Ile Arg Ile Thr Gly Ile Thr Val
                   85                  90                  95
   Ser Gln Val Gln Ala Ala Ile Ala Val Asp Cys Ala Arg Glu Arg Gly
               100                 105                 110
   Leu Ser His Gln Val Asp Phe Ser Cys Val Asp Ala Met Ser Leu Pro
           115                 120                 125
   Tyr Pro Asp Asn Ala Phe Asp Ala Ala Trp Ala Ile Gln Ser Leu Leu
       130                 135                 140
   Glu Met Ser Glu Pro Asp Arg Ala Ile Arg Glu Ile Val Arg Val Leu
   145                 150                 155                 160
   Lys Pro Gly Gly Ile Leu Gly Val Thr Glu Val Val Lys Arg Glu Ile
                   165                 170                 175
   Gly Ser Gly Ile Pro Val Ser Trp Asp Met Trp Pro Thr Gly Leu Arg
               180                 185                 190
   Ile Cys Leu Ala Glu Gln Leu Leu Glu Ser Leu Cys Ala Ala Gly Phe
           195                 200                 205
   Glu Ile Leu Ala Cys Asp Asp Val Ser Ser Arg Thr Arg Tyr Phe Met
       210                 215                 220
   Pro Gln Phe Ala Glu Ala Leu Ala Ala His Gln His Gly Ile Ala Glu
   225                 230                 235                 240
   Arg Tyr Gly Pro Ala Val Ala Asp Trp Ala Ala Ala Val Cys Asp Tyr
                   245                 250                 255
   Glu Lys Tyr Ala Asp Asp Met Gly Tyr Ala Ile Leu Thr Ala Arg Lys
               260                 265                 270
   Pro Val Gly
           275
<210> 9
   <211> 390
   <212> PRT
<213> Saccharopolyspora sp. NRRL30141
<400> 9
   Met Arg Val Leu Val Val Pro Leu Pro Tyr Pro Thr His Leu Met Ala
   1 5 10 15
   Met Val Pro Leu Cys Trp Ala Leu Arg Ala Ser Gly His Glu Val Leu
   20 25 30
   Val Ala Ala Pro Pro Glu Leu Gln Ala Thr Ala His Gly Ala Gly Leu
   35 40 45
   Thr Thr Ala Glu Ile Arg Gly Asn Asp Lys Thr Arg Asp Thr Gly Ser
   50 55 60
   Thr Thr Arg Leu Arg Phe Pro Asn Pro Ala Phe Gly Gln Arg Asp Thr
   65 70 75 80
   Glu Thr Gly Arg Gln Leu Trp Glu Gln Thr Ala Ser Tyr Val Val Gln
   85 90 95
   Ser Ser Leu Asp Gln Leu Pro Glu Tyr Leu Arg Leu Ala Glu Ala Trp
   100 105 110
   Arg Pro Ser Val Leu Leu Val Asp Val Cys Ala Leu Ile Gly Arg Val
   115 120 125
   Leu Gly Gly Leu Leu Asp Leu Pro Val Val Leu His Arg Trp Gly Val
   130 135 140
   Asp Pro Thr Ala Gly Pro Phe Ser Asp Arg Ala His Glu Leu Leu Asp
   145 150 155 160
   Pro Val Cys Arg His His Gly Leu Ala Gly Leu Pro Thr Pro Glu Leu
   165 170 175
   Ile Leu Asp Pro Cys Pro Pro Ser Leu Gln Ala Ser Asp Ala Pro Arg
   180 185 190
   Gly Val Pro Val Gln Tyr Val Pro Tyr Asn Gly Ser Gly Glu Leu Pro
   195 200 205
   Ala Trp Gly Ala Ala Arg Thr Ser Ala Arg Arg Val Cys Ile Cys Met
   210 215 220
   Gly Arg Met Val Leu Asn Ala Thr Gly Pro Ala Pro Leu Leu Arg Ala
   225 230 235 240
   Val Ala Ala Ala Thr Gly Leu Pro Gly Val Glu Ala Val Ile Ala Val
   245 250 255
   Pro Pro Glu His Arg Ala Leu Leu Thr Asp Leu Pro Asp Asn Ala Arg
   260 265 270
   Ile Ala Glu Ser Val Pro Leu Asn Leu Phe Leu Arg Thr Cys Glu Leu
   275 280 285
   Val Ile Cys Ala Gly Gly Ser Gly Thr Ala Phe Thr Ala Thr Arg Leu
   290 295 300
   Gly Ile Pro Gln Leu Val Leu Pro Gln Tyr Phe Asp Gln Phe Asp Tyr
   305 310 315 320
   Ala Arg Asn Leu Thr Ala Ala Gly Ala Gly Ile Cys Leu Pro Asp Glu
   325 330 335
   Gln Ala Gln Ser Asp His Glu Gln Phe Thr Gly Ser Ile Ala Thr Val
   340 345 350
   Leu Gly Asp Thr Gly Phe Ala Ala Ala Ala Thr Lys Leu Ser Asp Glu
   355 360 365
   Ile Thr Ala Met Pro Asn Pro Ala Glu Leu Val Arg Thr Leu Glu Ser
   370 375 380
   Ser Ala Ala Ile Gly Ala
   385 390
<210> 10
<211> 250
<212> PRT
   <213> Saccharopolyspora sp. NRRL30141
<400> 10
   Met Pro Ser Gln Asn Ala Leu Tyr Leu Asp Leu Leu Lys Lys Val Leu
   1               5                   10                  15
   Thr Asn Thr Ile Tyr Gly Asp Arg Pro His Thr Asn Val Trp Gln Asp
               20                  25                  30
   Asn Thr Asp Tyr Arg Gln Ala Ala Arg Ala Lys Gly Thr Asp Trp Pro
           35                  40                  45
   Thr Val Ala His Thr Met Ile Gly Leu Glu Arg Leu Asp Asn Leu Gln
       50                  55                  60
   His Cys Val Glu Ala Val Leu Ala Asp Gly Val Pro Gly Asp Phe Ala
   65                  70                  75                  80
   Glu Thr Gly Val Trp Arg Gly Gly Ala Cys Ile Phe Met Arg Ala Val
                   85                  90                  95
   Leu Gln Ala Phe Gly Asp Thr Gly Arg Thr Val Trp Val Val Asp Ser
               100                 105                 110
   Phe Gln Gly Met Pro Glu Ser Ser Ala Gln Asp His Glu Ser Asp Gln
           115                 120                 125
   Ala Met Ala Leu His Glu Tyr Asn Asp Val Leu Gly Val Pro Leu Glu
       130                 135                 140
   Thr Val Arg Gln Asn Phe Ala Arg Tyr Gly Leu Leu Asp Glu Gln Val
   145                 150                 155                 160
   Arg Phe Leu Pro Gly Trp Phe Arg Asp Thr Leu Pro Thr Ala Pro Ile
                   165                 170                 175
   Gln Glu Leu Ala Val Leu Arg Leu Asp Gly Asp Leu Tyr Glu Ser Thr
               180                 185                 190
   Met Asp Ser Leu Arg Asn Leu Tyr Pro Lys Leu Ser Pro Gly Gly Phe
           195                 200                 205
   Val Ile Ile Asp Asp Tyr Val Leu Pro Ser Cys Gln Asp Ala Val Lys
       210                 215                 220
   Gly Phe Arg Ala Glu Leu Gly Ile Thr Glu Pro Ile His Asp Ile Asp
   225                 230                 235                 240
   Gly Thr Gly Ala Tyr Trp Arg Arg Ser Trp
                   245                 250
<210> 11
   <211> 395
   <212> PRT
   <213> Saccharopolyspora sp. NRRL30141
<400> 11
   Met Gly Glu Ile Ala Val Ala Pro Trp Ser Val Val Glu His Leu Leu
   1 5 10 15
   Leu Ala Ala Gly Ala Gly Thr Glu Arg Leu Gln Glu Ala Val Gln Val
   20 25 30
   Ala Gly Leu Glu Ala Val Ala Asp Ala Ile Val Asp Glu Leu Val Val
   35 40 45
   Arg Cys Asp Pro Leu Ser Leu Asp Glu Ser Val Arg Ile Gly Leu Glu
   50 55 60
   Ile Thr Ser Gly Ala Gln Leu Val Arg Arg Thr Val Glu Leu Asp His
   65 70 75 80
   Ala Gly Leu Arg Leu Ala Ala Val Ala Glu Ala Pro Ala Val Leu Arg
   85 90 95
   Phe Asp Ala Val Asp Leu Leu Glu Gly Leu Phe Gly Pro Val Asp Gly
   100 105 110
   Arg Arg His Asn Ser Arg Glu Val Arg Trp Ser Asp Ser Met Thr Gln
   115 120 125
   Phe Ser Pro Asp Gln Gly Leu Ala Gly Ala Gln Arg Leu Leu Ala Phe
   130 135 140
   Arg Asn Lys Val Ser Thr Ala Val His Ala Val Leu Ala Ala Ala Ala
   145 150 155 160
   Thr Arg Cys Ser Asp Leu Gly Glu Leu Ala Val Arg Tyr Gly Ser Asp
   165 170 175
   Lys Trp Ala Asp Leu His Trp Tyr Thr Glu His Tyr Glu His His Phe
   180 185 190
   Ser Arg Phe Gln Asp Val Pro Val Arg Val Leu Glu Ile Gly Ile Gly
   195 200 205
   Gly Tyr His Ala Pro Glu Leu Gly Gly Ala Ser Leu Arg Met Trp Gln
   210 215 220
   Arg Tyr Phe Arg Arg Gly Leu Val Tyr Gly Leu Asp Ile Phe Glu Lys
   225 230 235 240
   Ala Gly Asn Glu Gly His Arg Val Arg Lys Leu Arg Gly Asp Gln Ser
   245 250 255
   Asp Ala Glu Phe Leu Ala Asp Met Ala Gly Lys Ile Gly Pro Phe Asp
   260 265 270
   Ile Val Ile Asp Asp Gly Ser His Val Asn Asp His Val Lys Lys Ser
   275 280 285
   Phe His Ala Leu Phe Pro His Val Arg Pro Gly Gly Leu Tyr Val Ile
   290 295 300
   Glu Asp Leu Gln Thr Ser Tyr Trp Pro Gly Tyr Gly Gly Arg Asp Thr
   305 310 315 320
   Glu Pro Ala Ala Gln Arg Thr Ser Ile Asp Met Leu Lys Glu Leu Ile
   325 330 335
   Asp Gly Leu His Tyr Gln Glu Arg Glu Ser Arg Arg Gly Thr Glu Pro
   340 345 350
   Cys Tyr Thr Glu Arg Asn Val Ala Ala Leu His Phe Tyr His Asn Leu
   355 360 365
   Val Phe Val Glu Lys Gly Leu Asn Ala Glu Pro Ala Ala Pro Gly Phe
   370 375 380
   Val Pro Arg Gln Ala Leu Gly Val Glu Asp Ser
   385 390 395
<210> 12
   <211> 539
   <212> PRT
   <213> Saccharopolyspora sp. NRRL30141
<400> 12
   Met Ile Ser Ala Glu Gly Glu Gln Ser Gly Pro Val Ser Lys Gly Gly
   1 5 10 15
   Ala Val Pro Asp Phe His Asp Pro Ala Thr Met Asn Arg Arg Thr Pro
   20 25 30
   Gly Thr Glu Ile Thr Val Glu Pro Gly Asp Pro Arg Tyr Pro Asp Leu
   35 40 45
   Val Val Gly His Asn Pro Arg Phe Thr Gly Lys Pro Glu Arg Ile His
   50 55 60
   Ile Ala Gly Ser Thr Glu Asp Val Val His Ala Val Ala Glu Ala Val
   65 70 75 80
   Arg Thr Gly Arg Arg Val Gly Val Arg Ser Gly Gly His Cys Phe Glu
   85 90 95
   Asn Leu Val Ala Asp Pro Ala Ile Arg Val Leu Val Asp Leu Ser Glu
   100 105 110
   Leu Asn Arg Val Tyr Phe Asp Ser Thr Arg Gly Ala Phe Ala Ile Glu
   115 120 125
   Ala Gly Ala Ala Leu Gly Gln Val Tyr Arg Thr Leu Phe Lys Asn Trp
   130 135 140
   Gly Val Thr Ile Pro Thr Gly Ala Cys Pro Gly Val Gly Ala Gly Gly
   145 150 155 160
   His Ile Pro Gly Gly Gly Tyr Gly Pro Leu Ser Arg Arg Phe Gly Ser
   165 170 175
   Val Val Asp Tyr Leu Gln Gly Val Glu Val Val Val Val Asp Arg Ala
   180 185 190
   Gly Glu Val His Ile Val Glu Val Asp Arg Asn Ser Ile Gly Ala Gly
   195 200 205
   His Asp Leu Trp Trp Ala His Thr Gly Gly Gly Gly Gly Asn Phe Gly
       210                 215                 220
   Val Val Thr Arg Phe Trp Leu Arg Ala Pro Asp Val Val Ser Thr Asp
   225                 230                 235                 240
   Pro Ser Glu Leu Leu Pro Arg Pro Pro Ala Thr Val Leu Leu Arg Ser
                   245                 250                 255
   Phe His Trp Pro Trp Cys Glu Leu Thr Glu Gln Ser Phe Ala Leu Leu
               260                 265                 270
   Leu Arg Asn Phe Gly Thr Trp Tyr Glu Gln His Ser Ala Pro Glu Ser
           275                 280                 285
   Thr Gln Leu Gly Leu Phe Ser Thr Leu Val Cys Ala His Arg Gln Ala
       290                 295                 300
   Gly Tyr Val Thr Leu Asn Ile His Leu Asp Gly Thr Asp Pro Asn Ala
   305                 310                 315                 320
   Glu Arg Thr Leu Ala Glu His Leu Ser Ala Ile Asn Asp Gln Val Gly
                   325                 330                 335
   Val Thr Pro Ala Glu Gly Leu Arg Glu Thr Leu Pro Trp Leu Arg Ser
               340                 345                 350
   Thr Gln Val Ser Gly Ser Leu Ala Glu Gly Gly Glu Pro Ser Gly Gln
           355                 360                 365
   Arg Thr Lys Val Lys Ala Ala Tyr Leu Arg Thr Gly Leu Ser Glu Ala
       370                 375                 380
   Gln Leu Ala Thr Val Tyr Arg Arg Leu Thr Asp Ser Gly Tyr Asp Asn
   385                 390                 395                 400
   Pro Ala Ala Ala Leu Leu Leu Leu Gly Tyr Gly Gly Arg Ala Asn Ala
                   405                 410                 415
   Val Ala Pro Ser Ala Thr Ala Leu Ala Gln Arg Asp Ser Val Leu Lys
               420                 425                 430
   Ala Leu Phe Val Thr Asn Trp Ser Glu Pro Ala Glu Asp Glu Arg His
           435                 440                 445
   Leu Thr Trp Ile Arg Gly Phe Tyr Arg Glu Met Tyr Ala Glu Thr Gly
       450                 455                 460
   Gly Val Pro Val Pro Gly Thr Arg Val Asp Gly Ser Tyr Ile Asn Tyr
   465                 470                 475                 480
   Pro Asp Thr Asp Leu Ala Asp Pro Leu Trp Asn Thr Ser Gly Val Ala
                   485                 490                 495
   Trp His Asp Leu Tyr Tyr Lys Asp Asn Tyr Pro Arg Leu Gln Arg Ala
               500                 505                 510
   Lys Ala Arg Trp Asp Pro Gln Asn Ile Phe Gln His Gly Leu Ser Ile
           515                 520                 525
   Lys Pro Pro Glu Arg Leu Ser Pro Gly Gln Pro
   530 535
<210> 13
   <211> 397
   <212> PRT
   <213> Saccharopolyspora sp. NRRL30141
<400> 13
   Met Ser Ala Thr His Glu Ile Glu Thr Val Glu Arg Ile Ile Leu Ala
   1               5                   10                  15
   Ala Gly Ser Ser Ala Ala Ser Leu Ala Glu Leu Thr Thr Glu Leu Gly
               20                  25                  30
   Leu Ala Arg Ile Ala Pro Val Leu Ile Glu Glu Ile Leu Phe Arg Ala
           35                  40                  45
   Glu Pro Ala Pro Asp Ile Glu Pro Thr Glu Val Ala Val Gln Ile Thr
       50                  55                  60
   His Gly Val Glu Thr Val Asp Phe Val Leu Lys Leu Gln Ser Gly Glu
   65                  70                  75                  80
   Leu Ile Lys Ala Glu Gln Arg Pro Val Gly Asp Val Pro Leu Arg Ile
                   85                  90                  95
   Gly Tyr Glu Leu Thr Asp Leu Ile Ala Glu Leu Phe Gly Pro Gly Ala
               100                 105                 110
   Pro Arg Ala Val Gly Ala Arg Ser Thr Asn Phe Leu Arg Thr Thr Thr
           115                 120                 125
   Ser Gly Ser Ile Pro Gly Pro Ser Glu Leu Ser Asp Gly Phe Gln Ala
       130                 135                 140
   Ile Ser Ala Val Val Ala Gly Cys Gly His Arg Arg Pro Asp Leu Asp
   145                 150                 155                 160
   Gln Leu Ala Ser His Tyr Arg Thr Asp Lys Trp Gly Gly Leu His Trp
                   165                 170                 175
   Phe Thr Pro Leu Tyr Glu Arg His Leu Gly Glu Phe Arg Asp Arg Pro
               180                 185                 190
   Val Arg Ile Leu Glu Ile Gly Val Gly Gly Tyr Asn Phe Asp Gly Gly
           195                 200                 205
   Gly Gly Glu Ser Leu Lys Met Trp Lys Arg Tyr Phe His Arg Gly Leu
       210                 215                 220
   Val Phe Gly Met Asp Val Phe Asp Lys Ser Phe Leu Asp Gln Gln Arg
   225                 230                 235                 240
   Leu Tyr Thr Val Arg Ala Asp Gln Ser Lys Pro Glu Glu Leu Ala Ala
                   245                 250                 255
   Val Asp Asp Glu Tyr Gly Pro Phe Asp Ile Ile Ile Asp Asp Gly Ser
                   260                     265                     270
   His Ile Asn Gly His Val Arg Thr Ser Leu Glu Thr Leu Phe Pro Arg
           275                     280                     285
   Leu Arg Ser Gly Gly Val Tyr Val Ile Glu Asp Leu Trp Thr Thr Tyr
       290                     295                     300
   Ala Pro Gly Phe Gly Gly Gln Ala Gln Ser Pro Ala Ala Pro Gly Thr
   305                     310                     315                     320
   Thr Val Ser Leu Leu Lys Asn Leu Leu Glu Gly Val Gln His Glu Glu
                   325                     330                     335
   Gln Pro His Ala Gly Ser Tyr Glu Pro Ser Tyr Leu Glu Arg Asn Val
               340                     345                     350
   Val Gly Leu His Val Tyr His Asn Ile Ala Phe Leu Glu Lys Gly Val
           355                     360                     365
   Asn Ala Glu Gly Ala Val Pro Ala Trp Val Pro Arg Ser Leu Asp Asp
       370                     375                     380
   Ile Leu His Leu Ala Asp Val Asn Ser Ala Glu Asp Lys
   385                     390                     395
<210> 14
   <211> 283
   <212> PRT
   <213> Saccharopolyspora sp. NRRL30141
<400> 14
   Val Glu Ser Ile Phe Asp Ala Leu Ala Gln Gly Arg Ala Leu His His
   1 5 10 15
   Gly Tyr Trp Ala Gly Gly Tyr Arg Glu Asp Ala Gly Ala Thr Pro Trp
   20 25 30
   Ser Asp Ala Ala Asp His Leu Thr Asp Leu Phe Ile Asp Lys Ala Ala
   35 40 45
   Leu Arg Pro Gly Ala His Leu Phe Asp Leu Gly Cys Gly Asn Gly Gln
   50 55 60
   Pro Val Val Arg Ala Ala Arg Thr Lys Gly Val Arg Val Thr Gly Ile
   65 70 75 80
   Thr Val Asn Ala Glu His Leu Ala Ala Ala Thr Arg Leu Ala Asn Glu
   85 90 95
   Thr Gly Leu Ala Asp Ser Leu Arg Phe Asp Leu Val Asp Gly Ala Arg
   100 105 110
   Leu Pro Tyr Pro Glu Gly Ser Phe His Ala Ala Trp Ala Met Gln Ser
   115 120 125
   Val Val Gln Ile Val Asp Gln Ala Ala Ala Ile Arg Glu Val His Arg
   130 135 140
   Ile Leu Glu Pro Gly Gly Gln Phe Val Leu Gly Asp Ile Ile Thr Arg
   145 150 155 160
   Ala Arg Leu Pro Glu Glu Tyr Ala Ala Val Trp Thr Gly Thr Thr Ala
   165 170 175
   His Thr Leu Asn Ser Leu Thr Ala Leu Val Ser Glu Ala Gly Phe Glu
   180 185 190
   Ile Leu Glu Val Thr Asp Leu Thr Ala Gln Thr Arg Cys Met Val Ser
   195 200 205
   Trp Tyr Val Asp Glu Leu Leu Arg Glu Leu Asp Glu Leu Ala Gly Val
   210 215 220
   Glu Pro Ala Ala Val Gly Thr Tyr Gln Gln Arg Tyr Leu Gly Asp Ile
   225 230 235 240
   Ala Ala Lys His Gly Pro Gly Pro Ala Gln Leu Ile Ala Ala Val Ala
   245 250 255
   Glu Tyr Arg Lys His Pro Asp Tyr Ala Arg Asn Glu Glu Ser Met Gly
   260 265 270
   Phe Met Leu Leu Gln Ala Arg Lys Lys Gln Ser
   275 280
<210> 15
   <211> 310
   <212> PRT
   <213> Saccharopolyspora sp. NRRL30141
<400> 15
   Val Pro Asn Ile Pro Trp Pro Gly Glu Asp Arg Pro Ile Ile Thr Phe
   1               5                   10                  15
   Ala Val Gly Thr His Gly Leu Gly Ser Gln Val Ala Pro Ser Tyr Leu
               20                  25                  30
   Leu Arg Thr Gly Thr Glu Pro Glu Thr Glu Leu Ile Ala Val Ala Leu
           35                  40                  45
   Asp Arg Gly Trp Ala Val Val Ile Thr Asp Tyr Glu Gly Leu Gly Thr
       50                  55                  60
   Pro Gly Thr His Thr Tyr Thr Val Gly Arg Pro Gln Gly His Ala Met
   65                  70                  75                  80
   Leu Asp Ala Ala Arg Ala Ala Gln Arg Leu Pro Gly Ser Gly Leu Gly
                   85                  90                  95
   Thr Asp Cys Pro Val Gly Ile Trp Gly Tyr Ala Gln Gly Gly Gln Ala
               100                 105                 110
   Ser Ala Phe Ala Gly Glu Leu His Pro Thr Tyr Ala Pro Glu Leu Pro
           115                 120                 125
   Ile Arg Ala Ala Ala Ala Gly Ala Val Pro Ile Asp Leu Leu Asp Ile
       130                 135                 140
   Leu His Arg Asn Asp Gly Val Phe Thr Gly Pro Val Leu Ala Gly Leu
   145 150 155 160
   Val Gly His Ala Ala Ala Tyr Pro Asp Leu Pro Phe Asp Glu Leu Leu
   165 170 175
   Thr Asp Ala Gly Arg Ile Ala Val Asp Gln Val Arg Glu Leu Gly Ala
   180 185 190
   Pro Glu Leu Val Thr Arg Phe Leu Gly Arg Glu Leu Ser Asp Phe Leu
   195 200 205
   Asp Thr Ser Gly Leu Phe Glu His Pro Arg Trp Arg Ala Arg Leu Val
   210 215 220
   Glu Ser Val Ala Gly Arg Asn Gly Gly Pro Val Val Pro Thr Leu Val
   225 230 235 240
   Tyr His Ser Thr Asp Asp Glu Ile Val Pro Phe Ala Phe Gly Glu Arg
   245 250 255
   Leu Arg Asp Ser Tyr Arg Ala Ala Gly Thr Pro Val Arg Trp His Pro
   260 265 270
   Leu Ser Gly Leu Ala His Phe Pro Ala Ala Leu Ala Ser Ser Arg Val
   275 280 285
   Val Val Ser Trp Phe Asp Glu His Phe Ser Gly Pro Ser Ala Ile Ser
   290 295 300
   Gly Pro Arg Asp Asp Gly
   305 310
<210> 16
   <211> 332
   <212> PRT
   <213> Saccharopolyspora sp. NRRL30141
<400> 16
   Met Arg Lys Pro Val Arg Ile Gly Val Leu Gly Cys Ala Ser Phe Ala
   1 5 10 15
   Trp Arg Arg Met Leu Pro Ala Met Cys Asp Val Ala Glu Thr Glu Val
   20 25 30
   Val Ala Val Ala Ser Arg His Pro Ala Lys Ala Glu Arg Phe Ala Ala
   35 40 45
   Arg Phe Glu Cys Glu Ala Val Leu Gly Tyr Gln Arg Leu Leu Glu Arg
   50 55 60
   Pro Asp Ile Asp Ala Val Tyr Val Pro Leu Pro Pro Gly Met His Ala
   65 70 75 80
   Glu Trp Ile Gly Lys Ala Leu Glu Ala Gly Lys His Val Leu Ala Glu
   85 90 95
   Lys Pro Leu Thr Thr Thr Ala Ser Glu Thr Ala Arg Leu Val Gly Leu
                   100                 105                 110
   Ala Arg Arg Lys His Leu Leu Leu Arg Glu Asn Tyr Leu Phe Leu His
           115                 120                 125
   His Gly Arg His Asp Val Val Arg Asp Leu Leu Gln Ser Glu Glu Ile
       130                 135                 140
   Gly Glu Leu Arg Glu Phe Thr Ala Val Phe Gly Ile Pro Pro Leu Ser
   145                 150                 155                 160
   Asp Thr Asp Ile Arg Tyr Arg Thr Glu Leu Gly Gly Gly Ala Leu Leu
                   165                 170                 175
   Asp Ile Gly Val Tyr Pro Ala Arg Ala Ala Arg Leu Phe Leu Leu Gly
               180                 185                 190
   Pro Leu Thr Val Ala Gly Ala Ser Ser His Glu Ala His Glu Ser Gly
           195                 200                 205
   Val Asp Leu Ser Gly Ser Val Leu Leu Gln Ser Glu Gly Gly Ala Val
       210                 215                 220
   Ala His Leu Gly Tyr Gly Phe Val His His Tyr Arg Ser Ala Tyr Glu
   225                 230                 235                 240
   Leu Trp Gly Ser Arg Gly Arg Ile Val Ile Asp Arg Ala Phe Thr Pro
                   245                 250                 255
   Pro Ala Glu Trp Gln Ala Val Ile Arg Ile Glu Arg Lys Gly Val Val
               260                 265                 270
   Asp Glu Leu Ser Leu Pro Ala Glu Asp Gln Val Arg Lys Ala Val Thr
           275                 280                 285
   Ala Phe Ala Arg Asp Ile Arg Ala Glu Ala Gly Val Asp Glu Pro Ala
       290                 295                 300
   Val Ala Gly Asp Ser Gly Glu Ser Met Ile Gln Gln Ala Ala Leu Val
   305                 310                 315                 320
   Glu Ala Ile Gly Gln Ala Cys Arg Cys Gly Ser Thr
                   325                 330
<210> 17
   <211> 486
   <212> PRT
   <213> Saccharopolyspora sp. NRRL30141
<400> 17
   Met Ser Ser Phe Ala Glu Ala Glu Ala Ser Ala Ala Ala Pro Leu Ser
   1               5                   10                  15
   Ser Asn Asn Thr Arg Arg Phe Val Asp Ser Ala Leu Ser Ala Cys Asn
               20                  25                  30
   Gly Arg Phe Pro Thr Thr Arg Phe His Cys Trp Leu Ala Asp Arg Leu
           35                  40                  45
   Gly Glu Asn Ser Phe Glu Thr Thr Arg Ile Pro Phe Asp Arg Leu Ser
       50                  55                  60
   Lys Trp Lys Phe Asp Ala Ser Thr Glu Asn Leu Val His Ala Asp Gly
   65                  70                  75                  80
   Arg Phe Phe Thr Val Glu Gly Leu Gln Val Glu Thr Asn Tyr Gly Ala
                   85                  90                  95
   Ala Thr Cys Trp His Gln Pro Ile Ile Asn Gln Ala Glu Val Gly Ile
               100                 105                 110
   Leu Gly Ile Leu Val Lys Glu Ile Asp Gly Val Leu His Cys Leu Met
           115                 120                 125
   Ser Ala Lys Met Glu Pro Gly Asn Val Asn Val Leu Gln Leu Ser Pro
       130                 135                 140
   Thr Val Gln Ala Thr Arg Ser Asn Tyr Thr Gln Ala His Arg Gly Ser
   145                 150                 155                 160
   Val Pro Pro Tyr Val Asp Tyr Phe Leu Gly Arg Gly Arg Ser Arg Val
                   165                 170                 175
   Leu Val Asp Val Leu Gln Ser Glu Gln Gly Ala Trp Phe Tyr Arg Lys
               180                 185                 190
   Arg Asn Arg Asn Met Val Val Glu Val Asp Glu Glu Val Pro Val Leu
           195                 200                 205
   Pro Asp Phe Cys Trp Leu Thr Leu Gly Gln Val Leu Asp Leu Leu Arg
       210                 215                 220
   Gln Asp Asn Ile Val Asn Met Asp Thr Arg Thr Val Leu Ser Cys Ile
   225                 230                 235                 240
   Pro Phe His Asp Ser Ala Thr Gly Pro Gly Leu Ala Ala Ser Ala Glu
                   245                 250                 255
   Pro Phe Arg Gln Ala Val Ala Arg Ser Leu Ser His Gly Ile Asp Ser
               260                 265                 270
   Ala Ser Ile Thr Glu Ala Val Gly Trp Phe Glu Glu Ala Lys Ala Arg
           275                 280                 285
   Tyr Ser Leu Arg Ala Thr Arg Val Pro Leu Ser Arg Val Asp Lys Trp
       290                 295                 300
   Tyr Arg Thr Asp Thr Glu Ile Ala His Gln Asp Gly Lys Tyr Phe Ser
   305                 310                 315                 320
   Val Ile Ala Val Ser Val Ser Ala Thr Asn Arg Glu Val Ser Ser Trp
                   325                 330                 335
   Thr Gln Pro Met Ile Glu Pro Arg Glu Pro Gly Glu Ile Ala Leu Leu
               340                 345                 350
   Val Lys Arg Ile Gly Gly Val Leu His Gly Leu Val Arg Ala Arg Val
           355                 360                 365
   Glu Ala Gly Tyr Lys Ser Thr Ala Glu Ile Ala Pro Thr Val Gln Cys
   370 375 380
   Ser Val Ala Asn Tyr Gln Ser Thr Pro Arg Asn Asp Trp Pro Pro Phe
   385 390 395 400
   Val Asp Asp Val Leu Thr Ala Asp Pro Glu Thr Val Arg Tyr Glu Ser
   405 410 415
   Ile Leu Ser Glu Glu Gly Gly Arg Phe Tyr Gln Ala Gln Asn Arg Tyr
   420 425 430
   Arg Ile Ile Glu Val His Glu Asp Phe Ala Ala Arg Pro Pro Ser Asp
   435 440 445
   Phe Arg Trp Met Thr Leu Gly Gln Leu Gly Glu Leu Leu Arg Ser Thr
   450 455 460
   His Ser Leu Asn Ile Gln Ala Arg Ser Leu Val Ala Ser Leu His Ser
   465 470 475 480
   Leu Trp Ala Leu Gly Arg
   485
<210> 18
   <211> 437
   <212> PRT
   <213> Saccharopolyspora sp. NRRL30141
<400> 18
   Met Arg Val Leu Phe Thr Pro Leu Pro Ala Ser Ser His Phe Phe Asn
   1               5                   10                  15
   Leu Val Pro Leu Ala Trp Ala Leu Arg Ala Ala Gly His Glu Val Arg
               20                  25                  30
   Val Ala Ile Cys Pro Asn Met Val Ser Met Val Thr Gly Ala Gly Leu
           35                  40                  45
   Thr Ala Val Pro Val Gly Asp Glu Leu Asp Leu Ile Ser Leu Ala Ala
       50                  55                  60
   Arg Asn Lys Leu Val Leu Gly Asn Gly Val Ala Phe Asp Glu Gly Arg
   65                  70                  75                  80
   Arg Pro Glu Leu Phe Asp Glu Leu Leu Ser Ile Asn Ser Gly Arg Asp
                   85                  90                  95
   Met Asp Ala Val Glu Gln Leu His Leu Val Asp Asp Arg Ser Leu Asp
               100                 105                 110
   Asp Leu Met Gly Phe Ala Glu Lys Trp Gln Pro Asp Leu Val Val Trp
           115                 120                 125
   Asp Ala Met Val Cys Ser Gly Pro Val Val Ala Gln Ala Leu Gly Val
       130                 135                 140
   Arg His Val Arg Met Leu Val Ala Leu Asp Val Ser Gly Trp Leu Arg
   145                 150                 155                 160
   Ser Gly Phe Leu Glu Tyr Leu Glu Ser Lys Pro Pro Glu Gln Arg Val
   165 170 175
   Asp Pro Leu Gly Ala Trp Leu Gly Ala Lys Leu Ser Lys Phe Gly Ala
   180 185 190
   Thr Phe Asp Glu Glu Ile Val Thr Gly Gln Ala Thr Ile Asp Pro Val
   195 200 205
   Ser Ser Trp Met Arg Leu Pro Val Asp Leu Asp Tyr Ile Ser Met Arg
   210 215 220
   Phe Val Pro Tyr Asn Gly Pro Ala Val Val Pro Glu Trp Leu Arg Glu
   225 230 235 240
   Pro Pro Thr Lys Pro Arg Val Cys Val Thr Arg Gly Leu Thr Lys Arg
   245 250 255
   Gln Gln Ser Arg Val Ala Glu Gln Trp Glu Gly Glu Ala Gln Glu Gln
   260 265 270
   Ala Met Val Glu Thr Leu Leu Arg Gly Ala Ala Gly Leu Asp Val Glu
   275 280 285
   Val Ile Ala Thr Leu Ser Gly Gly Glu Val Arg Glu Met Gly Glu Leu
   290 295 300
   Pro Pro Asn Val Arg Val His Glu Tyr Val Pro Leu Asn Glu Leu Leu
   305 310 315 320
   Glu Ser Cys Ser Ala Ile Ile His His Gly Ser Thr Thr Thr Gln Glu
   325 330 335
   Thr Ala Thr Val Asn Gly Val Pro Gln Leu Ile Leu Pro Gly Thr Phe
   340 345 350
   Trp Asp Glu Ser Arg Arg Ala Glu Leu Leu Ala Asp Arg Gly Ala Gly
   355 360 365
   Leu Val Leu Asp Arg Ala Thr Phe Thr Glu Asp Asp Val Arg Arg Gln
   370 375 380
   Leu Ala Arg Leu Leu Asp Glu Pro Ser Phe Ala Ala Asn Ala Ala Leu
   385 390 395 400
   Ile Arg Gly Glu Ile Glu Glu Asn Pro Ser Pro His Asp Ile Val Ala
   405 410 415
   Arg Leu Glu Lys Leu Val Ala Glu Gly Lys Asn Arg Arg Ala Gly Lys
   420 425 430
   Ser Asp Gly His Leu
   435
<210> 19
   <211> 447
   <212> PRT
   <213> Saccharopolyspora sp. NRRL30141
<400> 19
   Val Thr Ser Cys Asp Asp Thr Cys Ala Thr Ala Thr Glu Met Thr Pro
   1               5                   10                  15
   Asp Ala Lys Asp Arg Ile Leu Ala Ser Val Arg Asp Tyr His Arg Glu
               20                  25                  30
   Gln Lys Ser Ser Ile Phe Val Ala Gly Ser Thr Pro Ile Arg Pro Ser
           35                  40                  45
   Gly Ala Val Leu Asp Glu Asp Asp Arg Val Ala Leu Val Glu Ala Ala
       50                  55                  60
   Leu Glu Leu Arg Ile Ala Ala Gly Gly Asn Ala Arg Arg Phe Glu Ser
   65                  70                  75                  80
   Glu Phe Ala Arg Phe Phe Gly Leu Arg Lys Ala His Leu Thr Asn Ser
                   85                  90                  95
   Gly Ser Ser Ala Asn Leu Leu Ala Leu Ser Ser Leu Thr Ser Pro Asn
               100                 105                 110
   Leu Gly Glu Ala Arg Leu Arg Pro Gly Asp Glu Val Ile Thr Ala Ala
           115                 120                 125
   Val Gly Phe Pro Thr Thr Ile Asn Pro Ala Val Gln Asn Gly Leu Val
       130                 135                 140
   Pro Val Phe Val Asp Val Glu Leu Gly Thr Tyr Asn Ala Thr Pro Asp
   145                 150                 155                 160
   Arg Ile Lys Ala Ala Val Ser Glu Arg Thr Arg Ala Ile Met Leu Ala
                   165                 170                 175
   His Thr Leu Gly Asn Pro Phe Ala Ala Asp Glu Ile Ala Glu Ile Ala
               180                 185                 190
   Arg Glu His Glu Leu Phe Leu Ile Glu Asp Asn Cys Asp Ala Val Gly
           195                 200                 205
   Ser Thr Tyr Arg Gly Arg Leu Thr Gly Thr Phe Gly Asp Leu Thr Thr
       210                 215                 220
   Val Ser Phe Tyr Pro Ala His His Ile Thr Ser Gly Glu Gly Gly Cys
   225                 230                 235                 240
   Val Leu Thr Gly Ser Leu Glu Leu Ala Arg Ile Ile Glu Ser Leu Arg
                   245                 250                 255
   Asp Trp Gly Arg Asp Cys Trp Cys Glu Pro Gly Val Asp Asn Thr Cys
               260                 265                 270
   Arg Lys Arg Phe Asp Tyr Gln Leu Gly Thr Leu Pro Ala Gly Tyr Asp
           275                 280                 285
   His Lys Tyr Thr Phe Ser His Val Gly Tyr Asn Leu Lys Thr Thr Asp
       290                 295                 300
   Leu Gln Ala Ala Leu Ala Leu Ser Gln Leu Ser Lys Ile Ser Glu Phe
   305 310 315 320
   Gly Ser Ala Arg Arg Arg Asn Trp Arg Arg Leu Arg Glu Gly Leu Ser
   325 330 335
   Gly Val Pro Gly Leu Leu Leu Pro Val Pro Thr Pro His Ser Asp Pro
   340 345 350
   Ser Trp Phe Gly Phe Ala Ile Thr Val Ser Ala Asp Ala Gly Phe Thr
   355 360 365
   Arg Ala Ala Leu Val Asn Phe Leu Glu Ser Arg Asn Ile Gly Thr Arg
   370 375 380
   Leu Leu Phe Gly Gly Asn Ile Thr Arg His Pro Ala Phe Gln His Val
   385 390 395 400
   Arg Tyr Arg Ile Ala Asp Ala Leu Thr Asn Ser Asp Ile Val Thr Asp
   405 410 415
   Arg Thr Phe Trp Val Gly Val Tyr Pro Gly Ile Thr Asp Gln Met Ile
   420 425 430
   Asp Tyr Val Ala Glu Ser Ile Ala Glu Phe Val Ala Lys Asn Ser
   435 440 445
<210> 20
   <211> 378
   <212> PRT
   <213> Saccharopolyspora sp. NRRL30141
<400> 20
   Val Ile Asn Leu His Gln Pro Thr Leu Gly Ala Glu Glu Leu Asp Ala
   1               5                   10                  15
   Ile Ala Glu Val Phe Ala Ser Asn Trp Ile Gly Leu Gly Pro Arg Thr
               20                  25                  30
   Arg Thr Phe Glu Ala Asp Phe Ala His His Leu Gly Val Asp Pro Asp
           35                  40                  45
   Gln Ile Val Phe Val Asn Ser Gly Thr Ala Ala Leu Phe Leu Thr Val
       50                  55                  60
   Gln Val Leu Asp Leu Gly Pro Gly Asp Asp Val Val Leu Pro Ser Ile
   65                  70                  75                  80
   Ser Phe Val Ala Ala Ala Asn Ala Ile Ala Ser Ser Gly Ala Arg Pro
                   85                  90                  95
   Val Phe Cys Asp Val Asp Pro Arg Thr Leu Asn Pro Thr Leu Asp Asp
               100                 105                 110
   Val Ala Lys Ala Ile Thr Pro Thr Thr Lys Ala Val Leu Leu Leu His
           115                 120                 125
   Tyr Gly Gly Ser Pro Gly Glu Val Thr Glu Ile Ala Gly Phe Cys Arg
       130                 135                 140
   Glu Lys Gly Leu Val Leu Ile Glu Asp Thr Ala Cys Ala Val Ala Ser
   145 150 155 160
   Ser Val His Gly Thr Ala Cys Gly Thr Phe Gly Asp Leu Ala Thr Trp
   165 170 175
   Ser Phe Asp Ala Met Lys Ile Leu Val Thr Gly Asp Gly Gly Met Phe
   180 185 190
   Tyr Ala Ala Asp Arg Glu Leu Ala His Arg Ala Arg Arg Leu Ala Tyr
   195 200 205
   His Gly Leu Glu Gln Met Ser Gly Phe Asp Ser Ala Lys Ser Ser Asn
   210 215 220
   Arg Trp Trp Asp Ile Cys Val Glu Asp Ile Gly His Arg Leu Ile Gly
   225 230 235 240
   Asn Asp Met Thr Ala Ala Leu Gly Ser Val Gln Leu Arg Lys Leu Pro
   245 250 255
   Asp Phe Val Ser Arg Arg Arg Glu Ile Ala Thr Gln Tyr Asp Arg Leu
   260 265 270
   Leu Ser Asp Val Pro Gly Val His Leu Pro Pro Thr Leu Pro Asp Gly
   275 280 285
   His Val Ser Ser His Tyr Phe Tyr Trp Val Gln Leu Ala Pro Glu Ile
   290 295 300
   Arg Asp Arg Val Ala Gln Gln Met Leu Glu Arg Gly Ile Tyr Thr Ser
   305 310 315 320
   Phe Arg Tyr Pro Pro Leu His Lys Val Pro Ile Tyr Arg Ala Asp Cys
   325 330 335
   Lys Leu Pro Ser Ala Glu His Ala Cys Arg Arg Thr Leu Leu Leu Pro
   340 345 350
   Leu His Pro Ser Leu Asp Asp Ala Glu Val Arg Thr Val Ala Asp Glu
   355 360 365
   Phe Arg Lys Ala Val Glu Gln His Ile Ser
   370 375
<210> 21
   <211> 249
   <212> PRT
   <213> Saccharopolyspora sp. NRRL30141
<400> 21
   Met Ser Arg Val Ser Gly Thr Phe Glu Glu Leu Ser Ser Val Tyr Ser
   1 5 10 15
   Pro Asp His Ala Asp Ile Tyr Asp Ala Ile His Ser Ala Arg Gly Arg
   20 25 30
   Asp Trp Ala Thr Glu Ala Glu Glu Ile Ile Gln Leu Ile Arg Thr Arg
   35 40 45
   Leu Pro Glu Ala Gln Ser Leu Leu Asp Ile Ala Cys Gly Thr Gly Ala
   50 55 60
   His Leu Glu Arg Phe Arg Thr Glu Tyr Ala Lys Val Ala Gly Leu Glu
   65 70 75 80
   Leu Ser Asp Ala Met Arg Glu Ile Ala Ile Arg Arg Val Pro Glu Val
   85 90 95
   Pro Ile His Thr Gly Asp Ile Arg Asp Phe Asp Leu Gly Glu Pro Phe
   100 105 110
   Asp Val Val Thr Cys Leu Cys Phe Thr Ala Ala Tyr Met Arg Thr Val
   115 120 125
   Asp Glu Leu Arg Arg Val Thr Arg Asn Met Ala Arg His Leu Ala Pro
   130 135 140
   Gly Gly Val Ala Val Ile Glu Pro Trp Trp Phe Pro Asp Lys Phe Ile
   145 150 155 160
   Asp Gly Phe Val Thr Gly Ala Val Ala His His Gly Glu Arg Val Ile
   165 170 175
   Ser Arg Leu Ser His Ser Val Leu Glu Gly Arg Thr Ser Arg Met Thr
   180 185 190
   Val Arg Tyr Thr Val Ala Glu Pro Ala Gly Ile Arg Asp Phe Thr Glu
   195 200 205
   Phe Glu Ile Leu Ser Leu Phe Thr Glu Asp Glu Tyr Thr Ala Ala Leu
   210 215 220
   Glu Asp Ala Gly Ile Arg Ala Glu Tyr Leu Pro Gly Gly Pro Asn Gly
   225 230 235 240
   Arg Gly Leu Phe Val Gly Thr Arg Asn
   245
<210> 22
   <211> 470
   <212> PRT
   <213> Saccharopolyspora sp. NRRL30141
<400> 22
   Met Pro Ser Arg Arg Pro Leu Thr Ala Ile Gln Leu Asn Leu Tyr Pro
   1 5 10 15
   Arg Val Ala Arg His Pro Ala Val Val Gln Phe Cys Tyr Gly Gly Val
   20 25 30
   Tyr Cys Gly Pro Arg Trp Leu Gly Ser Trp Asp Trp Ile Met Ala His
   35 40 45
   Phe Val Phe Ala Thr Tyr Ala Asp His Ala His Ile Gly Pro Leu Val
   50 55 60
   Pro Val Ser Arg Ala Leu Val Glu Arg Asp His Gln Val Thr Trp Tyr
   65                  70                  75                  80
   Thr Gly Glu Asn Tyr Arg Ala Ala Val Glu Arg Ser Gly Ala Asp Phe
                   85                  90                  95
   Ala Ala Pro Val Glu Gly Arg Phe Ile Asp Gly Arg Glu Leu Glu Gln
               100                 105                 110
   Lys Phe Pro Glu Ser Ile Gln Met Ser Ala Arg Arg Arg Ala Arg Trp
           115                 120                 125
   Leu Met Asp Asn His Trp Val Pro Ala Tyr Glu Gly Gln Tyr Arg Asp
       130                 135                 140
   Leu Val Ala Val Val Asp Arg Thr Arg Ala Asp Val Leu Leu Ala Asp
   145                 150                 155                 160
   Ala Ser Trp Gly Pro Ala Lys Leu Val His Ala Val Thr Gly Val Leu
                   165                 170                 175
   Trp Ala Thr Ile Ser Gln Met Pro Ile Leu Leu Pro Asp Pro Ala Val
               180                 185                 190
   Pro Pro Ile Gly Thr Gly Trp Lys Phe Gly Thr Ser Pro Phe His Arg
           195                 200                 205
   Leu Arg Asn Arg Ile Gly Asn Arg Leu Ile Asn Ala Leu Val His Asp
       210                 215                 220
   Pro Gly Met Lys Lys Ile Asn Ala Phe Trp Asn Ser Ile Gly Val Pro
   225                 230                 235                 240
   Val Ser Arg Glu Val Ser Glu Ser Pro Tyr Leu Phe Met Gln Ala Gly
                   245                 250                 255
   Thr Arg Ser Leu Glu Tyr Pro Arg Ala Leu Pro Gln Gln Met His Phe
               260                 265                 270
   Ile Gly Arg Leu Glu Pro Asp Ser Pro Met Gly Val Gly Leu Pro Ser
           275                 280                 285
   Trp Trp Gly Glu Leu Asp Gly Asp Arg Pro Val Val Leu Val Thr Gln
       290                 295                 300
   Gly Thr Met Ala Val Asp Ala Asp Asp Leu Ile Arg Pro Ala Leu Arg
   305                 310                 315                 320
   Gly Leu Ala Gly Asp Gln Val Leu Val Val Ala Thr Thr Gly Arg Glu
                   325                 330                 335
   Gly Val Asp Leu Gly Tyr Val Pro Asp Asn Ala Arg Val Ala Ser Phe
               340                 345                 350
   Leu Pro Tyr Arg Glu Leu Met Pro Lys Leu Ala Ala Val Val Thr Asn
           355                 360                 365
   Gly Gly Phe Gly Thr Val Gln Gln Ala Leu Ser His Gly Leu Pro Leu
       370                 375                 380
   Val Val Ala Gly Arg Ser Glu Asp Lys Thr Asp Val Cys Ala Arg Val
   385 390 395 400
   Ala Trp Ser Gly Ala Gly Val Asp Leu Arg Thr Arg Arg Pro Ser Pro
   405 410 415
   Gln Gln Val Ala Gly Ala Val Lys Val Met Ser Thr Asp Pro Arg Tyr
   420 425 430
   Arg Gln Ala Ala Gln Arg Leu Ala Val Glu Tyr Ala Glu Tyr Asp Ala
   435 440 445
   Cys Gly Thr Ala Val Lys Leu Leu Glu Arg Leu Ala Thr Thr Arg Arg
   450 455 460
   Pro Val Ile Ala Ser Arg
   465 470
<210> 23
   <211> 169
   <212> PRT
   <213> Saccharopolyspora sp. NRRL30141
<400> 23
   Val Arg Cys Gly Cys Gly Arg Val His Thr Ala Ala Arg Pro Glu Gly
   1 5 10 15
   Ala Arg Pro Gly Ala Val Gly Tyr Gly Pro Asn Leu Gln Ala Phe Ala
   20 25 30
   Val Tyr Leu Met Val Val His Phe Ile Pro Val His Arg Cys Val Glu
   35 40 45
   Leu Leu Ala Ser Leu Thr Gly Ala Val Pro Ser Val Gly Phe Val His
   50 55 60
   Gly Val Leu Thr Arg Ala Ala Gly Val Leu Thr Glu Val Asp Lys Arg
   65 70 75 80
   Ile His Thr Leu Ala Tyr Ala Val Cys Cys Asp Glu Thr Pro Leu Arg
   85 90 95
   Val Gly Pro Arg Thr Pro Asn Gln Ala Glu Arg Asp Leu Arg Pro Ala
   100 105 110
   Lys Val Gln Gln Asn Ile Ser Gly Arg Leu Thr Ile Glu Lys Arg Thr
   115 120 125
   Lys Asp Arg Tyr Arg Ile Arg Gly Ser Leu Ser Thr Ala Gly Lys His
   130 135 140
   Gly Arg Asn Met Ile Glu Ala Leu Arg Glu Ala Ile Arg Gly His Pro
   145 150 155 160
   Trp Met Pro Pro Asp Pro Thr Pro Ala
   165
<210> 24
   <211> 165
   <212> PRT
   <213> Saccharopolyspora sp. NRRL30141
<400> 24
   Val Cys Ser Asp Arg Gly Ala Gly Val Ala Leu Cys Val Cys Trp Ser
   1               5                   10                  15
   Trp Cys Gly Phe Cys Val Gly Val Ala Glu Leu Ile Glu Leu Val Gly
               20                  25                  30
   Glu Gln Gly Ala Arg Ile Ala Val Leu Gly Glu Gln Ile Ala Val Arg
           35                  40                  45
   Asp Arg Gln Ile Thr Ala Met Ala Ala Gln Met Ala Glu Leu Ala Glu
       50                  55                  60
   Val Asn Glu Ala Leu Gly Glu Arg Leu Ala Lys Leu Glu His Ala Leu
   65                  70                  75                  80
   Ser Arg Asn Ser Lys Asn Ser Ser Ser Ala Pro Ser Lys Asp Asp Gly
                   85                  90                  95
   Pro Gly Arg Thr Pro Pro Pro Ala Lys Ala Lys Arg Gly Gly Ala Val
               100                 105                 110
   Lys Arg Lys Gly Lys Gln Pro Gly Ala Pro Gly Ala Asn Leu Ala Trp
           115                 120                 125
   Thr Asp Leu Pro Gly Asp His Lys Asp Arg Phe Pro Gly Gly Val Cys
       130                 135                 140
   Glu Cys Gly Ser Asp Leu Ala Arg Gly Thr Gly Ser Gly Gly Gly Gly
   145                 150                 155                 160
   Ser Leu Pro Ala Ala
                   165
<210> 25
   <211> 248
   <212> PRT
   <213> Saccharopolyspora sp. NRRL30141
<400> 25
   Met Glu Ile Ile Gly Arg Gly Phe Ile Ala Arg Asn Leu Leu Arg Ile
   1 5 10 15
   Ser Gly Arg His Ala Asp Ala Val Ala Leu Ala Ala Gly Val Ser Asn
   20 25 30
   Thr Ser Cys Arg Ser Glu Asp Glu Tyr Gln Arg Glu Ala Ala Leu Val
   35 40 45
   Tyr Arg Thr Ile Glu Arg Cys His Ala Ile Gly Arg Lys Leu Leu Phe
   50 55 60
   Phe Ser Thr Ala Ser Ala Ser Met Tyr Gly Ala Leu Thr Ser Pro Gly
   65 70 75 80
   Phe Glu Asp Gly Pro Val Tyr Pro Pro Thr Thr Tyr Gly Arg His Lys
   85 90 95
   Leu Ala Met Glu Ala Val Ile Lys Ala Ser Gly Val Asp Phe Leu Ile
   100 105 110
   Leu Arg Leu Ala Tyr Val Ile Gly Ala His Gln Arg Gly His Gln Leu
   115 120 125
   Leu Pro Ser Leu Val Thr Gln Leu Arg Ser Gly Ser Val Thr Val His
   130 135 140
   Arg Gly Ala His Arg Asp Val Ile Ala Ala Asp Asp Val Val Thr Ile
   145 150 155 160
   Val Asp Asp Leu Leu Thr Lys Ala Val Ala Gly Thr Val Val Asn Ile
   165 170 175
   Gly Ser Gly Phe Pro Val Pro Ala Glu Lys Ile Val Ala His Leu Glu
   180 185 190
   Tyr Arg Leu Gly Thr Ala Ala Ala Arg Gln Trp Ile Asp His Pro Thr
   195 200 205
   Glu Tyr Gln Ile Ser Leu Thr Arg Leu Asn Thr Leu Val Pro Arg Ile
   210 215 220
   Ala Glu Leu Gly Phe Gly Pro Asp Tyr Tyr Arg Gln Val Leu Asp His
   225 230 235 240
   Tyr Leu Asp Leu Tyr Pro Gln Ala
   245
<210> 26
   <211> 260
   <212> PRT
   <213> Saccharopolyspora sp. NRRL30141
<400> 26
   Met Phe Asp Thr Val Asp Asp Arg Ala Thr Gln Ala Leu Pro Asp Gly
   1               5                   10                  15
   Arg Leu Val Ala Cys Ala Asn Thr Leu Glu Val Leu Ala Ile Trp Gln
               20                  25                  30
   Asp Ile Ala Asn Asp Ser Ala Tyr Ala Arg Gly Leu Arg Gly Leu Gly
           35                  40                  45
   Ala Asp Ser Val Ile Val Asp Val Gly Ala His Val Gly Leu Ala Ser
       50                  55                  60
   Met Tyr Phe Ala Asp Arg Ile Pro Ala Ala Arg Ile Leu Ala Tyr Glu
   65                  70                  75                  80
   Pro Ala Pro Thr Thr Phe Ala Cys Leu Arg Glu Asn Phe Ala Arg His
                   85                  90                  95
   Val Pro Arg Gly Val Thr Phe Asp Leu Ala Val Gly Ala Glu Pro Gly
               100                 105                 110
   Thr Ser Arg Phe Val Tyr Tyr Pro Ala Gly Pro Ser Leu Ser Thr Leu
           115                 120                 125
   His Leu Asp Ala Ala Asp Glu Arg Arg Asn Ile Asp Thr Val Met Ser
       130                 135                 140
   Asn Val Gly Ser Pro Glu Leu Ala Gly Glu Ser Met Gln Gly Leu Val
   145                 150                 155                 160
   Arg Thr Lys Glu Glu Leu Asp Val Arg Val Thr Thr Leu Thr Glu Ile
                   165                 170                 175
   Ala Arg Gln His Arg Leu Asp Val Leu Asp Leu Leu Lys Ile Asp Val
               180                 185                 190
   Glu Arg Gly Glu Leu Asp Val Leu Asn Gly Ile Asp Asp Glu Met Trp
           195                 200                 205
   Pro Arg Ile Arg Arg Ile Val Val Glu Val His Asp Ile Cys Gly Arg
       210                 215                 220
   Leu Arg Gln Val Leu Asp Arg Leu Arg Lys Leu Asp Tyr Gln Val Glu
   225                 230                 235                 240
   Val Ser Gln Ser Pro Ile Phe Leu Gly Ala Ser Val His Ile Val Val
                   245                 250                 255
   Ala Val Arg Asp
               260
<210> 27
   <211> 399
   <212> PRT
   <213> Saccharopolyspora sp. NRRL30141
<400> 27
   Met Thr Asn Gly Asp Glu Pro Met Ala Tyr Pro Phe Gly Glu Ile Asp
   1               5                   10                  15
   Arg Leu Leu Leu Asp Asp Arg Tyr Ala Val Leu Arg Glu Gly Glu Pro
               20                  25                  30
   Val Ser Lys Ile Arg Leu Pro Tyr Gly Gly Asp Gly Trp Leu Val Thr
           35                  40                  45
   Arg Tyr Ala Asp Ile Lys Thr Val Leu Gly Asp Pro Arg Phe Ser Ala
       50                  55                  60
   Ala Ala Ile Leu Asn Arg Asp Val Pro Arg Gly Phe Pro Leu Ile Leu
   65                  70                  75                  80
   Arg Glu His Ser Leu Gly Thr Met Asp Pro Pro Glu His Thr Arg Leu
                   85                  90                  95
   Arg Lys Leu Val Gly Lys Ala Phe Thr Ala Arg Arg Val Glu Gln Leu
               100                 105                 110
   Arg Pro Arg Thr Gln Gln Leu Val Asp His Leu Leu Asp Arg Met Ala
           115                 120                 125
   Ala Asp Gly Pro Pro Gly Asp Leu Val Ser Ala Leu Ala Leu Pro Leu
   130 135 140
   Pro Ile Lys Val Ile Cys Asp Leu Leu Gly Ile Pro Val Ala Asp Arg
   145 150 155 160
   Glu Arg Phe Arg Val Trp Ser Asp Ile Ala Leu Ala Ile Thr Ser Asn
   165 170 175
   Ser Pro Glu Glu Ile Arg Glu Ser Arg Asp Gln Ile Arg Ala Tyr Ile
   180 185 190
   Gly Glu Leu Val Gln Gln Arg Lys Lys Met Pro Thr Glu Asp Leu Leu
   195 200 205
   Ser Val Leu Val Gln Ala Arg Ala Glu Gly Ala Gln Leu Ser Glu Glu
   210 215 220
   Glu Ile Val Val Thr Gly Ala Gly Leu Leu Ile Ala Gly Phe Glu Thr
   225 230 235 240
   Thr Ala Asn His Ile Ala Asn Phe Thr Phe Asn Leu Leu Thr His Pro
   245 250 255
   Asp Gln Leu Asp Lys Leu Ile Ala Asp Pro Glu Leu Val Pro Arg Ala
   260 265 270
   Val Glu Glu Leu Leu Arg Tyr Thr Pro Leu Gly Ala Thr Pro Gly Phe
   275 280 285
   Pro Arg Ile Ala Thr Glu Asp Leu Glu Leu Gly Gly Val Ser Ile Arg
   290 295 300
   Arg Gly Asp Ala Val Phe Phe Glu Ile Ala Ser Ala Asn Arg Asp Ser
   305 310 315 320
   Ala Val Phe Asp Gly Pro Asp Glu Leu Asp Leu Ala Arg Glu His Asn
   325 330 335
   Ser His Met Ala Leu Gly His Gly Pro His Tyr Cys Ile Gly Ala Gln
   340 345 350
   Leu Ala Arg Met Glu Leu Gln Val Ala Ile Gly Thr Leu Ile Lys Arg
   355 360 365
   Phe Pro Gln Leu Ser Phe Ala Val Pro Val Asp Glu Val Val Trp Lys
   370 375 380
   Arg Gly Arg Met Thr Arg Gly Pro Glu Ala Leu Pro Ile Thr Trp
   385 390 395
<210> 28
   <211> 248
   <212> PRT
   <213> Saccharopolyspora sp. NRRL30141
<400> 28
   Val Val Arg Asn Gly His Asp Gln Pro Arg Glu Val Leu Thr Ser Ala
   1 5 10 15
   Gly Ala Val Glu Val Thr Ala Pro Arg Val Asn Asp Lys Arg Thr Asp
   20 25 30
   Pro Asp Thr Gly Ala Arg Arg Arg Phe Ser Ser Ala Ile Leu Pro Pro
   35 40 45
   Trp Ala Arg Lys Thr Pro Lys Ile Thr Glu Met Leu Pro Leu Leu Tyr
   50 55 60
   Leu His Gly Leu Ser Ser Gly Asp Phe Val Pro Ala Leu Gly Gln Phe
   65 70 75 80
   Leu Gly Ser Ser Lys Gly Leu Ser Ala Thr Leu Ile Thr Lys Leu Thr
   85 90 95
   Glu Gln Trp Arg Thr Glu His Arg Ala Phe Asn Glu Arg Gly Leu Ser
   100 105 110
   Glu Val Asp Phe Val Tyr Leu Arg Ala Asp Gly Ile His Val Asn Ile
   115 120 125
   Arg Leu Glu Glu His Lys Leu Ser Leu Leu Val Val Ile Gly Val Arg
   130 135 140
   Ala Asp Gly Arg Lys Glu Leu Val Ala Leu Ala Asp Gly Tyr Arg Glu
   145 150 155 160
   Ser Thr Glu Ser Trp Ala Gly Leu Thr Tyr Cys Val Thr Ala Ser Ala
   165 170 175
   Ala Val Cys Val Pro Arg Tyr Trp Pro Ser Ala Thr Val His Trp Gly
   180 185 190
   Ser Gly Ala Arg Ser Ala Arg Leu Ser Leu Ile Arg Ala Ser Ser Ala
   195 200 205
   Thr Gly Ser Thr Arg Ser Ala Met Cys Ser Pro Arg Cys Arg Asn Arg
   210 215 220
   Arg Ile Pro Ala Arg Arg Arg Pro Trp Pro Arg Ser Gly Met Pro Arg
   225 230 235 240
   Thr Ala Gly Thr Cys Trp Thr Arg
   245
<210> 29
   <211> 276
   <212> PRT
   <213> Saccharopolyspora sp. NRRL30141
<400> 29
   Val Ala Glu Thr Ile Gly Leu Val Arg Arg Thr Ser Ser Gly Gln Leu
   1               5                   10                  15
   Ala Glu Thr Glu Leu Leu Ala Leu Leu Arg Arg Asp Gly Gly Arg Tyr
               20                  25                  30
   Arg Ser Thr Val Leu Ala Leu Thr Ala Pro Gly Phe Asn Arg Pro Ser
           35                  40                  45
   Glu Met Met His Arg Ala Val Leu Ser Gly Arg Ala His Thr Ala Gln
       50                  55                  60
   Val Leu Gly Thr Asp Leu Trp Gly Tyr Tyr Gly Thr Asn Pro Glu Glu
   65                  70                  75                  80
   Ala Lys Trp Phe Gly Gly Ala Met Thr Asp Leu Thr Asn Leu Val Ala
                   85                  90                  95
   Asp Leu Val Leu Ala Arg Tyr Glu Phe Ser Gly Arg Gly Thr Ile Met
               100                 105                 110
   Asp Val Gly Gly Ser His Gly Ile Phe Leu Ser Arg Ile Leu His Ala
           115                 120                 125
   Gln Pro Asp Ala Lys Gly Val Leu Phe Asp Arg Met Glu Val Val Glu
       130                 135                 140
   Glu Ala Arg Asn His Leu Asp Gln Asp Ile Arg Thr Arg Ile Gln Ile
   145                 150                 155                 160
   Val Gly Gly Asn Phe Phe Glu Gly Val Pro Glu Gly Gly Asp Leu Tyr
                   165                 170                 175
   Ile Leu Lys Ser Val Leu Cys Asp Trp Asp Asp Gln Ser Cys Leu Gln
               180                 185                 190
   Ile Leu Ser Arg Ile Arg Asn Ala Ala Met Pro Gly Ala Ser Leu Leu
           195                 200                 205
   Ile Val Asp Trp Leu Tyr Pro Asp Glu Ser Asp Pro Gly Leu Asp Ala
       210                 215                 220
   Ile Tyr Leu Gln Gln Ala Ile Ser Val Asn Gly Arg Val Arg Asn Gln
   225                 230                 235                 240
   Glu Gln Phe Glu Ser Leu Leu Lys Ala Thr Gly Phe Ala Val Thr Arg
                   245                 250                 255
   Val Glu Arg Thr Thr Pro Glu Asn Trp Ile Pro Ala Thr Ile Ile Glu
               260                 265                 270
   Ala Ile Arg Arg
           275
<210> 30
   <211> 616
   <212> PRT
   <213> Saccharopolyspora sp. NRRL30141
<400> 30
   Val Gly Cys Leu Arg Ser His Ala Ala Tyr Pro Ala Ser Ala Asp Gln
   1               5                   10                  15
   Gly Ala Leu Leu Arg Asp Pro Val Arg Arg Gly Val Gln Pro Gly Arg
   20 25 30
   Ala His Asp Arg His Arg Arg Arg Thr Arg Arg His Arg Ala Gly Arg
   35 40 45
   His Tyr Arg Pro Gly Gln Ser Gln Gly Ala Asn Arg Pro Leu Gly Thr
   50 55 60
   Leu Arg Gln Val Asp Asp Leu Gly Gly Val Gln Pro Gly Arg Gly Lys
   65 70 75 80
   Val Gly His Gln Arg Arg Arg Arg His Arg Cys Pro Val Val Pro Arg
   85 90 95
   Arg Pro Ala Ala Pro Thr Ala Ala Gly Asp Ala Gly Arg Pro Arg Arg
   100 105 110
   Gln Gly Val Arg Ser Gly Val Gln Leu Glu Arg Gly Gly Gly Leu His
   115 120 125
   Arg Gly Phe Leu Arg Asn Arg Arg Ser Arg Gly Ala Val Arg Gln Trp
   130 135 140
   Arg Ala Ala Asp Arg Ala Arg Pro Val Ala Thr Gly Leu Leu Asn Gly
   145 150 155 160
   His Glu Leu Arg Leu Thr Ala Val Ser Thr Val Asp Asp Ser Ala Leu
   165 170 175
   Ala Ile Ala Ser Lys Pro Arg Ser Pro Ile Pro Asp Pro Arg Cys Lys
   180 185 190
   Val Ala Thr Thr Ser Ser Ala Ser Ser Pro Leu Pro Gly Leu Gly Pro
   195 200 205
   Val Val Arg Ser Asn Phe Gly Pro Thr Arg Leu Gly Phe Val Leu Met
   210 215 220
   Leu Lys Phe Phe Glu Leu Glu Gly Arg Phe Pro Gln Phe Val Glu Glu
   225 230 235 240
   Phe Pro Gln Ala Ala Val Asp Tyr Val Ala Gly Val Val Lys Val Pro
   245 250 255
   Ala Glu Asp Leu Ala Lys Tyr Xaa Leu Ser Ser Arg Ser Ala Lys Gly
   260 265 270
   His Arg Thr Gln Ile Arg Glu Thr Leu Gly Tyr Xaa Pro Ala Thr Arg
   275 280 285
   Ala Asp Glu Glu Arg Leu Thr Ala Trp Leu Ala Asp Glu Val Cys Pro
   290 295 300
   Val Glu Met Val Glu Asp Arg Leu Arg Glu Ala Leu Leu Val Gln Cys
   305 310 315 320
   Arg Ser Asp His Val Glu Pro Pro Gly Arg Val Glu Arg Ile Val Ala
   325 330 335
   Ala Ala Arg Ala Arg Ala Asp Arg Val Phe Cys Ala Gln Thr Val Ala
   340 345 350
   Arg Leu Gly Glu Ala Cys Ala Gly Arg Leu Leu Thr Leu Val Ala Glu
   355 360 365
   Gly Asn Glu Glu Gly Thr Ala Leu Leu Ala Ser Leu Lys Arg Asp Pro
   370 375 380
   Gly Ala Val Gly Leu Asp Ser Leu Leu Ala Glu Ile Thr Lys Leu Thr
   385 390 395 400
   Ala Val Arg Arg Leu Gly Leu Pro Glu Gly Leu Phe Ala Asp Cys Ser
   405 410 415
   Glu Lys Leu Val Ala Ala Trp Ala Gly Ala Gly Asp Gln Asp Val Ser
   420 425 430
   Leu Gly Leu Pro Gly Arg Trp Gln Gly Cys Ala Asp His Ala Ala Gly
   435 440 445
   Gly Ala Val Arg Val Pro Ala Gly Gly Asp His Arg Cys Pro Gly Gly
   450 455 460
   Ala Ala Gly Arg Ser Gly Ser His Lys Ile Asn Ala Arg Ala Glu Arg
   465 470 475 480
   Arg Val Glu Arg Gln Leu Thr Ala Glu Leu Lys Lys Val Arg Gly Lys
   485 490 495
   Glu Gly Ile Leu Phe Gln Leu Ala Asp Ala Ser Val Gly Gln Pro Glu
   500 505 510
   Gly Thr Val Arg Arg Val Leu Phe Pro Val Val Gly Glu Lys Thr Leu
   515 520 525
   Arg Asp Leu Val Ala Glu Ala Asn Glu Lys Ala Phe Lys Ala Arg Val
   530 535 540
   Arg Thr Thr Leu Arg Ser Ser Tyr Ser Ser Tyr Tyr Pro Ala Asp Ala
   545 550 555 560
   Ala Val Thr Ala Ala Asp Ala Arg Leu Gln Val Gln Gln His Arg Leu
   565 570 575
   Pro Ala Gly Asp Gly Arg Ala Arg Ala Ala Gly Glu Val Arg Arg Arg
   580 585 590
   Arg Arg Gln Asp Pro Leu Leu Arg Arg Arg Arg Arg Gly Ala Asp Gly
   595 600 605
   Arg Pro Ser Pro Gln Gly Leu Ala
   610 615
<210> 31
   <211> 458
   <212> PRT
   <213> Saccharopolyspora sp. NRRL30141
<400> 31
   Val Leu Ile Phe Asp Arg Gly His Ala Glu Lys Ile Arg Gln Glu Tyr
   1               5                   10                  15
   Ala Cys His Phe Asn Thr His Arg Pro His Gln Ala His Asp Gln Gln
               20                  25                  30
   Ala Pro Tyr Val Ala Arg Ala Ser Tyr Arg Cys Arg Gln Leu Gly Ser
           35                  40                  45
   Asn Ala Asp Lys Pro Trp Gln Asp Ser Ser Thr Ser Thr Ala Lys Gln
       50                  55                  60
   Pro Asp Gly Pro Thr Lys Pro Gln Leu Thr Ala Ser Glu Pro Phe Leu
   65                  70                  75                  80
   Lys His Tyr Gly Ser Cys Cys Ser Pro Arg Ser Ser Arg Cys Arg Ser
                   85                  90                  95
   Ala Gly Arg Ser Arg Phe Gln Glu Asp Gln Pro Val Trp Ser Pro Cys
               100                 105                 110
   Arg Pro Ala Glu Ala Ala Pro Thr Thr Gly Trp Thr Ser Gly Val Leu
           115                 120                 125
   Ala Arg Arg Thr Ala Gly Val Arg Asn Arg Pro Tyr Arg Arg Gly Ser
       130                 135                 140
   Gln His Thr Cys Gly Thr Trp Ser Arg Gln Gly Pro Gly Arg Gly Thr
   145                 150                 155                 160
   Gly Val His His Phe Arg Arg Ser Gly Leu Asp His Asp Pro Ala Leu
                   165                 170                 175
   Pro Arg Tyr Cys Phe His Arg Glu Arg Ala Ala Val Gln Val Thr Phe
               180                 185                 190
   Glu Arg Phe Asp Ala Val Val Ala Glu His Ser Leu Gly His Ala Lys
           195                 200                 205
   Tyr Gly Gly Ser Val Tyr Glu Lys Arg Asp Leu Gly Gln Gln Val Pro
       210                 215                 220
   Cys Arg His Val Gln Leu Phe Leu Arg Glu Thr Ala Val Gly Ser Pro
   225                 230                 235                 240
   Pro Ala Pro Arg Asp Arg Phe Arg Leu Gly Gln Thr Gly Leu Pro Glu
                   245                 250                 255
   Val Phe Ile Arg Pro Glu Asp Leu Arg Asn Leu Val Pro Arg Ala Gln
               260                 265                 270
   Val Leu Leu Val Leu Val Glu Pro Gly Glu Val Asp Asp His Leu Leu
           275                 280                 285
   Gly Gly Arg Asp Ala Glu His Gly Lys Arg Pro Glu Tyr Leu Pro Ala
       290                 295                 300
   Gln Pro Ser Cys Leu Ala Ser Arg Leu Pro Leu Cys Phe His Arg Arg
   305                 310                 315                 320
   Ile Ala Ala Gly Leu His Arg Glu Ser Gly Gly Arg Val Arg Glu Glu
   325 330 335
   His Met Gln Arg Leu Glu Leu Gly His Arg Pro Pro Gln Glu Leu Val
   340 345 350
   Gln Ser Cys Leu Phe Glu Val Ala Val Glu Lys Ser Cys Ala Asn Lys
   355 360 365
   Gly Val Ser Thr Pro Leu Glu Asp Pro Gly Ala Leu Leu Gly Arg Arg
   370 375 380
   Phe Met Pro His His Lys Leu Gly Ile His Val His Ala Ser Ser Arg
   385 390 395 400
   His Gly Cys Ile His Val Val Cys Thr Phe Cys Leu Glu Thr Val Ala
   405 410 415
   Ser Ser Thr Leu Arg Gln Leu Arg Arg Ile Leu Tyr Ala Asn Leu Asp
   420 425 430
   Ala Leu Asp Ser Pro Leu Gln Arg Phe Arg Ile Cys Ala Arg Glu Pro
   435 440 445
   Arg Ala Glu Arg Gly Ile Pro Phe Pro Gln
   450 455
<210> 32
   <211> 305
   <212> PRT
   <213> Saccharopolyspora sp. NRRL30141
<400> 32
   Val Asp Val Ala Phe Cys Ala Ile Cys Gly Ser Asp Leu His Leu Arg
   1               5                   10                  15
   Ala Met Pro His Leu Val Pro Ala Asp Ala Val Leu Gly His Glu Ile
               20                  25                  30
   Ser Gly His Val Ala Ala Pro Gly Gly Glu Arg Leu Thr Ala Gly Gln
           35                  40                  45
   Ala Val Val Val Trp Pro Lys Ala Gly Cys Gly Asp Cys Asp Asp Cys
       50                  55                  60
   Arg Val Gly Asp Asn His Leu Cys Ala Val Gln Pro Trp Arg Leu Ser
   65                  70                  75                  80
   Ser Leu Gly Leu Gly Thr Arg Pro Gly Gly Tyr Ala Glu Ala Val Val
                   85                  90                  95
   Val Pro Glu His Thr Val Tyr Ala Val Pro Asp Gly Val Ser Leu Glu
               100                 105                 110
   His Ala Ala Leu Thr Glu Pro Leu Ser Cys Ala Val His Ala Val Asp
           115                 120                 125
   Arg Ser Gly Ile Ser Ala Ala Asp Thr Val Thr Val Leu Gly Gly Gly
       130                 135                 140
   Thr Val Gly Phe Leu Leu Ala His Val Leu Arg Leu Arg Gly Val Glu
   145 150 155 160
   Asp Val Arg Val Val Glu Pro His Pro Val Arg Arg Ala Arg Leu Thr
   165 170 175
   Ala Thr Gly Ile Thr Thr Val Asp Val Asp Glu Arg Gly Pro Asp Ala
   180 185 190
   Asp Val Val Phe Glu Cys Val Gly Ser Val Thr Ala Leu Thr Asp Ala
   195 200 205
   Ala Arg Arg Val Arg Thr Arg Gly Thr Ile Val Ala Leu Gly Val Asn
   210 215 220
   Glu Arg Pro Ser Glu Leu Asp Ser Val Ala Leu Ile Thr Lys Glu Ile
   225 230 235 240
   Arg Ile Val Gly Ser Phe Ala Gln Asn Arg Gly Ala Phe Glu Ala Ala
   245 250 255
   Leu Glu Leu Leu Gly Gly Gly Arg Ile Pro Val Glu Arg Ile Ile Thr
   260 265 270
   Asp Val Val Pro Leu Asp Ala Gly Pro Val Ser Ala Met Met Asp Ala
   275 280 285
   Leu Thr Gly Arg Pro Gly Asp His Gln Val Val Met Ile Ala Pro Gly
   290 295 300
   Gly
   305
<210> 33
   <211> 20
   <212> DNA
   <213> Saccharopolyspora spinosa
<400> 33
   gtgccgaata cgcgaaggtc 20
<210> 34
   <211> 20
   <212> DNA
   <213> Saccharopolyspora spinosa
<400> 34
   tccaggaagg tattccgcgc 20
<210> 35
   <211> 20
   <212> DNA
   <213> Saccharopolyspora spinosa
<400> 35
   gcgacaacgc gatccagatc 20
<210> 36
   <211> 22
   <212> DNA
   <213> Saccharopolyspora spinosa
<400> 36
   ccatgtcgtg ggcatatttc tc 22
<210> 37
   <211> 21
   <212> DNA
   <213> Saccharopolyspora spinosa
<400> 37
   tcccgatgcc tggattcatt g 21
<210> 38
   <211> 22
   <212> DNA
   <213> Saccharopolyspora spinosa
<400> 38
   cgtccatcat cgagaagtgg tc 22
<210> 39
   <211> 16
   <212> DNA
   <213> Saccharopolyspora sp. NRRL 30141
<400> 39
   cgtacgtggc gatcag 16
<210> 40
   <211> 21
   <212> DNA
   <213> Saccharopolyspora sp. NRRL 30141
<400> 40
   gtccaagttt cggttgcgtt c 21

## Description

### Summary of the Invention

The present invention provides novel butenyl-spinosyn biosynthetic genes, vectors incorporating the biosynthetic genes, *Saccharopolyspora* strains transformed with the biosynthetic genes, methods of using these genes to increase production of spinosyn-like insecticidal macrolides, and methods of using the genes or fragments thereof to change the metabolites made by spinosyn-producing strains of *Saccharopolyspora* spp.

### Background of the Invention

The naturally produced spinosyn compounds consist of a 5,6,5-tricylic ring system, fused to a 12-membered macrocyclic lactone, a neutral sugar (rhamnose) and an amino sugar (forosamine) (see Kirst *et al*., 1991). If the amino sugar is not present, the compounds have been referred to as pseudoaglycones, and if the neutral sugar is not present then the compounds have been referred to as reverse pseudoaglycones.

A83543 spinosyns are produced by *Saccharopolyspora spinosa* strain NRRL18395 and derivatives thereof. The known members of the A83543 spinosyn family and the strains producing them have been disclosed in US Patent No. 5,362,634; 5,202,242; 5,840,861; 5,539,089 and 5,767,253. The compounds are identified by letter designation: spinosyn A, B, etc. (see Kirst *et al*., 1991). The structure of A83543 spinosyn A is given in Table 1 hereinafter. The A83543 spinosyn compounds are useful for the control of arachnids, nematodes and insects, in particular *Lepidoptera* and *Diptera* species, and they have favorable environmental and toxicological profiles.

DNA sequences for the genes encoding the enzymes which direct the biosynthesis of A83543 spinosyn are disclosed in U.S. Patent No. 6,143,526, corresponding to WO99/46387. The cloned genes and open reading frames are designated as *spnA*, *spnB*, *spnC*, *spnD*, *spnE*, *spnF*, *spnG*, *spnH*, *spnI*, *spnJ*, *spnK*, *spnL, spnM*, *spnN*, *spnO*, *spnP*, *spnQ*, *spnR*, *spnS*, *S*. *spinosa gtt*, *S. spinosa gdh, S. spinosa epi,* and *S. spinosa kre.*

The spinosyn biosynthetic genes, excluding those for rhamnose biosynthesis, specifically genes *spnA*, *spnB*, *spnC*, *spnD, spnE*, *spnF*, *spnG*, *spnH*, *spnI*, *spnJ*, *spnK*, *spnL*, *spnM*, *spnN*, *spnO*, *spnP*, *spnQ*, *spnR*, and *spnS* are located contiguously on an approximately 74 kb region of the *S. spinosa* chromosome. The *spnA*, *spnB*, *spnC*, *spnD*, and *spnE* genes were shown to be similar to genes responsible for polyketide biosynthesis and disruption of *spnA, spnD*, or *spnE* eliminated all spinosyn production. A83543 spinosyn synthesis also involves bridging of the lactone nucleus, an activity that is rare in macrolide producers; the *spnF*, *spnJ*, *spnL*, and *spnM* genes were believed to be involved in this biosynthetic step. The *spnG*, *spnH*, *spnI*, and *spnK* genes were reported to be involved in rhamnose addition and modification, and the *spnN*, *spnO*, *spnP, spnQ, spnR* and *spnS* genes were reported to be involved in biosynthesis and addition of the forosamine sugar. The genes required for rhamnose biosynthesis were not located contiguously to the rest of the A83543 spinosyn biosynthetic genes. *S. spinosa gtt,* and *S. spinosa kre* were cloned on a distinct fragment, and *S. spinosa gdh* and *S. spinosa epi,* were cloned on other distinct fragments.

Recently a second class of spinosyns, the butenyl-spinosyns, produced by a novel organism, *Saccharopolyspora* sp. LW107129 (NRRL 30141) or derivatives thereof, was disclosed in WO 01/19840 and in US 5 817 820. Over 40 members of this chemical family have been defined. The butenyl-spinosyn compounds produced by Saccharopolyspora sp. LW107129 (NRRL 30141) are different from the compounds in the A83543 spinosyn series. The primary difference between the two classes of spinosyns is the substitution of the carbon tail attached to the macrocyclic ring at C-21. The natural butenyl spinosyns are substituted with a 3-4 carbon chain at C-21, preferably butenyl, while natural A83543 spinosyns are substituted with a 1-2 carbon chain at C-21, preferably ethyl.

The butenyl-spinosyn compounds are useful as reactants in the production of synthetically modified spinosoid compounds as disclosed in US 6 919 464. More preferably, the butenyl-spinosyn compounds and their synthetic derivatives are useful for the control of arachnids, nematodes and insects, in particular *Lepidoptera* and *Diptera* species.

In addition to the butenyl group at C-21, butenyl-spinosyns exhibit a number of other differences from the A83543 spinosyn series. A subset of the butenyl-spinosyn compounds and factors which demonstrate diversity relative to A83543 spinosyns are summarized in Table 1. The butenyl spinosyns are named in Table I and referred to hereinafter by the structural acronyms "for-rham-I", "for-rham-II", "for-rham-III" and derivatives thereof. In these cases I, II, and III refer to the appropriately-substituted macrolide structure (I: R⁴=R⁵=H; II: R⁵ = CH₃, R⁴ = H or OH; III: R⁵ = H, R⁴ = OH), 'for' represents the sugar at C-17 (for = forosamine), and 'rham' represents the sugar at C-9 (rham = tri-*O*-methylrhamnose). A second type of macrolide structure which is produced by strain NRRL 30141, with general Formula (2) having a 14-membered macrolide ring, is referred to hereinafter as IV and the fully glycosylated comound as "for-rham-IV". The butenyl-spinosyn compounds of formulae (1) and (2) are useful for the control of arachnids, nematodes and insects, in particular *Lepidoptera* and *Diptera* species, and they are quite environmentally friendly and have an appealing toxicological profile.

These differences include extensive modifications at the C-21 position, hydroxylation at the C-8 position and substitution of alternate sugars, including neutral sugars, for forosamine at C-17. In addition, a compound possessing a 5,6,5-tricylic ring system, fused to a 14 membered macro-cyclic ring with forosamine and rhamnose attached at C-17 and C-9, respectively was previously disclosed in the aforementioned application.

**Table 1**

| | | | | | | | |
|---|---|---|---|---|---|---|---|
| 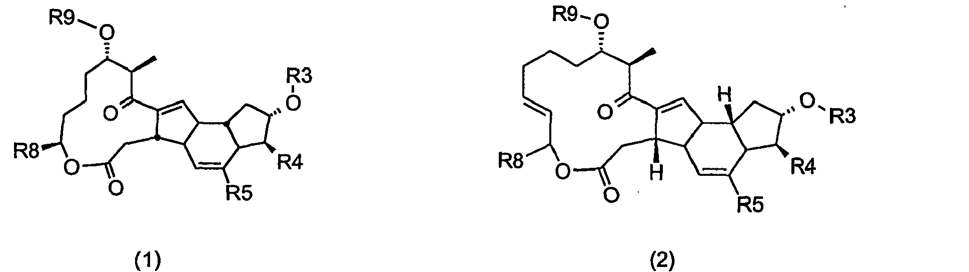 | | | | | | | |

| cmpd. no. | Name | formula | R³ * | R⁴ | R⁵ | R⁸ | R⁹ ** |
|---|---|---|---|---|---|---|---|
| 1 | A83543 spinosyn A | (1) | (3a) | H | H | ethyl | (9a) |
| 2 | for-rham-I | (1) | (3a) | H | H | 1-butenyl | (9a) |
| | (butenyl spinosyn) | | | | | | |
| 3 | 2"-hydroxy- for-rham-I | (1) | (3a) | H | H | 1-butenyl | (9d) |
| 4 | for-(3'-O-desmethyl rham)-I | (1) | (3c) | H | H | 1-butenyl | (9a) |
| | (3-ODM) | | | | | | |
| 5 | for-rham-II | (1) | (3a) | H | CH₃ | 1-butenyl | (9a) |
| 6 | for-rham-III | (1) | (3a) | OH | H | 1-butenyl | (9a) |
| 7 | 24,25-dehydro-forrham-I | (1) | (3a) | H | H | 1,3-butadienyl | (9a) |
| 8 | ami-rham-I | (1) | (3a) | H | H | 1-butenyl | (9e) |
| 9 | 3"-O-methyl-glu-rham-I | (1) | (3a) | H | H | 1-butenyl | (9f) |
| 10 | ami-rham-III | (1) | (3a) | OH | H | 1-butenyl | (9e) |
| 11 | mole-rham-III | (1) | (3a) | OH | H | 1-butenyl | (9g) |
| 12 | 24-demethyl-for-rham-I | (1) | (3a) | H | H | 1-propenyl | (9a) |
| 13 | rham-I | (1) | (3a) | H | H | 1-butenyl | H |
| 14 | 24-hydroxy-rham-I | (1) | (3a) | H | H | 3-hydroxy-1-butenyl | H |
| 15 | 24-hydroxy-rham-III | (1) | (3a) | OH | H | 3-hydroxy-1-butenyl | H |
| 16 | 22,23-dihydro-rham-I | (1) | (3a) | H | H | n-butyl | H |
| 17 | (4'-N-desmethyl-1",4"-diepi-for)-rham-I | (1) | (3a) | H | H | 1-butenyl | (9h) |
| 18 | 5"-epifor-rham-I | (1) | (3a) | H | H | 1-butenyl | (9i) |
| 19 | 24,25-dehydro-forrham-III | (1) | (3a) | OH | H | 1,3-butadienyl | (9a) |
| 20 | 24-desmethyl-forrham-III | (1) | (3a) | OH | | H 1-propenyl | (9a) |
| 21 | for-rham-IV | (2) | (3a) | H | H | ethyl | (9a) |
| 22 | for-(4'-*O*-desmethylrham)-(4-ODM) | (1A) | (3d) | H | H | 1-butenyl | (9a) |
| 23 | for-(3',4'-di-*O*-desmethyl-rham)-I | (1A) | (3e) | H | H | 1-butenyl | (9a) |

| | | | | | | | |
|---|---|---|---|---|---|---|---|
| *R³ is a group having one of the following formulas (3a) through (3c) 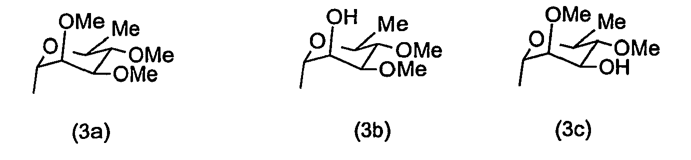 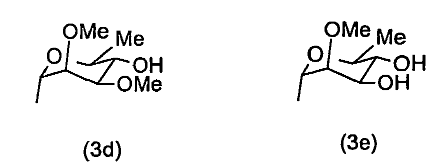 **R9 is a group having one of the following formulas (9a) to (9i) 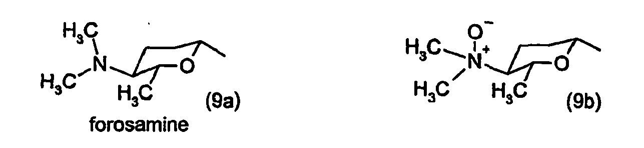 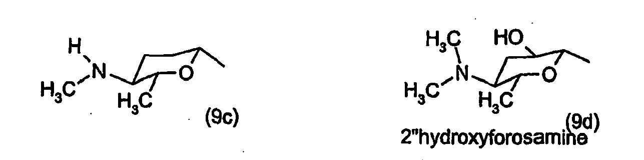 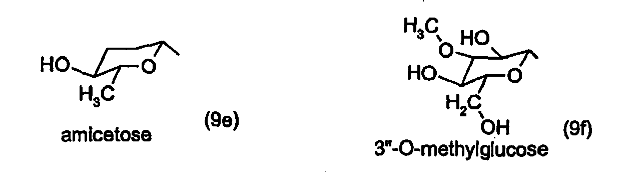 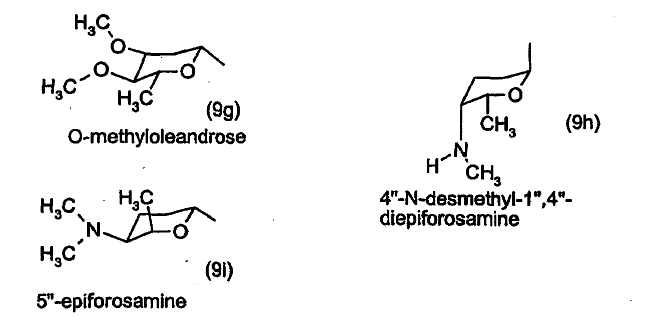 | | | | | | | |

Compounds 1-21 in Table I are produced by *Saccharopolyspora* sp. LW107129 (NRRL 30141) and are disclosed in WO 01/19840. Compounds 32823 are disclosed in US 5 817 820.

Despite the differences between the structures ofbutenyl-spinosyns and A83543 spinosyns, it can be deduced that some of the biosynthetic genes would be similar. However, as detailed above, Saccharopolyspora sp. LW107129 (NRRL 30141) produces a wide range of unique butenyl-spinosyn factors and compounds, which have not been observed in the A83543 spinosyns. Therefore, this organism must also posses novel biosynthetic enzymes, which are distinct from the A83543 spinosyn biosynthetic enzymes of *S. spinosa*. Specifically, relative to A83543 spinosyns, Saccharopolyspora sp. LW107129 (NRRL 30141) butenyl-spinosyn biosynthetic enzymes must be capable of extending the polyketide chain by 2 carbons (resulting in butenyl rather than ethyl at C-21). They must also be able to synthesize and attach alternate amino and neutral sugars at C-17, and hydroxylate at C-8 & C-24. In addition, rhamnose methylation must be different in Saccharopolyspora sp. LW107129 (NRRL 30141) relative to *S. spinosa*. Blocked mutants of *S. spinosa* which exhibited altered methylation of the rhamnose on A83543 spinosyn (as disclosed in USP 5,202,242 and 5,840,861) typically produced mono-desmethylated rhamnose derivatives of A83543 spinosyns. Di-desmethyl rhamnose derivatives of A83543 spinosyns were only detected in the presence of methylase inhibitors like sinefungin. Mutants of Saccharopolyspora sp. LW107129 (NRRL 30141) with altered methylation of rhamnose produced di- and tri-desmethyl rhamnose derivatives of butenyl-spinosyns in high amounts, in the absence of methylase inhibitors.

A challenge in producing butenyl-spinosyn compounds arises from the fact that a very large fermentation volume is required to produce a very small quantity of butenyl-spinosyns. A cloned fragment of DNA containing one or multiple genes for butenyl-spinosyn biosynthetic enzymes would enable duplication of genes to increase yield. A yield increase of this type was achieved in fermentation's of *Streptomyces fradiae* by duplicating the gene encoding a rate-limiting methyltransferase that converts macrocin to tylosin (Baltz *et al.,* 1997) and in *S. spinosa* by duplicating the *gtt & gdh* genes (Baltz *et al.,* 2000).

Cloned butenyl-spinosyn biosynthetic genes also provide a method for producing new derivatives of the butenyl-spinosyns, with a different spectrum of insecticidal activity. Specific intermediates (or their natural derivatives) can be synthesized by mutant strains of Saccharopolyspora sp. LW107129 (NRRL 30141) in which certain genes encoding enzymes for butenyl-spinosyn biosynthesis have been disrupted using recombinant DNA methods. Such a strategy was used effectively to generate a strain of *Saccharopolyspora erythraea* producing novel 6-deoxyerythromycin derivatives (Weber & McAlpine, 1992). Also butenyl-spinosyn biosynthetic genes can be expressed in other organisms, such as *S. spinosa,* which produce similar compounds. When expressed from the native butenyl-spinosyn promoter or a heterologous promoter these genes produce new hybrid molecules with some of the unique structural features of both spinosyns and butenyl-spinosyns.

Novel intermediates can also be synthesized by mutant strains of Saccharopolyspora sp. LW107129 (NRRL 30141) or *S. spinosa* in which parts of certain genes encoding enzymes for butenyl-spinosyn biosynthesis have been replaced with parts of the same gene which have been specifically mutated *in vitro*, or with corresponding parts of genes from other organisms. The hybrid gene will produce a protein with altered functions, either lacking an activity or performing a novel enzymatic transformation. A new chemical substance would accumulate upon fermentation of the mutant strain. Such a strategy was used to generate a strain of *Saccharopolyspora erythraea* producing a novel anhydroerythromycin derivative (Donadio *et al*., 1993).

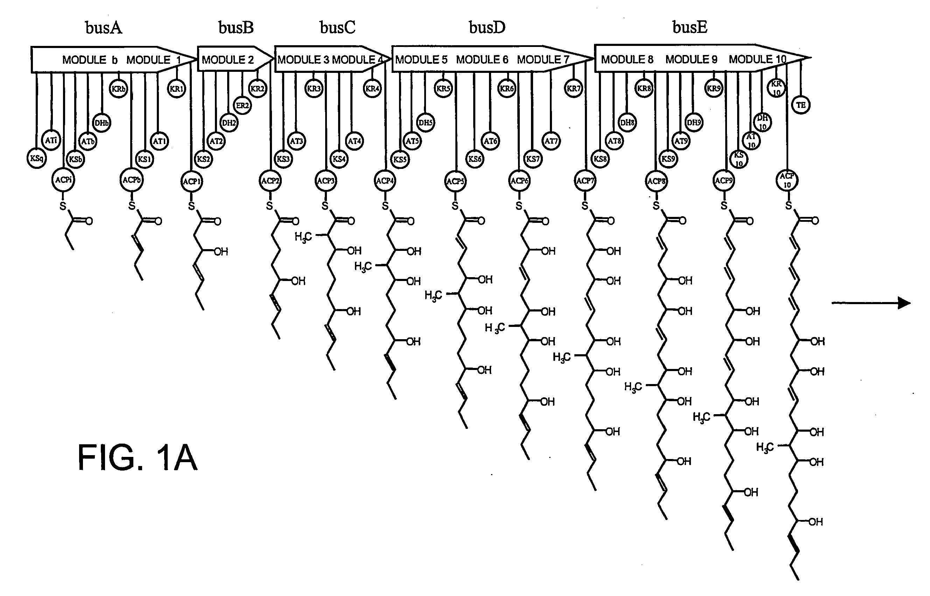

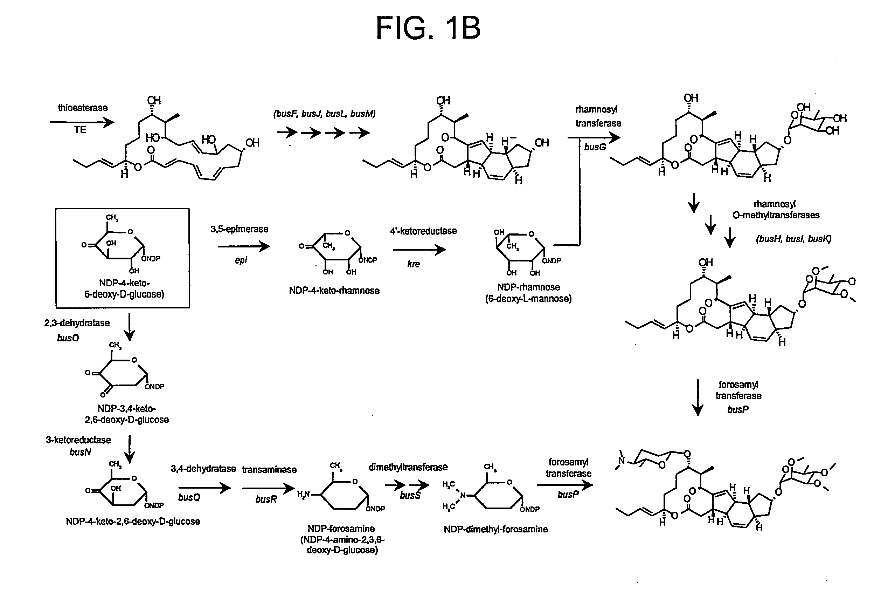

Biosynthesis of butenyl-spinosyns proceeds via stepwise condensation and modification of 2- and 3-carbon carboxylic acid precursors, generating a linear polyketide (Figure 1A) that is cyclized and bridged to produce the tetracyclic aglycone (Figure 1B). Pseudoaglycone (containing tri-O-methylated rhamnose) is formed next, then di-N-methylated forosamine or an alternate sugar is added to complete the biosynthesis (Figure 1B). Other macrolides, such as the antibiotic erythromycin, the antiparasitic avermectin and the immunosuppressant rapamycin, are synthesized in a similar fashion. In the bacteria producing these compounds, antibiotic biosynthesis is catalyzed by several very large, multifunctional proteins of a Type I polyketide synthase (PKS) (Donadio *et al*., 1991; Ikeda *et al*., 1999; Schwecke *et al*., 1995). Together the polypeptides form a complex consisting of an initiator module and several extender modules, each of which adds a specific acyl-CoA precursor to a growing polyketide chain, and modifies the β-keto group in a specific manner (Figure 1A). The structure of a polyketide is, therefore determined by the composition and order of the modules in the PKS. A module comprises several domains, each of which performs a specific function. The initiator module consists of an acyl transferase (AT) domain for addition of the acyl group from the precursor to an acyl carrier protein (ACP) domain. The initiator module may also contain a KSQ domain, which is highly similar to β-ketosynthase (KS) domains, but an essential active site cysteine has been replaced by glutamine (Bisang, *et al.,* 1999), so KSQ no longer has condensing activity. KSQ domains retain decarboxylase activity and determines the precursor specificity of the initiation module. The extender modules contain AT and ACP domains, along with an intact β-ketosynthase (KS) domain that adds the pre-existing polyketide chain to the new acyl-ACP by decarboxylative condensation. Additional domains may also be present in each extender modules to carry out specific β-keto modifications: a β-ketoreductase (KR) domain to reduce the β-keto group to a hydroxyl group, a dehydratase (DH) domain to remove the hydroxyl group and leave a double bond, and an enoyl reductase (ER) domain to reduce the double bond and leave a saturated carbon. The last extender module terminates with a thioesterase (TE) domain that liberates the polyketide from the PKS enzyme in the form of a macrocyclic lactone. Polyketide synthase enzymes are generally encoded by 3-7 large open reading frames (Donadio *et al*., 1991; Ikeda *et al*., 1999; Schwecke *et al*., 1995). Assembly of a functional polyketide synthase requires specific protein-protein interactions between these proteins.

Active macrolide antibiotics are derived from macrocyclic lactones by additional modifications, such as methylation and changes in reductive state, and the addition of unusual sugars. Most of the genes required for these modifications, and for the synthesis and attachment of the sugars, are clustered around the PKS genes. The genes encoding deoxysugar biosynthetic enzymes are similar in producers of macrolide antibiotics, such as erythromycin and tylosin (Donadio *et al*., 1993; Merson-Davies & Cundliffe, 1994), and producers of extracellular polysaccharides, such as the O-antigens of *Salmonella* and *Yersinia* (Jiang *et al.,* 1991; Trefzer *et al.,* 1999). All these syntheses involve activation of glucose by the addition of a nucleotide diphosphate, followed by dehydration, reduction and/or epimerization. The resultant deoxy-sugar could undergo one or more additional modifications such as deoxygenation, transamination and methylation. The sugars are incorporated into macrolides by the action of specific glycosyltransferases. Genes involved in the synthesis and attachment of a sugar may be tightly clustered - even transcribed as a single operon - or they may be dispersed (Ikeda *et al.,* 1999; Shen *et al.,* 2000; Aguirrezabalaga *et al.,* 1998).

The following terms are used herein as defined below:

a.a. - amino acid

AmR - the apramycin resistance-conferring gene.

ACP - acyl carrier protein domain.

AT - acyltransferase domain.

blocked mutant - mutant strain having a mutation blocking the function of a specific enzyme of a biosynthetic pathway such that a precursor or shunt product is produced.

bp - base pairs.

*bus -* butenyl-spinosyn biosynthetic gene.

Butenyl-spinosyn - a fermentation product structurally distinct from A83543 spinosyns (Table 1), disclosed in WO 01/19840, or a similar macrocyclic lactone fermentation product produced by a microorganism utilizing all or most of the butenyl-spinosyn genes.

Butenyl-spinosyn genes- the DNA sequences that encode the products required for butenyl-spinosyn biosynthesis, more specifically the genes *busA, busB, busC, busD, busE, busF, busG, busH, busI, busJ, busK, busL, busM, busN, busO, busP, busQ, busR*, and *busS*, as described hereinafter, or functional equivalents thereof.

Cloning - the process of incorporating a segment of DNA into a recombinant DNA cloning vector and transforming a host cell with the recombinant DNA.

Codon bias - the propensity to use a particular codon to specify a specific amino acid. In the case of Saccharopolyspora sp. LW107129 (NRRL 30141) , the propensity is to use a codon having cytosine or guanine as the third base.

Complementation - the restoration of a mutant strain to its normal phenotype by a cloned gene.

Conjugation - a process in which genetic material is transferred from one bacterial cell to another.

cos - the cohesive end sequence of bacteriophage lambda.

Cosmid - a recombinant DNA cloning vector which is a plasmid that not only can replicate in a host cell in the same manner as a plasmid but also can be packaged into phage heads.

DH - dehydratase domain.

ER - enoyl reductase domain.

Gene - a DNA sequence that encodes a polypeptide.

Genomic Library - a set of recombinant DNA cloning vectors into which segments of DNA, representing substantially all DNA sequences in a particular organism have been cloned.

Homology - degree of similarity between sequences

Hybridization - the process of annealing two single stranded DNA molecules to form a double stranded DNA molecule, which may or may not be completely base paired.

*In vitro* packaging - the *in vitro* encapsulation of DNA in coat protein to produce a virus-like particle that can introduce DNA into a host cell by infection

kb - kilo base pairs.

KR - β-keto reductase domain.

KS - ketosynthase domain.

Mutagenesis - creation of changes in DNA sequence. They can be random or targeted, generated *in vivo* or *in vitro.* Mutations can be silent, or can result in changes in the amino acid sequence of the translation product which alter the properties of the protein and produce a mutant phenotype.

ORF - open reading frame.

ori - a plasmid origin of replication (oriR) or transfer (oriT).

% Identity - the % identity value give by the BLAST program when two sequences are compared.

% Similarity - the % similarity value given by the BLAST program when two sequences are compared.

PCR - polymerase chain reaction - a method to specifically amplify a region of DNA.

PKS - polyketide synthase.

Promoter - a DNA sequence that directs the initiation of transcription.

Recombinant DNA cloning vector - any autonomously replicating or integrating agent, including, but not limited to, plasmids, comprising a DNA molecule to which one or more additional DNA molecules can be or have been added.

Recombinant DNA methodology - technologies used for the creation, characterization, and modification of DNA segments cloned in recombinant DNA vectors.

Restriction fragment - any linear DNA molecule generated by the action of one or more restriction enzymes.

Spinosyn - a fermentation product also known as A83543, typically characterized by a 5,6,5-tricylic ring system, fused to a 12-membered macrocyclic lactone, with a 1-2 carbon chain at C-21, a neutral sugar (rhamnose) and an amino sugar (forosamine), or a similar macrocyclic lactone fermentation product produced by a microorganism utilizing all or most of the A83543 spinosyn genes.

Spinosyn genes- the DNA sequences that encode the products required for A83543 spinosyn biosynthesis, more specifically the genes *spnA, spnB, spnC, spnD, spnE, spnF, spnG, spnH, spnI, spnJ, spnK, spnL, spnM, spnN, spnO, spnP, spnQ, spnR, spnS, S. spinosa gtt, S. spinosa gdh, S. spinosa epi,* and *S. spinosa kre,* as described hereinafter, or functional equivalents thereof.

*spn -* A83543 spinosyn biosynthetic gene.

Subclone - a cloning vector with an insert DNA derived from another DNA of equal size or larger.

TE - thioesterase domain.

Transconjugant - recombinant strain derived from a conjugal mating.

### Brief Description of the Figures

FIG.S 1A and 1B are a diagram illustrating the butenyl-spinosyn biosynthetic pathway.

FIG. 2 is a map illustrating the arrangement of *Hind*III, *Eco*RV *and Sca*I fragments and open reading frames in the cloned region of Saccharopolyspora sp. LW107129 (NRRL 30141) DNA and location of butenyl-spinosyn genes on the cloned DNA.

FIG. 3 is a restriction site and functional map of Cosmid pOJ436.

FIG. 4 is a diagram illustrating the biosynthetic pathway for 17-(4"-O-methyloleandrose)-butenyl-spinosyn [Table 1; compound (11)].

### Brief Description of the Invention

Butenyl-spinosyn biosynthetic genes and related ORFs were cloned and the DNA sequence of each was determined. The cloned genes and ORFs are designated hereinafter as *busA, busB, busC, busD, busE, busF, busG, busH, busI, busJ, busK, busL, busM, burN, busO, busP, busQ, busR, busS,* ORF LI, ORF LII, ORF LIII, ORF LIV, ORF LVI, ORF LVII, ORF LVIII, ORF LIX, ORF RI, ORF RII and ORF RIII. The proposed functions of the cloned genes in spinosyn biosynthesis are identified FIG. 1 and in the discussion hereinafter.

According to a first aspect, the invention provides an isolated DNA molecule comprising a DNA sequence that encodes one or more butenyl-spinosyn PKS domains selected from KSb, ATb, KRb, DHb, and ACPb, said domains being described by, respectively, amino acids 998-1413, 1495-1836, 1846-2028, 2306-2518, and 2621-2710 of SEQ ID NO:3. For example the DNA sequence may comprise bases 2992-4239, 4483-5508, 5538-6084, 6916-7554, and/or 7861-8130 of SEQ ID NO:1.

The DNA sequence may further encode one or more butenyl-spinosyn PKS domains selected from KSi, ATi, ACPi, KS1, AT1, KR1, and ACP1, said domains being described by, respectively, amino acids 7-423, 528-853, 895-977, 2735-3160, 3241-3604, 3907-4086, and 4181-4262 of SEQ ID NO:3. For example, the DNA sequence may comprise bases 16-1269, 1582-2559, 2683-2931, 8203-9480, 9721-10812, 11719-12258, and/or 12541-12786 of SEQ ID NO:1.

The DNA sequence may further encode one or more spinosyn PKS domains selected from KS2, AT2, DH2, ER2, KR2, and ACP2, said domains being described by, respectively, amino acids 1-421, 534-964, 990-1075, 1336-1681, 1685-1864, and 1953-2031 of SEQ ID NO:4. For example, the DNA sequence may comprise bases 13059-14321, 14658-15900, 16026-16283, 17064-18100, 18111-18650, and/or 18915-19151 of SEQ ID NO:1.

The DNA sequence may further encode one or more spinosyn PKS domains selected from KS3, AT3, KR3, ACP3, KS4, AT4, KR4, and ACP4, said domains being described by, respectively, amino acids 1-421, 528-814, 1157-1335, 1422-1503, 1526-1949, 2063-2393, 2697-2875, and 2969-3049 of SEQ ID NO:5. For example, the DNA sequence may comprise bases 19553-20815, 21143-22000, 23021-23557, 23816-24061, 24128-25399, 25739-26731, 27641-28183, and/or 28457-28699 of SEQ ID NO:1.

The DNA sequence may further encode one or more spinosyn PKS domains selected from KS5, AT5, DH5, KR5, ACP5, KS6, AT6, KR6, ACP6, KS7, AT7, KR7, and ACP7, said domains being described by, respectively, amino acids 1-422, 537-864, 891-1076, 1382-1563, 1643-1724, 1746-2170, 2281-2611, 2914-3093, 3186-3267, 3289-3711, 3823-4151, 4342-4636, and 4723-4804 of SEQ ID NO:6. For example, the DNA sequence may comprise bases 29092-30357, 30700-31683, 31762-32319, 33235-33780, 34018-34263, 34327-35601, 35932-36924, 37831-38370, 38647-38892, 38956-40224, 40560-41544, 42115-42999 and/or 43258-43503 of SEQ ID NO:1.

The DNA sequence may further encode one or more spinosyn PKS domains selected from KS8, AT8, DH8, KR8, ACP8, KS9, AT9, DH9, KR9, ACP9, KS10, AT10, DH10, KR10, ACP10, and TE10, said domains being described by, respectively, amino acids 1-424, 530-848, 885-1072, 1371-1554, 1650-1728, 1751-2175, 2289-2616, 2642-2775, 3131-3315, 3396-3474, 3508-3921, 4036-4366, 4389-4569, 4876-5054, 5148-5229, and 5278-5531 of SEQ ID NO:7. For example, the DNA sequence may comprise bases 43945-45216, 45532-46488, 46597-47160, 48055-48606, 48892-49083, 49195-50469, 50809-51792, 51868-52269, 53335-53889, 54130-54366, 54466-55707, 56050-57042, 57109-57651, 58570-59106, 59386-59631, and/or 59776-60537 of SEQ ID NO:1.

The DNA sequence may encode a spinosyn PKS module comprising amino acids 6-977 of SEQ ID NO:3, and/or a spinosyn PKS module comprising amino acids 998-2710 of SEQ ID NO:3. For example, the DNA sequence may comprise bases 16-2931 and/or 2992-8130 of SEQ ID NO:1. The DNA sequence may also encode one or more further spinosyn PKS modules selected from the group consisting of amino acids 2735-4262 of SEQ ID NO:3, 1-2031 of SEQ ID NO:4, 1-1503 of SEQ ID NO:5, 1526-3049 of SEQ ID NO:5, 1-1724 of SEQ ID NO:6, 1746-3267 of SEQ ID NO:6, 3289-4804 of SEQ ID NO:6, 1-1728 of SEQ ID NO:7, 1751-3474 of SEQ ID NO:7, and 3508-5531 of SEQ ID NO:7. For example, the DNA sequence may also comprise bases 8203-12786, 13059-19151, 19553-24061, 24128-28699, 29092-34263, 34327-38892, 38956-43503, 43945-49083, 49195-54366, and/or 54466-60537 of SEQ ID NO:1.

The DNA sequence may encode a butenyl-spinosyn biosynthetic enzyme comprised of an amino acid sequence at least 98% identical to SEQ ID NOS 3, provided that if the sequence is less than 100% identical to the selected sequence, then the differences do not substantially affect the functional properties of the encoded enzyme. The DNA sequence may comprise the *busA* gene described by bases 1-13032 of SEQ ID NO:1. The DNA sequence may encode one or more further butenyl-spinosyn biosynthetic enzymes comprised, respectively, of an amino acid sequence at least 98% identical to one selected from SEQ ID NOS 4-7 and 8-29, provided that if the sequence is less than 100% identical to the selected sequence, then the differences do not substantially affect the functional properties of the encoded enzyme. The DNA sequence may comprise the *busB, busC, busD, busE,* ORF RI, ORFRII, ORF RIII, *busF, busG, busH, busI, busJ, busK, busL, busM, busN, busO, busP, busQ, busR, busS,* ORF LI, ORF LII, ORF LIII, ORF LIV, ORF LVI, ORF LVII, ORF LVIII, and/or ORF LIX genes, said genes being described by, respectively, bases 13059-19505, 19553-29053, 29092-43890, 43945-60636, 62090-63937, 65229-66602 and 68762-69676 of SEQ ID NO:1 and 114-938, 1389-2558, 2601-3350, 3362-4546, 4684-6300, 6317-7507, 7555-8403, 8640-9569, 9671-10666, 10678-12135, 12867-14177, 14627-15967, 16008-17141, 17168-17914, 18523-19932, 19982-20488, 20539-21033, 21179-21922, 22674-23453, 23690-24886, 26180-26923, and 27646-28473 of SEQ ID NO:2.

According to a second aspect, the invention provides a recombinant DNA vector which comprises a DNA sequence according to the first aspect.

According to a third aspect, the invention provides a host cell transformed with a recombinant vector according to the second aspect.

According to a fourth aspect, the invention provides a process for preparing a butenyl-spinosyn which comprises either cultivating a microorganism having operative butenyl-spinosyn biosynthetic genes in its genome, provided that the genome of the organism has been modified so that duplicate copies of at least one DNA sequence according to the first aspect; or cultivating a heterologous microorganism that has been transformed so that its genome contains an operative butenyl spinosyn biosynthetic gene comprising a DNA sequence according to the first aspect.

### Detailed Description of the Invention

As a prerequisite for the characterization and utilization of butenyl-spinosyn genes, it is necessary to isolate and characterize the genes that encode the enzymes involved in the biosynthesis of this insect control agent. The approach described in the following Example 1 involves the construction of a genomic cosmid library and subsequent screening via DNA-hybridization.

### EXAMPLE 1

### a. Isolation of total cellular DNA from Saccharopolyspora sp. LW107129 (NRRL 30141)

Saccharopolyspora sp. LW107129 (NRRL 30141) was inoculated into 100 mL vegetative media (9.0 g/L dextrose, 30 g/L trypticase soy broth, 3.0 g/L yeast extract, 2.0 g/L magnesium sulfate 7 H₂0) in a 500-mL Erlenmeyer flask and incubated shaking at 150 rpm for 72 hours at 30°C. This culture was centrifuged for 10 min. at 3,000 rpm/4°C to pellet the cells. The supernatant fluid was removed and the cell pellet was washed with 20 mL of TE buffer (10 mM Tris/HCl pH 8.0; 1 mM EDTA pH 8.0). Cells were centrifuged again at 3,000 rpm and the pellet frozen at 20°C until it was thawed for total cellular DNA isolation.

Total cellular DNA was isolated from Saccharopolyspora sp. LW107129 (NRRL 30141) (NRRL30141) using a Genomic DNA purification kit (Qiagen Inc., Valencia, CA). Frozen bacterial cell pellets from 100 mL of culture were resuspended in 11 ml of Buffer B 1 (50 mM Tris/HCl, pH 8.0; 50 mM EDTA, pH 8.0; 0.5% Tween 20, 0.5% Triton X-100) containing 11 µl of Qiagen Rnase A solution (100 mg/ml) by vortexing. To this suspension, 300 µl of a lysozyme (100 mg/ml; Sigma Chemical Co., St. Louis, MO) stock solution and 500 µl of a proteinase K (50 mg/ml; Sigma Chemical Co.) stock solution was added. The suspension was mixed by vortexing and incubated at 37°C for 30 min. Four ml of Buffer B2 (3 M guanidine HCl; 20% Tween 20) was added to the bacterial lysates and mixed into solution by gentle inversion of the tubes. The bacterial lysates were incubated at 50°C for 30 min. Total cellular DNA was isolated from the bacterial lysates using Qiagen Genomic-tip 500/G tips as per manufacturer's instructions. The resulting purified DNA was dissolved in 5 mL TE buffer and stored at 4°C.

### b. Construction of genomic cosmid library

Total cellular DNA isolated from Saccharopolyspora sp. LW107129 (NRRL 30141) was partially digested with *Sau*3A I based on section 3.1.3 of Ausubel, et al. (Current Protocols in Molecular Biology, John Wiley and Sons, Inc., New York, NY). Small-scale (40 µg of total cellular DNA in an 80 µl reaction volume) reactions were performed to determine the proper enzyme to total cellular DNA ratio that resulted in the maximal concentration of partially-digested DNA fragments in the 25-50 Kb size range. Reactions were heated at 65°C for 15 min to inactivate the *Sau*3A I enzyme and aliquots of the reactions were analyzed by electrophoresis in 0.3% agarose gels to determine the relative abundance of partially-digested DNA fragments in the desired size range. Once an optimal enzyme to total cellular DNA ratio was observed, the reaction volume was increased to obtain sufficient quantities of partially-digested total cellular DNA for use as insert DNA in the construction of the cosmid libraries. A typical scaled reaction was 400 µg of Saccharopolyspora sp. LW107129 (NRRL 30141) total cellular DNA incubated with 9 units of *Sau*3A I (Gibco BRL, Gaithersburg, MD) for 15 min at 37°C in 800 µl total volume of 1X React 4 Buffer (supplied as 10X by the manufacturer). The reaction was heated at 65°C for 20 min to inactivate the enzyme. The partially-digested genomic DNA was mixed with an equal volume of an equilibrated phenol-chloroform (50:50; v/v) solution and mixed by gentle inversion. After centrifugation at 14,000 x g for 15 min, the aqueous phase was removed and mixed with an equal volume of a chloroform-isoamyl alcohol (24:1; v/v) solution. After mixing the two phases by gentle inversion, the solution was centrifuged at 14,000 x g for 15 min. The aqueous phase was removed to a fresh tube and 0.1 volume of 3 M sodium acetate (pH 5.2) was added. Two volumes of ice-cold 100% ethanol were added and the solution was mixed by inversion. To aid in the precipitation of the DNA, the samples were placed at -70°C overnight. The precipitated DNA was pelted by centrifugation at 14,000 x g for 20 min. The DNA pellet was resuspended in 50 µl double-distilled water and stored at -20°C.

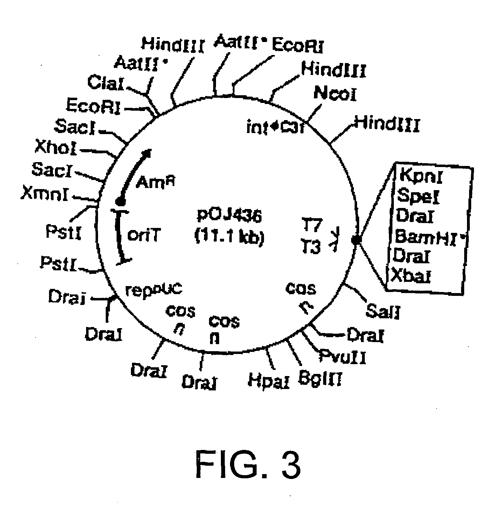

The vector used for construction of the cosmid library was pOJ436 (Figure 3) containing the apramycin resistance gene for selection. To minimize the re-ligation of cosmid vector DNA to itself, *Bam*H I-digested pOJ436 DNA was dephosphorylated by incubating the digested DNA with 20 units of shrimp alkaline phosphatase (Roche/Boehringer Mannheim, Indianapolis, IN) for 2 hrs at 37°C in 1.2 ml total volume of 1X SAP buffer (supplied as 10X by the manufacturer). *Sau*3A I-digested genomic DNA was ligated into the dephosphorylated *Bam*H I site of pOJ436 and using a 5:1 ratio of partially-digested insert to vector DNA. For this reaction, insert and vector DNAs were incubated with 20 units of T4 DNA Ligase (New England BioLabs Inc., Beverly, MA) overnight at 16°C in 1X T4 DNA Ligase Buffer (supplied as 10X by manufacturer). Ligation mixtures were packaged using Gigapack III Gold Packaging Extract (Stratagene, La Jolla, CA) and recombinant phage were titered using *Escherichia coli* strain DH5α-MRC⁺ cells (Gibco BRL), as described by the manufacturer's instructions. Aliquots (20-40 µl) of the recombinant phage and host cell culture were spread onto LB agar (10 g/l Bacto-tryptone, 10 g/l NaCl, 5 g/l Bacto-yeast extract, 15g/l Bacto agar; Difco Laboratories) containing apramycin (100mg/l; Sigma Chemical Co.) and incubated overnight at 37°C. To construct master plates of the cosmid libraries for freezer storage, single colonies were picked with sterile toothpicks and inoculated into individual wells of sterile 96-well microwell plates containing 250 µl of Terrific Broth (TB media: 12 g/l Bacto-tryptone, 24 g/l Bacto-yeast extract, 0.4% v/v glycerol, 17 mM KH₂PO₄, 72 mM K₂HPO₄) supplemented with 100mg/l apramycin and incubated without shaking overnight at 37°C. To generate copy plates from the master plates, a 96-well microplate replicator (V & P Scientific, Inc., San Diego, CA) was used to inoculate a sterile 96-well microwell plate containing 250 µl of TB media containing 100mg/l apramycin. Copy plates are incubated without shaking at 37°C overnight.

For both master and copy plates, a 7 % (v/v) dimethylsulfoxide solution was added to the plates and the cultures and mixed using a multichannel pipette. Plates were placed at -70°C for storage.

The average insert size of selected recombinant cosmids was assessed by isolating cosmid DNA using the NucleoSpin Nucleic Acid Purification Kit (CLONTECH Laboratories, Inc., Palo Alto, CA) and digesting the recovered DNA with 20 units of the restriction enzyme *Eco* RI (New England BioLabs) for 1 hr at 37°C. Restricted DNA was analyzed by electrophoresis in a 1.0% agarose gel. DNA fragments were visualized with UV light following 0.5% ethidium bromide (Sigma Chemical Co.) staining and relative size of fragments were estimated by comparison with 1 Kb DNA ladder (Gibco BRL). Insert size of the cosmid libraries constructed ranged from 20 Kb-40 Kb.

### c. Screening of cosmid libraries and identification of cosmids containing butenyl-spinosyn biosynthetic gene s

Representatives of each *E. coli* cosmid clone were inoculated using a 96-well microplate replicator (V & P Scientific, Inc.) in duplicate onto Hybond N+ (Amersham Pharmacia Biotech, Piscataway, NJ) nucleic acid binding membranes. Membranes were supported on LB agar plates supplemented with 100 mg/L apramycin and incubated overnight at 37°C. Membranes were processed according the manufacturers' protocols. Inoculated membranes were placed colony-side up onto 3MM-filter paper (Whatman, Clifton, NJ) saturated with 0.5 N NaOH for 1 minute. Filters were transferred to 3MM-filter paper saturated with 1 M Tris-HCl, pH 7.6 for 1 minute to denature the DNA. Membranes were neutralized by transfer to 3 MM-filter paper saturated with 1 M Tris-HCl, pH 7.6/1.5 M NaCl for 1 minute. Final washing was performed in a solution of 1 M Tris-HCl, pH 7.6/1.5 M NaCl where remaining colony debris was removed from the membranes. DNA was cross-linked to the membrane with 1200 µjoules using an UV Stratalinker 1800 (Stratagene).

The library of recombinant bacteria thus prepared was screened for homology to any of three radiolabelled DNA probes based upon the *spn* genes from *S. spinosa* (Baltz *et al*., 2000; Table 2). Pairs of oligonucleotides were used to amplify nucleotide regions specific for the *spn* biosynthetic gene cluster through polymerase chain reactions (PCR). Oligonucleotide primers were synthesized using a 394 DNA/RNA synthesizer (Applied Biosystems/PerkinElmer, Foster City, CA) and are listed in Table 2. PCR reactions were performed using AmpliTaq® DNA Polymerase Kit (Perkin Elmer/Roche, Branchburg, NJ) according to manufacturer's protocols. DNA fragments were amplified in a 48-sample DNA Thermal Cycler (Perkin Elmer Cetus) under the following cycle conditions: 1) 94°C, 1 min.; 55°C, 2 min.; 72°C, 3 min.; 25 cycles 2) 72°C, 10 min.; 1 cycle. Amplified products were analyzed using 0.1% agarose gel electrophoresis and bands corresponding to the appropriate size were gel-extracted utilizing Qiagen II Gel Extraction Kit (Qiagen Inc.) as outlined according to manufacturer's directions.

**Table 2**

| *S. spinosa* gene probe | Length of probe (bp) | Forward primer | Reverse primer |
|---|---|---|---|
| *spn S* | 499 | SEQ ID NO. 33 | SEQ ID NO. 34 |
| *spnF* | 536 | SEQ ID NO. 35 | SEQ ID NO. 36 |
| *spnE* (TE) | 506 | SEQ ID NO. 37 | SEQ ID NO. 38 |

Membranes were incubated at 65°C for 3 hours prior to addition of radiolabeled probe in 300 mls of pre-hybridization solution consisting of 6X SSC (52.59 g/L NaCl, 24.66 g/L sodium citrate, pH adjusted to 7.0 with 10 N NaOH), 0.1% sodium dodecyl sulfate (SDS), 10X Denhardt's Solution (50 mg/L Ficoll [Type 400, Pharmacia], 5.0 mg/L polyvinylpyrrolidone, 5.0 mg/L bovine serum albumin), 100 µg/ml denatured salmon sperm.

Concentration of DNA fragments was adjusted to 25 ng for all probes, denatured for 10 minutes in a boiling water bath and random-prime labeled with 50 µCi [α³²P]dCTP, 3000 Ci/mMol using 4 µl High Prime reaction mixture (Boehringer Mannheim) according to manufacturer's protocol. Separation of unincorporated nucleotides from radiolabeled DNA probes was performed using NucTrap Push Columns (Stratagene) and denatured for 10 minutes in a boiling water bath prior to addition to pre-hybridizing membranes. Approximately 2.0 x 10⁷ cpm were added to membranes for all DNA hybridizations. Hybridization conditions for all probes were for 16 hours in shaking water 65°C bath.

Hybridization solutions containing radiolabeled probes *spnF, spnS,* and *spnE* (TE) were decanted and each set of membranes washed under medium stringency conditions: 1) 15 min., room temperature in 300 ml 3X SSC/0.5 % SDS; 2) 30 min., 65°C shaking in 300 ml fresh 3X SSC/0.5 % SDS; 3) 30 min., room temperature in 300 ml 1X SSC/0.5 % SDS. Membranes screened with the radiolabeled probe derived from Saccharopolyspora sp. LW107129 (NRRL 30141) cosmid 9D3 sequence were washed under stringent conditions: 1) 30 min., 65°C shaking in 300 ml fresh 1X SSC/0.5 % SDS; 2) 30 min., 65°C shaking in 300 ml fresh 0.33X SSC/0.5 % SDS; 3) 30 min., 65°C shaking in 300 ml fresh 0.1X SSC/0.5 % SDS. Filters were monitored using a hand-held Geiger-Mueller counter to determine if background isotope detection was minimal. Membranes were mounted onto 3MM filter paper and covered with plastic wrap and exposed to x-ray film. Membranes were allowed to expose film for 24-72 hours at -70°C prior to development.

Putative positive cosmid clones were further characterized via restriction endonuclease digestion analysis and end-sequencing from the cosmid vector. Cosmid DNA was isolated using the NucleoSpin Nucleic Acid Purification Kit (CLONTECH Laboratories, Inc., Palo Alto, CA), and digested with 20 units of the restriction enzyme *Eco* RI (New England BioLabs) for 1 hr at 37 °C. Restricted DNA was electrophoresed in a 1.0% agarose gel. DNA fragments were visualized with UV light following 0.5% ethidium bromide staining and relative size of fragments were estimated by comparison with 1 Kb DNA ladder. Additionally, Saccharopolyspora sp. LW107129 (NRRL 30141) nucleotide sequence from the cosmid/vector junctions was obtained by fluorescent cycle sequencing according to the methods of Burgett and Rosteck (1994). Sequencing reactions consisted of 3 µl (2 µg purified cosmid DNA) template, 1 µl universal primer (4 pmole) or reverse primer (4 pmole), 8 µl Big Dye® reaction mixture, 1 µl DMSO, 7 ml H₂O under thermal cycler conditions: 96°C, 30 sec.; 50°C, 15 sec.; 60°C, 4 min.; 25 cycles with a 377 ABI Prism™ Sequencer (Applied Biosystems, Inc.).

Eight cosmid clones were identified as positively hybridizing to *S. spinosa* probes *spnS, spnF* and *spnE* (TE). Cosmid 8H3 was one of two clones that hybridized to both the *spnS* and *spnF* probes. Cosmid 9D3 was one of three clones that hybridized only to the *spnF* probe. Cosmid 10C1 was one of three clones that hybridized only to the *spnE* (TE) probe. Cosmid 9F4 was identified from the genomic library by hybidization to a radiolabeled PCR-fragment derived directly from Saccharopolyspora sp. LW107129 (NRRL 30141) sequence elucidated through nucleotide sequencing of the cosmid/vector ends from cosmid 9D3 (bases 297477-30163 in SEQ ID NO: 1). Two primers were synthesized based on the cosmid 9D3 DNA sequence (SEQ ID NO:39 & SEQ ID NO:40). A 416 bp DNA fragment was amplified from Saccharopolyspora sp. LW107129 (NRRL 30141) genomic DNA using these primes as detailed above and used for hybridization.

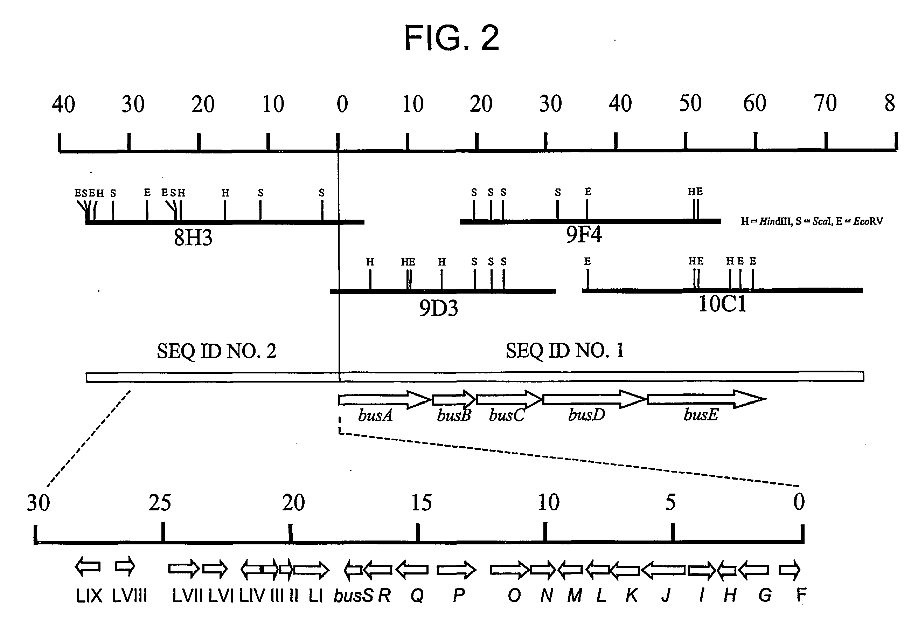

The complete sequences of cosmids 8H3, 9D3, 9F4 and 10C1 were determined by the method of fluorescent cycle sequencing of random DNA fragments cloned in phage M13 (SeqWright, Houston, TX). The inserts in cosmids 8H3 and 9D3 overlapped, The inserts in cosmids 9D3 and 9F4 overlapped, and the insert in 9F4 and 10C1 overlapped. See Fig. 2. Together, the four cosmid inserts spanned about 111 kb of unique sequence (SEQ ID NO: 1 & 2). SEQ ID NO 1 includes the start codon of busA and all DNA to the 3' of that (see Fig. 2.). SEQ ID NO. 2 begins the base before the busA start codon and includes all DNA to the 5' side of that base. The following Table 3 identifies the portions of SEQ ID NO:1 and SEQ ID NO. 2 included in each of the four inserts.

**Table 3**

| insert | Insert Size (base pairs) | bases in SEQ ID NO: 1 | bases in SEQ ID NO: 2 |
|---|---|---|---|
| cosmid 8H3 | 40,364 | 1-3,826 | 1-36,538 |
| cosmid 9D3 | 31,743 | 1-30,200 | 1-1,543 |
| cosmid 9F4 | 36,935 | 17,437-54,372 | none |
| cosmid 10C1 | 40,618 | 34,624-75,242 | none |

FIG. 2 gives a graphical representation of the relationship of the four inserts to the 110kb of sequence.

### PKS Genes

SEQ ID NO:1 includes a central region of about 60 kb with striking homology to the DNA encoding the polyketide synthases of known macrolide producers (Donadio *et al*., 1991; McDaniel & Katz., 2001; Dehoff *et al*., 1997). The butenyl-spinosyn PKS DNA region consists of 5 ORFs with in-frame stop codons at the end of ACP domains, similar to the PKS ORFs in the other macrolide-producing bacteria. The five butenyl-spinosyn PKS genes are arranged head-to-tail (see FIG. 2), without any intervening non-PKS functions such as the insertion element found between the erythromycin PKS genes AI and AII (Donadio *et al*., 1993). The PKS genes are designated *busA, busB, busC, busD,* and *busE*. The nucleotide sequence for each of the five spinosyn PKS genes, and the corresponding polypeptides, are identified in the following Table 4:

**Table 4**

| GENE | BASES IN SEQ ID NO:1 | CORRESPONDING POLYPEPTIDE |
|---|---|---|
| *busA* | 1-13,032 | SEQ ID NO: 3 |
| *busB* | 13,059-19,505 | SEQ ID NO: 4 |
| *busC* | 19,553-29,053 | SEQ ID NO: 5 |
| *busD* | 29,092-43,890 | SEQ ID NO: 6 |
| *busE* | 43,945-60,636 | SEQ ID NO: 7 |

*busA* encodes the initiator module (SEQ ID NO:1, bases 1-2931), extender module b (SEQ ID NO:1, bases 2992-8130) and extender module 1 (SEQ ID NO:1, bases 8205-13032). The nucleotide sequence and corresponding amino acid sequence for each of the functional domains within the initiator module and extender modules b and 1 are identified in the following Table 5:

**Table 5**

| busA | | |
|---|---|---|
| DOMAIN | BASES IN SEQ ID NO:1 | AMINO ACIDS IN SEQ ID NO:3 |
| KSi | 16-1269 | 6-423 |
| ATi | 1582-2559 | 528-853 |
| ACPi | 2683-2931 | 895-977 |
| KSb | 2992-4239 | 998-1413 |
| ATb | 4483-5508 | 1495-1836 |
| DHb | 5538-6084 | 1846-2028 |
| KRb | 6916-7554 | 2306-2518 |
| ACPb | 7861-8130 | 2621-2710 |
| KS | 8203-9480 | 2735-3160 |
| AT1 | 9721-10812 | 3241-3604 |
| KR1 | 11719-12258 | 3907-4086 |
| ACP1 | 12541-12786 | 4181-4262 |

*busB* encodes extender module 2 (SEQ ID NO:1, bases 13059-19505). The nucleotide sequence and corresponding amino acid sequence for each of the functional domains within extender module 2 are identified in the following Table 6:

**Table 6**

| busB | | |
|---|---|---|
| DOMAIN | BASES IN SEQ ID NO:1 | AMINO ACIDS IN SEQUENCE ID NO. 4 |
| KS2 | 13059-14321 | 1-421 |
| AT2 | 14658-15900 | 534-964 |
| DH2 | 16026-16283 | 990-1075 |
| ER2 | 17064-18101 | 1336-1681 |
| KR2 | 18111-18650 | 1685-1864 |
| ACP2 | 18915-19151 | 1953-2031 |

*busC* encodes extender module 3 (SEQ ID NO:1, bases 19553-24061) and extender module 4 (SEQ ID NO:1, bases 24128-29053). The nucleotide sequence and corresponding amino acid sequence for each of the functional domains within extender modules 3 and 4 are identified in the following Table 7:

**Table 7**

| busC | | |
|---|---|---|
| DOMAIN | BASES IN SEQ ID NO:1 | AMINO ACIDS IN SEQ ID NO:5 |
| KS3 | 19553-20815 | 1-421 |
| AT3 | 21134-22000 | 528-814 |
| KR3 | 23021-23557 | 1157-1335 |
| ACP3 | 23816-24061 | 1422-1503 |
| KS4 | 24128-25399 | 1526-1949 |
| AT4 | 25739-26731 | 2063-2393 |
| KR4 | 27641-28183 | 2697-2877 |
| ACP4 | 28457-28699 | 2969-3049 |

*busD* encodes extender module 5 (SEQ ID NO:1, bases 29092-34263), extender module 6 (SEQ ID NO:1, bases 34327-38892), and extender module 7 (SEQ ID NO:1, bases 38956-43503). The nucleotide sequence and corresponding amino acid sequence for each of the functional domains within extender modules 5, 6, and 7 is identified in the following Table 8:

**Table 8**

| busD | | |
|---|---|---|
| DOMAIN | BASES IN SEQ ID NO:1 | AMINO ACIDS IN SEQ ID NO:6 |
| KS5 | 29092-30357 | 1-422 |
| AT5 | 30700-31683 | 537-864 |
| DH5 | 31762-32319 | 891-1076 |
| KR5 | 33235-33780 | 1382-1563 |
| ACP5 | 34018-34263 | 1643-1724 |
| KS6 | 34327-35601 | 1746-2170 |
| AT6 | 35932-36924 | 2281-2611 |
| KR6 | 37831-38370 | 2914-3093 |
| ACP6 | 38647-38892 | 3186-3267 |
| KS7 | 38956-40224 | 3289-3711 |
| AT7 | 40560-41544 | 3823-4151 |
| KR7 | 42115-42999 | 4342-4636 |
| ACP7 | 43258-43503 | 4723-4804 |

*spnE* encodes extender module 8 (SEQ ID NO:1, bases 43945-49083), extender module 9 (SEQ ID NO:1, bases 49195-54366), and extender module 10 (SEQ ID NO:1, bases 54466-60707). The nucleotide sequence and corresponding amino acid sequence for each of the functional domains within extender modules 8, 9, and 10 is identified in the following Table 9:

**Table 9**

| busE | | |
|---|---|---|
| DOMAIN | BASES IN SEQ ID NO:1 | AMINO ACIDS IN SEQ ID NO:7 |
| KS8 | 43945-45216 | 1-424 |
| AT8 | 45532-46488 | 530-848 |
| DH8 | 46597-47160 | 885-1072 |
| KR8 | 48055-48606 | 1371-1554 |
| ACP8 | 48892-49083 | 1650-1728 |
| KS9 | 49195-50469 | 1751-2175 |
| AT9 | 50809-51792 | 2289-2616 |
| DH9 | 51868-52269 | 2642-2775 |
| KR9 | 53335-53889 | 3131-3315 |
| ACP9 | 54130-54366 | 3396-3474 |
| KS10 | 54466-55707 | 3508-3921 |
| AT10 | 56050-57042 | 4036-4366 |
| DH10 | 57109-57651 | 4389-4569 |
| KR10 | 58570-59106 | 4876-5054 |
| ACP10 | 59386-59631 | 5148-5229 |
| TE10 | 59776-60537 | 5278-5531 |

The boundaries and functions of the 55 domains identified in the foregoing Tables 7-11 are predicted based on similarities to the conserved amino acid sequences of the domains in other polyketide synthases, particularly the erythromycin polyketide synthase (Donadio *et al.,* 1992). Like the A83543 spinosyn PKS, the *bus* PKS has a KSQ domain at the amino terminus of the initiator module. This KSQ domain cannot function as a β-ketosynthase because it contains a glutamine residue at amino acid 172, in place of the cysteine required for β-ketosynthase activity (Siggard-Andersen, 1993). It has been reported that KSQ domains function to decarboxylate malonyl-ACP and are chain initiation factors (Bisang, *et al*., 1999). The other butenyl-spinosyn PKS domains are functional. None of them has the sequence characteristics of the inactive domains found in the erythromycin and rapamycin PKS genes (Donadio *et al.,* 1991; Aparicio *et al.,* 1996).

Although *busB-E* are comparable in size to *spnB-E, busA* is 5,244 bp larger than *spnA*. The first 4245 bp and the last 3,486 bp of *busA* have high similarity to *spnA*. However, bases 4246-9548 do not have counterparts in the *spnA* gene. This 5 kb region codes for an additional module with 5 functional domains: KSb, ATb, DHb, KRb, and ACPb. These functions together with the preceding initiation domain are responsible for the biosynthesis of the butenyl side chain, characteristic of butenyl-spinosyns relative to the A83543 spinosyns. The cloned *bus* PKS genes *busB, busC, busD* and *busE* were shown to be similar to the analogous A83543 spinosyn PKS genes *spnB, spnC, spnD* and *spnE* (table 10) (Baltz *et al.,* 2000).

**Table 10**

| butenyl-spinosyn | *bus* ORF length | Functional Domain | Best match in A83543 | *spn* ORF length bp(a.a.) | Functional Domain | ORF % Identity | ORF % Identity |
|---|---|---|---|---|---|---|---|
| Gene | bp(a.a.) | | spinosyn PKS | | | (DNA) | (a.a.) |
| *busA* | 13,032 | | *spnA* | 7,788 (2,595) | | | |
| | (4,344) | | | | | | |
| 1-4,245 | 4,245 (1,415) | KSQ-KSb | 21,111-25,214 | 4,245 (1,415) | KSQ-KS1 | 92 % | 91.2 % |
| 4,246-9,548 | 5,301 (1,767) | ATb-KS1 | none* | NA | | | |
| 9,549-13,032 | 3,486 (1,162) | AT1-ACP1 | 26,407-28,896 | 3,486 (1,162) | AT1-ACP1 | 91% | 87.6 % |
| *busB* | 6,450 (2,149) | KS2-ACP2 | *spnB* | 6,459 (2,152) | KS2-ACP2 | 93% | 93.1 % |
| *busC* | 9,546 (3,167) | KS3-ACP4 | *spnC* | 9,513(3,170) | KS3-ACP4 | 94% | 93.5% |
| *busD* | 14,805 | KS5-ACP7 | *spnD* | 14,787 (4,928) | KS5-ACP7 | 94% | 93.6 % |
| | (4,935) | | | | | | |
| *busE* | 16,692 | KS8-ACP10 | *spnE* | 16,767 (5,588) | KS8-ACP10 | 94% | 90.6 % |
| | (5,564) | | | | | | |

| | | | | | | | |
|---|---|---|---|---|---|---|---|
| * Similarity to *S. spinosa* PKS genes was in the same range as similarity to other like domains of the *bus & spn* PKS genes. | | | | | | | |

The proteins, which perform similar reactions in the biosynthesis of spinosyns, share 87-93 % amino acid identity and the genes range from 93-94 % DNA sequence identity. It should be noted that the *spn* PKS enzymes SpnB-E and the similar *bus* PKS enzymes BusB-E must maintain distinct substrate specificity since, although the reactions performed by the enzymes are identical, the substrate polyketides are different. In addition, aggregation of the 5 PKS enzymes into a functional PKS requires specific protein-protein interactions. The residues involved in this inter-subunit molecular recognition are unknown and may not be conserved between *S. spinosa* and Saccharopolyspora sp. LW107129 (NRRL 30141) .

### Genes Adjacent to the PKS Responsible for Additional Modifications

In the DNA upstream of the PKS genes (cloned in cosmid 8H3) there were 22 open reading frames (ORFs), each consisting of at least 100 codons, beginning with ATG or GTG and ending with TAA, TAG or TGA, and having the codon bias expected of protein-coding regions in an organism whose DNA contains a high percentage of guanine and cytosine residues (Bibb *et al.,* 1984). These 22 ORFs represented graphically in FIG. 2. Based on evidence that will be discussed hereinafter, 14 of the ORFs have been designated as butenyl-spinosyn biosynthetic genes, namely: *busF*, *busG, busH, busI*, *busJ*, *busK*, *busL, busM*, *busN*, *busO*, *busP*, *busQ*, *busR*, and *busS* (labeled F through S in FIG. 2). In the following Table 11, the DNA sequence and the amino acid sequence for the corresponding polypeptide are identified for each of these genes, as well as for ORFs found immediately downstream of *spnS* (ORF LI, ORF LII, ORF LIII, ORF LIV, ORF LVI, ORF LVII, ORF LVIII and ORF LIX in cosmid 8H3). Also identified in Table 11 are the nucleotide sequences for ORF RI, ORF RII and ORFRIII downstream of the PKS genes (in cosmid 2C10), and the amino acid sequences corresponding to them.

**Table 11**

| Gene | bases in Sequence ID NO: 2 | polypeptide |
|---|---|---|
| *busF* | 114-938 (C) | SEQ ID NO: 8 |
| *busG* | 1389-2558 | SEQ ID NO: 9 |
| *busH* | 2601-3350 | SEQ ID NO: 10 |
| *busI* | 3362-4546 (C) | SEQ ID NO: 11 |
| *busJ* | 4684-6300 | SEQ ID NO: 12 |
| *busK* | 6317-7507 | SEQ ID NO: 13 |
| *busL* | 7555-8403 | SEQ ID NO: 14 |
| *busM* | 8640-9569 | SEQ ID NO: 15 |
| *busN* | 9671-10666 (C) | SEQ ID NO: 16 |
| *busO* | 10678-12135 (C) | SEQ ID NO: 17 |
| *busP* | 12867-14177 (C) | SEQ ID NO: 18 |
| *busQ* | 14627-15967 | SEQ ID NO: 19 |
| *busR* | 16008-17141 | SEQ ID NO: 20 |
| *busS* | 17168-17914 | SEQ ID NO: 21 |
| ORF LI | 18523-19932 (C) | SEQ ID NO: 22 |
| ORF LII | 19982-20488 (C) | SEQ ID NO: 23 |
| ORF LIII | 20539-21033(C) | SEQ ID NO: 24 |
| ORF LIV | 21179-21922 | SEQ ID NO: 25 |
| ORF LVI | 22674-23453 (C) | SEQ ID NO: 26 |
| ORF LVII | 23690-24886 (C) | SEQ ID NO: 27 |
| ORF LVIII | 26180-26923 (C) | SEQ ID NO: 28 |
| ORF LIX | 27646-28473 | SEQ ID NO: 29 |
| | | |
| Gene | bases in Sequence ID NO:1 | Polypeptide |
| ORF RI | 62090-63937 | SEQ ID NO: 30 |
| ORF RII | 65229-66602 (C) | SEQ ID NO: 31 |
| ORF RIII | 68762-69676 (C) | SEQ ID NO: 32 |

| | | |
|---|---|---|
| (C) indicates complementary strand is given in the sequence listing | | |

To assign functions to the polypeptides identified in Table 11, four lines of evidence were utilized: similarity to sequences of known function, similarity to A83543 spinosyn biosynthetic genes, results of targeted gene disruption experiments, and results of bioconversion experiments.

The amino acid sequences of the predicted polypeptides were compared to sequences deposited in the databases at the National Center for Biotechnology Information (NCBI, Washington, DC), using the BLAST algorithm to determine how well they are related to known proteins. The BLAST searches of the NCBI databases were also repeated periodically to obtain new insights from additional homologies. Table 12 gives significant matches from a basic BLAST search on February 18, 2001:

**Table 12**

| Gene | Significant Protein Match | GenBank Accession | BLAST Score* | Reported function |
|---|---|---|---|---|
| *busF* | C-5-O-methyltransferase *aveD* (*Streptomyces avermitilis*) | T44579 | 156 | C-methylation |
| *busG* | Glycosyl transferase *urdGT1b* (*Streptomyces fradiae*) | AF164961 | 205 | glycosyl transfer |
| *busH* | 3"'macarocin O-methyltransferase *tylF* (*Streptomyces fradiae*) | AF147703 | 297 | sugar methylation |
| *busI* | 2"'macarocin O-methyltransferase *tylE* (*Streptomyces fradiae*) | AAD12164 | 287 | sugar methylation |
| *busJ* | Hexose oxidase (*Chondrus crispus*) | U89770 | 148 | Hexose oxidase |
| *busK* | 2"'macarocin O-methyltransferase *tylE* (*Streptomyces fradiae*) | AAD12164 | 310 | sugar methylation |
| *busL* | *mitM* methyltransferase (*Streptomyces lavenduale*) | AF127374 | 120 | C-methylation |
| *busM* | Lip4 secretory lipase (*Candida albicans*) | B70543 | 94 | lipase |
| *busN* | 3-ketoreductase *aknQ* (*Streptomyces galilaeus)* | AF264025 | 284 | hexose 3-ketoreductase |
| *busO* | *urdS* (*Streptomyces fradiae*) | AF269227 | 404 | hexose 2,3-dehydration |
| *busP* | *dnrH* glycosyl transferase (*Streptomyces puecetius*) | U77891 | 290 | glycosyl transfer |
| *busQ* | *urdQ* 3,4-dehydratase (*Streptomyces fradiae*) | AF269227 | 480 | hexose dehydratase |
| *busR* | *spsC* spore coat polysaccharide binding protein (*Bacillus subtillus*) | P39623 | 185 | hexose transamination |
| *busS* | *desVI* N,N-dimethyl transferase (*Streptomyces venezuelae*) | AF079762 | 240 | amino methylation |
| ORF LI | ngt N-glycosyltransferase (*Saccharothrix aerocologenies*) | AB023593 | 221 | glycosyltransfer |
| *ORF LIV* | *urdR* hexose-4-ketoreductase (*Streptomyces fradiae*) | AF080235 | 243 | hexose ketoreduction |
| *ORF LVI* | *fkbM*, FK506 O-methyltransferase | U65940 | 100 | methyltransfer |
| *ORF LVII* | *oleP*, P450 monooxygenase (*Streptomyces antibioticus*) | L37200 | 387 | monooxygenase |
| *ORFLVIII* | Transposase (*Mycobacterium avium*) | AF107207 | 180 | transposition |
| *ORF LLX* | *mmcR* (Streptomyces lavendulae) | AF127374 | 124 | Methyl transfer |
| *ORF RI* | resolvase-like protein (*Acidithiobacillus ferrooxidans*) | U73041 | 97 | transposition |
| *ORF RII* | hypothetical protein *yvmC (Bacillus subtillus*) | AF017113 | 120 | |
| *ORF RII* | alcohol dehydrogenase [*Streptomyces coelicolor* A3(2)] | AL133236 | 155 | alcohol dehydrogenase |

| | | | | |
|---|---|---|---|---|
| * Greater similarity is associated with higher BLAST scores (Altschul *et al*., 1990). | | | | |

The *bus* open reading frames were compared directly to the sequence of the A83543 spinosyn biosynthetic genes (Accession number AY007564). The high degree of similarity in both the DNA and protein sequence indicated that the genes performed similar functions in biosynthesis of spinosyns. Table 13 gives the similarity comparisons between the *bus* and *spn* genes

**Table 13**

| butenyl-spinosyn Gene | bus ORF length bp (a.a.) | A83543 spinosyn gene | spn ORF length bp (a.a.) | BLAST score | ORF % Identity (DNA) | ORF % Identity (a.a.) | Function reported in GenBank |
|---|---|---|---|---|---|---|---|
| *busF* | 828(275) | *spnF* | 828 (275) | 1247 | 94% | 91% | C-methylation |
| *busG* | 1173 (390) | *spnG* | 1173 (390) | 1844 | 95% | 90 % | sugar addition |
| *busH* | 753 (250) | *spnH* | 754 (250) | 1328 | 97% | 97% | sugar methylation |
| *busI* | 1188 (395) | *spnI* | 1188 (395) | 1966 | 96 % | 92 % | unknown |
| *busJ* | 1620 (539) | *spnJ* | 1620 (539) | 2587 | 95 % | 83 % | oxido-reduction |
| *busK* | 1194 (397) | *spnK* | 1194 (397) | 2163 | 96% | 88% | unknown |
| *busL* | 852 (283) | *spnL* | 852 (283) | 2274 | 94% | 94 % | C-methylation |
| *busM* | 933(310) | *spnM* | 963 (320) | 1909 | 95 % | 96% | unknown |
| *busN* | 999(332) | *spnN* | 999 (332) | 1772 | 96 % | 91 % | unknown |
| *busO* | 1461 (486) | *spnO* | 1461 (486) | 2319 | 95% | 92 % | deoxysugar synthesis |
| *busP* | 1314 (437) | *spnP* | 1368 (455) | 2004 | 94% | 89% | sugar addition |
| *busQ* | 1344 (447) | *spnQ* | 1389 (462) | 2355 | 94% | 81 % | dideoxysugar synthesis |
| *busR* | 1137 (378) | *spnR* | 1158 (385) | 1852 | 95% | 89 % | sugar transamination |
| *busS* | 750 (249) | *spnS* | 750 (249) | 1255 | 96 % | 93 % | aminosugar methylation |

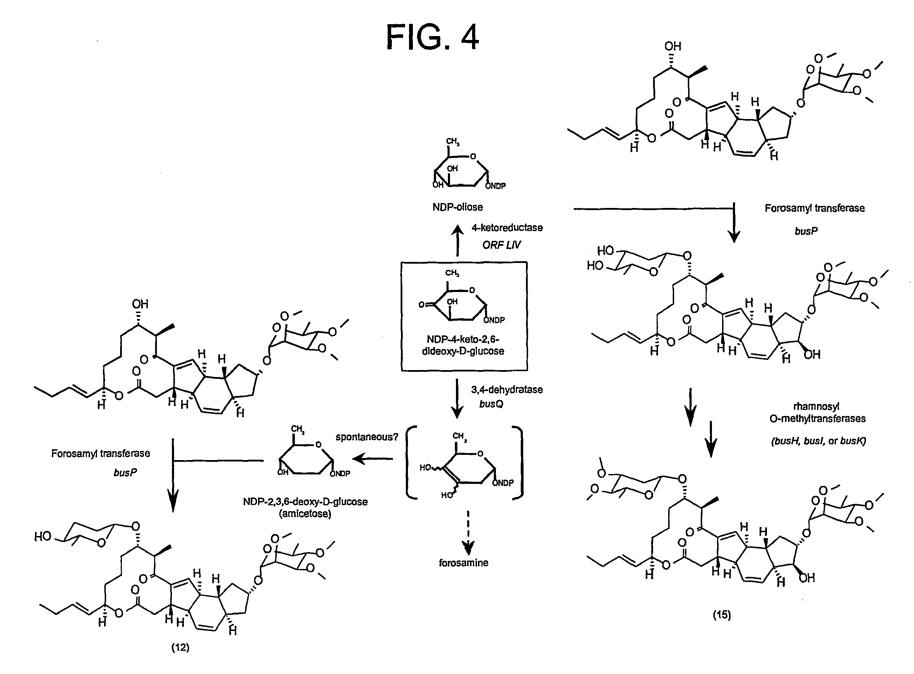

In spite of the high degree of DNA and amino acid similarity between some *bus & spn* genes, it should be noted that some of the *bus* gene products catalyze markedly different reactions in the biosynthesis of butenyl-spinosyn relative to A83543 spinosyns. These differences are manifested in the distinct butenyl-spinosyn compounds that have been isolated from Saccharopolyspora sp. LW107129 (NRRL 30141). All natural A83543 spinosyns disclosed are substituted at C-17 with forosamine or a specific forosamine isomer (Kirst, *et al*., 1992). Butenyl-spinosyns, on the other hand, are also substituted at C-17 with a wider range of forosamine isomers, as well as neutral sugars like amicetose, O-methyl-glucose and O-methyloleandrose. This C-17 glycosylation diversity relative to A83543 spinosyns requires biosynthetic enzymes to make the sugars and a glycosyltransferase capable of catalyzing these glycosylations. These sugars might be synthesized by specific synthase genes located near the *bus* genes or elsewhere in the chromosome, or they may be synthesized by alternate substrate specificity of the listed butenyl-spinosyn biosynthetic genes. Amicetose could be produced by genes outside of the *bus* gene cluster or it may be an intermediate in the biosynthesis of forosamine (FIG. 4). Methyloleandrose could be produced as a byproduct of forosamine biosynthesis and the rhamnose O-methyltransferases (*busH, busI* & *busK*). This sugar could be synthesized from NDP-4-keto-2,6-deoxy-D-glucose is an intermediate in the biosynthesis of forosamine. Therefore, ketoreduction and O-methylation of this precursor by the disclosed genes and other Saccharopolyspora sp. LW107129 (NRRL 30141) genes could lead to the biosynthesis of spinosyn derivatives containing methyloleandrose (FIG. 4).

In addition, nine genes listed in table 13 directly interact with the butenyl-spinosyn aglycone or PSA (*busF*, *busG*, *busH*, *busI*, *busJ*, *busK*, *busL*, *busM*, and *busP*). The aglycone and PSA substrate for these genes is distinct from the A83543 spinosyn aglycone and PSA. Therefore, these genes have distinct substrate specificity relative to their *spn* counterpart listed in Table 13.

Several butenyl-spinosyn analogs produced by Saccharopolyspora sp. LW107129 (NRRL 30141) are hydroxylated at C-8 or C-24 (Table 2). Macrolides can be hydroxylated post synthesis by P-450 monooxygenases as in hydroxylation at C-6 in erythromycin biosynthesis (Weber & McAlpine, 1992). ORF LVII is highly similar to P-450 monooxygenases and it or a monooxygenase encoded elsewhere on the Saccharopolyspora sp. LW107129 (NRRL 30141) chromosome may be responsible for the hydroxylations at C-8 or C-24 of butenyl-spinosyns. Alternatively, hydroxylated precursors such as glycolate or glycerol can be incorporated during polyketide synthesis as in leucomycin (Omura *et al*., 1983). It has been reported that the AT domain specific for addition of glycolate in the niddamycin producer (nid AT6) is similar to methyl-malonyl-CoA specific AT domains of the erythromycin and rapamycin PKS genes (Katz *et al*., 2000). PKS module 7 is responsible for addition of carbons 8 and 9 of the butenyl-spinosyn polyketide, however, the bus AT7 domain does not have the same methyl-malonyl-CoA specific sequences as *nid* AT6. There are unique sequences in bus AT7 relative to other AT domains and *nid* AT6 which could be responsible for glycolate specificity. The butenyl-spinosyn biosynthetic genes responsible for these modifications are unique relative to the A83543 spinosyns since no such hydroxylated spinosyns are produced by *S. spinosa*.

In addition, the specificity of rhamnose methylation is altered in *Saccharopolyspora* sp. LW107129 (NRRL 30141) relative to *S*. *spinosa*. Mutants of *S. spinosa* which exhibited altered methylation of the rhamnose on A83543 spinosyn as disclosed in US Patents 5,202,242 and 5,840,861, typically produced mono-desmethylated rhamnose derivatives of A83543 spinosyns. Di-desmethyl rhamnose derivatives of A83543 spinosyns were only detected in the presence of methyltransferase inhibitors like sinefungin. Mutants of *Saccharopolyspora* sp. LW107129 (NRRL 30141) with altered methylation of rhamnose (Hahn *et al*., 2001), produced di- and tri-desmethyl rhamnose derivatives of butenyl-spinosyns in high amounts, in the absence of methyltransferase inhibitors.

For complementation studies, cosmids containing Saccharopolyspora sp. LW107129 (NRRL 30141) *bus* DNA were conjugated into Saccharopolyspora sp. LW107129 (NRRL 30141) mutant strains in which butenyl-spinosyn synthesis was altered. (Details are given in the following Example 4.) Transconjugants were then tested for their ability to convert the products of blocked mutants into other spinosyns. The mutant used was 30141.8 which produced 3'-O-desmethylrhamnose-butenyl-spinosyn (3-ODM) and related factors. The 30141.8/8H3 transconjugants produced butenyl-spinosyns instead of 3-ODM, so the genes responsible for methylation at the 3' position of rhamnose must be present on cosmid 8H3.

In targeted gene disruptions, internal fragments are generated by PCR amplification from the cosmid DNAs, and cloned into a plasmid. The resulting plasmids were then conjugated into Saccharopolyspora sp. LW107129 (NRRL 30141, and apramycin-resistant transconjugants were isolated and fermented. The basis of disruption experiments is that when a plasmid bearing an internal gene fragment is integrated, two incomplete copies of the biosynthetic gene result, thereby eliminating the enzymatic function. Fermentation products are analyzed to determine which butenyl-spinosyns accumulate. Disruption of the *busO* gene leads to the accumulation of butenyl-spinosyn PSA indicating that *busO* is required for synthesis or addition of forosamine (See Example 5). Compounds containing sugars at C-17 which are not synthesized using the forosamine biosynthetic genes can also be accumulated in *busO* mutants.

The conclusions drawn from BLAST searches, the gene disruption experiments, and the bioconversion studies will now be discussed in greater detail on a gene by gene basis.

The 14 genes upstream of the PKS were determined to be involved in butenyl-spinosyn biosynthesis because of their high similarity to the *spnF-S* genes of *S. spinosa* (Table 13) and because BLAST searches showed that these genes had striking similarity to enzymes known to code for functions needed for the biosynthesis of butenyl-spinosyns.

### busF, busJ, busL, busM

The genes *busF, busJ*, *busL,* and *busM* show high similarity to *spnF*, *spnJ*, *spnL* and *spnM*. These A83543 spinosyn genes were reported to be involved in generation of the aglycone from the putative monocyclic lactone product of the PKS genes. The *busF* gene product has 91 % amino acid identity to *spnF*, likewise the *busL* gene product has 94 % amino acid identity to *spnL*. Both *spnF & spnL* gene products were reported to be methyltransferases and all 4 proteins have high similarity to enzymes from *Streptomyces* which are known to be involved in carbon-carbon bond formation, The *busJ* protein had 83 % amino acid identity to *spnJ*, reported to be an oxidoreductase. Both *busJ* & *spnJ* are highly similar to *dnrW*, which is known to be involved in C-C bond formation in the biosynthesis of daunorubicin. The *busM* gene product was 96 % identical to *spnM*. The gene products of both *busM & spnM* are highly similar to a new class of secreted lipases from *Candida albicans.* The roles of *busF*, and *busL* as methyltransferases, *busJ* as an oxidase and *busM as* a lipase are consistent with the reported roles of the *spnF*, *spnL, spnJ* and *spnM* genes in the formation of carbon-carbon bridges.

### busG, busH, busI, busK

The *busG*, *busH*, *busI*, and *busK* had high similarity to the *spnG*, *spnH*, *spnI and spnK* genes of *S. spinosa.* These genes were reported to be involved in rhamnose addition to the A83543 spinosyn aglycone and subsequent methylation. The *busG* gene has 90% similarity to *spnG* and was highly similar to several genes involved in sugar addition to polyketide derived antibiotics (table 11). The *busH*, *busI*, and *busK* gene products showed high amino acid similarity to the *spnH* (97 %), *spnI* (92 %), *and spnK* (88 %) gene products, respectively which were reported to be involved in methylation of rhamnose in spinosyn biosynthesis. All three genes had high amino acid similarity to the *tylE (busI & busK*) and *tylF* (*busH*) genes from *Streptomyces fradiae* which have been shown experimentally to be macarocin-O-methyltransferases (Bate & Cundliffe, 1999).

### busN, busO, busP, busQ, busR, busS

The *spnN*, *spnO, busP, busQ, busR,* and *busS* had high similarity to the *spnN, spnO, spnP, spnQ, spnR,* and *spnS* genes of S. *spinosa* (Table 12). These genes were reported to be involved in the biosynthesis or addition of the forosamine sugar. The similarity of *busP* to other glycosyl transferases (Table 11) indicates that it encodes the butenyl-spinosyn forosamyl transferase. The high degree of similarity between *busO* and the *urdS* 2,3 dehydratase (Table 11; Hoffmeister *et al.,* 2000) indicates that it is involved in the 2'-deoxygenation step of forosamine synthesis. The similarity between the *busQ* gene product and the *urdQ* 3,4-dehydratase (*S*. *fradiae*; Hoffmeister *et al.,* 2000), indicates that it is involved in the 3'-dehydration step of forosamine synthesis. *busR* had up to 40% identity to a group of proteins proposed to function as deoxysugar transaminases (Thorson *et al.,* 1993), indicating that *busR* is involved in the 4'-amination step of forosamine synthesis. Finally *busS* was highly similar to a group of amino methylases indicating that *busS* is involved in methylation of the 4' amino group of forosamine. Therefore, the *busN, busO, busP, busQ, busR,* and *busS* are involved in production of the forosamine moiety of butenyl-spinosyns.

Thus 19 genes from *Saccharopolyspora* sp. LW107129 (NRRL 30141) can be assigned roles in butenyl-spinosyn biosynthesis: 5 PKS genes to produce a macrocyclic lactone, 4 genes to modify this to the aglycone, 4 genes to add and methylate the rhamnose, and 6 genes to synthesize and add forosamine. The hypothetical biosynthetic pathway is summarized in FIGS. 1A and 1B.

### Utility

There are many uses for the cloned *Saccharopolyspora* sp. butenyl-spinosyn DNA. The cloned genes can be used to improve yields of butenyl-spinosyns and to produce new butenyl-spinosyns. Improved yields can be obtained by integrating into the genome of a particular butenyl-spinosyn producing strain, a duplicate copy of one or more butenyl-spinosyn biosynthetic genes. In the extreme case where the biosynthetic pathway is blocked in a particular mutant strain due to lack of a required enzyme, production of the desired spinosyns can be restored by integrating a copy of the required gene.

Novel compounds can be produced using fragments of the cloned DNA to disrupt steps in the biosynthesis ofbutenyl-spinosyns. Such disruption may lead to the accumulation of precursors or "shunt" products (the naturally-processed derivatives of precursors). Modified spinosyns produced by disrupting genes may be insect control agents themselves, or serve as substrates for further chemical modification, creating new semisynthetic spinosyns with unique properties and spectra of activity. A disruption of the *busO* gene results in the accumulation of butenyl-spinosyn PSA. Butenyl-spinosyn PSA is useful as a starting material for the synthesis of spinosyn analogs containing novel groups at C-17.

Novel butenyl-spinosyns can also be produced by transfer of one or more of the cloned *bus* genes, or a part thereof, into a heterologous host. These genes may provide enzymatic functions not present in the recipient host. Such genes might provide alternate sugars, modify existing sugars or aglycone carbons, allow alternate sugars to be attached to an aglycone or alter the base structure of the aglycone itself Compounds produced by transfer of the cloned *bus* genes into a heterologous host may be insect control agents themselves, or serve as substrates for further chemical modification, creating new semisynthetic spinosyns with unique properties and spectra of activity. Saccharopolyspora sp. LW107129 (NRRL 30141) DNA from cosmids 8H3 and 9D3 can be transferred into S. *spinosa,* producer of A83543 spinosyns, and the transconjugants produce novel spinosyns.

Novel butenyl-spinosyns can also be produced by mutagenesis of the cloned genes, and substitution of the mutated genes for their unmutated counterparts in a butenyl-spinosyn-producing organism. Mutagenesis may involve, for example: 1) deletion or inactivation of a KR, DH or ER domain so that one or more of these functions is blocked and the strain produces a spinosyn having a lactone nucleus with a double bond, a hydroxyl group, or a keto group that is not present in the nucleus of spinosyn A (*see* Donadio *et al*., 1993); 2) replacement of an AT domain so that a different carboxylic acid is incorporated in the lactone nucleus (*see* Ruan *et al.,* 1997); 3) addition of a KR, DH, or ER domain to an existing PKS module so that the strain produces a spinosyn having a lactone nucleus with a saturated bond, hydroxyl group, or double bond that is not present in the nucleus of spinosyn A (MacDaniel & Katz, 2001); or 4) addition or subtraction of a complete PKS module so that the cyclic lactone nucleus has a greater or lesser number of carbon atoms.

The DNA from the butenyl-spinosyn gene cluster region can be used as a hybridization probe to identify homologous sequences. Thus, the DNA cloned here could be used to locate additional plasmids from the *Saccharopolyspora* sp. LW107129 (NRRL 30141) gene libraries which overlap the region described here but also contain previously uncloned DNA from adjacent regions in the genome of *Saccharopolyspora* sp. LW107129 (NRRL 30141). Also, comparisons of the *Saccharopolyspora* sp. LW107129 (NRRL 30141 *bus* genes with the *S. spinosa spn* genes leads to the identification of regions of conserved sequence which are distinct from non-spinosyn producing biosynthetic genes such as the biosynthetic genes for erythromycin, rapamycin, tylosin and others. These spinosyn-specific gene probes as well as all DNA from the region cloned here may be used to identify non-identical but similar sequences in other organisms. Hybridization probes are normally at least about 20 bases long and are labeled to permit detection.

The modified strains provided by the invention may be cultivated to provide spinosyns using conventional protocols such as those disclosed in U. S. Patent No. 5,362,634. The above examples are non-limiting and should not be construed as limitations of the invention.

### EXAMPLE 2

### LC/MS Method for Analysis of Fermentation Broth for Butenyl-Spinosyn Metabolites

The following method utilizes HPLCseparation with electrospray (ESI) mass spectrometry to monitor fermentation for the production of Formula (1) and other components. Such a system was also used for determining molecular weights of the purified factors, by deduction from the electrospray adduct ions. These data are summarized in Table 15.

Add a volume of denatured ethanol equal to that of fermentation broth. Shake the mixture for 1 hour, then centrifuge and filtere (0.22 µm pore size) to remove the bulk cell debris. Microfuge a 1-mL aliquot, then analyze the clarified extract by the following LC-MS system:

HPLC system: Column stationary phase: 250 x 4.6-mm column, base-deactiviated silica gel, 5 µm C8 (Hypersil-C8-BDS). Mobile phase: 10 mM ammonium acetate-methanol-acetonitrile linear gradient summarized below:

**TABLE 14**

| Time (mins) | Percent solvent A | Percent solvent B |
|---|---|---|
| 0 | 100 | 0 |
| 20 | 0 | 100 |
| 25 | 0 | 100 |
| 30 | 100 | 0 |
| 35 | 100 | 0 |

| | | |
|---|---|---|
| where solvent A is 10 mM ammonium acetate and solvent B is methanol-acetonitrile (1:1); Flow rate: 1 mL/min; split after UV detector such that MS:waste ratio is approx. 5:95; Detection: positive ESI acquired in low and high cone voltage modes Characteristic LC retention times and mass spectrometry ions as summarized in Table 15. | | |

**TABLE 15**

| Compound No. (from Table 1) | LC retention time | m/z for [M+H]^{+ a} | m/z for secondary ion^{b} |
|---|---|---|---|
| 1 | 23.8 | 758.4 | 142.0 (forosamine) |
| 4 | 22.9 | 744.4 | 142.0 |
| 5 | 24.3 | 772.4 | 142.0 |
| 6 | 22.1 | 774.4 | 142.0 |
| 9 | 21.4 | 810.4 [M+NH₄] | 189.0 (tri-O-methyl-rhamnose) |
| 13 | 22.0 | 617.3 | 189.0 |

| | | | |
|---|---|---|---|
| ^{a} m/z for parent ion observed under +ESI mode, low cone voltage ^{b} m/z for most abundant fragment or adduct ion observed under +ESI, high cone voltage mode | | | |

### EXAMPLE 3

### Preparation Of Butenyl-Spinosyn Metabolites Through Fermentation

Metabolites of Formula (1) are produced by cultivation of the desired strain of *Saccharopolyspora* chosen from one of the following strains NRRL 30141, NRRL 30421, or derivatives thereof in fermentation medium as described below. A 1.8-mL frozen vegetative culture was thawed, inoculated into 25 mL vegetative media in a 125-mL Erlenmeyer baffled flask and grown at 30° C shaking at 150 rpm for 72-96 hours.

**TABLE 16**

| Vegetative Medium | |
|---|---|
| Ingredient | Amount (g) |
| Dextrose | 9.0 |
| Trypticase Soy Broth | 30.0 |
| Yeast Extract | 3.0 |
| Magnesium Sulfate. 7 H₂O | 2.0 |
| De-ionized water | 1000.0 |

### Shake Flask Fermentation

Twelve milliliters of mature first stage seed was used to inoculate 50-mL fermentation medium in a 500-mL baffled Erlenmeyer fermentation flask.

**TABLE 17**

| Fermentation Medium (per liter of water) | |
|---|---|
| Ingredient | Amount (g) |
| Dextrose | 80.0 |
| Cottonseed Flour | 32.0 |
| Soybean Flour | 8.0 |
| Corn Steep Powder | 8.0 |
| Calcium Carbonate | 5.0 |
| Yeast Extract | 2.0 |

Fermentation was maintained at 30° C, 200 rpm (50 mm stroke) for 7-12 days. Mature fermentation beer can be extracted with a suitable solvent and the metabolites recovered by chromatographic separation, as disclosed in Example 1.

### EXAMPLE 4

### Complementation of Rhamnose Methylation Defect in Strain NRRL 30421 by Cosmid 8H3

Strain NRRL 30421 is a mutant of *Saccharopolyspora* sp. NRRL 30141 which is unable to fully methylate the rhamnose on butenyl-spinosyns. Strain NRRL 30421 accumulates compound 4 and other butenyl-spinosyns which lack *O*-methylation at the 3' position of the rhamnose (3'-ODM). This methylation defect is presumed to be the result of a mutation in one of the *O*-methyltransferases encoded by the *busH, busI* or *busK* genes. All of these genes are present in cosmid 8H3 (Figure 2)

Cosmid 8H3 (Figure 3) was transferred from *Escherichia coli* ATCC 47055 into strain NRRL 30421 by conjugal transfer (Matsushima *et al.,* (1994)). Two independent isolates transformed with cosmid 8H3 were fermented as in Example 2 and analyzed for production of compound 1 and compound 4 as exemplified in Example 1.

**TABLE 18**

| Strain (Genotype) | compound 4 µg/ml | compound 1 µg/ml | ratio of compounds 1:4 |
|---|---|---|---|
| NRRL 30421 (3'-ODM*) | 1.0 | 0.7 | 0.7 |
| NRRL 30421 (3'- | 0.5 | 8.9 | 17.8 |
| ODM*)/8H3-42 | | | |
| NRRL 30421 (3'- ODM*)/8H3-45 | 0.1 | 3.0 | 30.0 |
| NRRL 30141 | 0.4 | 9.7 | 24.3 |

| | | | |
|---|---|---|---|
| * = mutation preventing methylation of rhamnose at 3' position | | | |

While NRRL 30421 produced predominantly compound 4, strains of NRRL 30421 containing cosmid 8H3 produced mostly compound 1 [Table 18]. The production of compounds 1 and 4 in NRRL 30421 containing cosmid 8H3 is similar to the non-mutant culture NRRL 30141 (Table 18). It has therefore been demonstrated that transformation with cosmid 8H3 is able to overcome a methylation defect in strain NRRL 30421 to restore enhanced production of compound 1.

### EXAMPLE 5

### Accumulationation of Butenyl-Spinosyn Precursor and Shunt Product Caused by Disruption of busO

The *busO* gene was inactivated by integration of a cloned internal fragment of the *busO* gene. A pair of oligonucleotides (the first corresponding to bases 11882-11861 in SEQ ID NO: 2 and the second corresponding to bases 10970-10993 in SEQ ID NO: 2) were used to amplify a 912 bp region internal to the 1,457 bp *busO* gene corresponding to bases 10970-11882 in SEQ ID NO: 2. Transformation of Saccharopolyspora sp. LW107129 (NRRL 30141) with a plasmid containing the fragment would result in partial duplication of the *busO* gene, to yield two truncated copies of the gene flanking the plasmid and antibiotic resistance gene.

The 912 bp internal *busO* PCR fragment was generated with primers SEQ ID NO: 33 & 34 using FailSafe™PCR (Epicenter) and cloned into pCRII according to the manufacturer's instructions (Invitrogen). The resulting plasmid was digested with *Eco*RI and the *busO* fragment was cloned into the *Eco*RI site of pOJ260 (Figure 3). The resultant plasmid was conjugated from *Escherichia coli* ATCC 47055 into a derivative of *Saccharopolyspora* sp. NRRL 30121 by conjugal transfer (Matsushima *et al.,* (1994)). Six independent apramycin resistant exconjugants were fermented as in Example 2 and analyzed for production of compound 1 and other spinosyn derivatives as in Example 1.

The parental strain, NRRL 30141 produced high levels of compound 1 and low levels of the pseudoaglycone (PSA; compound 13). It also produced a small amount of compound 9 [Table 19]. Compound 1 could not be detected in any of the six *busO* mutants, indicating that *busO* is required for complete butenyl spinosyn biosynthesis. In addition, levels of PSA were increased in all six *busO* mutants, as would be predicted from a deficiency in forosamine supply. The levels of compound 9 which has a sugar other than forosamine at C17, also increased in the *busO* mutants.

**TABLE 19**

| | | | |
|---|---|---|---|
| | | | |

| Strain (Genotype) | compound 1* | compound 13 | compound 9 |
|---|---|---|---|
| NRRL 30141 | 366.3 | 1.0 | 0.4 |
| NRRL 30141 | nd | 13.8 | 1.7 |
| *busO65* | | | |
| NRRL 30141 | nd | 12.3 | 3.7 |
| *busO67* | | | |
| NRRL 30141 | nd | 6.7 | 3.8 |
| *busO68* | | | |
| NRRL 30141 | nd | 9.3 | 1.3 |
| *busO70* | | | |
| NRRL 30141 | nd | 12.3 | 2.4 |
| *busO71* | | | |
| NRRL 30141 | nd | 5.4 | 1.6 |
| *busO72* | | | |

| | | | |
|---|---|---|---|
| * amounts reported are relative to compound 13 in NRRL 30141; nd = not detected | | | |

### References

1. Altschul, S. F., W. Gish, W. Miller, E. W. Myers, and D. J. Lipman (1990). Basic local alignment search tool. J. Molec. Biol. 215:403-10.
2. Aparicio, J. F., I. Molnar, T. Schwecke, A. Konig, S. F. Haydock, L. E. Khaw, J. Staunton & J. F. Leadlay (1996). "Organization of the biosynthetic gene cluster for rapamycin in Streptomyces hygroscopicus: analysis of the enzymatic domains in the modular polyketide synthase," Gene 169: 9-16.
3. Ausebel F., R. Brent, R. Kingston, D. Moore, J. Smith, J. Seidman, and K. Struhl, eds. (1987). Current Protocols in Molecular Biology. (John Wiley and Sons, New York).
4. Baltz, R. H., M. C. Broughton, K. P. Crawford, K. Madduri, D. J. Merlo, P. J. Treadway, J. R. Turner and C. Waldron (2000) Biosynthetic Genes for Spinosyn Insecticide Production. US Patent 6,143,526.
5. Bate, N., & E. Cundeliffe (1999) The mycinose-biosynthetic genes of Streptomyces fradiae, J. Ind. Microbiol. Biotechnol. 23:118-122.
6. Bibb, M. J., P. R. Findlay & M. W. Johnson (1984). "The relationship between base composition and codon usage in bacterial genes and its use for the simple and reliable identification of protein-coding sequences," Gene 30: 157-166.
7. Bierman, M., R. Logan, K. O'Brien, E. T. Seno, R. N. Rao & B. E. Schoner (1992). "Plasmid cloning vectors for the conjugal transfer of DNA from Escherichia coli to Streptomyces spp," Gene 116: 43-49.
8. Broughton, M. C., M. L. B. Huber, L. C. Creemer, H. A. Kirst & J. A. Turner (1991). "Biosynthesis of the macrolide insecticidal compound A83543 by Saccharopolyspora spinosa," Ann. Mtg. Amer. Soc. Microbiol.
9. Bsang, C., P. F. Long, J. Cortes, J. Westcott, J. Crosby, A.-L. Matharu, R. J. Cox, T. J. Simpson, J. Staunton and P. F. Leadlay (1999) "A chain initiation factor common to both modular and aromatic polyketide synthases." Nature 401:502-505.
10. Burgett, S. G. and P. R. J. Rosteck (1994) "Use of dimethyl sulfoxide to improve fluorescent, Taq cycle sequencing." in: Automated DNA Sequencing and Analysis. M. Adams, C. Fields and J. C. Venter, eds. NY, Academic Press: pp. 211-215.
11. Dehoff, B.S., S.A. Kuhstoss, P.R. Rosteck & K.L. Sutton (1997). "Polyketide synthase genes." EPA 0791655.
12. Donadio, S., J. B. McAlpine, P. S. Sheldon, M. Jackson & L. Katz (1993). "An erythromycin analog produced by reprogramming ofpolyketide synthesis," Proc. Natl. Acad. Sci. USA 90: 7119-7123.
13. Donadio, S. & L. Katz (1992). "Organization of the enzymatic domains in the multifunctional polyketide synthase involved in erythromycin formation in Saccharopolyspora erythrae," Gene 111: 51-60.
14. Donadio, S., M. J. Staver, J. B. McAlpine, S. J. Swanson & L. Katz (1991). "Modular organization of genes required for complex polyketide biosynthesis," Science 252: 675-679.
15. Hoffineister, D., K. Ichinose, S. Dormann, B. Foust, A. Trefzer, G. Drager, A. Kirschining, C. Fischer, E. Kunzel, D. W. Bearden, J. Rhor and A. Bechthold (2000) The NDP-sugar co-substrate concentration and the enzyme expression level influence the substrate specificity of glycosyltransferases: cloning and characterization of the deoxysugar biosynthesis genes of the urdamycin biosynthetic gene cluster. Chemistry & Biology 7:821-831.
16. Ikeda, H., T. Nomoniya, M. Usami, T. Ohta and S. Omura (1999) Organization of the biosynthetic gene cluster of the polyketide anthelmintic macrolide avermectin in Streptomyces avermitilis. Proc. Nat. Acad. Sci. USA 96:9509-9514.
17. Jiang, X. M., B. Neal, F. Santiago, S. J. Lee, L. K. Romana & P. R. Reeves (1991). "Structure and sequence of the rfb (O antigen) gene cluster of Salmonella serovar typhimurium (strain LT2)," Mol. Microbiol. 5: 695-713.
18. Katz, L., D. L. Stassi, R. G. Summers, Jr., X. Ruan, A. Pereda-Lopez and S. J. Kakavs. (2000) Polyketide derivatives and recombinant methods for making same. US Patent 6,060,234.
19. Kirst, H. A., K. H. Michel, J. W. Martin, L. C. Creemer, E. H. Chino, R. C. Yao, W. M. Nakatsukasa, L. D. Boeck, J. L. Occolowitz, J. W. Paschal, J. B. Deeter, N. D. Jones and G. D. Thompson. (1991) A83543A-D, unique fermentation-derived tetracyclic macrolides. Tetrahedron Lett. 32:4839-4842.
20. Liu, H.W. & J.S. Thorson (1994). "Pathways and mechanisms in the biogenesis of novel deoxysugars by bacteria," Ann Rev Microbiol 48: 223-256.
21. Matsushima, P., M. C. Broughton, J. R. Turner & R. H. Baltz (1994). "Conjugal transfer of cosmid DNA from Escherichia coli to Saccharopolyspora spinosa: effects of chromosomal insertion on macrolide A83543 production," Gene 146: 39-45.
22. McDaniel, R. & L. Katz (2001) Genetic engineering of novel macrolide antibiotics. In: Dev. Novel Antimicrob. Agents: Emerging Strategies; K. Lohner, Ed.; pp. 45-60; Horizon Scientific Press, Wymondham, UK.
23. Merson-Davies, L. A. and E. Cundeliffe (1994) Analysis of five tylosin biosynthetic genes from the tylIBA region of the Streptomyces fradiae genome. Mol Microbiol. 13:349-355.
24. Omura, S., K. Tsuzuki, A. Nakagawa, and G. Lukacs (1983) Biosynthetic origin of carbons 3 and 4 of leucomycin aglycone. J. Antibiot. 36:611-613.
25. Ruan, X., A. A Pereda, D. L. Stassi, D. Zeidner, R. G. Summers, M. Jackson, A. Shivakumar, S. Kakavas, M. J. Stavier, S. Donadio and L. Katz (1997). "Acyltransferase Domain Substitutions in Erythromycin Polyketide Synthase Yield Novel Erythromycin Derivatives," J. Bacteriology 179, 6416-6425.
26. Sambrook, J. E. F. Fritch, and T. Maniatis (1989) Molecular Cloning a Laboratory Manual, Second Edition. (Cold Spring Harbor Press, Cold Spring Harbor, NY)
27. Schwecke, T., J. F. Aparicio, I. Molnar, A. Konig, L. E. Khaw, S. F. Haydock, M. Oliynyk, P. Caffrey, J. Cortes, J. B. Lester, G. A. Bohm, J. Staunton and P. F. Leadlay (1995) The biosynthetic gene cluster for the polyketide immunosuppressant rapamycin. Proc. Nat. Acad. Sci. USA 92:7839-7843.
28. Shen, B., W. Liu, S. D. Christianson and S. Standage (2000) Gene cluster of the production of the enediyne antitumor antibiotic C-1027. WO App. 00/40596
29. Siggard-Andersen, M. (1993). "Conserved residues in condensing enzyme domains of fatty acid synthases and related sequences," Protein Seq. Data Anal. 5: 325-335.
30. Simon, R., U. Preifer & A. Puhler (1983). "A broad host range mobilization system for in vivo genetic engineering: transposon mutagenesis in Gram negative bacteria," Bio/Technology 1: 784-791.
31. Strobel, R. J. & W. M. Nakatsukasa (1993). "Response surface methods for optimizing Saccharopolyspora spinosa, a novel macrolide producer," J. Ind. Microbiol. 11: 121-127.
32. Thorson, J. S., S. F. Lo & H. Liu (1993). "Biosynthesis of 3,6-dideoxyhexoses: new mechanistic reflections upon 2,6-dideoxy, 4,6-dideoxy, and amino sugar construction, " J. Am. Chem. Soc. 115: 6993-6994.
33. Trefzer, A., J. A. Salas and A. Bechthold (1999) Genes and enzymes involved in deoxysugar biosynthesis. Nat. Prod. Rep. 16:283-299.
34. Weber, J. M. & J. B. McAlpine (1992). "Erythromycin derivatives," U.S. Patent 5,141,926.
35. Wohlert, S.-E., N. Lomovskaya, K. Kulowski, L. Fonstein, J. L. Occi, K. M. Gerwain, D. J. MacNeil and C. R. Hutchinson (2000) Biosynthesis of the avermectin deoxysugar L-oleandrose and novel avermectins. Genetics and Molecular Biology of Industrial Microorganisms Conference, Bloomington, IN, USA.

## Claims

1. An isolated DNA molecule comprising a DNA sequence that encodes one or more butenyl-spinosyn PKS domains selected from KSb, ATb, KRb, DHb, and ACPb, said domains being described by, respectively, amino acids 998-1413, 1495-1836, 1846-2028, 2306-2518, and 2621-2710 of SEQ ID NO:3.

2. The isolated DNA molecule of claim 1 wherein the DNA sequence comprises bases 2992-4239, 4483-5508, 5538-6084, 6916-7554, and/or 7861-8130 of SEQ ID NO:1.

3. The isolated DNA molecule of claim 1 or 2, the DNA sequence further encoding one or more butenyl-spinosyn PKS domains selected from KSi, ATi, ACPi, KS1, AT1, KR1, and ACP1, said domains being described by, respectively, amino acids 7-423, 528-853, 895-977, 2735-3160, 3241-3604, 3907-4086, and 4181-4262 of SEQ ID NO:3.

4. The isolated DNA molecule of claim 3, wherein the DNA sequence comprises bases 16-1269, 1582-2559, 2683-2931, 8203-9480, 9721-10812, 11719-12258, and/or 12541-12786 of SEQ ID NO:1.

5. The isolated DNA molecule of any preceding claim, the DNA sequence further encoding one or more spinosyn PKS domains selected from KS2, AT2, DH2, ER2, KR2, and ACP2, said domains being described by, respectively, amino acids 1-421, 534-964, 990-1075, 1336-1681, 1685-1864, and 1953-2031 of SEQ ID NO:4.

6. The isolated DNA molecule of claim 5 wherein the DNA sequence comprises bases 13059-14321, 14658-15900, 16026-16283, 17064-18100, 18111-18650, and/or 18915-19151 of SEQ ID NO:1.

7. The isolated DNA molecule of any preceding claim, the DNA sequence further encoding one or more spinosyn PKS domains selected from KS3, AT3, KR3, ACP3, KS4, AT4, KR4, and ACP4, said domains being described by, respectively, amino acids 1-421, 528-814, 1157-1335, 1422-1503, 1526-1949, 2063-2393, 2697-2875, and 2969-3049 of SEQ ID NO:5.

8. The isolated DNA molecule of claim 7 wherein the DNA sequence comprises bases 19553-20815, 21143-22000, 23021-23557, 23816-24061, 24128-25399, 25739-26731, 27641-28183, and/or 28457-28699 of SEQ ID NO:1.

9. The isolated DNA molecule of any preceding claim, the DNA sequence further encoding one or more spinosyn PKS domains selected from KS5, AT5, DH5, KR5, ACP5, KS6, AT6, KR6, ACP6, KS7, AT7, KR7, and ACP7, said domains being described by, respectively, amino acids 1-422, 537-864, 891-1076, 1382-1563, 1643-1724, 1746-2170, 2281-2611, 2914-3093, 3186-3267, 3289-3711, 3823-4151, 4342-4636, and 4723-4804 of SEQ ID NO:6.

10. The isolated DNA molecule of claim 9 wherein the DNA sequence comprises bases 29092-30357, 30700-31683, 31762-32319, 33235-33780, 34018-34263, 34327-35601, 35932-36924, 37831-38370, 38647-38892, 38956-40224, 40560-41544, 42115-42999 and/or 43258-43503 of SEQ ID NO:1.

11. The isolated DNA molecule of any preceding claim, the DNA sequence further encoding one or more spinosyn PKS domains selected from KS8, AT8, DH8, KR8, ACP8, KS9, AT9, DH9, KR9, ACP9, KS10, AT10, DH10, KR10, ACP10, and TE10, said domains being described by, respectively, amino acids 1-424, 530-848, 885-1072, 1371-1554, 1650-1728, 1751-2175, 2289-2616, 2642-2775, 3131-3315, 3396-3474, 3508-3921, 4036-4366, 4389-4569, 4876-5054, 5148-5229, and 5278-5531 of SEQ ID NO:7.

12. The isolated DNA molecule of claim 11 wherein the DNA sequence comprises bases 43945-45216, 45532-46488, 46597-47160, 48055-48606, 48892-49083, 49195-50469, 50809-51792, 51868-52269, 53335-53889, 54130-54366, 54466-55707, 56050-57042, 57109-57651, 58570-59106, 59386-59631, and/or 59776-60537 of SEQ ID NO:1.

13. The isolated DNA molecule of claim 1, wherein the DNA sequence encodes a spinosyn PKS module comprising amino acids 6-977 of SEQ ID NO:3, and/or a spinosyn PKS module comprising amino acids 998-2710 of SEQ ID NO:3.

14. The isolated DNA molecule of claim 13 wherein the DNA sequence comprises bases 16-2931 and/or 2992-8130 of SEQ ID NO:1.

15. The isolated DNA molecule of claim 13 or 14, wherein the DNA sequence encodes one or more further spinosyn PKS modules selected from the group consisting of amino acids 2735-4262 of SEQ ID NO:3, 1-2031 of SEQ ID NO:4, 1-1503 of SEQ ID NO:5, 1526-3049 of SEQ ID NO:5, 1-1724 of SEQ ID NO:6, 1746-3267 of SEQ ID NO:6, 3289-4804 of SEQ ID NO:6, 1-1728 of SEQ ID NO:7, 1751-3474 of SEQ ID NO:7, and 3508-5531 of SEQ ID NO:7.

16. The isolated DNA molecule of claim 15, wherein the DNA sequence comprises bases 8203-12786, 13059-19151, 19553-24061, 24128-28699, 29092-34263, 34327-38892, 38956-43503, 43945-49083, 49195-54366, and/or 54466-60537 of SEQ ID NO:1.

17. The isolated DNA molecule of claim 1, wherein the DNA sequence encodes a butenyl-spinosyn biosynthetic enzyme comprised of an amino acid sequence at least 98% identical to SEQ ID NOS 3, provided that if the sequence is less than 100% identical to the selected sequence, then the differences do not substantially affect the functional properties of the encoded enzyme.

18. The isolated DNA molecule of claim 17 wherein the DNA sequence comprises the *busA* gene described by bases 1-13032 of SEQ ID NO:1.

19. The isolated DNA molecule of claim 17 or 18, wherein the DNA sequence encodes one or more further butenyl-spinosyn biosynthetic enzymes comprised, respectively, of an amino acid sequence at least 98% identical to one selected from SEQ ID NOS 4-7 and 8-29, provided that if the sequence is less than 100% identical to the selected sequence, then the differences do not substantially affect the functional properties of the encoded enzyme.

20. The isolated DNA molecule of claim 19 wherein the DNA sequence comprises the *busB, busC, busD, busE,* ORF RI, ORFRII, ORF RIII, *busF, busG, busH, busI, busJ, busK, busL, busM, busN, busO, busP, busQ, busR, busS,* ORF LI, ORF LII, ORF LIII, ORF LIV, ORF LVI, ORF LVII, ORF LVIII, and/or ORF LIX genes, said genes being described by, respectively, bases 13059-19505, 19553-29053, 29092-43890, 43945-60636, 62090-63937, 65229-66602 and 68762-69676 of SEQ ID NO:1 and 114-938, 1389-2558, 2601-3350, 3362-4546, 4684-6300, 6317-7507, 7555-8403, 8640-9569, 9671-10666, 10678-12135, 12867-14177, 14627-15967, 16008-17141, 17168-17914, 18523-19932, 19982-20488, 20539-21033, 21179-21922, 22674-23453, 23690-24886, 26180-26923, and 27646-28473 of SEQ ID NO:2.

21. A recombinant DNA vector which comprises a DNA sequence of any one of claims 1-20.

22. A host cell transformed with a recombinant vector of claim 21.

23. A process for preparing a butenyl-spinosyn which comprises either:
cultivating a microorganism having operative butenyl-spinosyn biosynthetic genes in its genome, provided that the genome of the organism has been modified so that duplicate copies of at least one DNA sequence as claimed in any one of claims 1-20 are present; or
cultivating a heterologous microorganism that has been transformed so that its genome contains an operative butenyl spinosyn biosynthetic gene comprising a DNA sequence as claimed in any one of claims 1-20.

## Patentansprüche

1. Isoliertes DNA-Molekül, umfassend eine DNA-Sequenz, die für eine oder mehrere Butenyl-Spinosyn-PKS-Domänen codiert, ausgewählt aus KSb, ATb, KRb, DHb und ACPb, worin die Domänen beschrieben sind durch die Aminosäuren 998-1413, 1495-1836, 1846-2028, 2306-2518 bzw. 2621-2710 von SEQ ID No:3.

2. Isoliertes DNA-Molekül nach Anspruch 1, worin die DNA-Sequeriz die Basen 2992-4239, 4483-5508, 5538-6084, 6916-7554 und/oder 7861-8130 von SEQ ID No:1 umfasst.

3. Isoliertes DNA-Molekül nach Anspruch 1 oder 2, worin die DNA-Sequenz weiterhin codiert für eine oder mehrere Butenyl-Spinosyn-PKS-Domänen, ausgewählt aus KSi, ATi, ACPi, KS1, AT1, KR1 und ACP1, wobei die Domänen beschrieben sind durch die Aminosäuren 7-423,528-853, 895-977, 2735-3160, 3241-3604,3907-4086 bzw. 4181-4262 von SEQ ID No:3.

4. Isoliertes DNA-Molekül nach Anspruch 3, worin die DNA-Sequenz die Basen 16-1269, 1582-2559, 2683-2931, 8203-9480, 9721-10812, 11719-12258 und/oder 12541-12788 von SEQ ID No:1 umfasst.

5. Isoliertes DNA-Molekül nach einem der vorhergehenden Ansprüche, worin die DNA-Sequenz weiterhin codiert für eine oder mehrere Spinosyn-PKS-Domänen, ausgewählt aus KS2, AT2, DH2, ER2, KR2 und ACP2, wobei die Domänen beschrieben sind durch die Aminosäuren 1-421, 534-964, 990-1075, 1336-1681, 1685-1864 bzw. 1953-2031 von SEQ ID No:4.

6. Isoliertes DNA-Molekül nach Anspruch 5, worin die DNA-Sequenz die Basen 13059-14321, 14658-15900, 16026-16283, 17064-18100, 18111-18650 und/oder 18915-19151 von SEQ ID No:1 umfasst.

7. Isoliertes DNA-Molekül nach einem der vorhergehenden Ansprüche, worin die DNA-Sequenz weiterhin für eine oder mehrere Spinosyn-PKS-Domänen codiert, ausgewählt aus KS3, AT3, KR3, ACP3, KS4, AT4, KR4 und ACP4, wobei die Domänen beschrieben sind durch die Aminosäuren 1-421, 528-814, 1157-1335, 1422-1503, 1526-1949, 2063-2393, 2697-2875 bzw. 2969-3049 von SEQ ID No:5.

8. Isoliertes DNA-Molekül nach Anspruch 7, worin die DNA-Sequenz die Basen 19553-20815, 21143-22000, 23021-23557, 23816-24061, 24128-25399, 25739-26731, 27641-28183 und/oder 28457-28699 von SEQ ID No:1 umfasst.

9. Isoliertes DNA-Molekül nach einem der vorhergehenden Ansprüche, worin die DNA-Sequenz weiterhin für eine oder mehrere Spinosyn-PKS-Domänen codiert, ausgewählt aus KS5, AT5, DH5, KR5, ACP5, KS6, AT6, KR6, ACP6, KS7, AT7, KR7 und ACP7, wobei die Domänen beschrieben sind durch die Aminosäuren 1-422, 537-864, 891-1076, 1382-1563, 1643-1724, 1746-2170, 2281-2611, 2914-3093, 3186-3267, 3289-3711, 3823-4151, 4342-4636 bzw. 4723-4804 von SEQ ID No:6.

10. Isoliertes DNA-Molekül nach Anspruch 9, worin die DNA-Sequenz die Basen 29092-30357, 30700-31683, 31762-32319, 33235-33780, 34018-34263, 34327-35601, 35932-36924, 37831-38370, 38647-38892, 38956-40224, 40560-41544, 42115-42999 und/oder 43258-43503 von SEQ ID No:1 umfasst.

11. Isoliertes DNA-Molekül nach einem der vorhergehenden Ansprüche, worin die DNA-Sequenz weiterhin für eine oder mehrere Spinosyn-PKS-Domänen codiert, ausgewählt aus KS8, AT8, DH8, KR8, ACP8, KS9, AT9, DH9, KR9, ACP9, KS10, AT10, DH10, KR10, ACP10 und TE10, wobei die Domänen beschrieben sind durch die Aminosäuren 1-424, 530-848, 885-1072, 1371-1554, 1650-1728, 1751-2175, 2289-2616, 2642-2775, 3131-3315, 3396-3474, 3508-3921, 4036-4366, 4389-4569, 4876-5054, 5148-5229 bzw. 5278-5531 von SEQ ID No:7.

12. Isoliertes DNA-Molekül nach Anspruch 11, worin die DNA-Sequenz die Basen 43945-45216, 45532-46488, 46597-47160, 48055-48606, 48892-49083, 49195-50469, 50809-51792, 51868-52269, 53335-53889, 54130-54366, 54466-55707, 56050-57042, 57109-57651, 58570-59106, 59386-59631 und/oder 59776-60537 von SEQ ID No:1 umfasst.

13. Isoliertes DNA-Molekül nach Anspruch 1, worin die DNA-Sequenz codiert für ein Spinosyn-PKS-Modul, umfassend die Aminosäuren 6-977 von SEQ ID No:3 und/oder ein Spinosyn-PKS-Modul, umfassend die Aminosäuren 998-2710 von SEQ ID No:3.

14. Isoliertes DNA-Molekül nach Anspruch 13, worin die DNA-Sequenz die Basen 16-2931 und/oder 2992-8130 von SEQ ID No:1 umfasst.

15. Isoliertes DNA-Molekül nach Anspruch 13 oder 14, worin die DNA-Sequenz für eine oder mehrere weitere Spinosyn-PKS-Module codiert, ausgewählt aus der Gruppe, bestehend aus den Aminosäuren 2735-4262 von SEQ ID No:3, 1-2031 von SEQ ID No:4, 1-1503 von SEQ ID No:5, 1526-3049 von SEQ ID No:5, 1-1724 von SEQ ID No:6, 1746-3267 von SEQ ID No:6, 3289-4804 von SEQ ID No:6, 1-1728 von SEQ ID No:7, 1751-3474 von SEQ ID No:7 und 3508-5531 von SEQ ID No:7.

16. Isoliertes DNA-Molekül nach Anspruch 15, worin die DNA-Sequenz die Basen 8203-12786, 13059-19151, 19553-24061, 24128-28699, 29092-34263, 34327-38892, 38956-43503, 43945-49083, 49195-54366 und/oder 54466-60537 von SEQ ID No:1 umfasst.

17. Isoliertes DNA-Molekül nach Anspruch 1, worin die DNA-Sequenz codiert für ein biosynthetisches Butenyl-Spinosyn-Enzym, jeweils bestehend aus einer Aminosäuresequenz, die zu mindestens 98% identisch ist mit SEQ ID No:3, vorausgesetzt, dass wenn die Sequenz zu weniger als 100 % identisch ist mit der ausgewählten Sequenz, die Unterschiede nicht wesentlich die funktionalen Eigenschaften des codierten Enzyms beeinträchtigen.

18. Isoliertes DNA-Molekül nach Anspruch 17, worin die DNA-Sequenz das *busA*-Gen umfasst, das durch die Basen 1-13032 von SEQ ID No:1 beschrieben wird.

19. Isoliertes DNA-Molekül nach Anspruch 17 oder 18, worin die DNA-Sequenz für ein oder mehrere weitere biosynthetische Butenyl-Spinosyn-Enzyme codiert, jeweils bestehend aus einer Aminosäuresequenz, die zu mindestens 98% identisch ist mit einer, ausgewählt aus SEQ ID Nos:4-7 und 8-29, vorausgesetzt, dass wenn die Sequenz zu weniger als 100 % identisch ist mit der ausgewählten Sequenz, die Unterschiede nicht wesentlich die funktionalen Eigenschaften des codierten Enzyms beeinträchtigen.

20. Isoliertes DNA-Molekül nach Anspruch 19, worin die DNA-Sequenz die *busB-, busC-; busD-, busE-,* ORF RI-, ORFRII-, ORF RIII-, *busF-, busG-, busH-, busI-, busJ-, busK-, busL-, busM-, busN-, busO-, busP-, busQ-, busR-, busS-,* ORF LI-, ORF LII-, ORF LIII-, ORF LIV-, ORF LVI-, ORF LVII-, ORF LVIII- und/oder LIX-Gene umfasst, wobei die Gene beschrieben sind durch die Basen 13059-19505, 19553-29053, 29092-43890, 43945-60636, 62090-63937, 65229-66602 bzw. 68762-69676 von SEQ ID No:1 und 114-938, 1389-2558, 2601-3350, 3362-4546, 4684-6300, 6317-7507, 7555-8403, 8640-9569, 9671-10666, 10678-12135, 12867-14177, 14627-15967, 16008-17141, 17168-17914, 18523-19932, 19982-20488, 20539-21033, 21179-21922, 22674-23453, 23690-24886, 26180-26923 bzw. 27646-28473 von SEQ ID No:2.

21. Rekombinanter DNA-Vektor, umfassend eine DNA-Sequenz nach einem der Ansprüche 1-20.

22. Wirtszelle, transformiert mit einem rekombinanten Vektor nach Anspruch 21.

23. Verfahren zum Herstellen eines Butenyl-Spinosyns, umfassend entweder:
Kultivieren eines Mikroorganismus mit operativen biosynthetischen Butenyl-Spinosyn-Genen in seinem Genom, vorausgesetzt, dass das Genom des Organismus so modifiziert worden ist, dass doppelte Kopien von mindestens einer DNA-Sequenz nach einem der Ansprüche 1-20 vorliegen; oder
Kultivieren eines heterologen Mikroorganismus, der so transformiert worden ist, dass sein Genom ein operatives biosynthetisches Butenyl-Spinosyn-Gen enthält, das eine DNA-Sequenz nach einem der Ansprüche 1-20 umfasst.

## Revendications

1. Molécule d'ADN isolée, comprenant une séquence d'ADN qui code un ou plusieurs domaine(s) de butényl-spinosyne PKS (polycétide synthétase), choisi(s) parmi les domaines KSb, ATb, KRb, DHb et ACPb, lesquels domaines sont respectivement constitués par les acides aminés 998 à 1413, 1495 à 1836, 1846 à 2028, 2306 à 2518, et 2621 à 2710 de la Séquence N° 3.

2. Molécule d'ADN isolée, conforme à la revendication 1, dans laquelle la séquence d'ADN comprend les bases 2992 à 4239, 4483 à 5508, 5538 à 6084, 6916 à 7554, et/ou 7861 à 8130 de la Séquence N° 1.

3. Molécule d'ADN isolée, conforme à la revendication 1 ou 2, dont la séquence d'ADN code en outre un ou plusieurs domaine(s) de butényl-spinosyne PKS, choisi(s) parmi les domaines KSi, ATi, ACPi, KS1, AT1, KR1 et AP1, lesquels domaines sont respectivement constitués par les acides aminés 7 à 423, 528 à 853, 895 à 977, 2735 à 3160, 3241 à 3604, 3907 à 4086, et 4181 à 4262 de la Séquence N° 3.

4. Molécule d'ADN isolée, conforme à la revendication 3, dans laquelle la séquence d'ADN comprend les bases 16 à 1269, 1582 à 2559, 2683 à 2931, 8203 à 9480, 9721 à 10812, 11719 à 12258, et/ou 12541 à 12786 de la Séquence N° 1.

5. Molécule d'ADN isolée, conforme à l'une des revendications précédentes, dont la séquence d'ADN code en outre un ou plusieurs domaine(s) de butényl-spinosyne PKS, choisi(s) parmi les domaines KS2, AT2, DH2, ER2, KR2 et ACP2, lesquels domaines sont respectivement constitués par les acides aminés 1 à 421, 534 à 964, 990 à 1075, 1336 à 1681, 1685 à 1864, et 1953 à 2031 de la Séquence N° 4.

6. Molécule d'ADN isolée, conforme à la revendication 5, dans laquelle la séquence d'ADN comprend les bases 13059 à 14321, 14658 à 15900, 16026 à 16283, 17064 à 18100, 18111 à 18650, et/ou 18915 à 19151 de la Séquence N° 1.

7. Molécule d'ADN isolée, conforme à l'une des revendications précédentes, dont la séquence d'ADN code en outre un ou plusieurs domaine(s) de butényl-spinosyne PKS, choisi(s) parmi les domaines KS3, AT3, KR3, ACP3, KS4, AT4, KR4 et ACP4, lesquels domaines sont respectivement constitués par les acides aminés 1 à 421, 528 à 814, 1157 à 1335, 1422 à 1503, 1526 à 1949, 2063 à 2393, 2697 à 2875, et 2969 à 3049 de la Séquence N° 5.

8. Molécule d'ADN isolée, conforme à la revendication 7, dans laquelle la séquence d'ADN comprend les bases 19553 à 20815, 21143 à 22000, 23021 à 23557, 23816 à 24061, 24128 à 25399, 25739 à 26731, 27641 à 28183, et/ou 28457 à 28699 de la Séquence N° 1.

9. Molécule d'ADN isolée, conforme à l'une des revendications précédentes, dont la séquence d'ADN code en outre un ou plusieurs domaine(s) de butényl-spinosyne PKS, choisi(s) parmi les domaines KS5, AT5, DH5, KR5, ACP5, KS6, AT6, KR6, ACP6, KS7, AT7, KR7 et ACP7, lesquels domaines sont respectivement constitués par les acides aminés 1 à 422, 537 à 864, 891 à 1076, 1382 à 1563, 1643 à 1724, 1746 à 2170, 2281 à 2611, 2914 à 3093, 3186 à 3267, 3289 à 3711, 3823 à 4151, 4342 à 4636, et 4723 à 4804 de la Séquence N° 6.

10. Molécule d'ADN isolée, conforme à la revendication 9, dans laquelle la séquence d'ADN comprend les bases 29092 à 30357, 30700 à 31683, 31762 à 32319, 33235 à 33780, 34018 à 34263, 34327 à 35601, 35932 à 36924, 37831 à 38370, 38647 à 38892, 38956 à 40224, 40560 à 41544, 42115 à 42999, et/ou 43258 à 43503 de la Séquence N° 1.

11. Molécule d'ADN isolée, conforme à l'une des revendications précédentes, dont la séquence d'ADN code en outre un ou plusieurs domaine(s) de butényl-spinosyne PKS, choisi(s) parmi les domaines KS8, AT8, DH8, KR8, ACP8, KS9, AT9, DH9, KR9, ACP9, KS10, AT10, DH10, KR10, ACP10 et TE10, lesquels domaines sont respectivement constitués par les acides aminés 1 à 424, 530 à 848, 885 à 1072, 1371 à 1554, 1650 à 1728, 1751 à 2175, 2289 à 2616, 2642 à 2775, 3131 à 3315, 3396 à 3474, 3508 à 3921, 4036 à 4366, 4389 à 4569, 4876 à 5054, 5148 à 5229, et 5278 à 5531 de la Séquence N° 7.

12. Molécule d'ADN isolée, conforme à la revendication 11, dans laquelle la séquence d'ADN comprend les bases 43945 à 45216, 45532 à 46488, 46597 à 47160, 48055 à 48606, 48892 à 49083, 49195 à 50469, 50809 à 51792, 51868 à 52269, 53335 à 53889, 54130 à 54366, 54466 à 55707, 56050 à 57042, 57109 à 57651, 58570 à 59106, 59386 à 59631, et/ou 59766 à 60537 de la Séquence N° 1.

13. Molécule d'ADN isolée, conforme à la revendication 1, dont la séquence d'ADN code un module de spinosyne PKS comprenant les acides aminés 6 à 977 de la Séquence N° 3 et/ou un module de spinosyne PKS comprenant les acides aminés 998 à 2710 de la Séquence N° 3.

14. Molécule d'ADN isolée, conforme à la revendication 13, dans laquelle la séquence d'ADN comprend les bases 16 à 2931 et/ou 2992 à 8130 de la Séquence N° 1.

15. Molécule d'ADN isolée, conforme à la revendication 13 ou 14, dont la séquence d'ADN code un ou plusieurs autre(s) module(s) de spinosyne PKS choisi(s) parmi ceux qui comprennent les acides aminés 2735 à 4262 de la Séquence N° 3, 1 à 2031 de la Séquence N° 4, 1 à 1503 de la Séquence N° 5, 1526 à 3409 de la Séquence N° 5, 1 à 1724 de la Séquence N° 6, 1746 à 3267 de la Séquence N° 6, 3289 à 4804 de la Séquence N° 6, 1 à 1728 de la Séquence N° 7, 1751 à 3474 de la Séquence N° 7, et 3508 à 5531 de la Séquence N° 7.

16. Molécule d'ADN isolée, conforme à la revendication 15, dans laquelle la séquence d'ADN comprend les bases 8203 à 12786, 13059 à 19151, 19553 à 24601, 24128 à 28699, 29092 à 34263, 34327 à 38892, 38956 à 43503, 43945 à 49083, 49195 à 54366, et/ou 54466 à 60537 de la Séquence N° 1.

17. Molécule d'ADN isolée, conforme à la revendication 1, dont la séquence d'ADN code une enzyme de biosynthèse de butényl-spinosyne comprenant une séquence d'acides aminés identique, pour au moins 98 %, à la Séquence N° 3, étant entendu que, si la séquence est identique pour moins de 100 % à cette séquence, les différences n'affectent pas sensiblement les caractéristiques fonctionnelles de l'enzyme codée.

18. Molécule d'ADN isolée, conforme à la revendication 17, dans laquelle la séquence d'ADN comprend le gène *busA* constitué par les bases 1 à 13032 de la Séquence N° 1.

19. Molécule d'ADN isolée, conforme à la revendication 17 ou 18, dont la séquence d'ADN code une ou plusieurs autre(s) enzyme(s) de biosynthèse de butényl-spinosyne, comprenant chacune une séquence d'acides aminés identique, pour au moins 98 %, à une séquence choisie parmi les Séquences N° 4 à 7 et 8 à 29, étant entendu que, si la séquence est identique pour moins de 100 % à la séquence choisie, les différences n'affectent pas sensiblement les caractéristiques fonctionnelles de l'enzyme codée.

20. Molécule d'ADN isolée, conforme à la revendication 19, dans laquelle la séquence d'ADN comprend les gènes *busB, busC, busD, busE,* ORF RI, ORF RII, ORF RIII, *busF, busG, busH, busI, busJ, busK, busL, busM, busN, busO, busP, busQ, busR, busS,* ORF LI, ORF LII, ORF LIII, ORF LIV, ORF LVI, ORF LVII, ORF LVIII, et/ou ORF LIX, lesquels gènes sont constitués, respectivement, par les bases 13059 à 19505, 19553 à 29053, 29092 à 43890, 43945 à 60636, 62090 à 63937, 65229 à 66602, et 68762 à 69676 de la Séquence N° 1, et 114 à 938, 1389 à 2558, 2601 à 3350, 3362 à 4546, 4684 à 6300, 6317 à 7507, 7555 à 8403, 8640 à 9569, 9671 à 10666, 10678 à 12135, 12867 à 14177, 14627 à 15967, 16008 à 17141, 17168 à 17914, 18523 à 19932, 19982 à 20488, 20539 à 21033, 21179 à 21922, 22674 à 23453, 23690 à 24886, 26180 à 26923, et 27646 à 28473 de la Séquence N° 2.

21. Vecteur d'ADN recombiné, qui comprend une séquence d'ADN conforme à l'une des revendications 1 à 20.

22. Cellule hôte transformée avec un vecteur recombiné conforme à la revendication 21.

23. Procédé de préparation d'une butényl-spinosyne, lequel procédé comporte :
soit le fait de cultiver un microorganisme dont le génome comporte des gènes opérationnels de biosynthèse de butényl-spinosyne, sous réserve que l'on ait modifié le génome de cet organisme de manière à ce qu'y soient présentes des copies dupliquées d'au moins une séquence d'ADN revendiquée dans l'une des revendications 1 à 20 ;
soit le fait de cultiver un microorganisme hétérologue que l'on a transformé de manière à ce que son génome comporte un gène opérationnel de biosynthèse de butényl-spinosyne comprenant une séquence d'ADN revendiquée dans l'une des revendications 1 à 20.
